# EUROPÄISCHE PATENTSCHRIFT

(11) **EP 3 108 519 B1**
(45) Veröffentlichungstag und Bekanntmachung des Hinweises auf die Patenterteilung: **20.06.2018**
(21) Anmeldenummer: 15703461.2
(22) Anmeldetag: 10.02.2015
(51) Int. Cl.: H01L 51/46, C07D 317/36, H01L 51/30

(54) **KONJUGIERTE POLYMERE**
CONJUGATED POLYMERS
POLYMÈRES CONJUGUÉS

(30) Priorität: 20.02.2014 EP 14000593
(43) Veröffentlichungstag der Anmeldung: 28.12.2016
(73) Patentinhaber: InnovationLab GmbH, 69115 Heidelberg (DE); Karlsruher Institut für Technologie (KIT), 76131 Karlsruhe (DE); Technische Universität Braunschweig, 38106 Braunschweig (DE); Max-Planck-Gesellschaft zur Förderung der Wissenschaften e.V., 80539 München (DE)
(72) Erfinder: KUHN, Marius, 69120 Heidelberg (DE); ADERMANN, Torben, 69120 Heidelberg (DE); HAMBURGER, Manuel, 69120 Heidelberg (DE); MUELLEN, Klaus, 50939 Köln (DE); SCHINKE, Janusz, 38106 Braunschweig (DE); COLSMANN, Alexander, 76131 Karlsruhe (DE); HOEFLE, Stefan, 76131 Karlsruhe (DE); LEMMER, Ulrich, 76131 Karlsruhe (DE)
(74) Vertreter: Derow, Stephan
(86) Internationale Anmeldenummer: PCT/EP2015/000276
(87) Internationale Veröffentlichungsnummer: WO 2015/124272

(56) Entgegenhaltungen:
- US-A1- 2004 038 459
- US-A1- 2011 269 917
- US-A1- 2013 276 887
- MATTHEW M. DURBAN ET AL: "Synthesis and Characterization of Solution-Processable Ladderized n-Type Naphthalene Bisimide Copolymers for OFET Applications", MACROMOLECULES, Bd. 44, Nr. 12, 28. Juni 2011 (2011-06-28) , Seiten 4721-4728, XP055034788, ISSN: 0024-9297, DOI: 10.1021/ma2004822 in der Anmeldung erwähnt
- JUNGHOON LEE ET AL: "Inversion of Dominant Polarity in Ambipolar Polydiketopyrrolopyrrole with Thermally Removable Groups", ADVANCED FUNCTIONAL MATERIALS, Bd. 22, Nr. 19, 12. Juni 2012 (2012-06-12) , Seiten 4128-4138, XP055113862, ISSN: 1616-301X, DOI: 10.1002/adfm.201200940 in der Anmeldung erwähnt

## Beschreibung

Die Erfindung betrifft neue konjugierte Polymere enthaltend thermisch abspaltbare Seitengruppen, Monomere und Verfahren zu ihrer Herstellung, ihre Verwendung als Halbleiter in organischen elektronischen (OE) Vorrichtungen, insbesondere in organischen Photovoltaik (OPV)-Vorrichtungen, organischen Photodetektoren (OPD), organischen Leuchtdioden (OLED), organischen Feldeffekttransistoren (OFET), und diese Polymere enthaltende OE-, OPV-, OPD-, OLED und OFET-Vorrichtungen.

In den letzten Jahren wurden organische Halbleitermaterialien entwickelt, um vielseitigere und preisgünstigere OE-Vorrichtungen herzustellen. Derartige Materialien finden in einer Vielzahl von Vorrichtungen oder Geräten Verwendung, wie zum Beispiel in OFETs, OLEDs, OPV- oder OPD-Vorrichtungen, Sensoren, Speicherelementen und logischen Schaltungen. Die organischen Halbleitermaterialien liegen in der OE-Vorrichtung typischerweise in Form einer dünnen Schicht von z.B. weniger als 1 Mikrometer Dicke vor.

Ein besonders wichtiges Gebiet sind OPV-Vorrichtungen wie organische Solarzellen. Konjugierte Polymere finden als organische Halbleiter in organischen Solarzellen Verwendung, da sie eine einfache Herstellung der photoaktiven Schicht durch Verarbeitungstechniken aus der Lösung, wie Aufschleudern, Tauchbeschichtung oder Tintenstrahldruck ermöglichen. Im Vergleich zu Verdampfungstechniken, wie sie zur Herstellung anorganischer Halbleiterschichten verwendet werden, lässt sich die Verarbeitung aus der Lösung preiswerter und in größerem Maßstab durchführen. Für organische Solarzellen mit polymeren Halbleitern wurden Wirkungsgrade von über 7% berichtet.

Ein weiteres wichtiges Gebiet sind OFETs. Deren Leistung beruht hauptsächlich auf der Ladungsträgerbeweglichkeit des Halbleitermaterials und dem Ein/Aus-Verhältnis des Stromes, daher sollte der ideale Halbleiter eine geringe Leitfähigkeit im Aus-Zustand in Verbindung mit einer hohen Ladungsträgerbeweglichkeit (> 1 x 10⁻³ cm² V⁻¹ s⁻¹) aufweisen. Außerdem ist es wichtig, dass das Halbleitermaterial relativ oxidationsbeständig ist, d.h. ein hohes lonisierungspotential aufweist, da Oxidation zu einer Leistungsminderung der Vorrichtung führt. Weitere Anforderungen an das Halbleitermaterial sind eine gute Verarbeitbarkeit, insbesondere für die großmaßstäbliche Herstellung von dünnen Schichten und gewünschten Mustern, sowie hohe Stabilität, Einheitlichkeit des Films und Integrität der organischen Halbleiterschicht.

Es besteht jedoch immer noch ein Bedarf an organischen Halbleitermaterialien, die sich leicht synthetisieren lassen und sich insbesondere für die Massenproduktion eignen, gute Strukturorganisation und Filmbildungseigenschaften aufwiesen, gute elektronische Eigenschaften, insbesondere eine hohe Ladungsträgerbeweglichkeit, gute Verarbeitbarkeit, insbesondere eine hohe Löslichkeit in organischen Lösungsmitteln, und hohe Stabilität in Luft zeigen. Für die Verwendung in OPV-Elementen besteht insbesondere ein Bedarf an organischen Halbleitermaterialien mit einer niedrigen Energielücke, die verbesserte Lichtausnutzung durch die photoaktive Schicht ermöglichen und zu höherer Effizienz der Elemente führen können. Für die Verwendung in OFETs besteht insbesondere ein Bedarf an organischen Halbleitermaterialien mit hoher Ladungsträgerbeweglichkeit und hoher Oxidationsbeständigkeit.

Um eine verbesserte Löslichkeit der organischen Halbleiter in organischen Lösungsmitteln zu erreichen, werden im Stand der Technik als Halbleiter üblicherweise konjugierte Polymere verwendet, welche löslichkeitsfördernde, in der Regel unkonjugierte, Seitenketten wie zum Beispiel Alkylgruppen aufweisen. Diese Seitenketten können jedoch die Organisation der konjugierten Polymerhauptketten in der funktionellen Schicht und insbesondere die Kristallisation der Polymere stören, und damit den Ladungstransport zwischen den Polymermolekülen beeinträchtigen. Ausserdem zeigen die bisher verwendeten löslichkeitsfördernden Seitengruppen oft nicht die gewünschte Steigerung der Löslichkeit. Zudem können solche löslichkeitsfördernden Seitenketten dazu führen, dass die Polymere in Lösungsmitteln, die zum Auftrag weiterer Schichten auf die funktionellen Schicht verwendet werden, ebenfalls löslich sind. Als Folge davon kann der Auftrag einer weiteren Schicht die zuvor aufgetragene funktionellen Polymerschicht beschädigen. Wünschenswert wäre dagegen ein organisches Halbleitermaterial, welches in dem zum Auftrag der Halbleiterschicht verwendeten Lösungmittel eine hohe Löslichkeit besitzt, aber in einem zum Auftrag weiterer Schichten verwendeten Lösungmittel nicht oder nur schlecht löslich ist, d.h. eine hohe Orthogonalität aufweist.

Es war ein Ziel der vorliegenden Erfindung, Verbindungen für die Verwendung als organische Halbleitermaterialien bereitzustellen, welche die oben genannten Nachteile der Materialien des Standes der Technik nicht besitzen, sich leicht synthetisieren lassen, insbesondere nach Verfahren, die sich für die Massenproduktion eignen, und insbesondere gute Verarbeitbarkeit, hohe Stabilität, gute Löslichkeit in organischen Lösungsmitteln, hohe Orthogonalität gegenüber benachbarten Schichten in der Vorrichtung, hohe Ladungsträgerbeweglichkeit und eine niedrige Energielücke aufweisen. Ein weiteres Ziel war die Erweiterung des dem Fachmann zur Verfügung stehenden Spektrums an organischen Halbleitermaterialien. Weitere Ziele der vorliegenden Erfindung ergeben sich für den Fachmann aus der folgenden Offenbarung.

Es wurde gefunden, dass diese Ziele erreicht werden können durch die Bereitstellung von konjugierten Halbleiterpolymeren gemäß der vorliegenden Erfindung wie nachstehend beschrieben. Diese Polymere weisen löslichkeitsverbessernde Carbonatseitenketten oder Carbamatseitenketten auf, welche thermisch abspaltbar sind.

Konjugierte Polymere mit löslichkeitsverbessernden Gruppen sind aus dem Stand der Technik bekannt. Diese bestehen jedoch üblicherweise entweder aus Gruppen, wie z.B. sehr langen Alkyl- oder Fluoralkylresten, die nicht abspaltbar sind und damit nach der Prozessierung im Film verbleiben, oder aus Gruppen, die erst bei sehr hohen Temperaturen (>300 °C) abgespalten werden können.

So werden beispielsweise in US 2011/0045628 A1, J. Yu, S. Holdcroft, Macromolecules 2000, 33, 5073-5079, M. Helgesen, R. Søndergaard, F. C. Krebs, J. Mater. Chem. 2010, 20, 36-60, P. D. Kazarinoff, P. J. Shamburger, F. S. Ohuchi, C. K. Luscombe, J. Mater. Chem. 2010, 20, 3040-3045, E. Bundgaard, O. Hagemann, M. Bjerring, N. C. Nielsen, J. W. Andreasen, B. Andreasen, F. C. Krebs, Macromolecules 2012, 45, 3644-3646, F. C. Krebs, H. Spanggaard, Chem. Mater. 2005, 17, 5235-5237, J. Lee, A.-R. Han, J. Hong, J. H. Seo, J. H. Oh, C. Yang, Adv. Funct. Mater. 2012, 22, 4128-4138, M.M. Durban, P.D. Kazarinkoff, Y. Segawa, C.K. Luscombe, Macromolecules, 2011, 44, 4721-4728, J. Lee, A.-R. Han, J. Hong, J.H. Seo, J.H. Oh, C. Yang, Adv. Funct. Mater., 2012, 22, 4128-4138, oder B. Sun, W. Hong, H. Aziz, Y. Li, J. Mater. Chem. 2012, 22, 18950-18955 Polymere mit thermisch abspaltbaren Carbonyl-, Carbonyloxy- oder THP-Ethergruppen in der Seitenkette offenbart.

Der Abspaltungsprozess solcher Gruppen erfordert jedoch sehr hohe Temperaturen über 200°C und bis zu 310°C, wie im nachfolgenden Schema beispielhaft gezeigt. Solche hohen Prozesstemperaturen sind jedoch von Nachteil, da sie die aufgetragene Halbleiterschicht oder andere Komponenten der Vorrichtung beschädigen können. 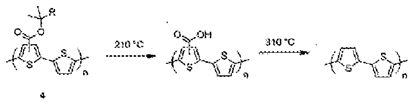 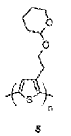 M. M. Durban, P. D. Kazarinoff, Y. Segawa, , C. K. Luscombe, J. Macromolecules 2011, 44, 4721-4728 offenbart Precursorpolymere der nachfolgenden Formeln (PNDI-1Boc, PNDI-2Boc), enthaltend eine Phenylen-2,5-diyl-Einheit mit einer oder zwei Carbamatseitengruppen sowie eine benachbarte Benzo[lmn][3,8]phenanthrolin-1,3,6,8-tetraon-4,9-diyl-Einheit (in der Literatur auch als "Naphthalin-bis(dicarboximid)" oder "NDI" bezeichnet), worin R einen 2-Octyldodecylrest bedeutet, 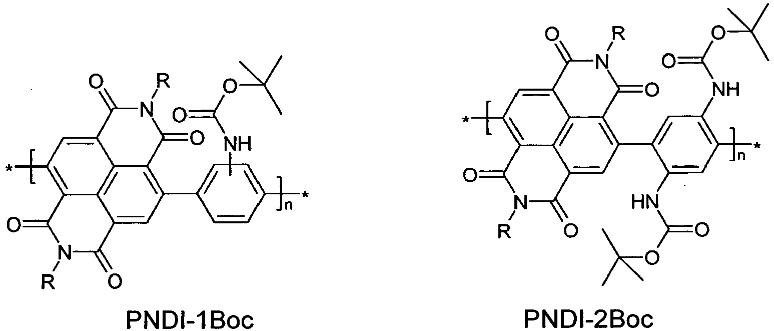 worin die Carbamatseitengruppen mit der benachbarten NDI-Einheit in einer Zyklisierungsreaktion ein Polymer oder Leiterpolymer (engl. "ladder polymer") der nachfolgenden Formeln (PNDI-1 BocL, PNDI-2BocL) bilden.

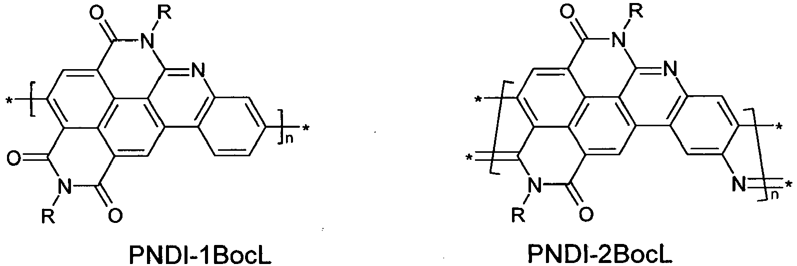

Die thermische Abspaltung der Carbamatseitengruppe, oder die Verwendung der Carbamat-substituierten Precursorpolymere ohne notwendige weitere Zyklisierungsreaktion in einer Halbleiterschicht, wird dort jedoch nicht offenbart.

Für die dort offenbarte Methode ist es notwendig, das thermisch labile Carbamat in unmittelbarer Nähe einer Carbonylgruppe (hier die ImidGruppe des NDI-Comonomers) zu positionieren. Dadurch wird die Variationsbreite dieses Ansatzes sehr stark eingeschränkt.

US 5,484,943 offenbart niedermolekulare Pyrrolo[3,4-c]pyrrole worin die N-Atome des Pyrrolrings auch mit einer Carbonylgruppe substituiert sein können (wodurch eine Carbamatgruppe entsteht), sowie deren Verwendung als fluoreszierende Pigmente zur Färbung von Kunststoffen. Dort wird auch beschrieben, dass die substituerten Pyrrolo[3,4-c]pyrrole durch thermische, photolytische oder chemische Behandlung unter Abspaltung der Carbonylgruppe in die entsprechenden Verbindungen mit unsubstituierten N-Ringatomen überführt werden können, wodurch eine neue Kristallform des Pigments entsteht, welche bessere Farbeigenschaften aufweist. Konjugierte Polymere oder deren Verwendung gemäß der vorliegenden Erfindung werden darin jedoch weder offenbart noch nahegelegt.

J. Lee et al., Adv. Funct. Mater. 2012, 22, 4128-4138 offenbart ambipolare Polymere der folgenden Formel 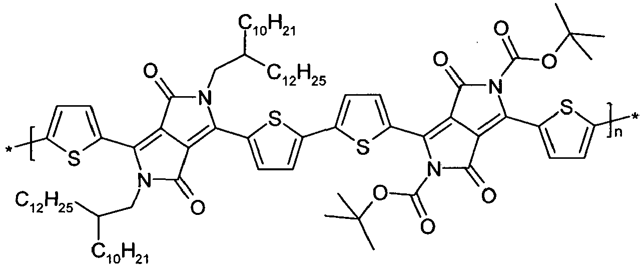 welche 3,6-Dithienyl-Dipyrrolopyrroleinheiten mit thermisch abspaltbaren Carbonylgruppen enthalten, sowie deren Verwendung in ambipolaren OFETs, worin nach Abspaltung der Carbonylgruppen die freien NH-Gruppen im Polymer H-Brücken ausbilden. Die Verwendung von DPP führt in den konjugierten Polymeren jedoch höchstens zu ambipolarem Ladungstransport. Konjugierte Polymere gemäß der vorliegenden Erfindung, welche als reines n-Kanal-Material verwendet werden können, werden nicht offenbart.

Es wurde nun überraschend gefunden, dass erfindungsgemäße Polymere mit löslichkeitsverbessernden Carbonatseitenketten oder Carbamatseitenketten eine teilweise oder vollständige Abspaltung der Seitenketten bereits bei Temperaturen unter 200°C ermöglichen. Die erfindungsgemäßen Polymere weisen zudem eine verbesserte Löslichkeit im Vergleich zu den aus dem Stand der Technik bekannten Materialien auf. Zudem wurde gefunden, dass diese Polymere nach Abspaltung der Seitenketten und ggf. thermischer Behandlung eine bessere Kristallisation und einen höhere Ordnung der Polymerketten ermöglichen, was zu verbessertem Ladungtransport in der Halbleiterschicht und bei Verwendung in organischen Solarzellen zu einer höheren Effizienz führt.

Die Abspaltbarkeit der Seitengruppen in einem erfindungsgemäßen Polymer ermöglicht ausserdem die Variation dessen Löslichkeit. So kann nach dem Auftrag der polymeren Halbleiterschicht aus der Lösung durch die Abspaltung der Seitenketten die Löslichkeit des Polymers deutlich reduziert werden. Dadurch kann die Stabilität der Halbeiterschicht gegenüber Lösungsmitteln, die zum Auftrag weiterer organischer Schichten verwendet werden, deutlich erhöht werden. Darüber hinaus kann für den Auftrag weiterer organischer Schichten dasselbe oder ein ähnliches Lösungsmittel verwendet werden wie für den Auftrag der Halbleiterschicht. Die Verwendung orthogonaler Lösungsmittel bzw. entsprechender Materialien für die nachfolgende Schicht ist somit nicht mehr zwingend erforderlich. Dies ermöglicht eine größere Variabilität bei der Auswahl der verwendeten Materialien und Prozesse.

Die Monomere und Polymere der vorliegenden Erfindung eignen sich insbesondere für die industrielle Herstellung im großen Maßstab. Gleichzeitig weisen sie gute Verarbeitbarkeit, hohe Löslichkeit in organischen Lösungsmitteln, hohe Ladungsträgerbeweglichkeit, hohe Langzeitstabilität, hohe Stabilität gegenüber Lösungsmitteln und hohe Oxidationsbeständigkeit auf, und stellen vielversprechende Materialien für organische elektronische OE-Vorrichtungen, insbesondere für OPV-Vorrichtungen dar.

Die Erfindung betrifft somit ein konjugiertes Polymer enthaltend eine oder mehrere gleiche oder verschiedene Wiederholungseinheiten der Formel I: 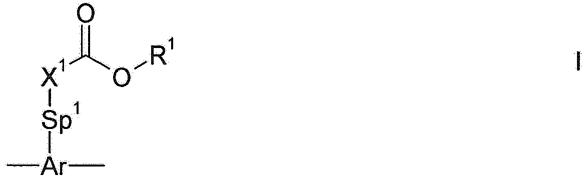 worin die einzelnen Reste folgende Bedeutung besitzen:
- Ar: mono- oder polycyclisches Aryl oder Heteroaryl, welches zusätzlich in einer oder mehreren Positionen substituiert sein kann,
- Sp¹: Alkylen mit 1 bis 20 C-Atomen, welches unsubstituiert oder ein- oder mehrfach durch F, Cl, Br, I oder CN substituiert ist, und worin auch eine oder mehrere nicht benachbarte CH₂-Gruppen jeweils unabhängig voneinander so durch -O-, -S-, -C(O)-, -C(S)-, -C(O)-O-, -O-C(O)-, -NR⁰-,-SiR⁰R⁰⁰)-, -CF₂-, -CHR⁰=CR⁰⁰-, -CY¹=CY²- oder -C≡C-ersetzt sein können, dass O- und/oder S-Atome nicht direkt miteinander verknüpft sind,
- X¹: NR⁰ oder O, vorzugsweise O,
- R¹: Hydrocarbyl mit 1 bis 40 C-Atomen,
- Y¹ und Y²: jeweils unabhängig voneinander H, F, Cl oder CN,
- R⁰ und R⁰⁰: jeweils unabhängig voneinander H oder Alkyl mit 1 bis 12 C-Atomen, ausgenommen

Wiederholungseinheiten der Formel I, worin Ar Phenylen bedeutet, welches ein- oder mehrfach mit -NH-C(O)-OR¹ substituiert ist, und welches in der Polymerhauptkette direkt benachbart zu einer gegebenenfalls substituierten Benzo[lmn][3,8]phenanthrolin-1,3,6,8-tetraon-4,9-diyl-Einheit ist, sowie

Wiederholungseinheiten der Formel I, worin Ar Pyrrolo[3,4-c]pyrrol-1,4-dion-3,6-diyl bedeutet worin beide N-Atome mit -C(O)-OR¹ substituiert sind.

Die Erfindung betrifft weiterhin ein konjugiertes Polymer, enthaltend eine oder mehrere Wiederholungseinheiten, die mit einer Carbonatgruppe oder einer Carbamatgruppe, vorzugsweise mit einer Carbonatgruppe, substituiert sind, und die aus den Wiederholungseinheiten der Formel I ausgewählt sind, sowie zusätzlich enthaltend eine oder mehrere, gegebenenfalls substituierte, mono- oder polycyclische Aryl- oder Heteroaryleinheiten.

Die Erfindung betrifft weiterhin ein Verfahren zur teilweisen oder vollständigen Abspaltung der Carbonat- oder Carbamatgruppen eines Polymers wie vor- und nachstehend beschrieben, durch Erhitzen des Polymers, oder einer Schicht enthaltend das Polymer, auf eine Temperatur von ≤200°C, sowie ein Polymer erhältlich durch dieses Verfahren.

Die Erfindung betrifft weiterhin Monomere enthaltend eine Einheit der Formel I und eine oder mehrere reaktive Gruppen, welche sich für die Herstellung von Polymeren wie vor- und nachstehend beschrieben eignen.

Die Erfindung betrifft weiterhin die Verwendung von Polymeren gemäß der vorliegenden Erfindung als Halbleiter, beispielsweise als Elektronendonor oder p-Typ-Halbleiter, oder als Elektronenakzeptor oder n-Typ-Halbleiter.

Die Erfindung betrifft weiterhin die Verwendung von Polymeren gemäß der vorliegenden Erfindung als halbleitendes Material, vorzugsweise als Elektronendonor, in einer organischen elektronischen Vorrichtung oder einer Komponente einer organischen elektronischen Vorrichtung.

Die Erfindung betrifft weiterhin die Verwendung von Polymeren gemäß der vorliegenden Erfindung als lichtemittierendes Material in einer organischen elektronischen Vorrichtung oder einer Komponente einer organischen elektronischen Vorrichtung.

Die Erfindung betrifft weiterhin ein halbleitendes und/oder lichtemittierendes Material, eine organische elektronische Vorrichtung oder eine Komponente einer organischen elektronischen Vorrichtung, enthaltend ein Polymer gemäß der vorliegenden Erfindung mit Elektronendonoreigenschaften und eine oder mehrere zusätzliche Verbindungen oder Polymere mit Elektronenakzeptoreigenschaften.

Die Erfindung betrifft weiterhin ein halbleitendes und/oder lichtemittierendes Material, eine organische elektronische Vorrichtung oder eine Komponente einer organischen elektronischen Vorrichtung, enthaltend ein Polymer gemäß der vorliegenden Erfindung mit Elektronenakzeptoreigenschaften und eine oder mehrere zusätzliche Verbindungen oder Polymere mit Elektronendonoreigenschaften. lichtemittierendes Material, eine organische elektronische Vorrichtung oder eine Komponente einer organischen elektronischen Vorrichtung, enthaltend ein oder mehrere Polymere gemäß der vorliegenden Erfindung mit Elektronendonoreigenschaften und ein oder mehrere Polymere gemäß der vorliegenden Erfindung mit Elektronenakzeptoreigenschaften.

Die Erfindung betrifft weiterhin eine Mischung oder einen Polymerblend enthaltend ein oder mehrere Polymere gemäß der vorliegenden Erfindung und eine oder mehrere zusätzliche Verbindungen oder Polymere, die vorzugsweise ausgewählt sind aus Verbindungen und Polymeren mit Halbleiter-, Ladungstransport-, Loch/Elektronentransport-, loch/elektronenblockierenden, elektrisch leitenden, photoleitenden oder lichtemittierenden Eigenschaften.

Die Erfindung betrifft weiterhin eine Mischung oder einen Polymerblend enthaltend ein oder mehrere Polymere gemäß der vorliegenden Erfindung mit Elektronendonoreigenschaften und eine oder mehrere zusätzliche Verbindungen aus gewählt aus Elektronenakzeptoren oder organischen Halbleitern des n-Typs, vorzugsweise ausgewählt aus der Gruppe bestehend aus Fullerenen und substituierten Fullerenen.

Die Erfindung betrifft weiterhin eine Formulierung enthaltend ein oder mehrere Polymere, Mischungen oder Polymerblends gemäß der vorliegenden Erfindung und ein oder mehrere Lösungsmittel, vorzugsweise ausgewählt aus organischen Lösungsmitteln.

Die Erfindung betrifft weiterhin die Verwendung von Polymeren, Formulierung, Mischungen und Polymerblends gemäß der vorliegenden Erfindung als Ladungstransportmaterial, Halbleitermaterial, elektrisch leitendes Material, photoleitendes Material oder lichtemittierendes Material, vorzugsweise in einer Vorrichtung mit optischen, elektrooptischen, elektronischen, Elektrolumineszenz- oder Photolumineszenz-Eigenschaften, in einer Komponente einer solchen Vorrichtung, oder in einem Produkt enthaltend eine solche Vorrichtung.

Die Erfindung betrifft weiterhin ein Ladungstransport-, Halbleiter-, elektrisch leitendes, photoleitendes oder lichtemittierendes Material, enthaltend ein Polymer, eine Formulierung, eine Mischung oder einen Polymerblend gemäß der vorliegenden Erfindung.

Die Erfindung betrifft weiterhin eine optische, elektrooptische, elektronische, Elektrolumineszenz- oder Photolumineszenz-Vorrichtung, eine Komponente einer solchen Vorrichtung, oder ein Produkt enthaltend eine solche Vorrichtung, welche ein Polymer, eine Formulierung, eine Mischung oder einen Polymerblend gemäß der vorliegenden Erfindung enthält.

Die optischen, elektrooptischen, elektronischen Elektrolumineszenz- und Photolumineszenz-Vorrichtungen umfassen, ohne hierauf beschränkt zu sein, organische Feldeffekttransistoren (organic field effect transistors - OFETs), organische Dünnschichttransistoren (organic thin film transistors - OTFTs), organische Leuchtdioden (organic light emitting diodes - OLEDs), organische lichtemittierende Transistoren (organic light emitting transistors - OLETs), organische Photovoltaikvorrichtungen (OPV), organische Photodetektoren (OPD), organische Solarzellen, Schottky-Dioden, Laserdioden, und organische Photoleiter.

Besonders bevorzugt sind OFETs, OPV-Vorrichtungen, organische Solarzellen und OPDs, insbesondere OPV-Vorrichtungen und organische Solarzellen worin die organischen Halbleiter in der photoaktiven Schicht einen Volumen-Heteroübergang (englisch "bulk heterojunction" oder "BHJ") bildet (BHJ-OPV).

Die Komponenten der erfindungsgemäßen Vorrichtungen umfassen, ohne hierauf beschränkt zu sein, Ladungsinjektionsschichten, Ladungstransportschichten, Zwischenschichten, Planarisierungsschichten, Antistatikfolien, Polymerelektrolytmembranen (PEMs), leitende Substrate, leitende Muster.

Die Produkte enthaltend die erfindungsgemäßen Vorrichtungen umfassen, ohne hierauf beschränkt zu sein, integrierte Schaltungen (integrated circuits - ICs), Kondensatoren, RFID-Tags (RFID - radio frequency identification) oder diese enthaltende Sicherheitsmarkierungen oder Sicherheitsvorrichtungen, Flachbildschirme, Hintergrundbeleuchtungen für Anzeigen, elektrophotographische Vorrichtungen, organische Speichervorrichtungen, Sensorvorrichtungen, Biosensoren und Biochips.

Die Erfindung betrifft weiterhin die Verwendung der Polymere, Formulierung, Mischungen und Polymerblends gemäß der vorliegenden Erfindung als Elektrodenmaterialien in Batterien und in Komponenten oder Vorrichtungen für den Nachweis und die Unterscheidung von DNA-Sequenzen.

**Abbildung 1a****-c** zeigen die Topographie einer Schicht eines Polymers aus Beispiel 3.2 vor (1a) bzw. nach thermischer Behandlung (1b) sowie nach Abspülen mit Toluol (1c).

**Abbildung 2** zeigt das Emissionsspektrum (2a) und den Farbort (2b) einer OLED mit einem Polymer nach Beispiel 3.2 vor und nach thermischer Nachbehandlung.

**Abbildung 3a****-d** zeigen die optoelektronischen Kenndaten einer OLED mit einem Polymer nach Beispiel 3.2 als Emitter.

**Abbildung 4** zeigt den Vergleich der Elektroluminszenz-Spektren eines Polymers nach Beispiel 3.3 bei einer Spannung von 6 V vor (löslich) und nach (unlöslich) thermischer Behandlung.

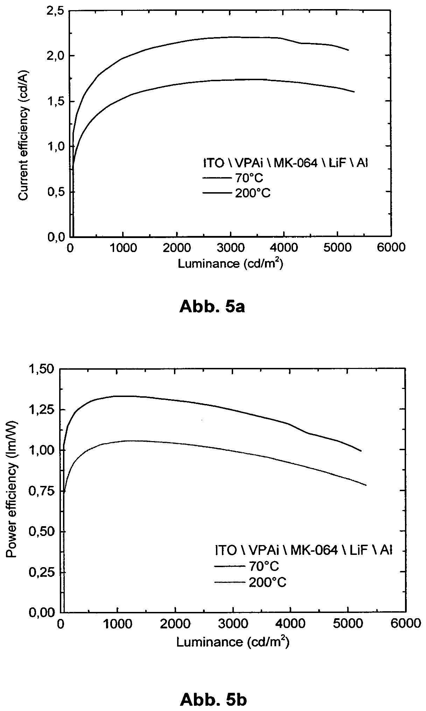

**Abbildung 5** zeigt die optoelektronischen Kenndaten einer OLED mit einem Polymer nach Beispiel 3.3 als Emitter.

**Abbildung 6** zeigt die Ul-Kennlinie einer organischen Solarzelle mit einer photoaktive Schicht enthaltend eine Mischung aus einem Polymer nach Beispiel 3.1 als Donor und PC₆₀BM als Akzeptor.

**Abbildung 7** zeigt exemplarisch den schematischen Aufbau der Top-Gate/Bottom-Contact OFETs gemäß den Beispielen 4.7 bis 4.9.

**Abbildung 8a, 8b****,** **9a, 9b****,** **10a** und **10b** zeigen die TFT Transfer Kennlinien von OFETs gemäß den Beispielen 4.7 bis 4.9.

Die Monomere und Polymere der vorliegenden Erfindung lassen sich leicht synthetisieren und weisen mehrere vorteilhafte Eigenschaften auf, wie eine niedrige Energielücke, eine hohe Ladungsträgerbeweglichkeit, eine hohe Löslichkeit in organischen Lösungsmitteln, eine gute Verarbeitbarkeit bei der Herstellung der Vorrichtung, eine hohe Oxidationsbeständigkeit und lange Lebensdauer in elektronischen Vorrichtungen.

Die Carbonat- oder Carbamat-Seitenketten erhöhen die Löslichkeit des Polymers in üblichen organischen Lösungsmitteln, was eine einfachere Verarbeitung des Materials aus der Lösung gestattet.

Die Carbonat- oder Carbamat-Seitenketten können nach Verarbeitung des Polymers aus der Lösung, z.B. in Form einer dünnen Schicht, thermisch abgespalten werden. Hierdurch wird die Stabilität der Polymerschicht gegenüber Lösungsmitteln, die z.B. zum nachfolgenden Auftrag zusätzlicher Schichten verwendet werden, erhöht. Ausserdem wird die Kristallisation des Polymers verbessert und eine verbesserte pi-pi-Stapelung der Polymerketten im festen Zustand ermöglicht, was zu verbesserten Ladungstransporteigenschaften in Form höherer Ladungsträgerbeweglichkeit führt.

Die löslichkeitsverbessernden Gruppen in den erfindungsgemäßen Polymeren zeigen eine bessere Löslichkeit gegenüber Standardgruppen, wie Alkylreste, in herkömmlichen Lösungsmitteln. Dies konnte durch Gel-Permeations-Chromatographie (GPC) anhand der Mₙ-Werte der Polymere nachgewiesen werden. Dabei wurden Standardpolymere, wie Poly-3-hexyl-thiophen (P3HT), mit den erfindungsgemäßen Polymeren in verschiedenen Lösungsmitteln (Vergleich zwischen Aceton und Chloroform) untersucht.

Mit dem Begriff "Polymer" wird allgemein ein Molekül mit hoher relativer Molekülmasse bezeichnet, dessen Struktur im Wesentlichen die mehrfache Wiederholung von Einheiten umfasst, die tatsächlich oder vom Konzept her von Molekülen mit geringer relativer Molekülmasse abgeleitet sind (PAC, 1996, 68, 2291). Mit dem Begriff "Oligomer" wird allgemein ein Molekül mit mittlerer relativer Molekülmasse bezeichnet, dessen Struktur im Wesentlichen eine kleine Anzahl von Einheiten umfasst, die tatsächlich oder vom Konzept her von Molekülen mit geringerer relativer Molekülmasse abgeleitet sind (PAC, 1996, 68, 2291). In einer bevorzugten Bedeutung gemäß der vorliegenden Erfindung bezeichnet ein Polymer eine Verbindung mit > 1, vorzugsweise ≥ 5 Wiederholungseinheiten, und ein Oligomer bezeichnet eine Verbindung mit > 1 und < 10, vorzugsweise < 5 Wiederholungseinheiten.

Wenn nicht anders angegeben, ist als Molekulargewicht das zahlenmittlere Molekulargewicht Mₙ oder gewichtsmittlere Molekulargewicht M_{w} angegeben, das durch Gelpermeationschromatographie (GPC) gegen Polystyrolstandards in eluierenden Lösungsmitteln wie Tetrahydrofuran, Trichlormethan (TCM, Chloroform), Chlorbenzol oder 1,2,4-Trichlorbenzol bestimmt wird. Wenn nicht anders angegeben, wird Trichlormethan als Lösungsmittel eingesetzt. Der Polymerisationsgrad (n) bezeichnet den zahlenmittleren Polymerisationsgrad, gegeben durch n = Mₙ/M_{U}, worin M_{U} das Molekulargewicht der einzelnen Wiederholungseinheit ist, wie in J. M. G. Cowie, Polymers: Chemistry & Physics of Modem Materials, Blackie, Glasgow, 1991, beschrieben.

Vor- und nachstehend gibt in einer Formel, die ein Polymer oder eine Wiederholungseinheit zeigt, wie die Formel I und ihre Unterformeln, ein Sternchen (*) eine Verknüpfung mit der benachbarten Wiederholungseinheit in der Polymerkette an.

Die Begriffe "Wiederholungseinheit" und "Monomereinheit" bezeichnen die zu Grunde liegende Wiederholungseinheit (constitutional repeating unit - CRU), bei der es sich um die kleinste zu Grunde liegende Einheit handelt, deren Wiederholung ein reguläres Makromolekül, ein reguläres Oligomermolekül, einen regulären Block oder eine reguläre Kette darstellt (PAC, 1996, 68, 2291). Der Begriff "Einheit" bezeichnet eine Struktureinheit, welche selbst eine Wiederholungseinheit sein kann oder zusammen mit anderen Einheiten eine Wiederholungseinheit bilden kann. Der Begriff "Abgangsgruppe" bezeichnet ein Atom oder eine Gruppe (geladen oder ungeladen), das/die durch Teilnahme an einer spezifischen Reaktion von einem Atom in dem Teil, der als Rest- oder Hauptteil des Moleküls betrachtet wird, getrennt wird (siehe auch PAC, 1994, 66, 1134).

Der Begriff "konjugiert" bezeichnet eine Verbindung, die überwiegend C-Atome mit sp²-Hybridisierung (oder gegebenenfalls auch sp-Hybridisierung) enthält, die auch durch Heteroatome ersetzt sein können. Im einfachsten Fall ist dies beispielsweise eine Verbindung mit alternierenden C-C-Einfach- und -Doppel- (oder -Dreifach-)bindungen, beinhaltet aber auch Verbindungen mit Einheiten wie 1,3-Phenylen. "Hauptsächlich" bedeutet in diesem Zusammenhang, dass eine Verbindung mit natürlich (spontan) auftretenden Defekten, die zu einer Unterbrechung der Konjugation führen können, ebenfalls als konjugierte Verbindung betrachtet wird.

Die Begriffe "Donor" und "Akzeptor" bezeichnen jeweils einen Elektronendonor bzw. -akzeptor. Der Begriff "Elektronendonor" bezeichnet eine chemische Verbindung oder Gruppe, die Elektronen an eine andere chemische Verbindung oder Gruppe abgibt. Der Begriff "Elektronenakzeptor" bezeichnet eine chemische Verbindung oder Gruppe, die Elektronen von einer anderen chemischen Verbindung oder Gruppe annimmt (siehe U.S. Environmental Protection Agency, 2009, Glossary of technical terms) http://www.epa.gov/oust/cat/TUMGLOSS.HTM

Der Begriff "n-Typ" oder "n-Typ-Halbleiter" bezeichnet einen extrinsischen Halbleiter worin die Dichte der leitenden Elektronen höher ist als die Dichte der beweglichen Löcher. Der Begriff "p-Typ" oder "p-Typ-Halbleiter" bezeichnet einen extrinsischen Halbleiter worin die Dichte der leitenden Elektronen geringer ist als die Dichte der beweglichen Löcher (siehe, J. Thewlis, Concise Dictionary of Physics, Pergamon Press, Oxford, 1973).

Der Begriff "Benzo[lmn][3,8]phenanthrolin-1,3,6,8-tetraon-4,9-diyl" bezeichnet eine Gruppe der folgenden Formel ("NDI") 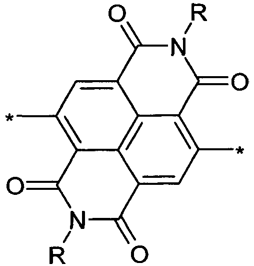 worin R einen Substituenten, beispielsweise eine Carbyl- oder Hydrocarbylgruppe wie vor- und nachstehend definiert, oder ein H-Atom bedeutet.

Der Begriff "Pyrrolo[3,4-c]pyrrol-1,4-dion-3,6-diyl" bezeichnet eine Gruppe der folgenden Formel 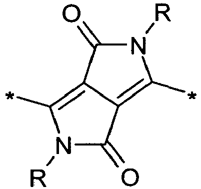 worin R einen Substituenten, beispielsweise eine Carbyl- oder Hydrocarbylgruppe wie vor- und nachstehend definiert, oder ein H-Atom bedeutet.

Der Begriff "Carbylgruppe" wie vor- und nachstehend verwendet steht für einen beliebigen einwertigen oder mehrwertigen organischen Molekülrest, der mindestens ein Kohlenstoffatom entweder ohne irgendwelche Nicht-Kohlenstoffatome (wie z.B. -C≡C-) oder gegebenenfalls in Verbindung mit mindestens einem Nicht-Kohlenstoffatom wie N, O, S, P, Si, Se, As, Te oder Ge (z.B. Carbonyl usw.) enthält. Der Begriff "Hydrocarbylgruppe" bezeichnet eine Carbylgruppe, welche zusätzlich ein oder mehrere H-Atome und gegebenenfalls ein oder mehrere Heteroatome wie beispielsweise N, O, S, P, Si, Se, As, Te oder Ge enthält.

Eine Carbyl- oder Hydrocarbylgruppe mit einer Kette von 3 oder mehr C-Atomen kann auch geradkettig, verzweigt und/oder cyclisch sein, einschließlich Spiro- und/oder anellierte Ringe.

Zu den bevorzugten Carbyl- und Hydrocarbylgruppen zählen Alkyl, Alkoxy, Alkylcarbonyl, Alkoxycarbonyl, Alkylcarbonyloxy und Alkoxycarbonyloxy, das jeweils gegebenenfalls substituiert ist und 1 bis 40, vorzugsweise 1 bis 25, sehr bevorzugt 1 bis 18 C-Atome aufweist, weiterhin gegebenenfalls substituiertes Aryl oder Aryloxy mit 6 bis 40, vorzugsweise 6 bis 25 C-Atomen, weiterhin Alkylaryloxy, Arylcarbonyl, Aryloxycarbonyl, Arylcarbonyloxy und Aryloxycarbonyloxy, das jeweils gegebenenfalls substituiert ist und 6 bis 40, vorzugsweise 7 bis 40 C-Atome aufweist, wobei alle diese Gruppen gegebenenfalls ein oder mehrere Heteroatome enthalten, vorzugsweise ausgewählt aus N, O, S, P, Si, Se, As, Te und Ge.

Bei der Carbyl- oder Hydrocarbylgruppe kann es sich um eine gesättigte oder ungesättigte acyclische Gruppe oder eine gesättigte oder ungesättigte cyclische Gruppe handeln. Ungesättigte acyclische oder cyclische Gruppen sind bevorzugt, insbesondere Aryl-, Alkenyl- und Alkinylgruppen (insbesondere Ethinyl). Eine acyclische C₁-C₄₀ Carbyl- oder Hydrocarbylgruppe kann geradkettig oder verzweigt sein. Die C₁-C₄₀ Carbyl- oder Hydrocarbylgruppe umfasst beispielsweise: eine C₁-C₄₀ Alkylgruppe, eine C₁-C₄₀ Alkoxy- oder Oxaalkylgruppe, eine C₂-C₄₀ Alkenylgruppe, eine C₂-C₄₀ Alkinylgruppe, eine C₃-C₄₀ Allylgruppe, eine C₄-C₄₀ Alkyldienylgruppe, eine C₄-C₄₀ Polyenylgruppe, eine C₆-C₁₈ Arylgruppe, eine C₆-C₄₀ Alkylarylgruppe, eine C₆-C₄₀ Arylalkylgruppe, eine C₄-C₄₀ Cycloalkylgruppe, eine C₄-C₄₀ Cycloalkenylgruppe und dergleichen. Bevorzugt unter den vorstehenden Gruppen sind eine C₁-C₂₀Alkylgruppe, eine C₂-C₂₀ Alkenylgruppe, eine C₂-C₂₀ Alkinylgruppe, eine C₃-C₂₀ Allylgruppe, eine C₄-C₂₀ Alkyldienylgruppe, eine C₆-C₁₂ Arylgruppe bzw. eine C₄-C₂₀ Polyenylgruppe. Ebenfalls eingeschlossen sind Kombinationen von Gruppen mit Kohlenstoffatomen und Gruppen mit Heteroatomen, wie z.B. eine Alkinylgruppe, vorzugsweise Ethinyl, die mit einer Silylgruppe, vorzugsweise einer Trialkylsilylgruppe, substituiert ist.

Aryl und Heteroaryl bedeuten vorzugsweise eine mono-, bi- oder tricyclische aromatische oder heteroaromatische Gruppe mit 4 bis 30 Ring-C-Atomen, die auch kondensierte Ringe enthalten kann und gegebenenfalls mit einer oder mehreren Gruppen L wie oben definiert substituiert ist.

Besonders bevorzugte Substituenten L sind aus Halogen, insbesondere bevorzugt F, oder Alkyl, Alkoxy, Oxaalkyl, Thioalkyl, Fluoralkyl und Fluoralkoxy mit 1 bis 12 C-Atomen oder Alkenyl, Alkinyl mit 2 bis 12 C-Atomen ausgewählt.

Besonders bevorzugte Aryl- und Heteroarylgruppen sind Phenyl, in dem zusätzlich eine oder mehrere CH-Gruppen durch N ersetzt sein können, Naphthalin, Thiophen, Selenophen, Thienothiophen, Dithienothiophen, Fluoren und Oxazol, die alle jeweils unsubstituiert oder ein- oder mehrfach mit L wie oben definiert substituiert sein können. Sehr bevorzugte Ringe sind ausgewählt aus Pyrrol, vorzugsweise N-Pyrrol, Pyridin, vorzugsweise 2- oder 3-Pyridin, Pyrimidin, Thiophen vorzugsweise 2-Thiophen, Selenophen, vorzugsweise 2-Selenophen, Thieno[3,2-b]thiophen, Thiazol, Thiadiazol, Oxazol und Oxadiazol, besonders bevorzugt Thiophen-2-yl, 5-substituiertes Thiophen-2-yl oder Pyridin-3-yl, die alle jeweils unsubstituiert oder ein- oder mehrfach mit L wie oben definiert substituiert sein können.

Ein Alkyl- oder Alkoxyrest, d.h. wo die CH₂-Endgruppe durch -O- ersetzt ist, kann geradkettig oder verzweigt sein. Vorzugsweise ist er geradkettig, weist 2, 3, 4, 5, 6, 7 oder 8 Kohlenstoffatome auf und steht somit vorzugsweise z.B. für Ethyl, Propyl, Butyl, Pentyl, Hexyl, Heptyl, Octyl, Ethoxy, Propoxy, Butoxy, Pentoxy, Hexoxy, Heptoxy oder Octoxy, weiterhin Methyl, Nonyl, Decyl, Undecyl, Dodecyl, Tridecyl, Tetradecyl, Pentadecyl, Nonoxy, Decoxy, Undecoxy, Dodecoxy, Tridecoxy oder Tetradecoxy.

Eine Alkenylgruppe, worin eine oder mehrere CH₂-Gruppen durch -CH=CH- ersetzt sind, kann geradkettig oder verzweigt sein. Vorzugsweise ist sie geradkettig, weist 2 bis 10 C-Atome auf und steht somit vorzugsweise für Vinyl, Prop-1- oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Besonders bevorzugte Alkenylgruppen sind C₂-C₇-1E-Alkenyl, C₄-C₇-3E-Alkenyl, C₅-C₇-4-Alkenyl, C₆-C₇-5-Alkenyl und C₇-6-Alkenyl, insbesondere C₂-C₇-1E-Alkenyl, C₄-C₇-3E-Alkenyl und C₅-C₇-4-Alkenyl. Beispiele für besonders bevorzugte Alkenylgruppen sind Vinyl, 1E-Propenyl, 1E-Butenyl, 1E-Pentenyl, 1E-Hexenyl, 1E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl, 6-Heptenyl und dergleichen. Gruppen mit bis zu 5 C-Atomen sind im Allgemeinen bevorzugt.

Eine Oxaalkylgruppe, d.h. wo eine CH₂-Gruppe durch -O- ersetzt ist, steht vorzugsweise z.B. für geradkettiges 2-Oxapropyl (=Methoxymethyl), 2-(=Ethoxymethyl) oder 3-Oxabutyl (=2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl oder 2-, 3-, 4-, 5-, 6-, 7-, 8- oder 9-Oxadecyl. Oxaalkyl, d.h. wo eine CH₂-Gruppe durch -O- ersetzt ist, steht vorzugsweise z.B. für geradkettiges 2-Oxapropyl (=Methoxymethyl), 2- (=Ethoxymethyl) oder 3-Oxabutyl (=2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl oder 2-, 3-, 4-, 5-, 6-, 7-, 8- oder 9-Oxadecyl.

In einer Alkylgruppe, in der eine CH₂-Gruppe durch -O- und eine durch -CO- ersetzt ist, sind diese Reste vorzugsweise benachbart. Somit bilden diese Reste zusammen eine Carbonyloxygruppe -CO-O- oder eine Oxycarbonylgruppe -O-CO-. Vorzugsweise ist diese Gruppe geradkettig und weist 2 bis 6 C-Atome auf. Somit steht sie vorzugsweise für Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 3-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)propyl, 4-(Methoxycarbonyl)-butyl.

Eine Alkylgruppe, in der zwei oder mehr CH₂-Gruppen durch -O- und/oder -COO- ersetzt sind, kann geradkettig oder verzweigt sein. Vorzugsweise ist sie geradkettig und weist 3 bis 12 C-Atome auf. Sie steht somit vorzugsweise für Bis-carboxy-methyl, 2,2-Bis-carboxy-ethyl, 3,3-Bis-carboxypropyl, 4,4-Bis-carboxy-butyl, 5,5-Bis-carboxy-pentyl, 6,6-Bis-carboxyhexyl, 7,7-Bis-carboxy-heptyl, 8,8-Bis-carboxy-octyl, 9,9-Bis-carboxynonyl, 10,10-Bis-carboxy-decyl, Bis-(methoxycarbonyl)-methyl, 2,2-Bis-(methoxycarbonyl)-ethyl, 3,3-Bis-(methoxycarbonyl)-propyl, 4,4-Bis-(methoxycarbonyl)-butyl, 5,5-Bis-(methoxycarbonyl)-pentyl, 6,6-Bis-(methoxycarbonyl)-hexyl, 7,7-Bis-(methoxycarbonyl)-heptyl, 8,8-Bis-(methoxycarbonyl)-octyl, Bis-(ethoxycarbonyl)-methyl, 2,2-Bis-(ethoxycarbonyl)-ethyl, 3,3-Bis-(ethoxycarbonyl)-propyl, 4,4-Bis-(ethoxycarbonyl)-butyl, 5,5-Bis-(ethoxycarbonyl)-hexyl.

Bei einer Thioalkylgruppe, d.h. wo eine CH₂-Gruppe durch -S- ersetzt ist, handelt es sich vorzugsweise um geradkettiges Thiomethyl (-SCH₃), 1-Thioethyl (-SCH₂CH₃), 1-Thiopropyl (= -SCH₂CH₂CH₃), 1-(Thiobutyl), 1-(Thiopentyl), 1-(Thiohexyl), 1-(Thioheptyl), 1-(Thiooctyl), 1-(Thiononyl), 1-(Thiodecyl), 1-(Thioundecyl) oder 1-(Thiododecyl), in denen vorzugsweise die dem sp²-hybridisierten Vinyl-Kohlenstoffatom benachbarte CH₂-Gruppe ersetzt ist.

Bei einer Fluoralkylgruppe handelt es sich vorzugsweise um geradkettiges Perfluoralkyl CᵢF₂ᵢ₊₁, worin i für eine ganze Zahl von 1 bis 15 steht, insbesondere CF₃, C₂F₅, C₃F₇, C₄F₉, C₅F₁₁, C₆F₁₃, C₇F₁₅ oder C₈F₁₇, sehr bevorzugt C₆F₁₃.

Bei den oben genannten Alkyl-, Alkoxy-, Alkenyl-, Oxaalkyl-, Thioalkyl-, Carbonyl- und Carbonyloxygruppen kann es sich um achirale oder chirale Gruppen handeln. Besonders bevorzugte chirale Gruppen sind z.B. 2-Butyl (=1-Methylpropyl), 2-Methylbutyl, 2-Methylpentyl, 3-Methylpentyl, 2-Ethylhexyl, 2-Propylpentyl, insbesondere 2-Methylbutyl, 2-Methylbutoxy, 2-Methylpentoxy, 3-Methylpentoxy, 2-Ethylhexoxy, 1-Methylhexoxy, 2-Octyloxy, 2-Oxa-3-methylbutyl, 3-Oxa-4-methylpentyl, 4-Methylhexyl, 2-Hexyl, 2-Octyl, 2-Nonyl, 2-Decyl, 2-Dodecyl, 6-Methoxyoctoxy, 6-Methyloctoxy, 6-Methyloctanoyloxy, 5-Methylheptyloxycarbonyl, 2-Methylbutyryloxy, 3-Methylvaleroyloxy, 4-Methylhexanoyloxy, 2-Chlorpropionyloxy, 2-Chlor-3-methylbutyryloxy, 2-Chlor-4-methylvaleryloxy, 2-Chlor-3-methyl-valeryloxy, 2-Methyl-3-oxapentyl, 2-Methyl-3-oxahexyl, 1-Methoxypropyl-2-oxy, 1-Ethoxypropyl-2-oxy, 1-Propoxypropyl-2-oxy, 1-Butoxypropyl-2-oxy, 2-Fluoroctyloxy, 2-Fluordecyloxy, 1,1,1-Trifluor-2-octyloxy, 1,1,1-Trifluor-2-octyl, 2-Fluormethyloctyloxy. Sehr bevorzugt sind 2-Hexyl, 2-Octyl, 2-Octyloxy, 1,1,1-Trifluor-2-hexyl, 1,1,1-Trifluor-2-octyl und 1,1,1-Trifluor-2-octyloxy.

Als achirale verzweigte Gruppen sind Isopropyl, Isobutyl (=Methylpropyl), Isopentyl (=3-Methylbutyl), tert.-Butyl, Isopropoxy, 2-Methylpropoxy und 3-Methylbutoxy bevorzugt.

In einer weiteren bevorzugten Ausführungsform der vorliegenden Erfindung sind die Alkyl- und Alkoxygruppen ausgewählt aus primärem, sekundärem oder tertiärem Alkyl oder Alkoxy mit 1 bis 30 C-Atomen, bei dem ein oder mehrere H-Atome gegebenenfalls durch F ersetzt sind, oder Aryl, Aryloxy, Heteroaryl oder Heteroaryloxy, das gegebenenfalls alkyliert oder alkoxyliert ist und 4 bis 30 Ringatome aufweist. Sehr bevorzugte Gruppen dieser Art sind aus der Gruppe ausgewählt, die aus den folgenden Formeln besteht 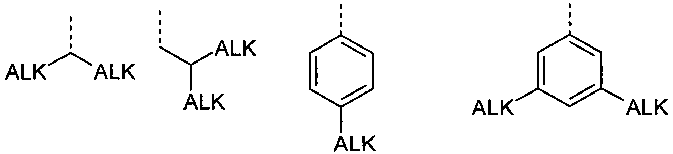 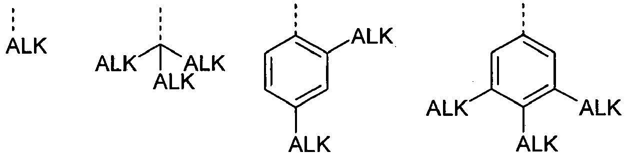 in denen "ALK" gegebenenfalls fluoriertes, vorzugsweise lineares, Alkyl oder Alkoxy mit 1 bis 20, vorzugsweise 1 bis 12 C-Atomen, bei tertiären Gruppen sehr bevorzugt 1 bis 9 C-Atomen, bedeutet, und die gestrichelte Linie die Verbindung zum Ring angibt, mit dem diese Gruppen verknüpft sind. Besonders bevorzugt unter diesen Gruppen sind diejenigen, in denen alle ALK-Teilgruppen gleich sind.

-CY¹=CY²- bedeutet vorzugsweise -CH=CH-, -CF=CF- oder -CH=C(CN)-.

Halogen bedeutet F, Cl, Br oder I, vorzugsweise F, Cl oder Br.

Die erfindungsgemäßen konjugierten Polymere enthalten vorzugsweise keine Wiederholungseinheiten ausgewählt aus gegebenenfalls substituiertem Pyrrolo[3,4-c]pyrrol-1,4-dion-3,6-diyl.

Die erfindungsgemäßen konjugierten Polymere enthalten vorzugsweise keine Wiederholungseinheiten ausgewählt aus Phenylen, welches mit einer oder mehreren Carbamatgruppen substituert ist, besonders bevorzugt keine gegebenenfalls substituierten Phenyleneinheiten.

Eine bevorzugte Ausführungsform richtet sich auf erfindungsgemäße konjugierte Polymere, worin die mit einer Carbonatgruppe oder Carbamatgruppe substituierten Wiederholungseinheiten, oder die Einheiten der Formel I, aus der Gruppe bestehend aus den folgenden Formeln ausgewählt ist, welche vorzugsweise Elektronendonoreigenschaften aufweisen: 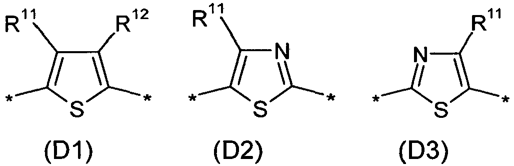 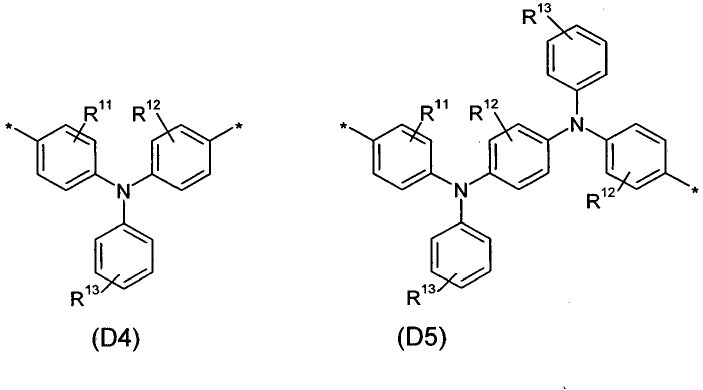 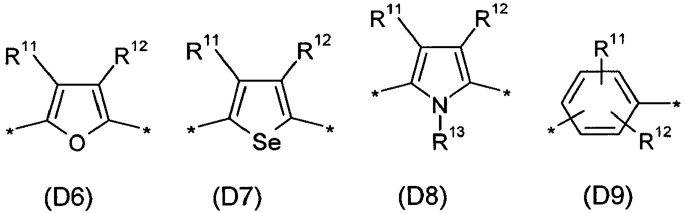 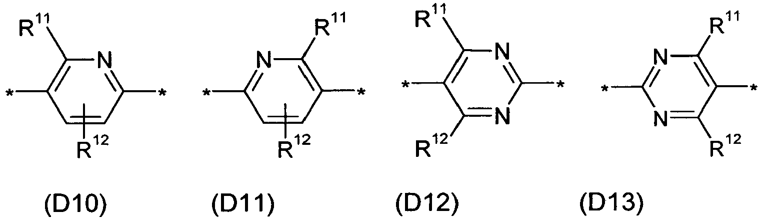 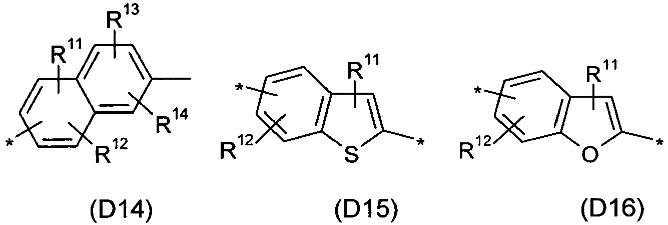 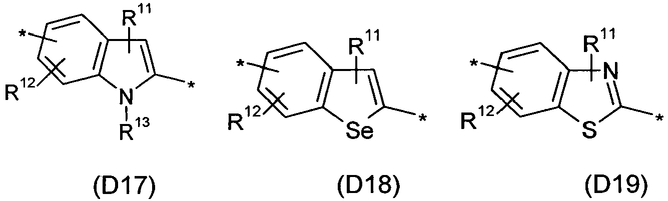 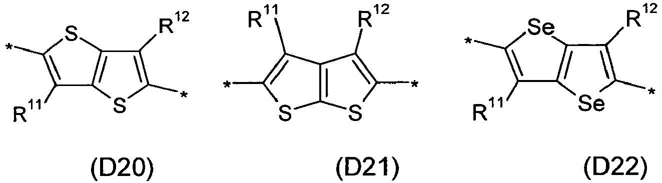 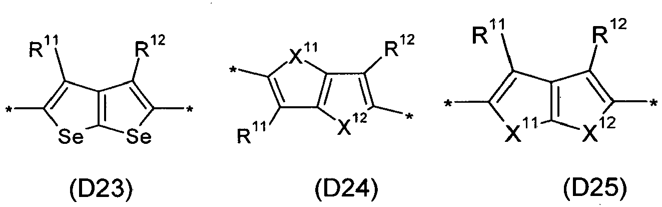 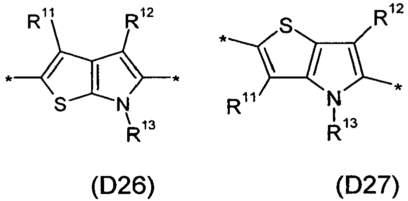 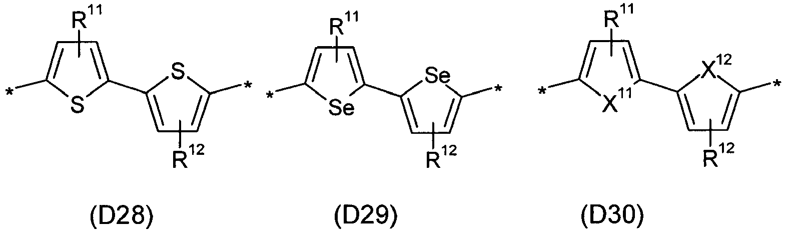 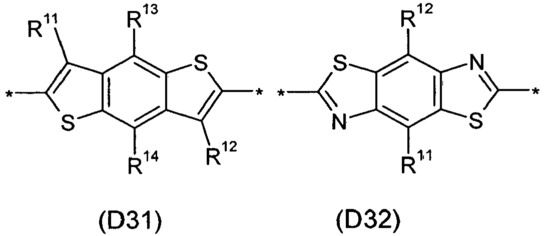 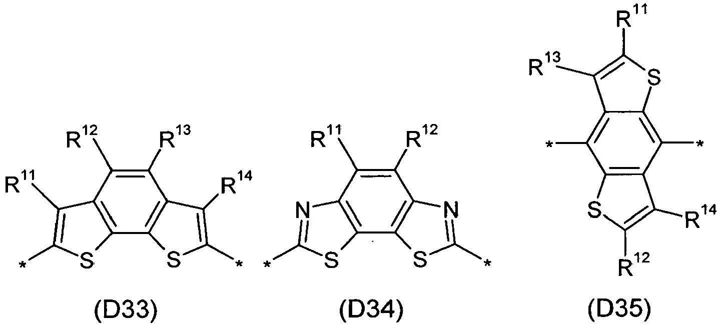 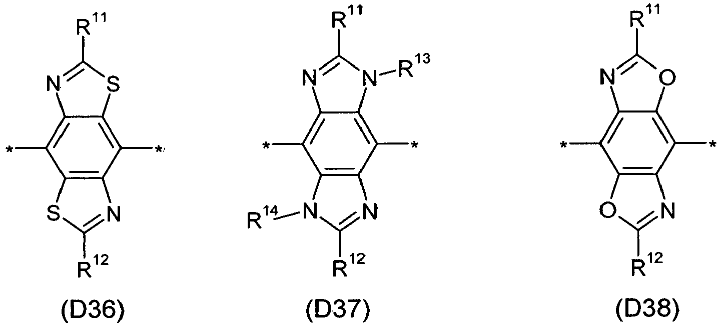 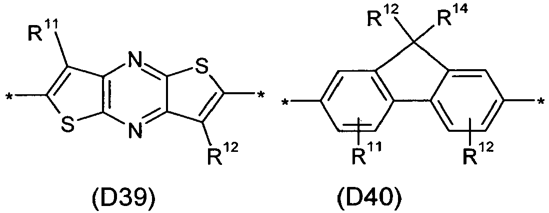 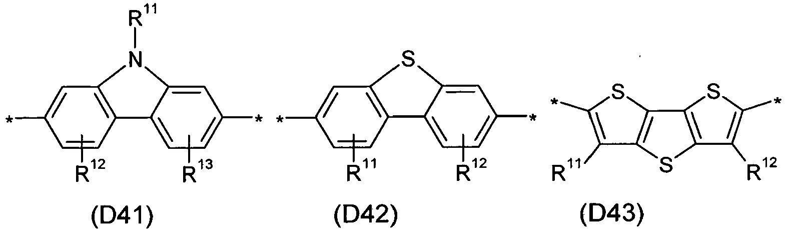 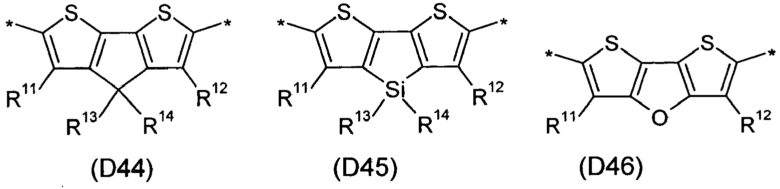 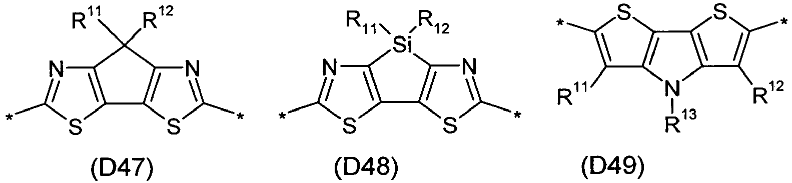 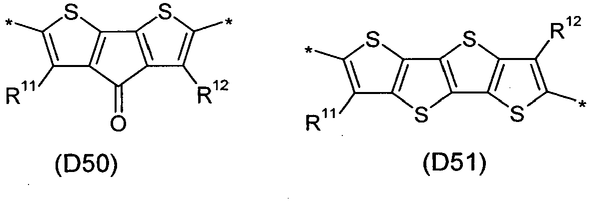 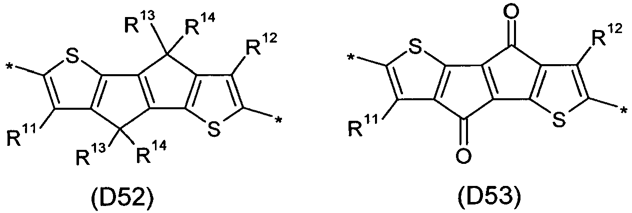 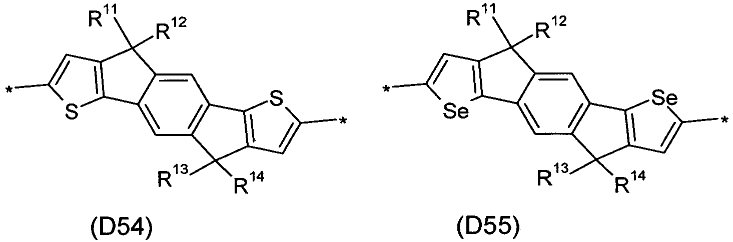 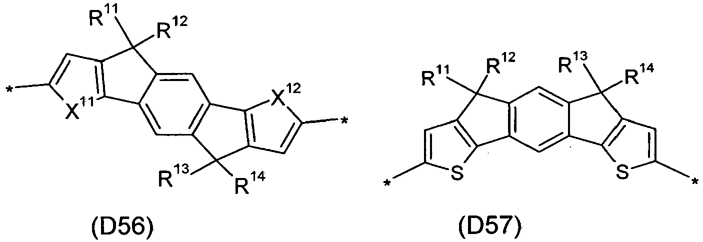 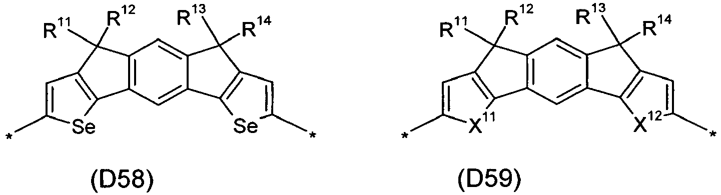 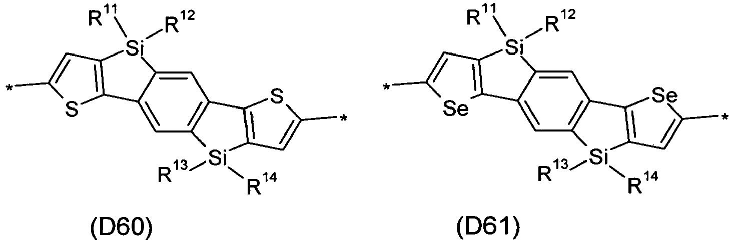 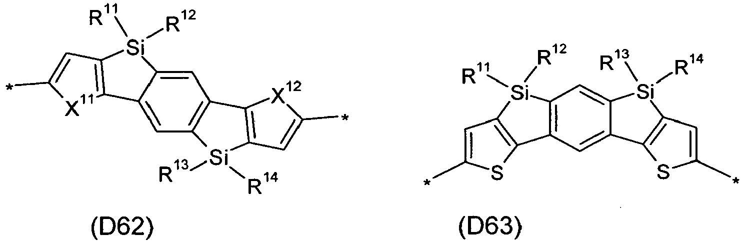 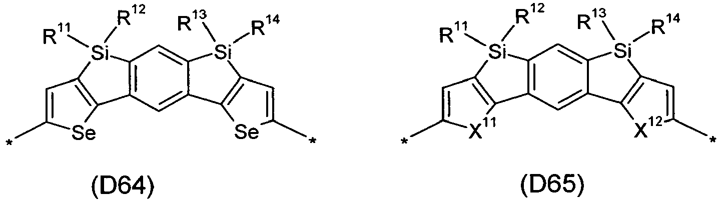 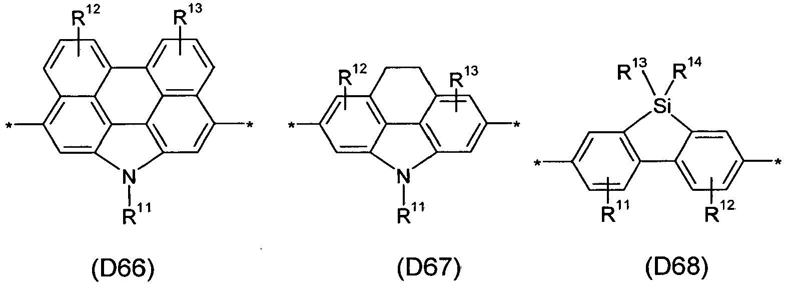 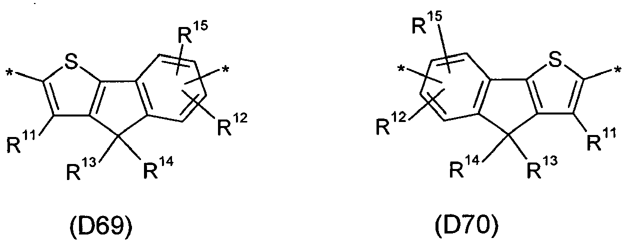 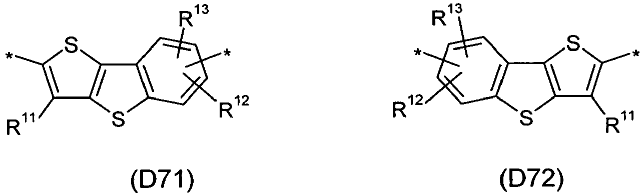 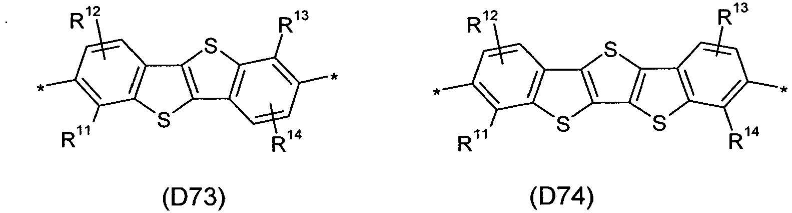 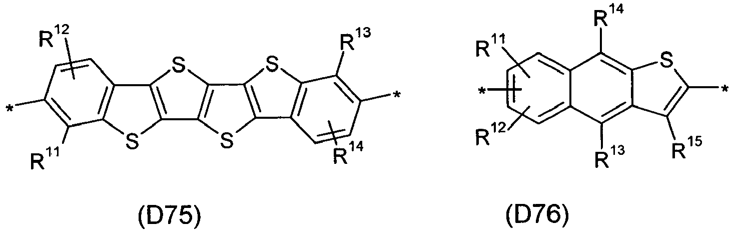 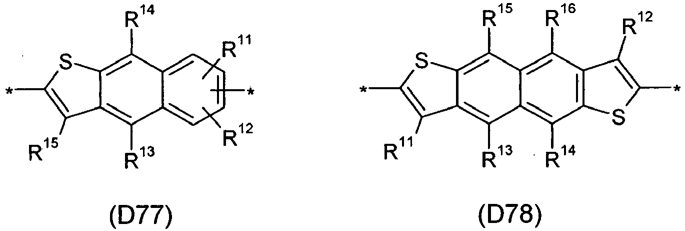 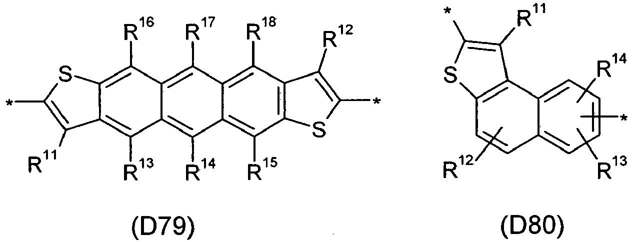 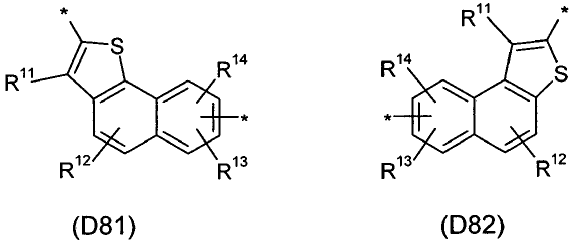 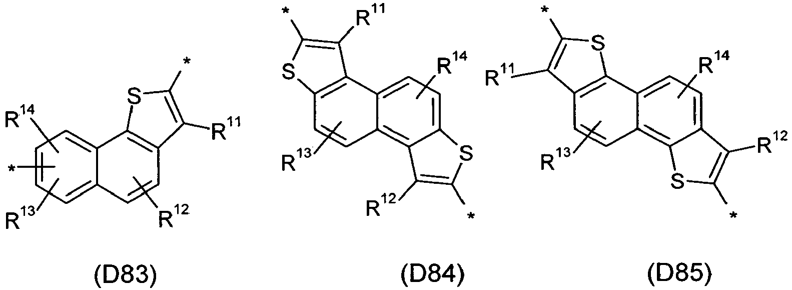 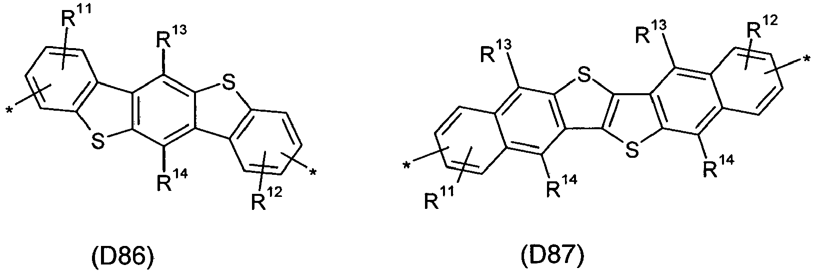 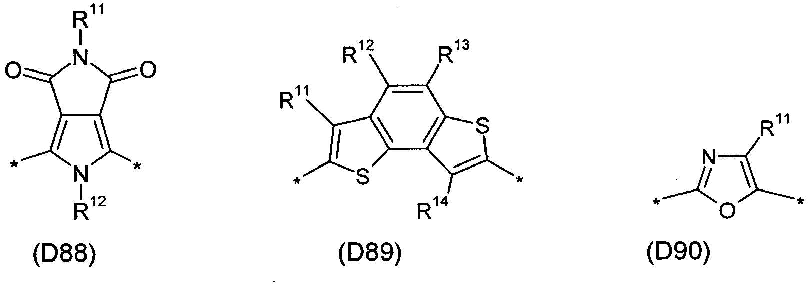 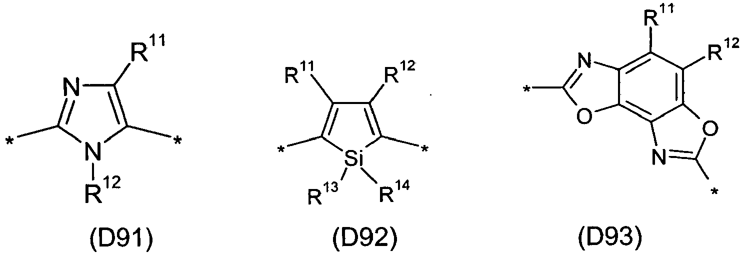 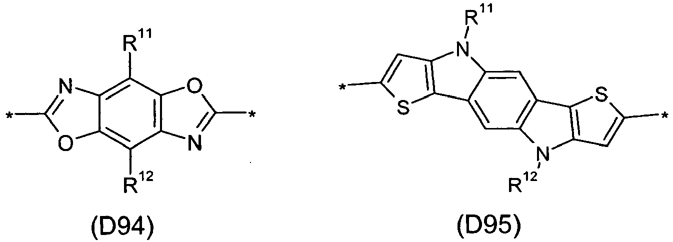 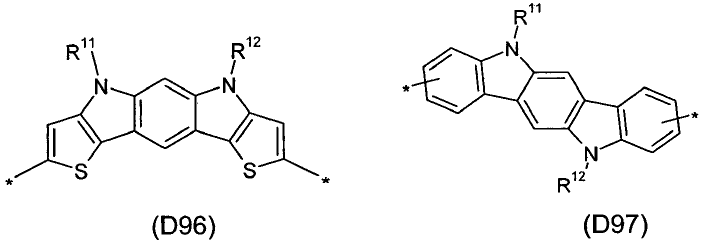 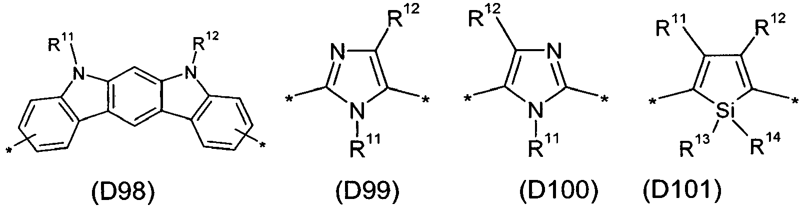 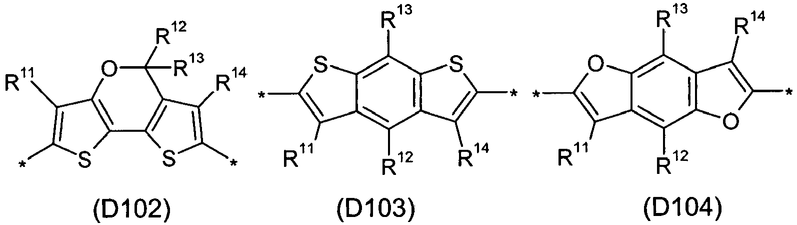 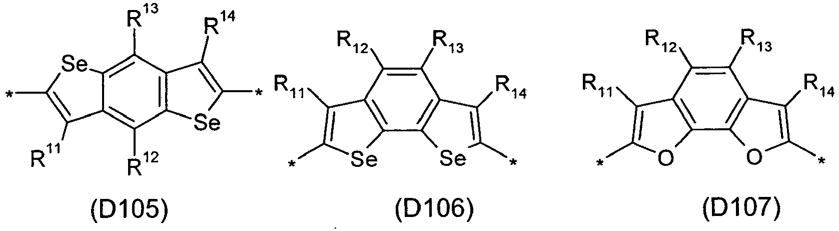 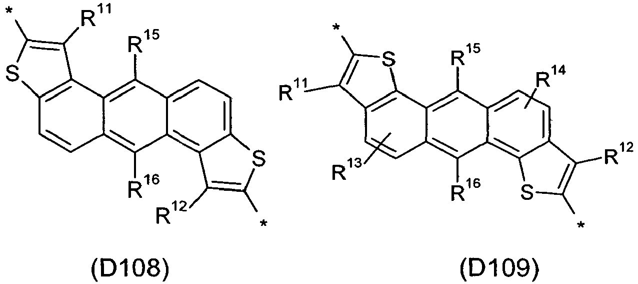 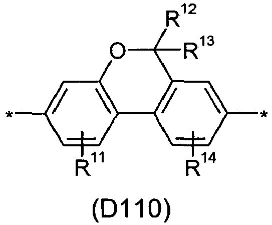 worin einer der Reste X¹¹ und X¹² S und der andere Se bedeutet, und R¹¹, R¹², R¹³, R¹⁴, R¹⁵, R¹⁶, R¹⁷ and R¹⁸ jeweils unabhängig voneinander H oder R¹ wie in Formel I definiert bedeuten, und worin einer oder mehrere der Reste R¹¹, R¹², R¹³, R¹⁴, R¹⁵, R¹⁶, R¹⁷ und R¹⁸ eine Gruppe -Sp¹-X¹-C(O)-O-R¹ wie in Formel I definiert bedeutet.

Eine bevorzugte Ausführungsform richtet sich auf erfindungsgemäße konjugierte Polymere, worin die mit einer Carbonatgruppe oder Carbamatgruppe substituierten Wiederholungseinheiten, oder die Einheiten der Formel I, aus der Gruppe bestehend aus den folgenden Formeln ausgewählt sind, welche vorzugsweise Elektronenakzeptoreigenschaften aufweisen: 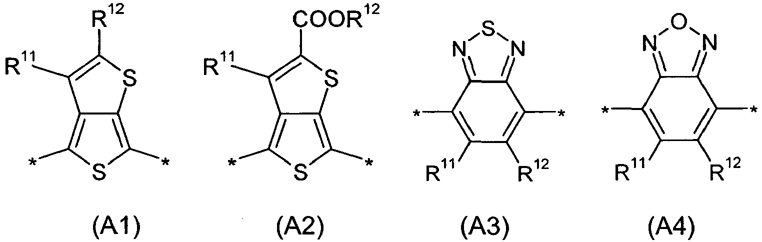 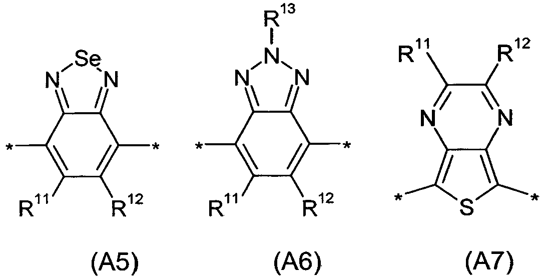 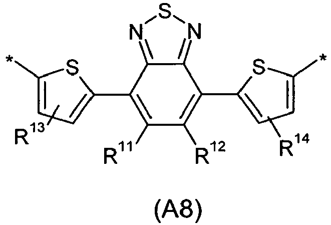 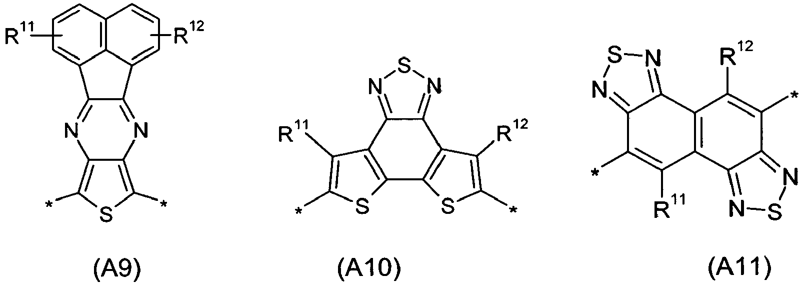 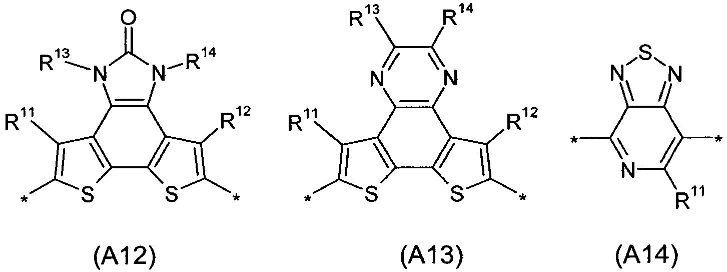 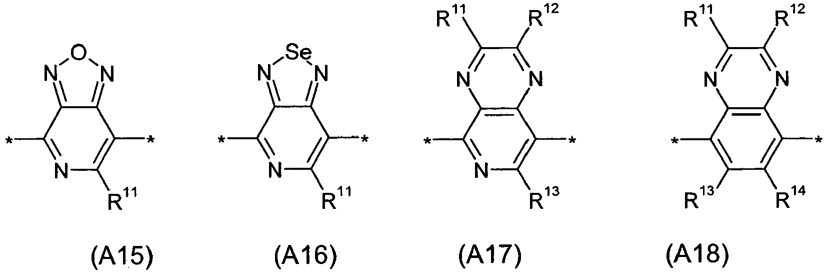 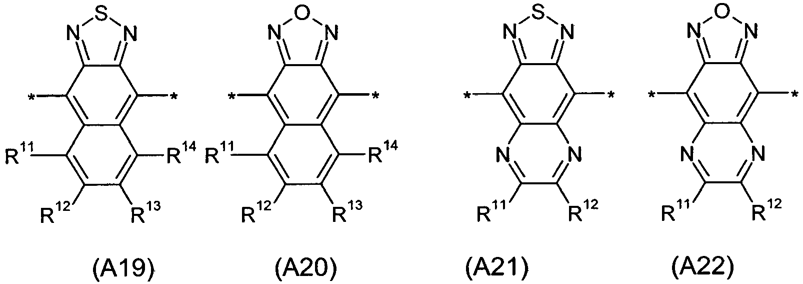 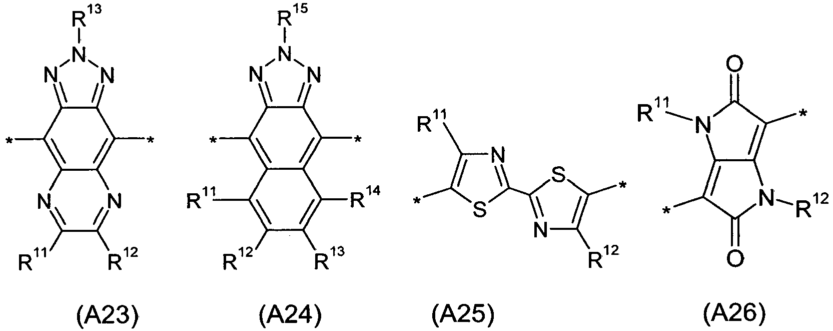 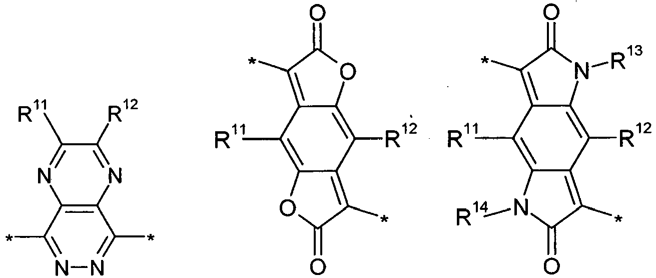 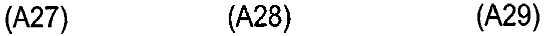 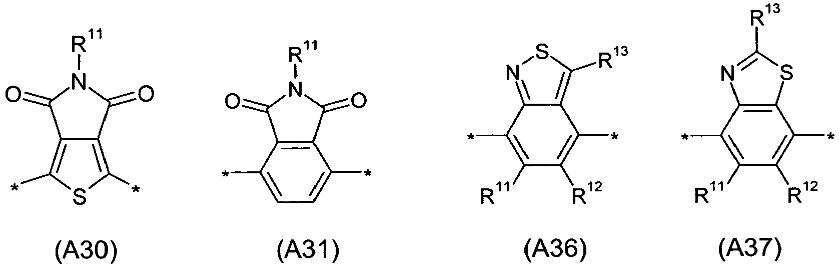 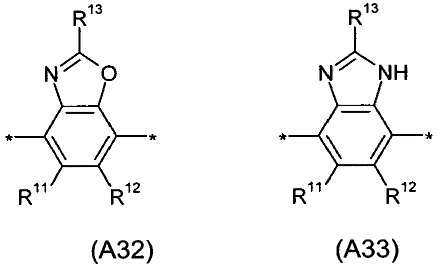 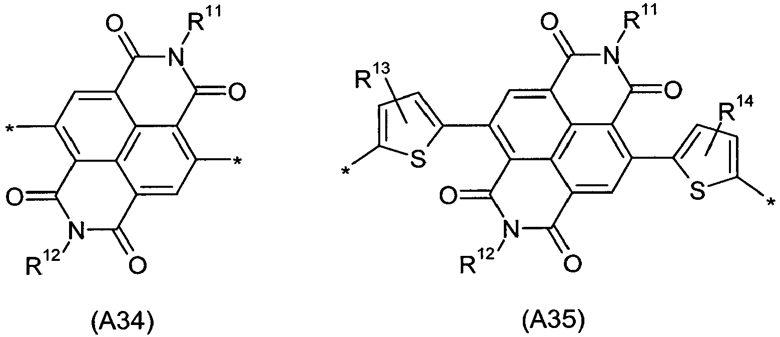 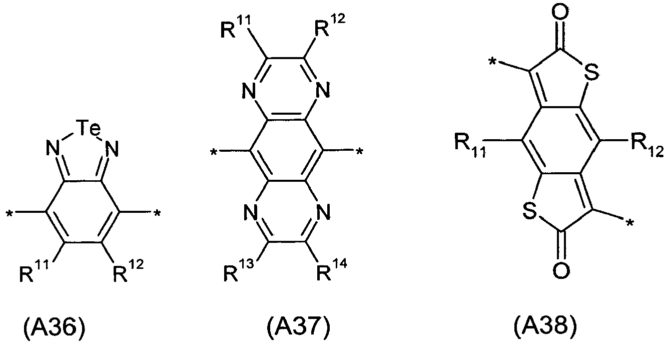 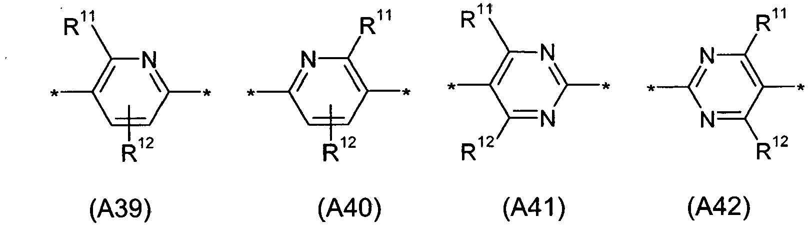 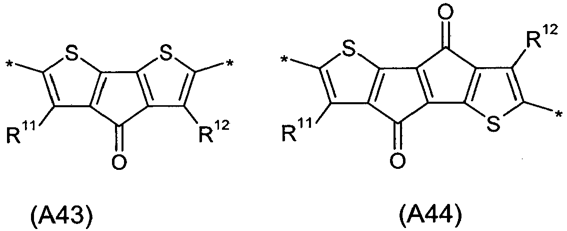 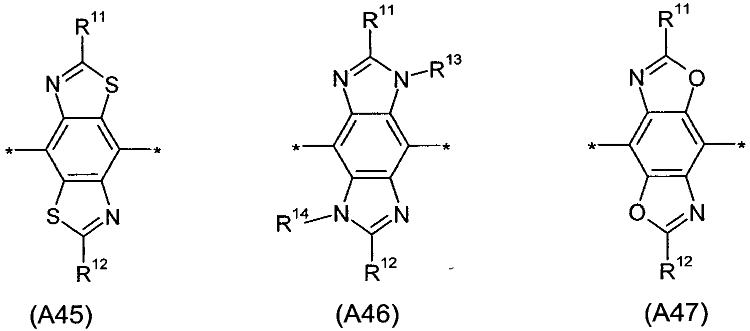 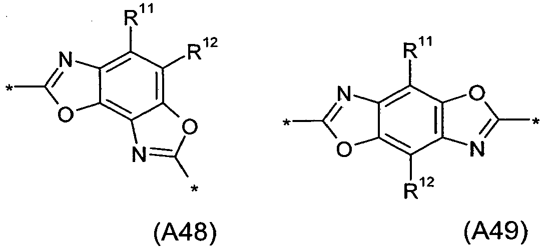 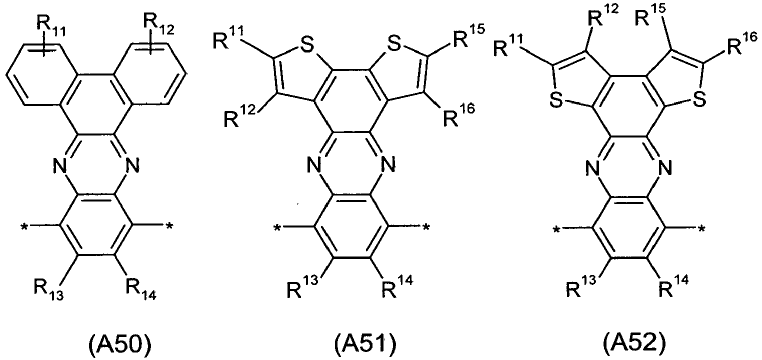 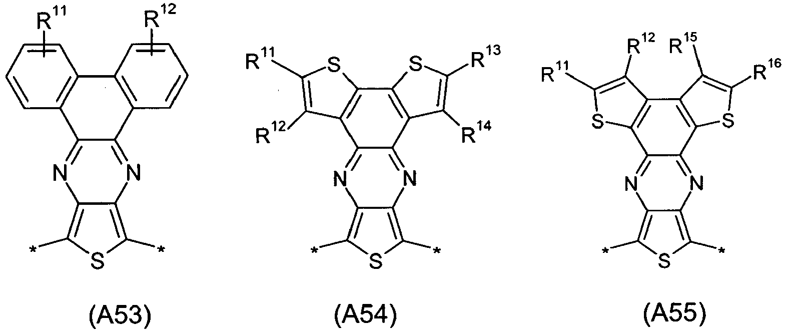 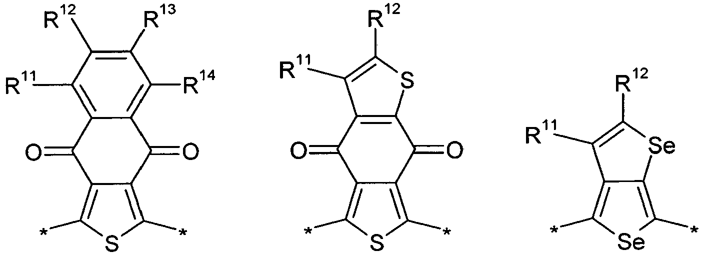 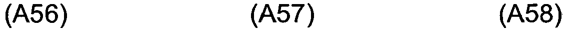 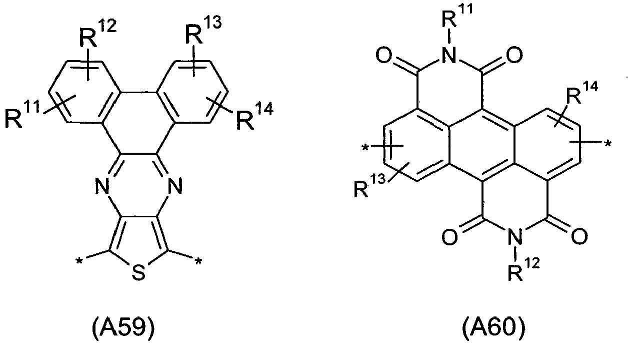 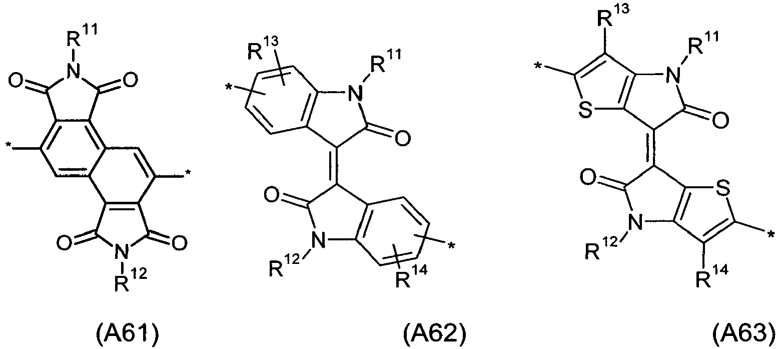 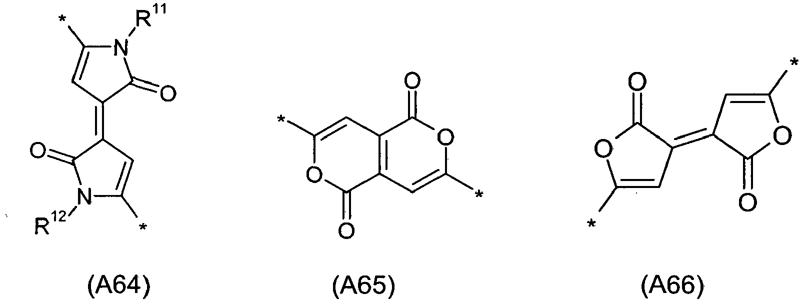 worin einer der Reste X¹¹ und X¹² S und der andere Se bedeutet, und R¹¹, R¹², R¹³, R¹⁴ und R¹⁵ jeweils unabhängig voneinander H oder R¹ wie in Formel I definiert bedeuten, und worin einer oder mehrere der Reste R¹¹,

R¹², R¹³, R¹⁴ und R¹⁵ eine Gruppe -Sp¹-X¹-C(O)-O-R¹ wie in Formel I definiert bedeutet.

Besonders bevorzugte Wiederholungseinheiten der Formel I sind ausgewählt aus folgenden Unterformeln: 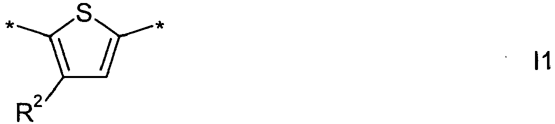 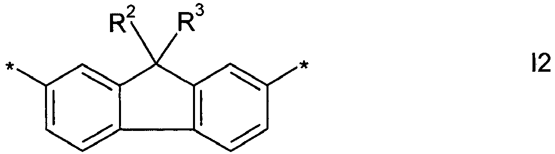 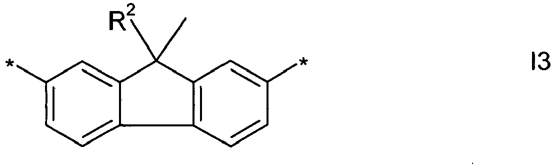 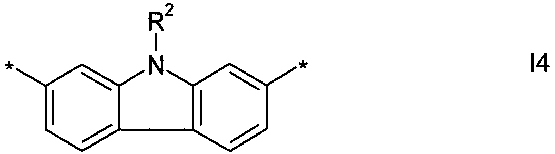 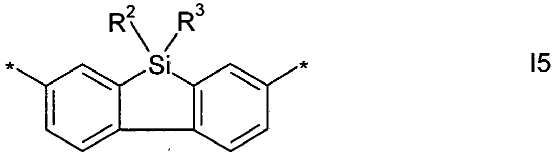 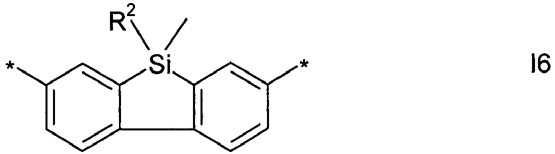 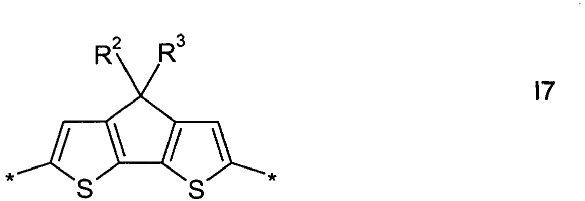 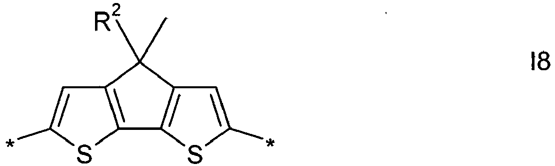 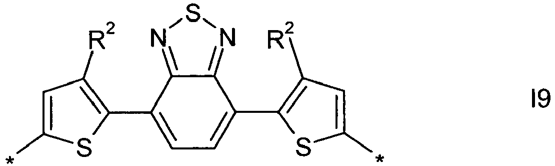 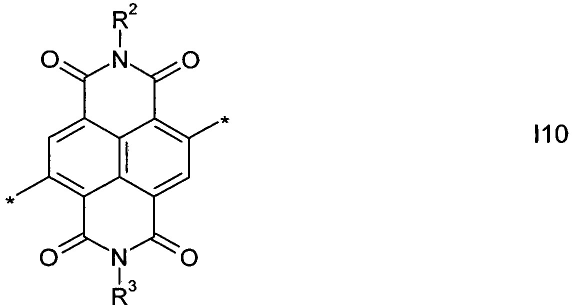 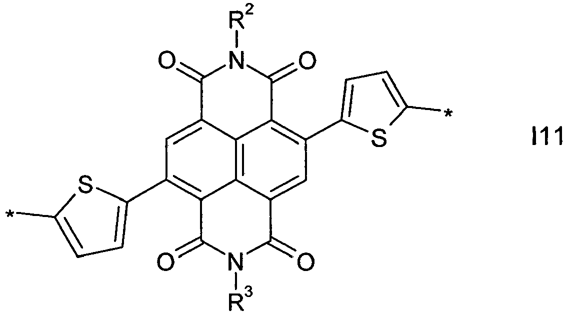 worin R² und R³ jeweils unabhängig voneinander, und bei jedem Auftreten gleich oder verschieden, einen Rest -Sp¹-X¹-C(O)-O-R¹ bedeutet, und Sp¹, X¹ und R¹ die vor- oder nachstehend angegebenen Bedeutungen besitzen.

Der Rest X¹ in Formel I und deren Unterformeln bedeutet vorzugsweise O oder NH, besonders bevorzugt O.

Der Rest Sp¹ in Formel I und deren Unterformeln bedeutet vorzugsweise Alkylen mit 1 bis 20, besonders bevorzugt mit 1 bis 8 C-Atomen, ganz besonders bevorzugt Methylen, Ethylen oder Propylen.

Der Rest R¹ in Formel I und deren Unterformeln bedeutet vorzugsweise geradkettiges, verzweigtes oder cyclisches Alkyl mit 1 bis 25 C-Atomen, welches unsubstituiert oder ein- oder mehrfach durch F, Cl, Br, I oder CN substituiert ist, und worin auch eine oder mehrere nicht benachbarte CH₂-Gruppen jeweils unabhängig voneinander so durch -O-, -S-, -C(O)-, -C(S)-, -C(O)-O-, -O-C(O)-, -NR⁰-, -SiR⁰R⁰⁰-, -CF₂-, -CHR⁰=CR⁰⁰-, -CY¹=CY²- oder -C≡C- ersetzt sein können, dass O- und/oder S-Atome nicht direkt miteinander verknüpft sind, wobei R⁰ und R⁰⁰ die in Formel I angegebene Bedeutung besitzen.

Besonders bevorzugt bedeutet R¹ Alkyl oder Alkenyl mit 1 bis 25, vorzugsweise 1 bis 20 C-Atomen, welches geradkettig, verzweigt und/oder cyclisch sein kann.

Bevorzugte Reste -Sp¹-X¹-C(O)-O-R¹ sind ausgewählt aus der Formel S: 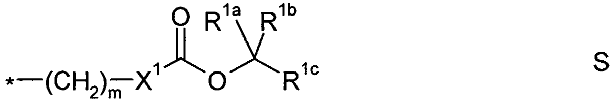 worin X¹ die vor- und nachstehend angegebene Bedeutung hat, R^{1b}, R^{1b} und R^{1c} jeweils unabhänging voneinander H, einen geradkettigen, verzweigten oder cyclischen Alkylrest mit 1 bis 25 C-Atomen oder einen geradkettigen, verzweigten oder cyclischen Alkenylrest oder Alkinylrest mit jeweils 2 bis 25 C-Atomen bedeuten, wobei auch zwei der Reste R^{1a}, R^{1b} und R^{1c} zusammen einen cyclischen Alkylrest, Alkenylrest oder Alkinylrest mit jeweils 5 bis 12 C-Atomen bilden können, m 0 oder eine ganze Zahl von 1 bis 12, vorzugsweise 2, 3, 4, 5 oder 6 bedeutet, und das Symbol * die Verknüpfung mit dem Rest Ar bedeutet. Vorzugsweise sind mindestens zwei, besonders bevorzugt drei, Reste R^{1a}, R^{1b} und R^{1c} von H verschieden.

Besonders bevorzugte Reste -Sp¹-X¹-C(O)-O-R¹ sind ausgewählt aus den folgenden Unterformeln: 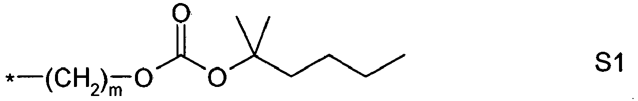 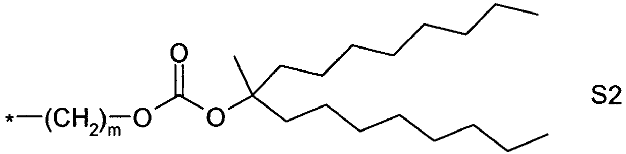 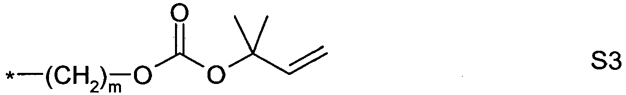 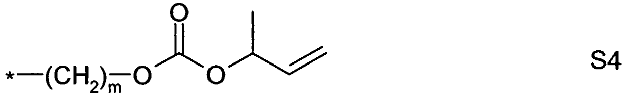 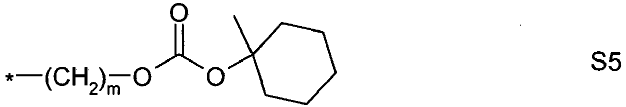 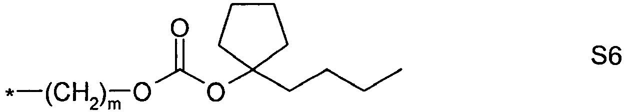 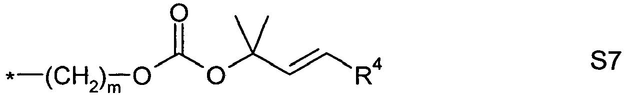 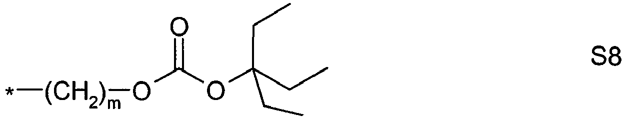 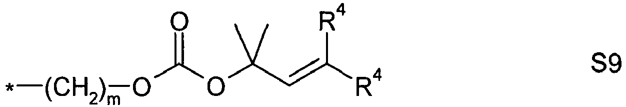 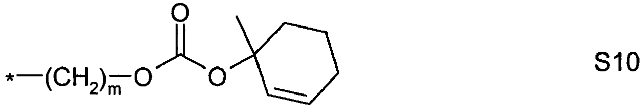 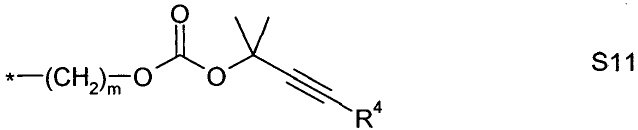 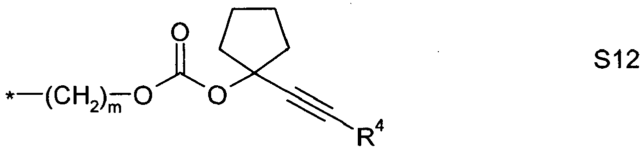 worin R⁴ bei jedem Auftreten gleich oder verschieden H oder einen geradkettigen oder verzweigten Alkylrest mit 1 bis 25 C-Atomen bedeutet, m eine ganze Zahl von 1 bis 12, vorzugsweise 2, 3, 4, 5 oder 6 bedeutet, und das Symbol * die Verknüpfung mit dem Rest Ar bedeutet. Vorzugsweise ist R⁴ verschieden von H.

Eine alternative Ausführungsform, die nicht unter die beanspruchte Erfindung fällt und in der Sp¹ eine Einfachbindung darstellt, richtet sich auf Wiederholungseinheiten der Formel I, worin der Rest Ar ein oder mehrere N-Atome enthält, und die Seitenkette Sp¹-X¹-C(O)-O-R¹ an eines dieser N-Atome im Rest Ar gebunden ist, wie z.B.

Wiederholungseinheiten der Formel I4, I10 oder I11. In einer dieser nicht erfindungsgemäßen Ausführungsform bedeutet in diesen Wiederholungseinheiten der Rest Sp¹-X¹ eine Einfachbindung, so dass das im Rest Ar enthaltene N-Atom, welches als Verknüpfungsstelle für die Seitenkette dient, zusammen mit dem Rest -C(O)-O-R¹ eine Carbamatgruppe bildet.

Wiederholungseinheiten dieser bevorzugten Ausführungsform sind solche ausgewählt aus folgenden Formeln: 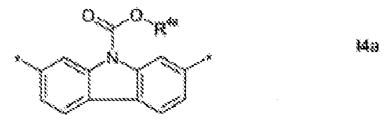 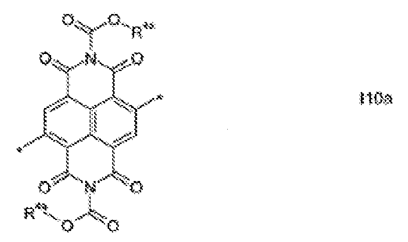 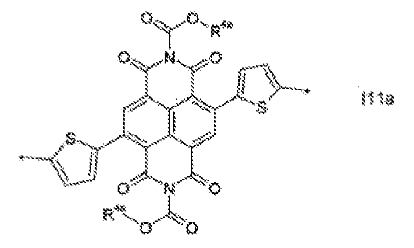 worin R^{4a} einen geradkettigen oder verzweigten Alkylrest mit 1 bis 25 C-Atomen bedeutet. Bevorzugte Reste R^{4a} sind 2-Methylhexyl und 9-Methylheptadecyl.

Bevorzugte Polymere gemäß der vorliegenden Erfindung enthalten eine oder mehrere Wiederholungseinheiten der Formel IIa oder IIb:

-[(Ar¹)ₐ-(U)_{b}-(Ar²)_{c}-(Ar³)_{d}]- IIa

-[(U)_{b}-(Ar¹)ₐ-(U)_{b}-(Ar²)_{c}-(Ar³)_{d}]- IIb

worin die einzelnen Reste folgende Bedeutung besitzen
- U: bei jedem Auftreten gleich oder verschieden eine Einheit der Formel I oder deren Unterformeln I1 bis I14,
- Ar¹, Ar², Ar³: bei jedem Auftreten gleich oder verschieden, und jeweils unabhängig voneinander, Aryl oder Heteroaryl welches von U verschieden ist, vorzugsweise 5 bis 30 Ringatome aufweist, und gegebenenfalls substitutiert ist, vorzugsweise mit einer oder mehreren Gruppen R^{S},
- R^{S}: bei jedem Auftreten gleich oder verschieden F, Br, Cl, -CN,-NC, -NCO, -NCS, -OCN, -SCN, -C(O)NR⁰R⁰⁰, -C(O)X⁰,-C(O)R⁰, -NH₂, -NR⁰R⁰⁰, -SH, -SR⁰, -SO₃H, -SO₂R⁰, -OH, -NO₂, -CF₃, -SF₅, gegebenenfalls substitutiertes Silyl oder Hydrocarbyl mit 1 bis 40 C-Atomen, welches gegebenenfalls substitutiert ist und gegebenenfalls ein oder mehrere Heteroatome enthält,
- R⁰ und R⁰⁰: wie in Formel I definiert,
- X⁰: Halogen, vorzugsweise F, Cl oder Br,
- a, b und c: bei jedem Auftreten gleich oder verschieden und jeweils unabhängig voneinander 0, 1 oder 2,
- d: bei jedem Auftreten gleich oder verschieden 0 oder eine ganze Zahl von 1 bis 10,

wobei das erfindungsgemäße Polymer mindestens eine Wiederholungseinheit der Formel IIa oder IIb enthält, worin b mindestens 1 ist.

R^{S} bedeutet vorzugsweise, bei jedem Auftreten gleich oder verschieden, H, geradkettiges, verzweigtes oder cyclisches Alkyl mit 1 bis 30 C-Atomen, worin auch eine oder mehrere nicht benachbarte CH₂-Gruppen jeweils unabhängig voneinander so durch -O-, -S-, -C(O)-, -C(S)-, -C(O)-O-, -O-C(O)-, -NR⁰-, -SiR⁰R⁰⁰-, -CF₂-, -CHR⁰=CR⁰⁰-, -CY¹=CY²- oder -C≡Cersetzt sein können, dass dass O- und/oder S-Atome nicht direkt miteinander verknüpft sind, und worin auch ein oder mehrere H-Atome durch F, Cl, Br, I oder CN ersetzt sein können, oder bedeutet Aryl, Heteroaryl, Aryloxy oder Heteroaryloxy mit 4 bis 20 Ringatomen, welches gegebenenfalls substituiert ist, vorzugsweise durch Halogen oder Alkyl oder Cycloalkyl wie vorstehend definiert.

Weitere bevorzugte Polymere gemäß der vorliegenden Erfindung enthalten, zusätzlich zu den Einheiten der Formel I, IIa oder IIb, eine oder mehrere Wiederholungseinheiten ausgewählt aus der Gruppe bestehend aus mono- oder polycyclischen Aryl- oder Heteroarylgruppen die gegebenenfalls substitutiert sind.

Diese zusätzlichen Wiederholungseinheiten sind vorzugsweise aus Formel IIIa oder IIIb ausgewählt

-[(Ar¹)ₐ-(Ar⁴)_{b}-(Ar²)_{c}-(Ar³)_{d}]- IIIa

-[(Ar⁴)_{b}-(Ar¹)ₐ-(Ar-⁴)_{b}-(Ar²)_{c}-(Ar³)_{d}]- IIIb

worin Ar¹, Ar², Ar³, a, b, c und d wie in Formel II definiert sind, und Ar⁴ eine Aryl- oder Heteroarylgruppe bedeutet, welche von U und Ar¹⁻³ verschieden ist, vorzugsweise 5 bis 30 Ringatome aufweist, und gegebenenfalls substitutiert ist, vorzugsweise mit einer oder mehreren Gruppen R^{S}, wobei das erfindungsgemäße Polymer mindestens eine Wiederholungseinheit der Formel IIIa oder IIIb enthält worin b mindestens 1 ist.

Besonderes bevorzugte Gruppen Ar¹, Ar², Ar³ und Ar⁴ sind ausgewählt aus der Gruppe bestehend aus den oben genannten Formeln D1 bis D110, worin einer der Reste X¹¹ und X¹² S und der andere Se bedeutet, und R¹¹, R¹², R¹³, R¹⁴, R¹⁵, R¹⁶, R¹⁷ and R¹⁸ jeweils unabhängig voneinander H oder R¹ wie in Formel I definiert bedeuten.

Weitere bevorzugte Gruppen Ar¹, Ar², Ar³ und Ar⁴ sind ausgewählt aus der Gruppe bestehend aus den oben genannten Formeln A1 bis A66, worin einer der Reste X¹¹ und X¹² S und der andere Se bedeutet, und R¹¹, R¹², R¹³, R¹⁴ und R¹⁵ jeweils unabhängig voneinander H oder R¹ wie in Formel I definiert bedeuten.

Falls Ar in Formel I, bzw. U in Formel IIa und IIb, aus der Gruppe bestehend aus den Formeln D1 bis D110 ausgewählt ist, bedeutet Ar⁴ in Formel IIIa und IIIb vorzugsweise eine Aryl- oder Heteroarylgruppe mit Elektronenakzeptoreigenschaften. Besonders bevorzugt ist Ar⁴ in diesem Fall aus der Gruppe bestehend aus den Formeln A1 bis A66 ausgewählt, worin einer der Reste X¹¹ und X¹² S und der andere Se bedeutet, und R¹¹, R¹², R¹³, R¹⁴ und R¹⁵ jeweils unabhängig voneinander H oder R¹ wie in Formel I definiert bedeuten.

Falls Ar in Formel I, bzw. U in Formel IIa und IIb, aus der Gruppe bestehend aus den Formeln A1 bis A66 ausgewählt ist, bedeutet Ar⁴ in Formel IIIa und IIIb vorzugsweise eine Aryl- oder Heteroarylgruppe mit Elektronendonoreigenschaften. Besonders bevorzugt ist Ar⁴ in diesem Fall aus der Gruppe bestehend aus den Formeln D1 bis D110 ausgewählt, worin einer der Reste X¹¹ und X¹² S und der andere Se bedeutet, und R¹¹, R¹², R¹³, R¹⁴, R¹⁵, R¹⁶, R¹⁷ and R¹⁸ jeweils unabhängig voneinander H oder R¹ wie in Formel I definiert bedeuten.

Die konjugierten Polymere der vorliegenden Erfindung sind vorzugsweise ausgewählt aus Formel IV: 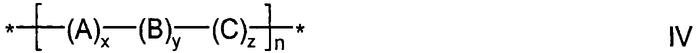 worin die einzelnen Reste folgende Bedeutung besitzen
- A, B, C: jeweils unabhängig voneinander eine verschiedene Einheit der Formel I, I1 bis I9, IIa, IIb, lila oder IIIb,
- x: > 0 und ≤ 1,
- y: ≥ 0 und < 1,
- z: ≥ 0 und < 1,
- x+y+z: 1, und
- n: eine ganze Zahl >1.

Bevorzugte Polymere der Formel IV sind ausgewählt aus folgenden Unterfomeln

*-[(Ar¹-U-Ar²)ₓ-(Ar³)_{y}]ₙ-* IVa

*-[(Ar¹-U-Ar²)ₓ-(Ar³-Ar³)_{y}]ₙ-* IVb

*-[(Ar¹-U-Ar²)ₓ-(Ar³-Ar³-Ar³)_{y}]ₙ-* IVc

*-[(Ar¹ₐ-(U)_{b}-Ar²)_{c}(Ar³)_{d}]ₙ-* IVd

*-([(Ar¹)ₐ-(U)_{b}-(Ar²)_{c}-(Ar³)_{d}]ₓ-[(Ar¹)ₐ-(Ar⁴)_{b}-(Ar²)_{c}-(Ar³)_{d}]_{y})ₙ-* IVe

*-[(U-Ar¹-U)ₓ-(Ar²-Ar³)_{y}]ₙ-* IVf

*-[(U-Ar¹-U)ₓ-(Ar²-Ar³-Ar²)_{y}]ₙ-* IVg

*-[(U)_{b}-(Ar¹)ₐ-(U)_{b}-(Ar²)_{c}]ₙ-* IVh

*-([(U)_{b}-(Ar¹)ₐ-(U)_{b}-(Ar²)_{c}]ₓ-[(A^{c})_{b}-(Ar¹)ₐ-(Ar⁴)_{b}-(Ar²)_{d}]_{y})ₙ-* IVi

*-[(U-Ar¹)ₓ-(U-Ar²)_{y}-(U-Ar³)_{z}]ₙ-* IVk

worin U, Ar¹, Ar², Ar³, a, b, c und d bei jedem Auftreten gleich oder verschieden eine der in Formel IIa angegebenen Bedeutungen besitzen, Ar⁴ bei jedem Auftreten gleich oder verschieden eine der in Formel lila angegebenen Bedeutungen besitzt, und x, y, zund n wie in Formel IV definiert sind, wobei diese Polymere alternierende oder statistische Copolymere sein können, und wobei in Formel IVd und IVe in mindestens einer Wiederholungseinheit [(Ar¹)ₐ-(U)_{b}-(Ar²)_{c}-(Ar³)_{d}] und in in mindestens einer Wiederholungseinheit [(Ar¹)ₐ-(Ar⁴)_{b}-(Ar²)_{c}-(Ar³)_{d}] b mindestens 1 ist, und in Formel IVh und IVi in mindestens einer Wiederholungseinheit [(U)_{b}-(Ar¹)ₐ-(U)_{b}-(Ar²)_{d}] und in mindestens einer Wiederholungseinheit [(U)_{b}-(Ar¹)ₐ-(U)_{b}-(Ar²)_{d}] b 1 ist.

In den Polymeren der Formeln IV und IVa bis IVk ist b vorzugsweise in allen Wiederholungseinheiten mindestens 1.

In den Polymeren der Formeln IV und IVa bis IVk ist x vorzugsweise 0.1 bis 0.9, besonders bevorzugt 0.3 bis 0.7.

In einer bevorzugten Ausführungsform der Erfindung sind y und z 0. In einer bevorzugten Ausführungsform der Erfindung sind y und z >0. In einer weiteren bevorzugten Ausführungsform der Erfindung ist einer der Indices y und z 0 und der andere >0. In den Polymeren der Formeln IV und IVa bis IVk worin y oder z >0 ist, ist dieser vorzugsweise 0.1 bis 0.9, besonders bevorzugt 0.3 bis 0.7.

In den Polymeren gemäß der vorliegenden Erfindung ist die Gesamtanzahl der Wiederholungseinheiten n vorzugsweise ≥ 5, sehr bevorzugt ≥ 10, insbesondere bevorzugt ≥ 50, und vorzugsweise bis zu 500, sehr bevorzugt bis zu 1.000, insbesondere bevorzugt bis zu 2.000, einschließlich beliebiger Kombinationen der vorgenannten unteren und oberen Grenzwerte für n.

Die Polymere der vorliegenden Erfindung umfassen Homopolymere und Copolymere, wie statistische Copolymere, alternierende Copolymere und Blockcopolymere, sowie deren Kombinationen.

Besonders bevorzugt sind Polymere ausgewählt aus den folgenden Gruppen:
- Gruppe A bestehend aus Homopolymeren der Wiederholungseinheit U oder (Ar¹-U) oder (Ar¹-U-Ar²) oder (Ar¹-U-Ar³) oder (U-Ar²-Ar³) oder (Ar¹-U-Ar²-Ar³), d.h. worin alle Wiederholungseinheiten identisch sind,
- Gruppe B bestehend aus statistischen oder alternierenden Copolymeren gebildet aus identischen Wiederholungseinheiten (Ar¹-U-Ar²) und identischen Wiederholungseinheiten (Ar³),
- Gruppe C bestehend aus statistischen oder alternierenden Copolymeren gebildet aus identischen Wiederholungseinheiten (Ar¹-U-Ar²) und identischen Wiederholungseinheiten (A¹),
- Gruppe D bestehend aus statistischen oder alternierenden Copolymeren gebildet aus identischen Wiederholungseinheiten Ar¹-U-Ar²) und identischen Wiederholungseinheiten (Ar¹-Ar⁴-Ar²),
worin in all diesen Gruppen U, D¹, Ar¹, Ar² und Ar³ wie vor- und nachstehend definiert sind, in den Gruppen A, B und C Ar¹, Ar² und Ar³ von einer Einfachbindung verschieden sind, und in Gruppe D einer der Reste Ar¹ und Ar² auch eine Einfachbindung bedeuten kann.

Besonders bevorzugte Polymere der Formel IV sind ausgewählt aus folgenden Unterformeln, wobei die Polymere der Formeln IV8 und IV9 nicht erfindungsgemäß sind. 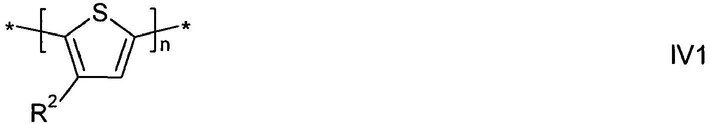 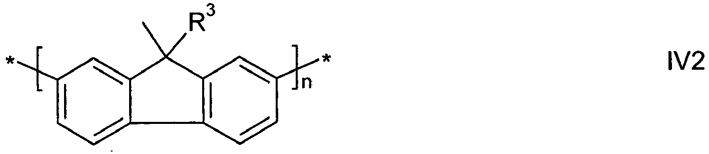 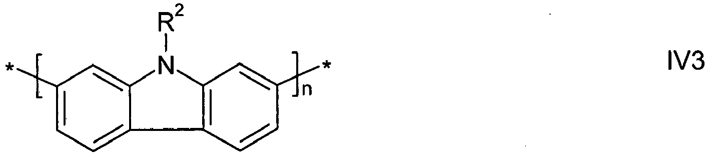 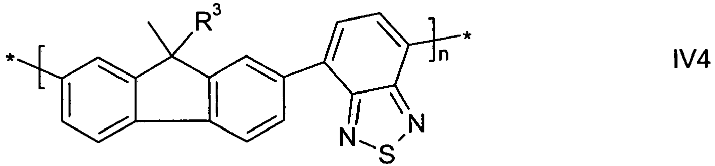 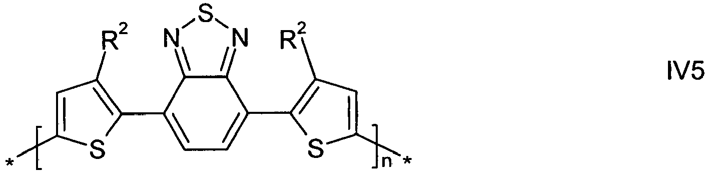 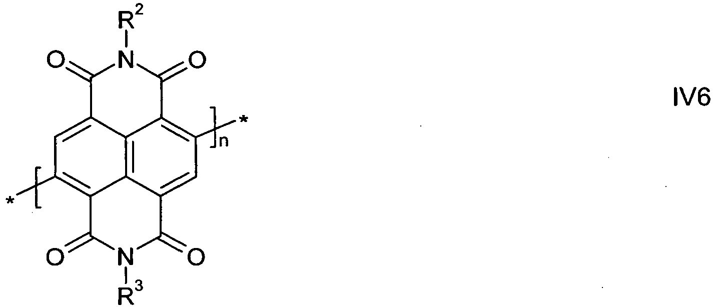 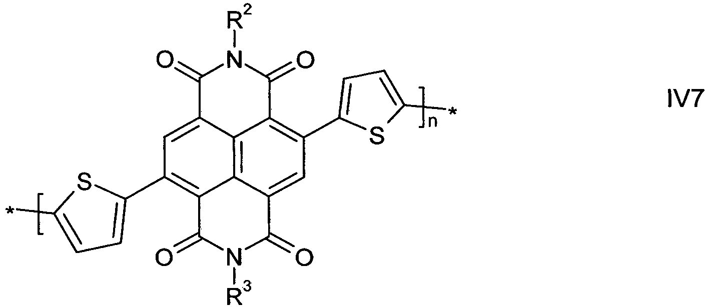 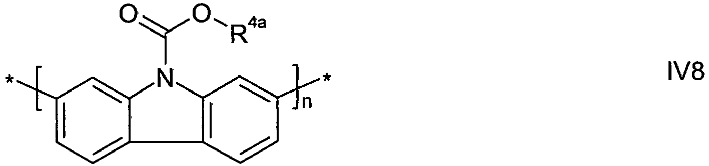 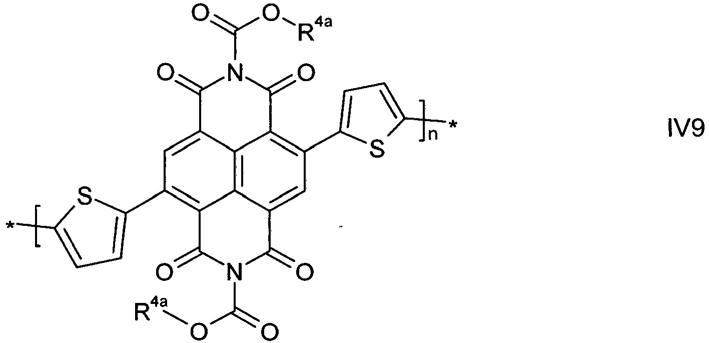 worin R², R³ und R^{4a} die in Formel I1-I14 angegebene Bedeutung besitzen und n die in Formel IV angegebene Bedeutung besitzt.

Bevorzugte Polymere der Formeln IV, IVa bis IVk und VI1 bis IV9 sind aus folgender Formel ausgewählt:

R⁵-Kette-R⁶ V

worin "Kette" eine aus den obigen Formeln IV, IVa bis IVk und IV1 bis IV9 ausgewählte Polymerkette bedeutet, und R⁵ und R⁶ jeweils unabhängig voneinander eine der für R^{S} in Formel IIa angegebenen Bedeutungen besitzen, oder H, F, Br, Cl, I, -CH₂Cl, -CHO, -CR'=CR"₂, -SiR'R"R"', - SiR'X'X", -SiR'R"X', -SnR'R"R"', -BR'R", -B(OR')(OR"), -B(OH)₂, -O-SO₂-R', -C≡CH, -C≡C-SiR'₃ oder -ZnX' bedeuten, worin X' und X" Halogen bedeuten, R', R" und R'" jeweils unabhängig voneinander eine der für R⁰ in Formel I angegebenen Bedeutungen besitzen, und zwei der Reste R', R" und R"' zusammen mit dem jeweiligen Heteroatom, an das sie gebunden sind, auch eine Cyclosilyl-, Cyclostannyl-, Cycloboran- oder Cycloboronatgruppe mit 2 bis 20 C-Atomen bilden können.

R⁵ und R⁶ bedeuten vorzusgweise H, C₁-C₂₀-Alkyl, C₁-C₂₀-Alkoxy, C₂-C₂₀-Alkenyl, C₁-C₂₀-Fluoralkyl, oder gegebenenfalls substitutiertes C₆₋₁₂ Aryl oder C₂₋₁₀ Heteroaryl, besonders bevorzugt H Phenyl, oder Triphenylamin welches auch ein oder mehrfach mit C1-C4-Alkylgruppen wie z.B. Methyl substituiert sein kann.

In den Polymeren der Formeln IV, IVa bis IVk, IV1 bis IV9 und V bedeuten x, y und z jeweils den molaren Anteil der Einheiten A, B und C, und n bedeutet den Polymerisationsgrad bzw. die Gesamtzahl aller Einheiten A, B und C. Diese Formeln beinhalten Blockcopolymere, statistische Copolymere und alternierende Copolymere von A, B und C, sowie Homopolymere von A für den Fall x>0 und y=z=0.

Ein weiterer Gegenstand der Erfindung sind Monomere der Formeln VIa und VIb:

R⁷-(Ar¹)ₐ-U-(Ar²)_{c}-R⁸ VIa

R⁷-U-(Ar¹)ₐ-U-R⁸ VIb

worin U, Ar¹, Ar², a und b die in Formel IIa angegeben Bedeutung besitzen und R⁷ und R⁸ jeweils unabhängig voneinander ausgewählt sind aus der Gruppe bestehend aus Cl, Br, I, O-Tosylat, O-Triflat, O-Mesylat, O-Nonaflat, -SiMe₂F, -SiMeF₂, -O-SO₂Z¹, -B(OZ²)₂ , -CZ³=C(Z³)₂, -C≡CH, - C≡CSi(Z¹)₃, -ZnX⁰ und -Sn(Z⁴)₃, worin X⁰ Halogen, vorzugsweise Cl, Br oder I bedeutet, Z¹⁻⁴ ausgewählt ist aus der Gruppe bestehend aus Alkyl und Aryl, welche gegebenenfalls substitutiert sein können, und zwei Reste Z² zusammen mit den B- und O-Atomen auch eine Cycloboronatgruppe mit 2 bis 20 C-Atomen bilden können, ausgenommen Monomere der Formel VIa, worin a und c 0 sind und U eine mit einer Carbamatgruppe substituierte Phenylen-2,5-diylgruppe bedeutet.

Besonders bevorzugt sind Monomere ausgewählt aus folgenden Formeln:

R⁷-Ar¹-U-Ar²-R⁸ VI1

R⁷-U-R⁸ VI2

R⁷-Ar¹-U-R⁸ VI3

R⁷-U-Ar²-R⁸ VI4

R⁷-U-Ar¹-U-R⁸ VI5

worin U, Ar¹, Ar², R⁷ und R⁸ wie in Formel VIa definiert sind.

Besonders bevorzugt sind Wiederholungseinheiten, Polymer und Monomere der Formeln I, IIa, IIb, IIIa, IIIb, IVa, IVb, V, VIa und VIb sowie deren Unterformeln gemäß den folgenden bevorzugten Ausführungsformen sowie deren Kombinationen:
- y ist > 0 und < 1 und z ist 0,
- y ist > 0 und < 1 und z ist > 0 und < 1,
- n ist mindestens 5, vorzugsweise mindestens 10, besonders bevorzugt mindestens 50, und bis zu 2,000, vorzugsweise bis zu 500.
- M_{w} ist mindestens 5,000, vorzugsweise mindestens 8,000, besonders bevorzugt mindestens 10,000, und vorzugsweise bis zu 300,000, besonders bevorzugt bis zu 100,000,
- M_{w} beträgt mindestens 5.000, vorzugsweise mindestens 8.000, besonders bevorzugt mindestens 10.000, und bis zu 300.000, vorzugsweise bis zu 100.000,
- X¹ bedeutet O oder NH, besonders bevorzugt O,
- Sp¹ bedeutet Alkylen mit 1 bis 20, besonders bevorzugt mit 1 bis 8 C-Atomen, ganz besonders bevorzugt Methylen, Ethylen, Propylen oder Hexylen,
- Ar¹ und Ar² sind, jeweils unabhängig voneinander, und bei jedem Auftreten gleich oder verschieden, ausgewählt aus 1,4-Phenylen, Thiophen-2,5-diyl, Thiazol-2,5-diyl, Selenophen-2,5-diyl, Furan-2,5-diyl, Thieno[3,2-*b*]thiophen-2,5-diyl, Thieno[2,3-*b*]thiophen-2,5-diyl, Selenopheno[3,2-*b*]selenophen-2,5-diyl, Selenopheno[2,3-*b*]selenophen-2,5-diyl, Selenopheno[3,2-*b*]thiophen-2,5-diyl oder Selenopheno[2,3-*b*]thiophen-2,5-diyl, wobei alle diese Reste unsubstituiert oder ein- oder mehrfach, vorzugsweise mit R^{S} wie vor- und nachstehend definiert, substituiert sein können,
- Ar³ und Ar⁴ sind, jeweils unabhängig voneinander, und bei jedem Auftreten gleich oder verschieden, ausgewählt aus 1,4-Phenylen, Thiophen-2,5-diyl, Selenophen-2,5-diyl, Thieno[3,2-*b*]thiophen-2,5-diyl, Thieno[2,3-*b*]thiophen-2,5-diyl, Selenopheno[3,2-*b*]selenophen-2,5-diyl, Selenopheno[2,3-*b*]selenophen-2,5-diyl, Selenopheno[3,2-*b*]thiophen-2,5-diyl, Selenopheno[2,3-*b*]thiophen-2,5-diyl, Benzo[1,2-*b*:4,5-*b*']dithiophen-2,6-diyl, 2,2-Dithiophen, 2,2-Diselenophen, Dithieno[3,2-*b*:2',3'-*d*]silol-5,5-diyl, 4*H*-Cyclopenta[2,1-b:3,4-*b*']dithiophen-2,6-diyl, Carbazol-2,7-diyl, Fluoren-2,7-diyl, Indaceno[1,2-*b*:5,6-*b*']dithiophen-2,7-diyl,Benzo[1",2":4,5;4",5":4',5']bis(silolo[3,2-*b*:3',2'-*b*']thiophen)-2,7-diyl, Phenanthro[1,10,9,8-*c,d,e,f,g*]carbazol-2,7-diyl, Benzo[2,1,3]thiadiazoI-4,7-diyl, Benzo[2,1,3]selenadiazol-4,7-diyl, Benzo[2,1,3]oxadiazol-4,7-diyl, 2H-Benzotriazol-4,7-diyl, 3,4-Difluorthiophen-2,5-diyl, Thieno[3,4-*b*]pyrazin-2,5-diyl, Chinoxalin-5,8-diyl, Thieno[3,4-*b*]thiophen-4,6-diyl, Thieno[3,4-*b*]thiophen-6,4-diyl, 3,6-Di-thien-2-yl-pyrrolo[3,4-c]pyrrol-1,4-dion oder [1,3]Thiazolo[5,4-d][1,3]thiazol-2,5-diyl, wobei alle diese Reste unsubstituiert oder ein- oder mehrfach, vorzugsweise mit R^{S} wie vor- und nachstehend definiert, substituiert sein können,
- Ar ist, bei jedem Auftreten gleich oder verschieden, ausgewählt aus 1,4-Phenylen, Thiophen-2,5-diyl, Selenophen-2,5-diyl, Thieno[3,2-*b*]thiophen-2,5-diyl, Thieno[2,3-*b*]thiophen-2,5-diyl, Selenopheno[3,2-*b*]selenophen-2,5-diyl, Selenopheno[2,3-b]selenophen-2,5-diyl, Selenopheno[3,2-*b*]thiophen-2,5-diyl, Selenopheno[2,3-*b*]thiophen-2,5-diyl, Benzo[1,2-b:4,5-*b*']dithiophen-2,6-diyl, 2,2-Dithiophen, 2,2-Diselenophen, Dithieno[3,2-b:2',3'-*d*]silol-5,5-diyl, 4*H*-Cyclopenta[2,1-*b*:3,4-*b*']dithiophen-2,6-diyl, Carbazol-2,7-diyl, Fluoren-2,7-diyl, Indaceno[1,2-6:5,6-*b*']dithiophen-2,7-diyl, Benzo[1",2":4,5;4"5":4',5']-bis(silolo[3,2-*b*:3',2'-*b*']thiophen)-2,7-diyl, Phenanthro[1,10,9,8-*c,d,e,f,g*]-carbazol-2,7-diyl, Benzo[2,1,3]thiadiazol-4,7-diyl, Benzo[2,1,3]selena-diazol-4,7-diyl, Benzo[2,1,3]oxadiazol-4,7-diyl, 2H-Benzotriazol-4,7-diyl, 3,4-Difluorthiophen-2,5-diyl, Thieno[3,4-*b*]pyrazin-2,5-diyl, Chinoxalin-5,8-diyl, Thieno[3,4-*b*]thiophen-4,6-diyl, Thieno[3,4-*b*]thiophen-6,4-diyl, [1,3]Thiazolo[5,4-d][1,3]thiazol-2,5-diyl oder Benzo[lmn][3,8]phenanthrolin-1,3,6,8-tetraon-4,9-diyl, wobei alle diese Reste unsubstituiert oder ein- oder mehrfach, vorzugsweise mit R^{S} wie vor- und nachstehend definiert, substituiert sein können, und alle Reste mindestens einfach mit Sp¹-X¹-C(O)-O-R¹ wie vor- und nachstehend definiert substituiert sind,
- das Polymer enthält keine gegebenenfalls substituierten Benzo[*lmn*][3,8]phenathrolin-1,3,6,8-tetraon-4,9-diyl-Einheiten, die zu einer mit einer Carbamat- oder Carbonatgruppe substituierten Phenylen-1,4-diyl-Einheit direkt benachbart sind,
- Ar und Ar¹⁻⁴ sind verschieden von gegebenenfalls substituiertem Pyrrolo[3,4-c]pyrrol-1,4-dion-3,6-diyl,
- Ar und Ar¹⁻⁴ sind verschieden von gegebenenfalls substituiertem Phenylen-1,4-diyl,
- R¹ ist von H verschieden,
- R¹ bedeutet primäres Alkyl mit 1 bis 20 C-Atomen, sekundäres Alkyl mit 3 bis 30 C-Atomen oder tertiäres Alkyl mit 4 bis 30 C-Atomen, wobei in allen diesen Gruppen gegebenenfalls ein oder mehrere H-Atome durch F ersetzt sind,
- R¹ bedeutet geradkettiges oder verzweigtes Alkenyl mit 2 bis 20 C-Atomen, worin gegebenenfalls ein oder mehrere H-Atome durch F ersetzt sind,
- R¹ bedeutet cyclisches Alkyl 3 bis 20 C-Atomen, worin gegebenenfalls ein oder mehrere H-Atome durch F ersetzt sind,
- R¹ bedeutet einen Rest enthaltend geradkettige und cyclische Alkylgruppen mit insgesamt 5 bis 20 C-Atomen, worin gegebenenfalls ein oder mehrere H-Atome durch F ersetzt sind,
- R⁰ und R⁰⁰ sind aus H oder C₁-C₁₀-Alkyl ausgewählt,
- R^{S} ist, bei jedem Auftreten gleich oder verschieden, ausgewählt aus geradkettigem, verzweigtem und/oder cyclischem Alkyl, Alkoxy oder Sulfanylalkyl mit 1 bis 30 C-Atomen, worin gegebenenfalls ein oder mehrere H-Atome durch F ersetzt sind,
- R^{S} ist, bei jedem Auftreten gleich oder verschieden, ausgewählt aus Aryl, Aryloxy, Heteroaryl und Heteroaryloxy, welche gegebenenfalls mit F, Alkyl oder Alkoxy substituiert ist und 4 bis 30 Ringatome aufweist,
- R^{S} ist, bei jedem Auftreten gleich oder verschieden, ausgewählt aus geradkettigem, verzweigtem und/oder cyclischem Alkylcarbonyl, Alkoxycarbonyl und Alkylcarbonyloxy, worin gegebenenfalls ein oder mehrere H-Atome durch F ersetzt sind,
- R^{S} bedeutet, bei jedem Auftreten gleich oder verschieden, F, Cl, Br, I, CN, R⁹, -C(O)-R⁹, -C(O)-O-R⁹, -O-C(O)-R⁹, -SO₂-R⁹ oder -SO₃-R⁹, worin R⁹ geradkettiges, verzweigtes und/oder cyclisches Alkyl mit 1 bis 20 C-Atomen bedeutet, worin auch eine oder mehrere nicht benachbarte CH₂-Gruppen jeweils unabhängig voneinander so durch - O-, -S-, -C(O)-, -C(S)-, -C(O)-O-, -O-C(O)-, -NR⁰-, -SiR⁰R⁰⁰-, -CF₂-, - CHR⁰=CR⁰⁰-, -CY¹=CY²- oder -C≡C- ersetzt sein können, dass dass O- und/oder S-Atome nicht direkt miteinander verknüpft sind, und worin auch ein oder mehrere H-Atome durch F, Cl, Br, I oder CN ersetzt sein können, oder bedeutet Aryl, Heteroaryl, Aryloxy oder Heteroaryloxy mit 4 bis 20 Ringatomen, welches gegebenenfalls substituiert ist, vorzugsweise durch Halogen oder Alkyl oder Cycloalkyl wie vorstehend definiert,
- R⁰ und R⁰⁰ bedeuten jeweils unabhängig voneinander H oder C₁-C₁₀-Alkyl,
- R⁵ und R⁶ sind, jeweils unabhängig voneinander, und bei jedem Auftreten gleich oder verschieden, ausgewählt aus H, Halogen, -CH₂Cl, -CHO, -CH=CH₂ -SiR'R"R"', -SnR'R"R"', -BR'R", -B(OR')(OR"), -B(OH)₂, C₁-C₂₀-Alkyl, C₁-C₂₀-Alkoxy, C₂-C₂₀-Alkenyl, C₁-C₂₀-Fluoralkyl und gegebenenfalls substituertem Aryl oder Heteroaryl mit 4 bis 10 Ringatomen, vorzugsweise Phenyl,
- R⁷ und R⁸ sind, jeweils unabhängig voneinander, und bei jedem Auftreten gleich oder verschieden, ausgewählt aus Cl, Br, I, O-Tosylat, O-Triflat, O-Mesylat, O-Nonaflat, -SiMe₂F, -SiMeF₂, -O-SO₂Z¹, - B(OZ²)₂,-CZ³=C(Z⁴)₂, -C≡CH, C=CSi(Z¹)₃, -ZnX⁰ und -Sn(Z⁴)₃, worin X⁰ Halogen, vorzugsweise Cl, Br oder I bedeutet, Z¹⁻⁴ ausgewählt ist aus der Gruppe bestehend aus Alkyl und Aryl, welche gegebenenfalls substitutiert sein können, und zwei Reste Z² zusammen mit den B- und O-Atomen auch eine Cycloboronatgruppe mit 2 bis 20 C-Atomen bilden können.

Die Polymere und Monomere gemäß der vorliegenden Erfindung können nach oder in Analogie zu Verfahren synthetisiert werden, die dem Fachmann bekannt und in der Literatur beschrieben sind. Andere Herstellungsverfahren lassen sich den Beispielen entnehmen.

Beispielsweise lassen sich erfindungsgemäße Polymere in geeigneter Weise durch Aryl-Aryl-Kupplungsreaktionen, wie Yamamoto-Kupplung, Suzuki-Kupplung, Stille-Kupplung, Kumada-Kupplung, Negishi-Kupplung, Sonogashira-Kupplung, Heck-Kupplung, Buchwald-Kupplung oder Synthese nach Yokozawa, herstellen. Suzuki-Kupplung, Yamamoto-Kupplung und Stille-Kupplung sind besonders bevorzugt.

Die Monomere, die zur Bildung der Wiederholungseinheiten der Polymere polymerisiert werden, können nach dem Fachmann bekannten Verfahren hergestellt werden.

Die Polymere werden vorzugsweise aus Monomeren der Formel VIa oder VIb oder ihren bevorzugten Unterformeln wie vor- und nachstehend beschrieben hergestellt.

Ein weiterer Aspekt der Erfindung ist ein Verfahren zur Herstellung eines erfindungsgemäßen Polymers durch Kuppeln einer oder mehrerer gleicher oder verschiedener Monomereinheiten der Formel I oder eines oder mehrerer gleicher oder verschiedener Monomere der Formel VIa oder VIb miteinander und/oder mit einem oder mehreren Comonomeren in einer Polymerisierungsreaktion, vorzugsweise in einer Aryl-Aryl-Kupplungsreaktion.

Geeignete und bevorzugte Co-Monomere sind ausgewäht aus folgenden Formeln:

R⁷-(Ar¹)ₐ-Ar⁴-(Ar²)_{c}-R⁸ VIII

R⁷-Ar¹-R⁸ IX

R⁷-Ar³-R⁸ X

worin Ar¹, Ar², Ar³, Ar⁴, a und c die in Formel IIa und lila angegebene Bedeutung besitzen, und R⁷ und R⁸ die in Formel VIa angegebene Bedeutung besitzen.

Besonders bevorzugt ist ein Verfahren zur Herstellung eines Polymers durch Reaktion eines oder mehrerer gleicher oder verschiedener Monomere der Formel Via oder VIb mit einem oder mehreren Monomeren der Formel VIII, und gegebenenfalls mit einem oder mehreren Monomern ausgewählt aus den Formeln IX und X, in einer Aryl-Aryl-Kupplungsreaktion, worin vorzugsweise R⁷ und R⁸ ausgewählt sind aus Cl, Br, I, -B(OZ²)₂ und -Sn(Z⁴)₃.

Besonders bevorzugte Verfahren sind ausgewählt aus folgenden Ausführungsformen
a) ein Verfahren zur Herstellung eines Polymers durch Aryl-Aryl-Kupplungsreaktion eines Monomers der Formel VI1

   R⁷-Ar¹-U-Ar²-R⁸ VI1

   mit einem Monomer der Formel IX

   R⁷-Ar¹-R⁸ IX
b) ein Verfahren zur Herstellung eines Polymers durch Aryl-Aryl-Kupplungsreaktion eines Monomers der Formel VI2

   R⁷-U-R⁸ VI2

   mit einem Monomer der Formel VIII1

   R⁷-Ar¹-Ar⁴-Ar²-R⁸ VIII1
c) ein Verfahren zur Herstellung eines Polymers durch Aryl-Aryl-Kupplungsreaktion eines Monomers der Formel VI2

   R⁷-U-R⁸ VI2

   mit einem Monomer der Formel VIII2

   R⁷-Ar⁴-R⁸ VIII2
d) ein Verfahren zur Herstellung eines Polymers durch Aryl-Aryl-Kupplungsreaktion eines Monomers der Formel VI2

   R⁷-U-R⁸ VI2

   mit einem Monomer der Formel VIII2

   R⁷-Ar⁴-R⁸ VIII2

   und einem Monomer der Formel IX

   R⁷-Ar¹-R⁸ IX
e) ein Verfahren zur Herstellung eines Polymers durch Aryl-Aryl-Kupplungsreaktion eines Monomers der Formel VI1

   R⁷-U-Ar¹-U-R⁸ VI5

   mit einem Monomer der Formel IX

   R⁷-Ar¹-R⁸ IX
f) ein Verfahren zur Herstellung eines Polymers durch Aryl-Aryl-Kupplungsreaktion eines Monomers der Formel VI2

   R⁷-U-R⁸ VI2

   Mit einem Monomer der Formel IX

   R⁷-Ar¹-R⁸ IX

   und einem Monomer der Formel X

   R⁷-Ar³-R⁸ X

   worin R⁷, R⁸, U, Ar^{1,2,3,4} wie in Formel IIa, IIIa und VIa definiert sind, und R⁷ und R⁸ vorzugsweise aus Cl, Br, I, -B(OZ²)₂ und -Sn(Z⁴)₃ wie in Formel VIa definiert ausgewählt sind.

Bevorzugte Aryl-Aryl-Kupplungsverfahren und Polymerisationsverfahren sind Yamamoto-Kupplung, Kumada-Kupplung, Negishi-Kupplung, Suzuki-Kupplung, Stille-Kupplung, Sonogashira-Kupplung, Heck-Kupplung, C-H-Aktivierings-Kupplung, Ullmann-Kupplung unde Buchwald-Kupplung, und Synthese nach Yokozawa. Besonders bevorzugt sind die Suzuki-, Negishi-, Stille- und Yamamoto-Kupplung und die Synthese nach Yokozawa. Letztgenannte ist z.B. in Yokozawa (R. Miyakoshi, A. Yokoyama, T. Yokozawa, J. Am. Chem. Soc. 2005, 127, 17542-17547.) beschrieben, Die Suzuki-Kupplung ist z.B. in der WO 00/53656 A1 oder M. Ranger, D. Rondeau, M. Leclerc, Macromolecules 1997, 30, 7686-7691 beschrieben. Die Negishi-Kupplung ist z.B. in J. Chem. Soc., Chem. Commun., 1977, 683-684 beschrieben. Die Yamamoto-Kupplung ist z.B. in T. Yamamoto et al., Prog. Polym. Sci., 1993, 17, 1153-1205, WO 2004/022626 A1 oder N. Kobayashi, R. Koguchi, M. Kijima, Macromolecules 2006, 39, 9102-9111 beschrieben, und die Stille-Kupplung ist z.B. in Z. Bao et al., J. Am. Chem. Soc., 1995, 117, 12426-12435 beschrieben.

Beispielsweise werden für die Yamamoto-Kupplung vorzugsweise Monomere wie oben beschrieben mit zwei reaktiven Halogenidgruppen verwendet. Für die Suzuki-Kupplung werden vorzugsweise Monomere wie oben beschrieben mit zwei reaktiven Boronsäure- oder Boronsäreestergruppen sowie Monomere mit zwei reaktiven Halogenidgruppen, oder Monomere mit einer reaktiven Boronsäure- oder Boronsäureestergruppe und einer reaktiven Halogenidgruppe verwendet. Für die Stille-Kupplung werden vorzugsweise Monomere wie oben beschrieben mit zwei reaktiven Stannylgruppen sowie Monomere mit zwei reaktiven Halogenidgruppen, oder Monomere mit einer reaktiven Stannlygruppe und einer reaktiven Halogenidgruppe verwendet. Für die Negishi-Kupplung werden vorzugsweise Monomere wie oben beschrieben mit zwei reaktiven Organozinkgruppen sowie Monomere mit zwei reaktiven Halogenidgruppen, oder Monomere mit einer reaktiven Organozinkgruppe und einer reaktiven Halogenidgruppe verwendet.

Bevorzugte Katalysatoren, insbesondere für die Suzuki-, Negishi- oder Stille-Kupplung, sind ausgewählt aus Pd(0)-Komplexen und Pd(II)-Salzen. Bevorzugte Pd(0)-Komplexe sind solche, die mindestens einen Phosphinliganden wie Pd(PPh₃)₄ tragen. Ein weiterer bevorzugter Phosphinligand ist Tris(*ortho*-tolyl)phosphin, d.h. Pd(o-Tol)₄. Zu den bevorzugten Pd(II)-Salzen zählt Palladiumacetat, d.h. Pd(OAc)₂. Der Pd(0)-Komplex kann auch z.B. hergestellt werden durch Mischen eines Pd(0)-Dibenzylidenaceton-Komplexes, wie z.B. Tris(dibenzylidenaceton)dipalladium(0), Bis(dibenzylidenaceton)palladium(0), oder eines Pd(II)-Salzes wie z.B. Palladiumacetat, mit einem Phosphinligand, wie z.B. Triphenylphosphin, Tris(*ortho*-tolyl)phosphin oder Tri(tert-butyl)phosphin. Die Suzuki-Polymerisation wird in Gegenwart einer Base, z.B. Natriumcarbonat, Kaliumphosphat, Lithiumhydroxid, oder Kaliumphosphat, oder einer organischen Base, wie Tetraethylammoniumcarbonat oder Tetraethylammoniumhydroxid, durchgeführt. Bei der Yamamoto-Polymerisation wird ein Ni(0)-Komplex, z.B. Bis(1,5-cyclooctadienyl)nickel(0) eingesetzt.

Die Suzuki-Polymerisation kann verwendet werden, um sowohl Homopolymere als auch statistische und alternierende Copolymere und zufällig verteilte Blockcopolymere herzustellen. Statistische oder Blockcopolymere lassen sich beispielsweise aus den obigen Monomeren herstellen, in der eine der reaktiven Gruppen Halogen bedeutet und die andere reaktive Gruppe eine Boronsäure- oder Boronsäurederivat-Gruppe bedeutet. Die Synthese der statistischen, alternierenden und Blockcopolymere ist beispielsweise in der WO 03/048225 A2 oder WO 2005/014688 A2 ausführlicher beschrieben.

Als Alternativen zu Halogenen wie oben beschrieben können Abgangsgruppen der Formel -O-SO₂Z¹ verwendet werden, in der Z¹ wie oben beschrieben ist. Spezifische Beispiele derartiger Abgangsgruppen sind Tosylat, Mesylat und Triflat.

Monomere der Formel VIa und VIb können z.B. gemäß Schema 1 oder in Analogie dazu hergestellt werden

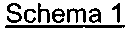

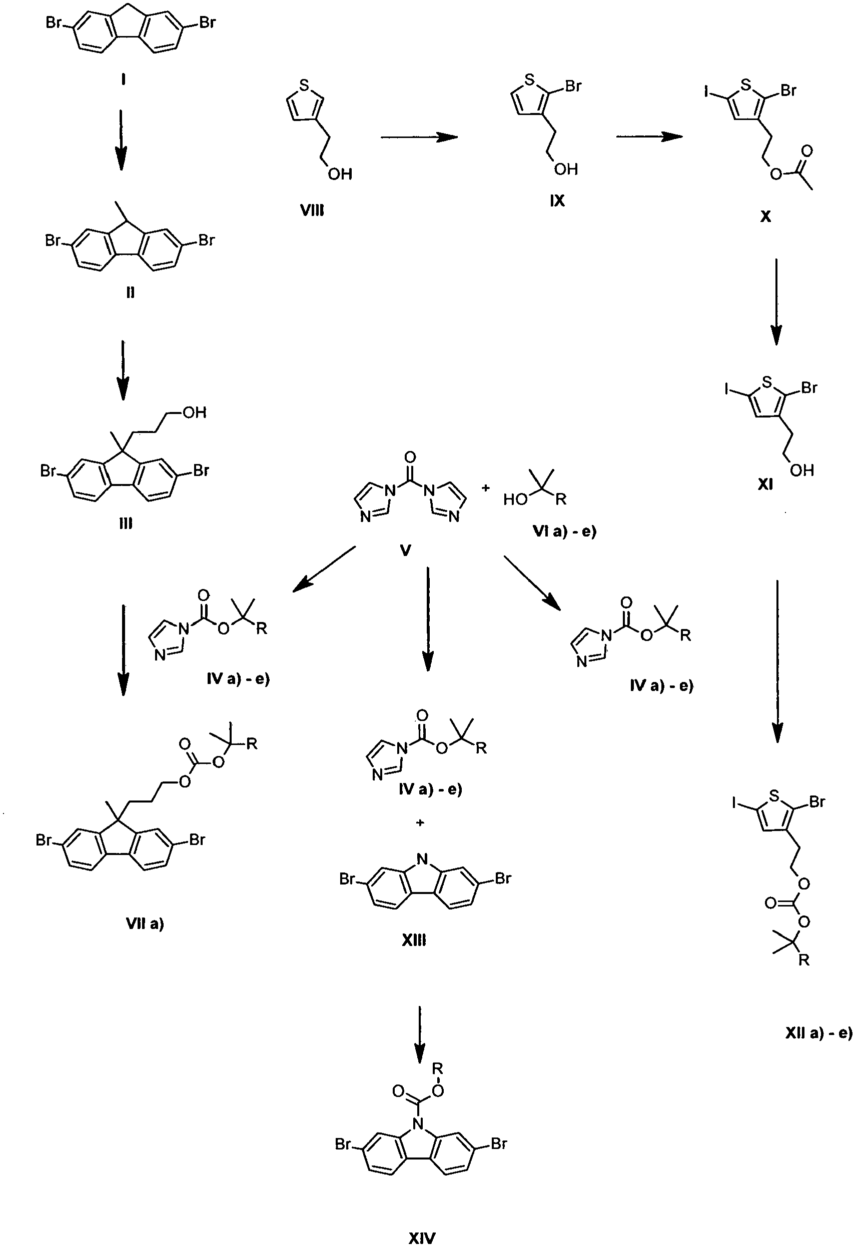

Die Monomere der Formel VIa und VIb basierend auf dem Benzo[lmn][3,8]phenanthrolin-1,3,6,8-tetraon-4,9-diyl-Bausteinen IV6 und IV7 können z.B: gemäß Schema 2 oder in Analogie dazu hergestellt werden.

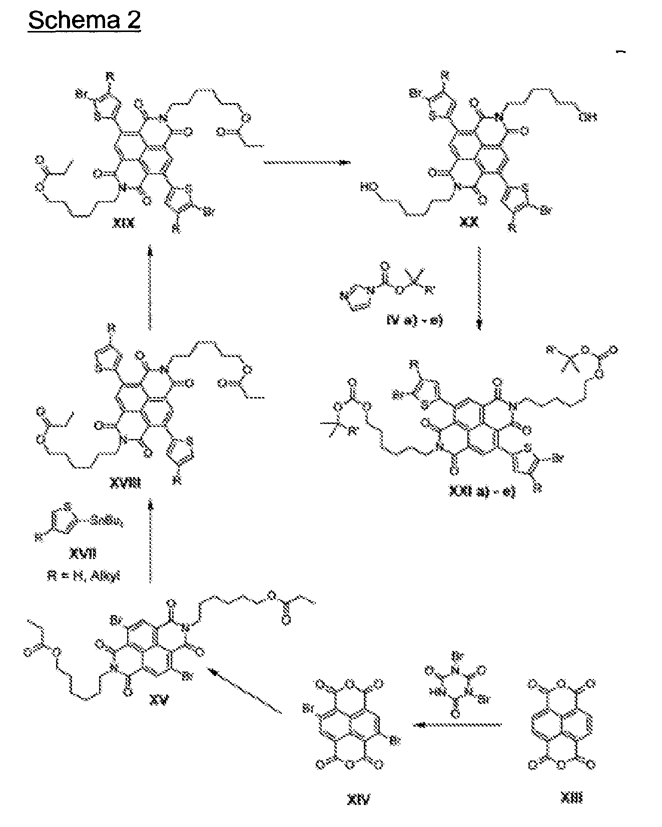

Die nicht erfindungsgemäßen Monomere der Formel IV9 und IV10 basierend auf dem Benzo[lmn][3,8]phenanthrolin-1,3,6,8-tetraon-4,9-diyl-Bausteinen I10a und I11a können z.B: gemäß Schema 3 oder in Analogie dazu hergestellt werden.

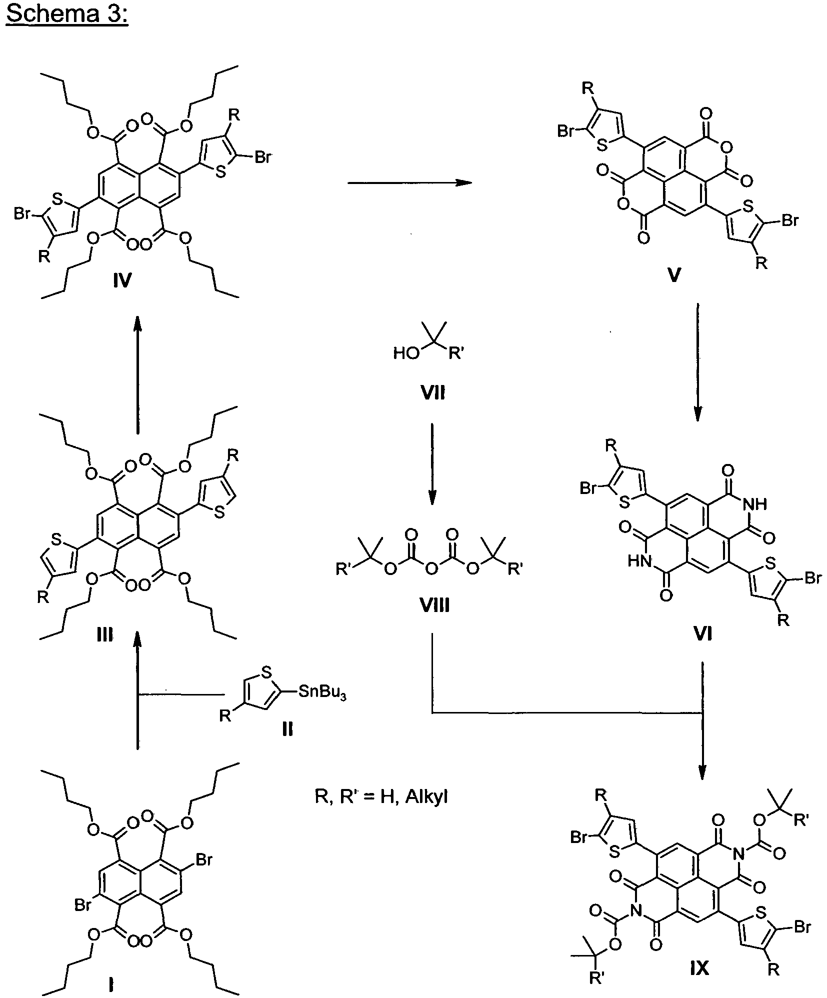

Die neuen Verfahren zur Herstellung von Monomeren und Polymeren wie vor- und nachstehend beschrieben sind ein weiterer Gegenstand der Erfindung.

Die Abspaltung der Carbonat- oder Carbamatseitenketten aus einem erfindungsgemäßen Polymer kann einfach durch Erhitzen erfolgen, z.B. nachdem das Polymer, oder ein das Polymer enthaltende Material, als Halbleiterschicht auf ein Substrat oder ein elektronisches Bauteil aufgebracht worden ist.

Die Temperatur der Abspaltung kann anhand Thermogravimetrischer Analyse (TGA) und Dynamischer Differenzkalometrie (*engl.* Differential Scanning Calorimetry DSC) bestimmt werden. Bei den aus der Literatur bekannten Seitenketten liegt die Temperatur typischerweise bei 210 °C bzw. 310 °C, um zum komplett defunktionalisiertem Polymerrückgrat zu gelangen. Bei den erfindungsgemäße thermisch abspaltbaren Gruppen liegt diese Temperatur dagegen bei <200 °C und kann noch weiter gesenkt werden, z.B. durch Ersatz der Alkylreste durch z.B. Alkenylreste. Die in dieser Erfindung beschriebenen halbleitenden Polymere besitzen nach der Abspaltung üblicherweise eine Alkoholgruppe, eine Amin- oder eine Imidfunktion. Diese können Wasserstoffbrücken bilden, welche die Löslichkeit weiter senken.

Als Beispiel ist in Schema 4 die thermische Abspaltungsreaktion für ein erfindungsgemäßes Polymer der Formel IV2 gezeigt.

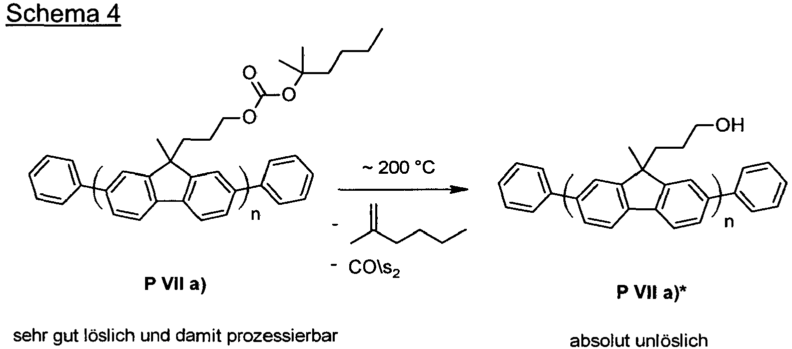

Vorzugsweise wird die Abspaltungsreaktion durch Erhitzen des erfindungsgemäßen Polymers auf eine Temperatur von <200°C durchgeführt.

Ein weiterer Gegenstand der Erfindung ist ein Verfahren zur teilweisen oder vollständigen Abspaltung der Carbonat- oder Carbamatgruppen eines erfindungsgemäßen Polymers, durch Erhitzen des Polymers oder einer Schicht, z.B. einer Halbleiterschicht, enthaltend das erfindungsgemäße Polymer, auf eine Temperatur von ≤200°C, vorzugsweise von 150°C bis 200°C. Weiterer Gegenstand der Erfindung ist ein Polymer oder eine Halbleiterschicht erhältlich durch dieses Verfahren.

Die Polymere gemäß der vorliegenden Erfindung können auch in Mischungen oder Polymerblends verwendet werden, zum Beispiel zusammen mit monomeren Verbindungen oder zusammen mit anderen Polymeren mit Ladungstransport-, Halbleiter-, elektrisch leitenden, photoleitenden und/oder lichtemittierenden Halbleitereigenschaften oder zum Beispiel mit Polymeren mit lochblockierenden oder elektronenblockierenden Eigenschaften für die Verwendung als Zwischenschichten oder Ladungsblockierungsschichten in OLED-Vorrichtungen. Ein weiterer Aspekt der Erfindung betrifft daher einen Polymerblend, der ein oder mehrere Polymere gemäß der vorliegenden Erfindung und ein oder mehrere weitere Polymere mit einer oder mehreren der oben genannten Eigenschaften enthält. Diese Blends können nach herkömmlichen Verfahren hergestellt werden, die im Stand der Technik beschrieben und dem Fachmann bekannt sind. Typischerweise werden die Polymere miteinander vermischt oder in geeigneten Lösungsmitteln gelöst und die Lösungen zusammengegeben.

Ein weiterer Aspekt der Erfindung betrifft eine Formulierung, die ein oder mehrere Polymere, Mischungen oder Polymerblends wie vor- und nachstehend beschrieben und ein oder mehrere organische Lösungsmittel enthält.

Bevorzugte Lösungsmittel sind aliphatische Kohlenwasserstoffe, chlorierte Kohlenwasserstoffe, aromatische Kohlenwasserstoffe, Ketone, Ether und Gemische von diesen. Zu weiteren Lösungsmitteln, die verwendet werden können, zählen 1,2,4-Trimethylbenzol, 1,2,3,4-Tetramethylbenzol, Pentylbenzol, Mesitylen, Cumol, Cymol, Cyclohexylbenzol, Diethylbenzol, Tetralin, Decalin, 2,6-Lutidin, 2-Fluor-m-xylol, 3-Fluor-o-xylol, 2-Chlorbenzotrifluorid, Dimethylformamid, 2-Chlor-6-fluortoluol, 2-Fluoranisol, Anisol, 2,3-Dimethylpyrazin, 4-Fluoranisol, 3-Fluoranisol, 3-Trifluor-methylanisol, 2-Methylanisol, Phenetol, 4-Methylanisol, 3-Methylanisol, 4-Fluor-3-methylanisol, 2-Fluorbenzonitril, 4-Fluorveratrol, 2,6-Dimethylanisol, 3-Fluorbenzonitril, 2,5-Dimethylanisol, 2,4-Dimethylanisol, Benzonitril, 3,5-Dimethylanisol, N,N-Dimethylanilin, Benzoesäureethylester, 1-Fluor-3,5-dimethoxybenzol, 1-Methylnaphthalin, N-Methylpyrrolidinon, 3-Fluorbenzotrifluorid, Benzotrifluorid, Benzotrifluorid, 1,4-Dioxan, Trifluormethoxybenzol, 4-Fluorbenzotrifluorid, 3-Fluorpyridin, Toluol, 2-Fluortoluol, 2-Fluorbenzotrifluorid, 3-Fluortoluol, 4-Isopropylbiphenyl, Phenylether, Pyridin, 4-Fluortoluol, 2,5-Difluortoluol, 1-Chlor-2,4-difluorbenzol, 2-Fluorpyridin, 3-Chlorfluorbenzol, 3-Chlorfluorbenzol, 1-Chlor-2,5-difluorbenzol, 4-Chlorfluorbenzol, Chlorbenzol, o-Dichlorbenzol, 2-Chlorfluorbenzol, p-Xylol, m-Xylol, o-Xylol oder Gemisch aus o-, m- und p-Isomeren. Lösungsmittel mit relativ geringer Polarität werden im Allgemeinen bevorzugt. Für den Tintenstrahldruck werden Lösungsmittel mit hohen Siedetemperaturen und Lösungsmittelgemische bevorzugt. Für das Spincoating werden alkylierte Benzole wie Xylol und Toluol bevorzugt.

Zu den Beispielen besonders bevorzugter Lösungsmittel gehören, ohne hierauf beschränkt zu sein, Dichlormethan, Trichlormethan, Monochlorbenzol, o-Dichlorbenzol, Tetrahydrofuran, Anisol, Morpholin, Toluol, o-Xylol, m-Xylol, p-Xylol, 1,4-Dioxan, Aceton, Methylethylketon, 1,2-Dichlorethan, 1,1,1-Trichlorethan, 1,1,2,2-Tetrachlorethan, Essigsäureethylester, n-Butylacetat, Dimethylformamid, Dimethylacetamid, Dimethylsulfoxid, Tetralin, Decalin, Indan, Benzoesäuremethylester, Benzoesäureethylester, Mesitylen sowie Gemische der vorstehend genannten Lösungsmittel.

Die Konzentration der Polymere in der Lösung beträgt vorzugsweise 0,1 bis 10 Gew.-%, besonders bevorzugt 0,5 bis 5 Gew.-%. Gegebenenfalls enthält die Lösung auch ein oder mehrere Bindemittel zur Einstellung der rheologischen Eigenschaften, wie z.B. in der WO 2005/055248 A1 beschrieben.

Nach entsprechendem Mischen und Altern werden die Lösungen in eine der folgenden Kategorien eingestuft: vollständige Lösung, Grenzlösung oder unlöslich. Die Konturlinie wird gezeichnet, um die Löslichkeitsparameter-Wasserstoffbindungsgrenzen anzugeben, die Löslichkeit und Unlöslichkeit trennen. "Vollständige" Lösungsmittel, die in den Löslichkeitsbereich fallen, können aus Literaturwerten ausgewählt werden, wie sie in "Crowley, J.D., Teague, G.S. Jr und Lowe, J.W. Jr., Journal of Paint Technology, 38, Nr 496, 296 (1966)" veröffentlicht sind. Lösungsmittelgemische können ebenfalls verwendet werden und können wie in "Solvents, W.H. Ellis, Federation of Societies for Coatings Technology, S. 9-10, 1986" beschrieben identifiziert werden. Eine derartige Vorgehensweise kann zu einem Gemisch von "Nicht"-Lösungsmitteln führen, welches beide Polymere der vorliegenden Erfindung löst, auch wenn es wünschenswert ist, wenn mindestens ein echtes Lösungsmittel in einem Gemisch vorhanden ist.

Die Polymere gemäß der vorliegenden Erfindung können auch in strukturierten Halbleiterschichten in den Vorrichtungen wie vor- und nachstehend beschrieben verwendet werden. Für Anwendungen in der modernen Mikroelektronik ist es im Allgemeinen wünschenswert, kleine Strukturen oder Muster zu erzeugen, um die Kosten (mehr Vorrichtungen/Flächeneinheit) und den Energieverbrauch zu reduzieren. Die Strukturierung dünner Schichten, die ein Polymer gemäß der vorliegenden Erfindung enthalten, kann beispielsweise durch Photolithographie, Elektronenstrahllithographie oder Laserstrukturierung durchgeführt werden.

Zur Verwendung als dünne Schichten in elektronischen oder elektrooptischen Vorrichtungen können die Polymere, Polymerblends oder Formulierungen der vorliegenden Erfindung mit jedem geeigneten Verfahren abgeschieden werden. Flüssigbeschichtung von Vorrichtungen ist wünschenswerter als Vakuumbedampfungstechniken. Abscheidungsverfahren aus der Lösung sind besonders bevorzugt. Die Formulierungen der vorliegenden Erfindung ermöglichen die Verwendung einer Reihe von Flüssigbeschichtungstechniken. Zu den bevorzugten Abscheidungstechniken zählen, ohne hierauf beschränkt zu sein, Dipcoating, Spincoating, Tintenstrahldruck, Hochdruck, Siebdruck, Rakelbeschichtung, Walzendruck, Umkehrwalzendruck, Offset-Lithographiedruck, Flexodruck, Rollendruck, Spraycoating, Brushcoating oder Tampondruck. Der Tintenstrahldruck ist besonders bevorzugt, da er die Herstellung hochauflösender Schichten und Vorrichtungen ermöglicht.

Ausgewählte Formulierungen der vorliegenden Erfindung können durch Tintenstrahldruck oder Mikrodosierung auf vorgefertigte Substrate für Vorrichtungen aufgebracht werden. Vorzugsweise können zum Aufbringen der organischen Halbleiterschicht auf ein Substrat großtechnische piezoelektrische Druckköpfe wie die von Aprion, Hitachi-Koki, InkJet Technology, On Target Technology, Picojet, Spectra, Trident, Xaar vertriebenen verwendet werden, ohne hierauf beschränkt zu sein. Auch können halbtechnische Köpfe wie die von Brother, Epson, Konica, Seiko Instruments, Toshiba TEC hergestellten oder Einzeldüsen-Mikrodosierer wie die von Microdrop und Microfab hergestellten verwendet werden.

Zum Aufbringen durch Tintenstrahldruck oder Mikrodosierung sollten die Polymere zunächst in einem geeigneten Lösungsmittel gelöst werden. Lösungsmittel müssen die oben angegebenen Anforderungen erfüllen und dürfen keinen nachteiligen Einfluss auf den gewählten Druckkopf haben. Des weiteren sollten Lösungsmittel Siedepunkte >100°C, vorzugsweise >140°C und besonders bevorzugt >150°C besitzen, um durch Eintrocknen der Lösung im Druckkopf verursachte Betriebsprobleme zu verhindern. Neben den oben genannten Lösungsmitteln zählen substituierte und nicht substituierte Xylolderivate, Di-C₁₋₂-alkylformamid, substituierte und nicht substituierte Anisole und andere Phenoletherderivate, substituierte Heterocyclen wie substituierte Pyridine, Pyrazine, Pyrimidine, Pyrrolidinone, substituierte und nicht substituierte *N,N*-Di-C₁₋₂-alkylaniline und andere fluorierte oder chlorierte Aromaten zu den geeigneten Lösungsmitteln.

Ein bevorzugtes Lösungsmittel zum Abscheiden eines Polymers gemäß der vorliegenden Erfindung durch Tintenstrahldruck enthält ein Benzolderivat, bei dem ein Benzolring durch einen oder mehrere Substituenten substituiert ist, wobei die Gesamtzahl der Kohlenstoffatome bei dem einen oder den mehreren Substituenten mindestens drei beträgt. Beispielsweise kann das Benzolderivat mit einer Propylgruppe oder drei Methylgruppen substituiert sein, wobei in beiden Fällen insgesamt mindestens drei Kohlenstoffatome vorhanden sind. Ein derartiges Lösungsmittel gestattet die Bildung einer das Lösungsmittel mit dem Polymer enthaltenden Tintenstrahlflüssigkeit, was ein Verstopfen der Düsen und Separieren der Komponenten während des Sprühens reduziert oder verhindert. Zu dem/den Lösungsmittel(n) können die aus der folgenden Liste von Beispielen ausgewählten zählen: Dodecylbenzol, 1-Methyl-4-tert.-butylbenzol, Terpineol, Limonen, Isodurol, Terpinolen, Cymol, Diethylbenzol. Bei dem Lösungsmittel kann es sich um ein Lösungsmittelgemisch, das heißt eine Kombination von zwei oder mehr Lösungsmitteln, handeln, wobei jedes Lösungsmittel vorzugsweise einen Siedepunkt >100°C, besonders bevorzugt >140°C besitzt. Derartige Lösungsmittel verbessern auch die Filmbildung in der abgeschiedenen Schicht und verringern Defekte in der Schicht.

Die Tintenstrahlflüssigkeit (das heißt die Mischung aus Lösungsmittel, Bindemittel und Halbleiterverbindung) weist vorzugsweise bei 20°C eine Viskosität von 1-100 mPa·s, besonders bevorzugt 1-50 mPa·s und insbesondere bevorzugt 1-30 mPa·s auf.

Die Polymere oder Formulierungen gemäß der vorliegenden Erfindung können zusätzlich eine oder mehrere weitere Komponenten oder Zusätze enthalten, die für z.B. aus oberflächenaktiven Verbindungen, Schmier-, Netz-, Dispergier-, Hydrophobier-, Haftmitteln, Fließverbesserern, Entschäumern, Entgasungsmitteln, Verdünnungsmitteln, die reaktiv oder nicht reaktiv sein können, Hilfsstoffen, Farbmitteln, Farbstoffen oder Pigmenten, Sensibilisierern, Stabilisatoren, Nanopartikeln oder Inhibitoren ausgewählt sind.

Die Polymere gemäß der vorliegenden Erfindung sind als Ladungstransport-, Halbleiter-, elektrisch leitende, photoleitende oder lichtemittierende Materialien in optischen, elektrooptischen, elektronischen, Elektrolumineszenz- oder Photolumineszenz-Komponenten oder -Vorrichtungen verwendbar. Bei diesen Vorrichtungen werden die Polymere der vorliegenden Erfindung typischerweise als dünne Schichten oder Filme aufgebracht.

Ein weiterer Gegenstand der vorliegenden Erfindung ist daher die Verwendung eines erfindungsgemäßen halbleitenden Polymers, Polymerblends, oder einer erfindungsgemäßen Formulierung oder Schicht in einer elektronischen Vorrichtung. Die Formulierung kann in verschiedenen Vorrichtungen und Geräten als Halbleitermaterial mit hoher Beweglichkeit verwendet werden. Die Formulierung kann beispielsweise in Form einer Halbleiterschicht oder eines Halbleiterfilms verwendet werden. Dementsprechend stellt die vorliegende Erfindung in einem weiteren Aspekt eine Halbleiterschicht für die Verwendung in einer elektronischen Vorrichtung bereit, wobei die Schicht ein Polymer, ein Polymerblend oder eine Formulierung gemäß der Erfindung enthält. Die Schicht oder der Film kann weniger als etwa 30 Mikron sein. Für die Anwendung in verschiedenen elektronischen Vorrichtungen kann die Dicke weniger als etwa 1 Mikron dick sein. Die Schicht kann mittels einer beliebigen der vorstehend genannten Lösungsbeschichtungs- oder Drucktechniken abgeschieden werden, zum Beispiel auf einem Teil einer elektronischen Vorrichtung.

Ein weiterer Gegenstand der vorliegenden Erfindung ist eine elektronische Vorrichtung, die ein Polymer, ein Polymerblend, eine Formulierung oder eine organische Halbleiterschicht gemäß der vorliegenden Erfindung enthält. Besonders bevorzugte Vorrichtungen sind OFETs, TFTs, ICs, logische Schaltungen, Kondensatoren, RFID-Tags, OLEDs, OLETs, OPEDs, OPVs, OPDs, Solarzellen, Laserdioden, Photoleiter, Photodetektoren, elektrophotographische Vorrichtungen, elektrophotographische Aufzeichnungsvorrichtungen, organische Speichervorrichtungen, Sensorvorrichtungen, Ladungsinjektionsschichten, Schottky-Dioden, Planarisierungsschichten, Antistatikfolien, leitende Substrate und leitende Muster.

Besonders bevorzugte elektronische Vorrichtung sind OFETs, OLEDs, OPV- und OPD-Vorrichtungen, insbesondere OPV-Vorrichtungen mit Volumen-Heteroübergang (englisch "bulk heterojunction" oder "BHJ"). In einem OFET kann zum Beispiel der aktive Halbleiterkanal zwischen Drain und Source die erfindungsgemäße Schicht enthalten. Als weiteres Beispiel kann in einer OLED-Vorrichtung die Ladungs(Loch- oder Elektronen-)injektions- oder -transportschicht die erfindungsgemäße Schicht enthalten.

Für die Verwendung in OPV- oder OPD-Vorrichtungen wird das erfindungsgemäße Polymer vorzugsweise in einer Formulierung verwendet, die einen Halbleiter des p-Typs (Elektronendonor) und einen Halbleiter des n-Typs (Elektronenakzeptor) umfasst oder enthält, besonders bevorzugt im Wesentlichen hieraus besteht und sehr bevorzugt ausschließlich hieraus besteht. Der Halbleiter des p-Typs ist vorzugsweise ein erfindungsgemäßes Polymer. Der Halbleiter des n-Typs ist z.B. ein anorganisches Material wie Zinkoxid (ZnOₓ), Zink-Zinn-Oxid (ZTO), Titanoxid (TiOₓ), Molybdänoxid (MoOₓ), Nickeloxid (NiOₓ), oder Cadmiumselenid (CdSe), oder ein organisches Material wie Graphen, ein Fulleren oder ein substituertes Fulleren, z.B. ein Inden-C₆₀-Fulleren-Bisaddukt wie ICBA, oder (6,6)-Phenyl-buttersäuremethylesterderivatisiertes Methano-C₆₀-Fulleren, auch als "PCBM" oder "C₆₀PCBM" bekannt, wie beispielsweise in G. Yu, J. Gao, J.C. Hummelen, F. Wudl, A.J. Heeger, Science 1995, Bd. 270, S. 1789 ff offenbart und mit der unten gezeigten Struktur, oder eine strukturanaloge Verbindung mit z.B. einer C₇₀-Fullerengruppe (C₇₀PCBM) oder ein Polymer (siehe z.B. Coakley, K. M. und McGehee, M. D. *Chem. Mater.* 2004, 16, 4533) handeln. Erfindungsgemäße Polymere, die eine Akzeptorgruppe, wie z.B. eine Wiederholungseinheit der Formel I10 oder I11 enthalten, können auch als n-Typ Halbleiter eingesetzt werden.

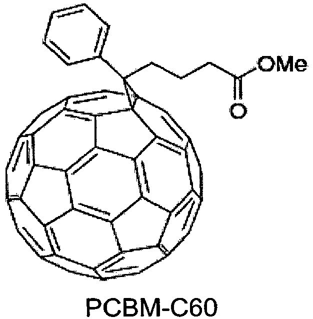

Um eine photoaktive Schicht in einer erfindungsgemäßen OPV- oder OPD-Vorrichtung zu bilden, wird ein erfindungsgemäßes Polymer vorzugsweise gemischt mit einem Fulleren oder einem substituierten Fulleren wie PCBM-C₆₀, PCBM-C₇₀, bis-PCBM-C₆₀, bis-PCBM-C₇₀, ICBA (1',1",4',4"- tetrahydro-di[1,4]methano-naphthaleno

[1,2:2',3';56,60:2",3"][5,6]fullerene-C60-lh), oder mit Graphen oder einem Metalloxid wie z.B. ZnOₓ, TiOₓ, ZTO, MoOₓ, NiOₓ.

Eine erfindungsgemäße OPV- oder OPD-Vorrichtung umfasst vorzugsweise ausser der photoaktiven Schicht eine erste transparente oder semi-transparente Elektrode auf einem transparenten oder semitransparenten Substrat auf einer Seite der photoaktiven Schicht, sowie eine zweite metallische oder semi-transparente Elektrode auf der anderen Seite der photoaktiven Schicht.

In einer weiteren bevorzugten Ausführungsform umfasst eine erfindungsgemäße OPV- oder OPD-Vorrichtung, zwischen der photoaktiven Schicht und der ersten und/oder zweiten Elektrode, eine oder mehrere Zwischenschichten oder Pufferschichten, welche als Lochtransport- und/oder elektronenblockierende Schicht fungieren können und ein Material enthalten wie beispielsweise ein Metalloxid, z.B. ZTO, MoOₓ, NiOₓ, einen konjugierten Polymerelektrolyten, z.B. PEDOT:PSS, ein konjugiertes Polymer, z.B. Polytriarylamin (PTAA), oder eine organische Verbindung, z.B. N,N'-Diphenyl-N,N'-bis(1-naphthyl)(1,1'-biphenyl)-4,4'diamin (NPB), N,N'-Diphenyl-N,N-(3-methylphenyl)-1,1'-biphenyl-4,4'-diamin (TPD), oder eine oder mehrere Zwischenschichten oder Pufferschichten, welche als lochblockierende und/oder Elektrontransportschicht fungieren können und ein Material enthalten wie beispielsweise ein Metalloxid,z.B., ZnOₓ, TiOₓ, ein Salz, z.B. LiF, NaF, CsF, einen konjugierten Polymerelektrolyten, z.B. Poly[3-(6-trimethylammoniumhexyl)thiophen], Poly(9,9-bis(2-ethylhexyl)-fluoren]-*b*-poly[3-(6-trimethylammoniumhexyl)thiophen], oder Poly [(9,9-bis(3'-(N,N-dimethylamino)propyl)-2,7-fluoren)-alt-2,7-(9,9-dioctylfluoren)], oder eine organische Verbindung, z.B. tris(8-Chinolinolato)-Aluminium(III) (Alq₃), oder 4,7-Diphenyl-1,10-phenanthrolin.

In einer Mischung eines erfindungsgemäßen Polymers oder Polymerblends mt einem Fulleren oder substituiertem Fulleren beträgt das Verhältnis Polymer:Fulleren vorzugsweise von 5:1 bis 1:5 Gewichtsanteilen, besonders bevorzugt von 1:1 bis 1:3 Gewichtsanteilen, ganz besonders bevorzugt von 1:1 bis 1:2 Gewichtsanteilen. Ein polymeres Bindemittel kann auch zugefügt werden, vorzugsweise in einer Konzentration von 5 bis 95 Gew.-%. Beispiele für geeignete und bevorzugte Bindemittel umfassen Polystyrol (PS), Polypropylen (PP) und Polymethylmethacrylat (PMMA).

Zur Herstellung dünner Schichten in BHJ-OPV-Vorrichtungen können die Polymere, Polymerblends oder Formulierungen der vorliegenden Erfindung mit jedem geeigneten Verfahren abgeschieden werden. Flüssigbeschichtung von Vorrichtungen ist wünschenswerter als Vakuumbedampfungstechniken. Abscheidungsverfahren aus der Lösung sind besonders bevorzugt. Die Formulierungen der vorliegenden Erfindung ermöglichen die Verwendung einer Reihe von Flüssigbeschichtungstechniken. Zu den bevorzugten Abscheidungstechniken zählen, ohne hierauf beschränkt zu sein, Dipcoating, Spincoating, Tintenstrahldruck, Hochdruck, Siebdruck, Rakelbeschichtung, Walzendruck, Umkehrwalzendruck, Offset-Lithographiedruck, Flexodruck, Rollendruck, Slot-Dye-Coating, Spraycoating, Brushcoating oder Tampondruck. Der Tintenstrahldruck ist besonders bevorzugt, da er die Herstellung hochauflösender Schichten und Vorrichtungen ermöglicht.

Für die Herstellung von OPV-Vorrichtungen und OPV-Modulen besonders bevorzugt sind Flächendruckverfahren die mit flexiblen Substraten kompatibel sind, wie z.B. Slot-Dye-Coating oder Spraycoating .

Bei der Auswahl geeigneter Lösungmittel zur Herstellung von BHJ-Schichten aus einem erfindungsgemäßen Polymer und einem Fulleren sollte auf eine komplette Lösung beider Komponenten geachtet werden, sowie auf die Grenzflächenbedingungen (z.B.. die rheologischen Eigenschaften) die aus dem gewählten Druck- oder Beschichtungsverfahren resultieren.

Zu diesem Zweck werden organische Lösungsmittel verwendet.Typische Lösungsmittel sind beispielsweise aromatische und/oder chlorierte Lösungsmittel. Beispiele für geeignete und bevorzugte Lösungsmittel beinhalten, ohne darauf beschränkt z sein, Chlorobenzol, 1,2-Dichlorbenzol, Chloroform, 1,2-Dichlorethan, Dichlormethan, Kohlenstofftetrachlorid, Toluol, Cyclohexanon, Ethylacetat, Tetrahydrofuran, Anisol, Morpholin, o-Xylol, m-Xylol, p-Xylol, 1,4-Dioxan, Aceton, Methylethylketon, 1,2-Dichlorethan, 1,1,1-Trichlorethan, 1,1,2,2-Tetrachlorethan, n-Butylacetat, Dimethylformamid, Dimethylacetamid, Dimethylsulfoxid, Tetralin, Decalin, Indan, Methylbenzoat, Ethylbenzoat, Mesitylen sowie Gemische der vorstehend genannten Lösungsmittel.

Die OPV-Vorrichtung kann beispielsweise eine beliebige literaturbekannte Art sein [siehe z.B. Waldauf et al., Appl. Phys. Lett. 89, 233517 (2006)].

Eine erste bevorzugte erfindungsgemäße OPV-Vorrichtung enthält (in der Reihenfolge beginnend mit der dem einfallenden Licht zugewandten Seite):
- gegebenenfalls ein Substrat,
- eine Elektrode mit hoher Austrittsarbeit, die vorzugsweise ein Metalloxid wie z.B. ITO enthält, als Anode,
- gegebenenfalls eine leitende Polymerschicht oder Lochtransportschicht, die vorzugsweise ein organisches Polymer oder Polymerblend oder eine organische Verbindung wie z.B. PEDOT:PSS (Poly(3,4-ethylendioxythiophen):Poly(styrolsulfonat)), TBD (N,N'-Diphenyl-N-N'-bis(3-methylphenyl)-1,1'biphenyl-4,4'-diamin) oder NBD (N,N'-Diphenyl-N-N'-bis(1-napthylphenyl)-1,1'biphenyl-4,4'-diamin) enthält,
- eine Schicht (auch als "aktive Schicht" oder "photoaktive Schicht" bezeichnet), die einen organischen Halbleiter vom p-Typ und einen organischen Halbleiter vom n-Typ enthält, welche beispielsweise in Form einer Doppelschicht oder zweier getrennter Schichten eines p-Typ- und n-Typ-Halbleiters, oder als Blend eines p-Typ- und n-Typ-Halbleiters vorliegt, die einen Volumen-Heteroübergang (BHJ) bilden,
- gegebenenfalls eine Elektronentransportschicht welche z.B. LiF enthält,
- eine Elektrode mit geringer Austrittsarbeit, die vorzugsweise ein Metall wie z.B. Aluminium enthält, als Kathode,
wobei mindestens eine der Elektroden, vorzugsweise die Anode, transparent ist, und
wobei der p-Typ-Halbleiter und/oder der n-Typ-Halbleiter ein erfindungsgemäßes Polymer ist.

Eine zweite bevorzugte erfindungsgemäße OPV-Vorrichtung ist eine invertierte OPV-Vorrichtung und enthält (in der Reihenfolge beginnend mit der dem einfallenden Licht zugewandten Seite):
- gegebenenfalls ein Substrat,
- eine Elektrode mit hoher Austrittsarbeit, die vorzugsweise ein Metalloxid wie z.B. ITO enthält, als Kathode,
- eine lochblockierende Schicht, welche vorzugsweise eine Metalloxid wie z.B. TiOₓ oder ZnOₓ enthält,
- eine Schicht (auch als "aktive Schicht" oder "photoaktive Schicht" bezeichnet), die einen organischen Halbleiter vom p-Typ und einen organischen Halbleiter vom n-Typ enthält, welche beispielsweise in Form einer Doppelschicht oder zweier getrennter Schichten eines p-Typ- und n-Typ-Halbleiters, oder als Blend eines p-Typ- und n-Typ-Halbleiters vorliegt, die einen Volumen-Heteroübergang (BHJ) bilden,
- gegebenenfalls eine leitende Polymerschicht oder Lochtransportschicht, die vorzugsweise ein organisches Polymer oder Polymerblend oder eine organische Verbindung wie z.B. PEDOT:PSS, TBD oder NBD enthält,
- eine Elektrode die ein Metall mit hoher Austrittsarbeit wie z.B. Silber enthält, als Anode,
wobei mindestens eine der Elektroden, vorzugsweise die Kathode, transparent ist, und
wobei der p-Typ-Halbleiter und/oder der n-Typ-Halbleiter ein erfindungsgemäßes Polymer ist..

In den OPV-Vorrichtungen der vorliegenden erfinden Erfindung sind die Halbleitermaterialien des p-Typs und n-Typs vorzugsweise aus den oben beschriebenen Materialien, wie den Polymer/Fulleren-Systemen, ausgewählt. Wird eine aktive Schicht enthaltend ein solches Polymer/Fulleren-System auf ein Substrate aufgebracht, so bildet es eine BHJ die Phasenseparation im Nanometerbereich zeigt, siehe z.B. Dennler et al, Proceedings of the IEEE, 2005, 93(8), 1429 oder Hoppe et al, Adv. Func. Mater, 2004, 14(10), 1005. Gegebenenfalls kann ein Temperungsschritt erforderlich sein, um die Morphologie des Blends und die Leistung der OPV-Vorrichtung zu optimieren.

Eine weitere Methode zur Optimierung der Leistung der OPV-Vorrichtung ist die Herstellung einer Formulierung zur Produktion von OPV(BHJ)-Vorrichtungen, welche hochsiedende Additive enthält die die Phasenseparation beeinflussen und in die gewünschte Richtung lenken können. zu diesem Zweck können beispielsweise 1,8-Oktandithiol, 1,8-Dijodoktan, Nitrobenzol, Chlornaphthalin oder andere Additive verwendet werden, um hocheffiziente Solarzellen herzustellen. Bespiele hierfür sind in J. Peet, et al, Nat. Mater., 2007, 6, 497 oder Frechet et al. J. Am. Chem. Soc., 2010, 132, 7595-7597 beschrieben.

Die erfindungsgemäßen Polymere, Polymerblends, Formulierungen und Schichten eignen sich auch für die Verwendung in einem OFET als Halbleiterkanal. Dementsprechend stellt die Erfindung auch einen OFET bereit, der eine Gate-Elektrode, eine isolierende (oder Gate-Isolator-) Schicht, eine Source-Elektrode, eine Drain-Elektrode und einen die Source- und Drain-Elektroden verbindenden organischen Halbleiterkanal enthält, wobei der organische Halbleiterkanal ein Polymer, Polymerblend, Formulierung oder organische Halbleiterschicht gemäß der vorliegenden Erfindung enthält. Weitere Merkmale des OFET sind dem Fachmann bekannt und können ohne erfinderisches Zutun hinzugefügt werden.

OFETs, bei denen ein organisches Halbleitermaterial als dünner Film zischen einem Gate-Dielektrikum und einer Drain- und einer Source-Elektrode angeordnet ist, sind allgemein bekannt und beispielsweise in den US 5 892 244, US 5 998 804 oder US 6 723 934 beschrieben. Aufgrund der Vorteile, wie preisgünstige Herstellung unter Ausnutzung der Löslichkeitseigenschaften der erfindungsgemäßen Verbindungen und damit Verarbeitbarkeit großer Oberflächen, sind bevorzugte Anwendungen dieser FETs solche wie integrierte Schaltungen, TFT-Anzeigen und Sicherheitsanwendungen.

Die Gate-, Source- und Drain-Elektroden und die isolierenden und Halbleiterschichten in der OFET-Vorrichtung können in beliebiger Abfolge angeordnet sein, solange die Source- und Drain-Elektrode von der Gate-Elektrode durch die isolierende Schicht getrennt sind, die Gate-Elektrode und die Halbleiterschicht beide Kontakt mit der isolierenden Schicht haben und die Source-Elektrode und die Drain-Elektrode beide Kontakt mit der Halbleiterschicht haben.

Eine OFET-Vorrichtung gemäß der vorliegenden Erfindung enthält vorzugsweise:
- eine Source-Elektrode,
- eine Drain-Elektrode,
- eine Gate-Elektrode,
- eine Halbleiterschicht,
- eine oder mehrere Gate-Isolatorschichten,
- gegebenenfalls ein Substrat,
wobei die Halbleiterschicht vorzugsweise ein Polymer, Polymerblend oder eine Formulierung gemäß der vorliegenden Erfindung enthält.

Bei der OFET-Vorrichtung kann es sich um eine Top-Gate-Vorrichtung oder eine Bottom-Gate-Vorrichtung handeln. Geeignete Strukturen und Herstellungsverfahren für eine OFET-Vorrichtung sind dem Fachmann bekannt und in der Literatur beschrieben, beispielsweise in der US 2007/0102696 A1.

Die Gate-Isolatorschicht kann vorzugsweise ein Fluorpolymer, wie z.B. das im Handel erhältliche Cytop 809M® oder Cytop 107M® (Fa. Asahi Glass) enthalten. Vorzugsweise wird die Gate-Isolatorschicht aus einer Formulierung, welche ein Isolatormaterial und ein oder mehrere Lösungsmittel mit einem oder mehreren Fluoratomen (Fluorlösungsmittel), vorzugsweise ein Perfluorlösungsmittel, enthält, abgeschieden, z.B. durch Spincoating, Aufrakeln, Drahtrakelbeschichtung, Spray- oder Dipcoating oder andere bekannte Verfahren. Ein geeignetes Perfluorlösungsmittel ist z.B. FC75® (Fa. Acros, Katalognummer 12380). Andere geeignete Fluorpolymere und Fluorlösungsmittel sind aus dem Stand der Technik bekannt, wie beispielsweise die Perfluorpolymere Teflon AF® 1600 oder 2400 (Fa. DuPont) oder Fluoropel® (Fa. Cytonix) oder das Perfluorlösungsmittel FC 43® (Acros, Nr. 12377). Besonders bevorzugt sind organische dielektrische Materialien mit einer niedrigen Permittivität (oder dielektrischen Konstante) von 1,0 bis 5,0, sehr bevorzugt von 1,8 bis 4,0 ("Low-k-Materialien"), wie zum Beispiel in der US 2007/0102696 A1 oder US 7 095 044 offenbart.

In Sicherheitsanwendungen können OFETs und andere Vorrichtungen mit Halbleitermaterialien gemäß der vorliegenden Erfindung, wie Transistoren oder Dioden, für RFID-Tags oder Sicherheitsmarkierungen verwendet werden, um Wertdokumente wie Banknoten, Kreditkarten oder Ausweise, nationale Ausweisdokumente, Berechtigungsscheine oder beliebige Produkte mit Geldwert, wie Briefmarken, Eintritts- und Fahrkarten, Lotteriescheine, Aktien, Schecks usw. zu authentifizieren und deren Fälschung zu verhindern.

Alternativ können die erfindungsgemäßen Materialien in OLEDs verwendet werden, z.B. als aktives Anzeigematerial in einer Flachbildschirmanwendungen oder als Hintergrundbeleuchtung eines Flachbildschirms wie z.B. einer Flüssigkristallanzeige. Übliche OLEDs werden unter Verwendung von Mehrschichtstrukturen realisiert. Im Allgemeinen ist eine Emissionsschicht zwischen einer oder mehreren Elektronentransport- und/oder Lochtransportschichten angeordnet. Durch Anlegen einer elektrischen Spannung bewegen sich Elektronen und Löcher als Ladungsträger zur Emissionsschicht, wo ihre Rekombination zur Erregung und damit Lumineszenz der in der Emissionsschicht enthaltenen Lumophoreinheiten führt. Die erfindungsgemäßen Verbindungen, Materialien und Filme können in einer oder mehreren der Ladungstransportschichten und/oder in der Emissionsschicht eingesetzt werden, entsprechend ihren elektrischen und/oder optischen Eigenschaften. Weiterhin ist ihre Verwendung in der Emissionsschicht besonders vorteilhaft, wenn die erfindungsgemäßen Verbindungen, Materialien und Filme selber Elektrolumineszenzeigenschaften zeigen oder elektrolumineszierende Gruppen oder Verbindungen enthalten. Die Auswahl, Charakterisierung sowie die Verarbeitung geeigneter monomerer, oligomerer und polymerer Verbindungen oder Materialien für die Verwendung in OLEDs ist dem Fachmann allgemein bekannt, siehe z.B. Müller et al, Synth. Metals, 2000, 111-112, 31-34, Alcala, J. Appl. Phys., 2000, 88, 7124-7128 und die darin aufgeführte Literatur. Gemäß einer anderen Verwendung kann man die Materialien gemäß dieser Erfindung, insbesondere diejenigen, die Photolumineszenzeigenschaften zeigen, als Materialien für Lichtquellen, z.B. für Anzeigevorrichtungen, wie in der EP 0 889 350 A1 oder von C. Weder et al., Science, 1998, 279, 835-837 beschrieben, einsetzen.

Ein weiterer Aspekt der Erfindung betrifft sowohl die oxidierte als auch die reduzierte Form der erfindungsgemäßen Verbindungen. Abgabe oder Aufnahme von Elektronen führt zur Bildung einer stark delokalisierten Ionenform mit hoher Leitfähigkeit. Dies kann durch Einwirkung üblicher Dotierstoffe auftreten. Geeignete Dotierstoffe und -methoden sind dem Fachmann bekannt, z.B. aus der EP 0 528 662, US 5 198 153 oder WO 96/21659.

Das Dotierverfahren sieht typischerweise die Behandlung des Halbleitermaterials mit einem Oxidations- oder Reduktionsmittel in einer Redoxreaktion vor, so dass sich im Material delokalisierte ionische Zentren bilden, wobei die entsprechenden Gegenionen aus den angewendeten Dotierstoffen stammen. Geeignete Dotiermethoden umfassen beispielsweise die Einwirkung eines dotierenden Dampfes unter atmosphärischem oder vermindertem Druck, elektrochemische Dotierung in einer dotierstoffhaltigen Lösung, das In-Kontakt-Bringen eines Dotierstoffes mit dem Halbleitermaterial zur thermischen Diffundierung und lonenimplantierung des Dotierstoffes in das Halbleitermaterial.

Wenn Elektronen als Träger verwendet werden, sind geeignete Dotierstoffe beispielsweise Halogene (z.B. I₂, Cl₂, Br₂, ICl, ICl₃, IBr und IF), Lewissäuren (z.B. PF₅, AsF₅, SbF₅, BF₃, BCl₃, SbCl₅, BBr₃ und SO₃), Protonensäuren, organische Säuren oder Aminosäuren (z.B. HF, HCl, HNO₃, H₂SO₄, HClO₄, FSO₃H und ClSO₃H), Übergangsmetallverbindungen (z.B. FeCl₃, FeOCl, Fe(ClO₄)₃, Fe(4-CH₃C₆H₄SO₃)₃, TiCl₄, ZrCl₄, HfCl₄, NbF₅, NbCl₅, TaCl₅, MoF₅, MoCl₅, WF₅, WCl₆, UF₆ und LnCl₃ (worin Ln ein Element der Lanthanreihe bedeutet), Anionen (z.B. Cl⁻, Br⁻, I⁻, I₃⁻, HSO₄⁻, SO₄²⁻, NO₃⁻, ClO₄⁻, BF₄⁻, PF₆⁻, AsF₆⁻, SbF₆⁻, FeCl₄⁻, Fe(CN)₆³⁻ und Anionen verschiedener Sulfonsäuren, wie Aryl-SO₃⁻). Wenn Löcher als Träger verwendet werden, sind beispielhafte Dotierstoffe Kationen (z.B. H⁺, Li⁺, Na⁺, K⁺, Rb⁺ und Cs⁺), Alkalimetalle (z.B. Li, Na, K, Rb und Cs), Erdalkalimetalle (z.B. Ca, Sr und Ba), O₂, XeOF₄, (NO₂⁺) (SbF₆⁻), (NO₂⁺) (SbCl₆⁻), (NO₂⁺)(BF₄⁻), AgClO₄, H₂IrCl₆, La(NO₃)₃ · 6H₂O, FSO₂OOSO₂F, Eu, Acetylcholin, R₄N⁺ (R bedeutet eine Alkylgruppe), R₄P⁺ (R bedeutet eine Alkylgruppe), R₆As⁺ (R bedeutet eine Alkylgruppe) und R₃S⁺ (R bedeutet eine Alkylgruppe).

Die leitende Form der Verbindungen der vorliegenden Erfindung kann als organisches "Metall" in Anwendungen wie beispielsweise Ladungsinjektionsschichten und ITO-Planarisierungsschichten in OLED-Anwendungen, Folien für Flach- und Tastbildschirme, Antistatikfolien, gedruckten leitenden Substraten, Mustern oder Leiterbahnen in Elektronikanwendungen wie Leiterplatten und Kondensatoren verwendet werden, ohne hierauf beschränkt zu sein.

Die Verbindungen und Formulierungen gemäß der vorliegenden Erfindung können sich auch für die Verwendung in organischen plasma-emittierenden Dioden (OPEDs), wie zum Beispiel in Koller et al., Nat. Photonics, 2008, 2, 684 beschrieben, eignen.

Gemäß einer anderen Verwendung kann man die Materialien gemäß der vorliegenden Erfindung allein oder zusammen mit anderen Materialien in oder als Orientierungsschichten in LCD- oder OLED-Vorrichtungen, wie beispielsweise in der US 2003/0021913 beschrieben, verwenden. Die Verwendung von Ladungstransportverbindungen gemäß der vorliegenden Erfindung kann die elektrische Leitfähigkeit der Orientierungsschicht erhöhen. Bei Verwendung in einer LCD kann diese erhöhte elektrische Leitfähigkeit ungünstige Restgleichstromeffekte in dem schaltbaren LCD-Element herabsetzen und Einbrennen unterdrücken oder, beispielsweise in ferroelektrischen LCDs, die durch das Schalten der spontanen Polarisationsladung der ferroelektrischen LCs erzeugte restliche Ladung herabsetzen. Bei Verwendung in einer OLED-Vorrichtung mit einem auf der Orientierungsschicht angebrachten lichtemittierenden Material kann diese erhöhte elektrische Leitfähigkeit die Elektrolumineszenz des lichtemittierenden Materials verstärken. Die Verbindungen oder Materialien gemäß der vorliegenden Erfindung mit mesogenen oder flüssigkristallinen Eigenschaften können ausgerichtete anisotrope Filme wie oben beschrieben bilden, die sich insbesondere als Orientierungsschichten zur Induzierung oder Verstärkung der Orientierung in einem auf dem anisotropen Film angebrachten Flüssigkristallmedium eignen. Die Materialien gemäß der vorliegenden Erfindung können auch mit photoisomerisierbaren Verbindungen und/oder Chromophoren zur Verwendung in oder als Photoorientierungsschichten kombiniert werden, wie in der US 2003/0021913 beschrieben.

Gemäß einer anderen Verwendung können die Materialien gemäß der vorliegenden Erfindung, insbesondere ihre wasserlöslichen Derivate (beispielsweise mit polaren oder ionischen Seitengruppen) oder ionisch dotierten Formen, als chemische Sensoren oder Materialien zum Nachweis und zur Unterscheidung von DNA-Sequenzen eingesetzt werden. Derartige Verwendungen sind z.B. in L. Chen, D. W. McBranch, H. Wang, R. Helgeson, F. Wudl und D. G. Whitten, Proc. Natl. Acad. Sci. U.S.A., 1999, 96, 12287; D. Wang, X. Gong, P. S. Heeger, F. Rininsland, G. C. Bazan und A. J. Heeger, Proc. Natl. Acad. Sci. U.S.A., 2002, 99, 49; N. DiCesare, M. R. Pinot, K. S. Schanze und J. R. Lakowicz, Langmuir, 2002, 18, 7785; D. T. McQuade, A. E. Pullen, T. M. Swager, Chem. Rev., 2000, 100, 2537 beschrieben.

Sofern aus dem Zusammenhang nicht deutlich anders ersichtlich, sind hier verwendete Pluralformen der Begriffe als die Singularform enthaltend zu verstehen und umgekehrt.

In der gesamten Beschreibung und den Ansprüchen dieser Spezifikation haben die Begriffe "umfassen" und "enthalten" sowie Variationen hiervon, wie beispielsweise "enthaltend" und "enthält", die Bedeutung "beinhaltend, ohne hierauf beschränkt zu sein", und sind nicht so zu verstehen, dass sie andere Komponenten ausschließen.

Es versteht sich, dass Variationen der vorhergehenden Ausführungsformen der Erfindung durchgeführt werden können, die weiterhin unter den Schutzumfang der vorliegenden Erfindung fallen. Jedes in diesem Anmeldungstext offenbarte Merkmal kann, wenn nicht anders angegeben, durch alternative Merkmale ersetzt werden, die einem gleichen, gleichwertigen oder ähnlichen Zweck dienen. Wenn nicht anders angegeben, ist jedes offenbarte Merkmal daher nur ein Beispiel einer gattungsmäßigen Reihe von gleichwertigen oder ähnlichen Merkmalen.

Alle der in diesem Anmeldungstext offenbarten Merkmale können in jeder beliebigen Kombination miteinander kombiniert werden, mit Ausnahme von Kombinationen, bei denen wenigstens einiger dieser Merkmale und/oder Schritte einander ausschließen. Insbesondere sind die bevorzugten Merkmale der Erfindung auf alle Aspekte der Erfindung anwendbar und können in jeder beliebigen Kombination verwendet werden. Ebenso können in nicht wesentlichen Kombinationen beschriebene Merkmale auch getrennt voneinander (d.h. nicht in Kombination miteinander) verwendet werden.

Sofern nicht anders vermerkt, sind vor- und nachstehend alle Prozentwerte Gewichtsprozent, und alle Temperaturwerte Grad Celsius. Die Werte der dielectrischen Konstante ε ("Permittivität") meinen die bei 20°C und 1,000 Hz ermittelten Werte.

Die Erfindung wird nun unter Bezugnahme auf die folgenden Beispiele ausführlicher beschrieben, welche die Erfindung veranschaulichen sollen, ohne sie einzuschränken.

### Beispiel 1 - Bausteine

### Synthese des Polvthiophen-Bausteins:

### 1.1 2-(2-Bromthiophen-3-yl)ethanol:

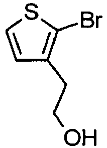

N-Bromsuccinimid (13.9 g, 78.0 mmol, 1.00 Äquiv.) wurde bei 0 °C portionsweise zu einer Lösung aus 2-(Thiophen-3-yl)ethanol (10.0 g, 78.0 mmol, 1.00 Äquiv.) in Tetrahydrofuran (200 mL) gegeben. Die Suspension wurde unter Rühren langsam über Nacht auf Raumtemperatur aufgewärmt. Zum Reaktionsgemisch wurden 50 mL Wasser gegeben, die Phasen getrennt und die wässrige Phase mit Chloroform (50 mL) extrahiert. Die vereinigten organischen Phasen wurden mit einer wässrigen Natriumhydrogencarbonat-Lösung (50 mL) gewaschen, über Magnesiumsulfat getrocknet und das Lösungsmittel wurde unter vermindertem Druck entfernt. Das Rohprodukt wurde säulenchromatographisch an Kieselgel mit Petrolether/Essigsäureethylester (2:1) als Eluent gereinigt. Es wurden 14.5 g (90%) des Produktes als gelbes Öl erhalten. - *R*_{f} = 0.39 (Petrolether/Essigsäureethylester 2:1). - ¹H-NMR (300 MHz, CDCl₃): δ = 7.26 (d, ³*J* = 5.6 Hz, 1 H, *H*ₐᵣ), 6.90 (d, ³*J* = 5.6 Hz, 1 H, *H*ₐᵣ), 3.86 (t, ³*J* = 6.6 Hz, 2 H, C*H*₂-OH), 2.89 (t, ³*J* = 6.6 Hz, 2 H, Cₐᵣ-C*H*₂), 1.75 (s, 1 H, OH). - ¹³C-NMR (75 MHz, CDCl₃): δ = 138.1 (*C*ₐᵣ-CH₂), 128.7 (*C*ₐᵣ), 125.8 (*C*ₐᵣ), 110.5 (*C*ₐᵣ-Br), 62.2 (*C*H₂-OH), 32.9 (Cₐᵣ-*C*H₂). - MS (EI), *m*/*z* (%): 208/206 (52/51) [M⁺], 177/175 (100/91) [(C₅H₄BrS)⁺], 97 (38). - HRMS (C₆H₇BrOS): ber. 205.9401, gef. 205.9402.

### 1.2 2-(2-Brom-5-iodthiophen-3-yl)ethylacetat:

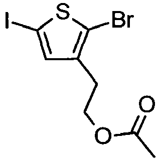

2-(2-Bromthiophen-3-yl)ethanol (9.11 g, 44.0 mmol, 1.00 Äquiv.) wurde in Essigsäure (50 mL) gelöst. Nach der Zugabe von n-Iodsuccinimid (10.4 g, 46.2 mmol, 1.05 Äquiv.) wurde die entstandene Suspension bei 100 °C für 4 h gerührt. Das Reaktionsgemisch wurden auf Wasser (50 mL) gegeben, die Phasen getrennt und die wässrige Phase mit Dichlormethan (50 mL) extrahiert. Die vereinigten organischen Phasen wurden mit einer wässrigen 1 M Natriumhydroxid-Lösung (50 mL), 10%-iger Natriumthiosulfat-Lösung (50 mL), Wasser (50 mL) und gesättigter Natriumchlorid-Lösung (50 mL) gewaschen, über Magnesiumsulfat getrocknet und das Lösungsmittel wurde unter vermindertem Druck entfernt. Das Rohprodukt wurde säulenchromatographisch an Kieselgel mit Petrolether/Essigsäureethylester (3:1) als Eluent gereinigt. Es wurden 15.4 g (93%) des Produktes als orangenes Öl erhalten.
*R*_{f} = 0.54 (Petrolether/Essigsäureethylester 3:1). - ¹H-NMR (300 MHz, CDCl₃): δ = 7.01 (s, 1 H, *H*ₐᵣ), 4.20 (t, ³*J* = 6.9 Hz, 2 H, C*H*₂-O), 2.88 (t, ³*J* = 6.9 Hz, 2 H, Cₐᵣ-C*H*₂) 2.05 (s, 3 H, C*H*₃). -¹³C-NMR (75 MHz, CDCl₃): δ = 171.0 (*C*=O), 140.0 (*C*ₐᵣ-CH₂), 138.1 (*C*ₐᵣ), 113.7 (*C*ₐᵣ-Br), 71.7 (*C*ₐᵣ-I), 63.2 (*C*H₂-O), 28.8 (Cₐᵣ-*C*H₂), 21.2 (CH₃). - FTIR: ṽ = 2952, 1735, 1363, 1228, 1036 cm⁻¹. - MS (EI), *m*/*z* (%): 376/374 (15/15) [M⁺], 316/314 (100/100) [(C₆H₄BrIS)⁺], 190/188 (8/9) [(C₆H₅BrS)⁺]. - HRMS (C₈H₈BrIO₂S): ber. 373.8473, gef. 373.8463.

### 1.3 2-(2-Brom-5-iodthiophen-3-vl)ethanol:

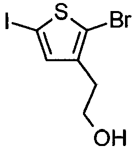

2-(2-Brom-5-iodthiophen-3-yl)ethylacetat (17.1 g, 45.7 mmol, 1.00 Äquiv.) wurde in einer Mischung aus Tetrahydrofuran (300 mL) und wässriger 1 M Natriumhydroxid-Lösung (200 mL) gelöst und bei 70 °C für 4 h gerührt. Das Reaktionsgemisch wurde mit Toluol (100 mL) verdünnt und die organische Phase dreimal mit je 100 mL Wasser gewaschen und über Magnesiumsulfat getrocknet und das Lösungsmittel wurde unter vermindertem Druck entfernt. Das Rohprodukt wurde säulenchromatographisch an Kieselgel mit Petrolether/Essigsäure-ethylester (3:1) als Eluent gereinigt. Es wurden 14.9 g (98%) des Produktes als leicht gelber Feststoff erhalten.

*R*_{f} = 0.23 (Petrolether/Essigsäureethylester 3:1).
¹H-NMR (300 MHz, CDCl₃): δ = 7.06 (s, 1 H, *H*ₐᵣ), 3.82 (t, ³*J* = 6.4 Hz, 2 H, C*H*₂-OH), 2.82 (t, ³*J* = 6.4 Hz, 2 H, Cₐᵣ-C*H*₂), 1.44 (s, 1 H, O*H*). - ¹³C-NMR (75 MHz, CDCl₃): δ = 140.5 (*C*ₐᵣ-CH₂), 138.4 (*C*ₐᵣ), 113.4 (*C*ₐᵣ-Br), 71.8 (*C*ₐᵣ-I), 62.0 (*C*H₂-OH), 32.7 (Cₐᵣ-*C*H₂). - FTIR: ṽ = 3257, 2952, 999 cm⁻¹. - MS (EI), *m*/*z* (%): 334/332 (91/94) [M⁺], 303/301 (100/99) [(C₅H₃BrIS)⁺]. - HRMS (C₆H₆BrIOS): ber. 331.8367, gef. 331.8363.

### Synthese des Fluoren-Bausteins:

### 1.4 2,7-Dibrom-9-methylfluoren:

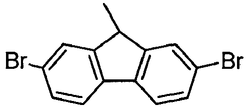

1.54 M n-Butyllithium-Lösung (35 mL, 3.42 g, 53.4 mmol, 1.01 Äquiv.) in Hexan wurde zu einer Lösung aus 2,7-Dibromfluoren (17.1 g, 52.9 mmol, 1.00 Äquiv.) in Tetrahydrofuran (300 mL) bei -78 °C langsam zugetropft. Nach fünf Minuten wurde lodmethan (8.26 g, 58.2 mmol, 1.10 Äquiv.) langsam zugetropft. Das Reaktionsgemisch wurde 4 h gerührt und dann auf Dichlormethan/Wasser (2:1, 100 mL) gegeben. Die wässrige Phase wurde weiter mit Dichlormethan extrahiert, die vereinigten organischen Phasen wurden über Magnesiumsulfat getrocknet und das Lösungsmittel unter vermindertem Druck entfernt. Der Rückstand wurde aus Petrolether umkristallisiert. Es wurden 11.8 g (66%) des Produktes als gelber Feststoff erhalten.

### 1.5 2,7-Dibrom-9-(3-hydroxypropyl)-9-methylfluoren:

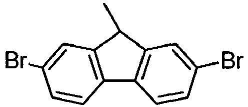

2,7-Dibrom-9-methylfluoren (9.03 g, 26.7 mmol, 1.00 Äquiv.) wurde in Dimethylsulfoxid (150 mL) gelöst. Dazu wurden Kaliumhydroxid (1.65 g, 29.4 mmol, 1.10 Äquiv.), 18-Krone-6 (141 mg, 534 µmol, 0.02 Äquiv.), Wasser (5 mL) und 3-Brompropan-1-ol (3.90 g, 28.0 mmol, 1.05 Äquiv.) gegeben. Das Reaktionsgemisch wurde bei Raumtemperatur über Nacht gerührt und dann vorsichtig auf Dichlormethan (100 mL) gegeben. Die organische Phase wurde mit gesättigter Natriumchlorid-Lösung (50 mL) und Wasser (50 mL) gewaschen. Die organische Phase wurde über Magnesiumsulfat getrocknet und das Lösungsmittel unter vermindertem Druck entfernt. Das Rohprodukt wurde säulenchromatographisch an Kieselgel mit Dichlormethan als Eluent gereinigt. Es wurden 5.73 g (54%) des Produktes als gelber Feststoff erhalten. - *R*_{f} = 0.40 (Dichlormethan). - ¹H-NMR (300 MHz, CDCl₃): δ = 7.53 (d, ³*J* = 8.1 Hz, 2 H, 2 × *H*ₐᵣ), 7.50 (d, ⁴*J* = 1.5 Hz, 2 H, 2 × *H*ₐᵣ), 7.45 (d, ³*J* = 8.1 Hz, ⁴*J* = 1.5 Hz, 2 H, 2 × *H*ₐᵣ), 3.34-3.41 (m, 2 H, C*H*₂-OH), 1.98-2.05 (m, 2 H, C*H*₂), 1.46 (s, 3 H, C*H*₃), 0.83-0.94 (m, 2 H, C*H*₂). - ¹³C-NMR (75 MHz, CDCl₃): δ = 153.3 (2 × *C*ₐᵣ), 138.2 (2 × *C*ₐᵣ), 130.5 (2 × *C*ₐᵣH), 126.3 (2 × *C*ₐᵣH), 121.7 (2 × *C*ₐᵣ-Br), 121.5 (2 × *C*ₐᵣH), 62.8 (*C*H₂-OH), 51.0 (*C*H₂), 36.6 (*C*H₂), 27.6 (*C*H₃) 26.6 (*C*H₂). - MS (EI), *m*/*z* (%): 398/396/394 (50/99/53) [M⁺], 339/337/335 (56/100/51) [(C₁₄H₁₉Br₂)⁺], 258/256 (76/75) [(C₁₄H₁₉Br)⁺], 176 (89). - HRMS (C₁₇H₁₆Br₂O): ber. 393.9568, gef. 393.9605.

### Synthese des Carbazol-Bausteins:

### 1.6 4,4-Dibrom-2-nitrobiphenyl:

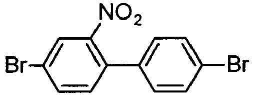

Eine Mischung aus rauschender Salpetersäure (92 mL) und Wasser (8 mL) wurde langsam bei 100 °C zu einer Lösung aus 4,4-Dibrombiphenyl (20 g, 0.064 mol, 1.00 Äquiv.) in Essigsäure (300 mL) zugegeben. Das Reaktionsgemisch wurde für 30 Minuten bei 100 °C gerührt. Nachdem Abkühlen auf Raumtemperatur wurde das Reaktionsgemisch auf Wasser (100 mL) gegeben und mit Dichlormethan (100 mL) extrahiert. Die vereinigten organischen Phasen wurden über Magnesiumsulfat getrocknet und das Lösungsmittel unter vermindertem Druck entfernt. Der Rückstand wurde aus Ethanol umkristallisiert. Es wurden 18.1 g (79%) des Produktes als gelber Feststoff erhalten. - ¹H-NMR (300 MHz, CDCl₃): δ = 8.03 (d, ⁴*J* = 2.0 Hz, 1 H, *H*ₐᵣ), 7.76 (dd, ³*J* = 8.3 Hz, ⁴*J* = 2.0 Hz, 1 H, *H*ₐᵣ), 7.56 (d, ³*J* = 8.6 Hz, 2 H, 2 × *H*ₐᵣ), 7.29 (q, ³*J* = 8.3 Hz, 1 H, *H*ₐᵣ), 7.16 (d, ³*J* = 8.6 Hz, 2 H, 2 × *H*ₐᵣ). - ¹³C-NMR (75 MHz, CDCl₃): δ = 145.8 (*C*ₐᵣ-NO₂), 135.7 (*C*ₐᵣH), 135.4 (*C*ₐᵣ), 134.3 (*C*ₐᵣ), 133.2 (*C*ₐᵣH), 132.2 (2 × *C*ₐᵣH), 129.6 (2 × *C*ₐᵣH), 127.4 (*C*ₐᵣH), 123.2 (*C*ₐᵣ-Br), 122.0 (*C*ₐᵣ-Br). - MS (EI), *m*/*z* (%): 359/357/355 (50/100/51) [M⁺], 232/230 (50/47) [(C₁₂H₇Br)⁺], 151 (65) [(C₁₂H₇)⁺]. - HRMS (C₁₂H₇Br₂NO₂): ber. 354.8844, gef. 354.8846.

### 1.7 2,7-Dibromcarbazol:

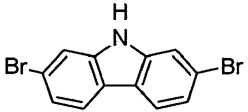

Eine Mischung aus 4,4-Dibrom-2-nitrobiphenyl (18.2 g, 51.0 mmol, 1.00 Äquiv.) und Triethylphosphit (100 mL) wurde bei 160 °C für 18 h gerührt. Nachdem dem Abkühlen auf Raumtemperatur wurde das Reaktionsgemisch auf Wasser (100 mL) gegeben und mit Dichlormethan extrahiert. Die vereinigten organischen Phasen wurden über Magnesiumsulfat getrocknet und der Überschuss an Triethylphosphit wurde bei 160 °C abdestilliert. Das Rohprodukt wurde säulenchromatographisch an Kieselgel mit Petrolether/Essigsäureethylester (20:1) als Eluent gereinigt. Es wurden 10.0 g (60%) des Produktes als gelber Feststoff erhalten. - *R*_{f} = 0.23 (Petrolether/Essigsäureethylester 20:1). - ¹H-NMR (300 MHz, Aceton-d₆): δ = 10.60 (bs, 1 H, N*H*), 8.06 (d, ³*J* = 8.5 Hz, 2 H, 2 × *H*ₐᵣ), 7.73 (d, ⁴*J* = 1.7 Hz, 2 H, 2 × *H*ₐᵣ), 7.35 (dd, ³*J* = 8.5 Hz, ⁴*J* = 1.7 Hz, 2 H, 2 × *H*ₐᵣ). - ¹³C-NMR (75 MHz, Aceton-d₆): δ = 141.9 (2 × *C*ₐᵣ-NH), 123.3 (2 × *C*ₐᵣH), 122.6 (2 × *C*ₐᵣH), 119.9 (2 × *C*ₐᵣ-Br), 114.9 (2 × *C*ₐᵣ), 114.8 (2 × *C*ₐᵣH). - MS (EI), *m*/*z* (%): 327/325/323 (50/100/51) [M⁺], 246/244 (14/15) [(C₁₂H₇BrN)⁺]. - HRMS (C₁₂H₇Br₂N): ber. 322.8945, gef. 322.8927.

### Synthese der Alkohole für die Imidazolester:

### 1.8 1-Butylcyclopentanol:

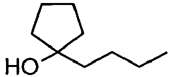

Eine 1.6 M n-Butyllithium-Lösung in Hexan (31.3 mL, 3.20 g, 50.0 mmol, 1.00 Äquiv.) wurde zu einer Lösung aus Cyclopentanon (4.21 g, 50.0 mmol, 1.00 Äquiv.) und 0.6 M Lanthanchlorid Bis(lithiumchlorid)-Komplex-Lösung (40.0 mL, 4.13 g, 12.5 mmol, 0.25 Äquiv.) in Tetrahydrofuran bei - 78 °C langsam zugetropft. Die Lösung wurde unter Rühren langsam über Nacht auf Raumtemperatur aufgewärmt. Zum Reaktionsgemisch wurde gesättigte wässrige Ammoniumchlorid-Lösung (20 mL) gegeben, die Phasen getrennt und Diethylether extrahiert. Die vereinigten organischen Phasen wurden über Natriumsulfat getrocknet und das Lösungsmittel wurde unter vermindertem Druck entfernt. Das Rohprodukt wurde säulenchromatographisch an Kieselgel mit Petrolether/Essigsäureethylester (10:1) als Eluent gereinigt. Es wurden 5.46 g (70%) des Produktes als gelbes Öl erhalten. - *R*_{f} = 0.24 (Petrolether/Essigsäureethylester 10:1). - ¹H-NMR (300 MHz, CDCl₃): δ = 1.74-1.86 (m, 3 H, O*H* + C*H*₂), 1.52-1.66 (m, 8 H, 4 × C*H*₂), 1.26-1.43 (m, 4 H, 2 × C*H*₂), 0.91 (t, ³*J* = 7.0 Hz, 3 H, C*H*₃). - ¹³C-NMR (75 MHz, CDCl₃): δ = 82.7 (*C*_{q}-OH), 41.4 (*C*H₂), 39.8 (2 × *C*H₂), 27.1 (*C*H₂), 24.0 (2 × *C*H₂), 23.5 (*C*H₂), 14.3 (*C*H₃). - FTIR: ṽ = 3376, 2954, 2871, 1696, 1634, 988 cm⁻¹. - MS (EI), *m*/*z* (%): 142 (6) [M⁺], 113 (57) [(C₇H₁₃O)⁺], 110 (23) [(C₇H₁₃O)⁺], 85 (100) [(C₅H₉O)⁺], 85 (100) [(C₅H₉O)⁺], 58 (29). - HRMS (C₉H₁₈O): ber. 142.1358, gef. 142.1345.

### 1.9 2-Methyloct-3-in-2-ol:

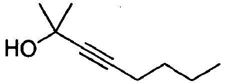

Eine 1.6 M n-Butyllithium-Lösung in Hexan (34.4 mL, 3.52 g, 55.0 mmol, 1.10 Äquiv.) wurde zu einer Lösung aus 1-Hexin (4,11 g, 50.0 mmol, 1.00 Äquiv.) in Tetrahydrofuran (50 mL) bei -78 °C langsam zugetropft. Nach einer Stunde wurde das Reaktionsgemisch auf Raumtemperatur erwärmt und anschließend wieder auf-78 °C gekühlt. Dann wurde Aceton (3.49 g, 60.0 mmol, 1.20 Äquiv.) langsam hinzugetropft. Die Lösung wurde unter Rühren langsam über Nacht auf Raumtemperatur aufgewärmt. Zum Reaktionsgemisch wurde Wasser (20 mL) gegeben und die organische Phase dreimal mit je 20 mL Wasser gewaschen. Die vereinigten organischen Phasen wurden über Magnesiumsulfat getrocknet und das Lösungsmittel wurde unter vermindertem Druck entfernt. Das Rohprodukt wurde säulenchromatographisch an Kieselgel mit Petrolether/Essigsäureethylester (10:1) als Eluent gereinigt. Es wurden 5.47 g (78%) des Produktes als klares Öl erhalten. - *R*_{f} = 0.32 (Petrolether/Essigsäureethylester 10:1). - ¹H-NMR (300 MHz, CDCl₃): δ = 2.17 (t, ³*J* = 6.9 Hz, 2 H, C*H*₂), 1.96 (bs, 1 H, OH), 1.32-1.52 (m, 4 H, 2 × C*H*₂), 1.48 (s, 6 H, 2 × C*H*₃), 0.89 (t, ³*J* = 7.0 Hz, 3 H, C*H*₃). - ¹³C-NMR (75 MHz, CDCl₃): δ = 85.2 (C_{q}-*C*), 82.7 (*C*-CH₂), 65.4 (*C*_{q}-OH), 31.9 (2 × *C*H₃), 30.9 (*C*H₂), 22.0 (*C*H₂), 18.03 (*C*H₂), 13.7 (*C*H₃). - FTIR: ṽ = 3354, 2978, 2934, 2871, 2235, 1462, 1362, 1238, 1163, 944, 554 cm⁻¹. - MS (EI), *m*/*z* (%): 140 (1) [M⁺], 125 (100) [(C₈H₁₃O)⁺], 43 (100) [(C₃H₇)⁺]. - HRMS (C₉H₁₆O): ber. 140.1201, gef. 140.1197.

### 1.10 1-(Hex-1-yn-1-yl)cyclohexanol

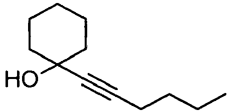

Eine 1.6 M n-Butyllithium-Lösung in Hexan (34.4 mL, 3.52 g, 55.0 mmol, 1.10 Äquiv.) wurde zu einer Lösung aus 1-Hexin (4,11 g, 50.0 mmol, 1.00 Äquiv.) in Tetrahydrofuran (50 mL) bei -78 °C langsam zugetropft. Nach einer Stunde wurde das Reaktionsgemisch auf Raumtemperatur erwärmt und anschließend wieder auf -78 °C gekühlt. Dann wurde Cyclohexanon (5.89 g, 60.0 mmol, 1.20 Äquiv.) langsam hinzugetropft. Die Lösung wurde unter Rühren langsam über Nacht auf Raumtemperatur aufgewärmt. Zum Reaktionsgemisch wurde Wasser (20 mL) gegeben und die organische Phase dreimal mit je 20 mL Wasser gewaschen. Die vereinigten organischen Phasen wurden über Magnesiumsulfat getrocknet und das Lösungsmittel wurde unter vermindertem Druck entfernt. Das Rohprodukt wurde säulenchromatographisch an Kieselgel mit Petrolether/Essigsäureethylester (10:1) als Eluent gereinigt. Es wurden 8.21 g (91%) des Produktes als gelbes Öl erhalten. - *R*_{f} = 0.23 (Petrolether/Essigsäureethylester 10:1). - ¹H-NMR (300 MHz, CDCl₃): δ = 2.21 (t, ³*J* = 6.9 Hz, 2 H, C*H*₂), 1.80-1.91 (m, 2 H, C*H*₂), 1.77 (s, 1 H, O*H*), 1.60-1.72 (m, 2 H, C*H*₂), 1.23-1.58 (m, 10 H, 5 × C*H*₂), 0.90 (t, ³*J* = 7.2 Hz, 3 H, C*H*₃). - ¹³C-NMR (75 MHz, CDCl₃): δ = 84.8 (C_{q}-*C*), 84.0 (*C*-CH₂), 68.9 (*C*_{q}-OH), 40.4 (2 × *C*H₂), 31.0 (*C*H₂), 25.4 (*C*H₂), 23.6 (2 × *C*H₂), 22.0 (*C*H₂), 18.5 (*C*H₂), 13.7 (*C*H₃). - FTIR: ṽ = 3362, 2929, 2859, 1447, 1061, 963 cm⁻¹. - MS (EI), *m*/*z* (%): 180 (18) [M⁺], 137 (100) [(C₉H₁₃O)⁺]. - HRMS (C₁₁H₁₈O): ber. 180.1514, gef. 180.1521.

### 1.11 9-Methylheptadecan-9-ol

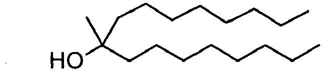

Eine Lösung aus 1-Bromoctan (19.3 g, 100 mmol, 1.00 Äquiv.) in Tetrahydrofuran (100 mL) wurde zu einer Suspension aus Magnesiumspänen (2.67 g, 110 mmol, 1.10 Äquiv.) ind. Tetrahydrofuran (10 mL) getropft und anschließend 6 Stunden bei bei 80 °C gerührt. Danach wurde eine Lösung aus Essigsäureethylester (2.20 g, 50 mmol, 0.50 Äquiv.) in Tetrahydrofuran (80 mL) dazugetropft und weitere 5 Stunden bei 80 °C gerührt. Die Reaktionsmischung wurde eine gesättigte Ammoniumchlorid-Lösung (100 mL) gegeben und mit Diethylether (30 mL) extrahiert. Die vereinigten organischen Phasen wurden über Magnesiumsulfat getrocknet und das Lösungsmittel wurde unter vermindertem Druck entfernt. Das Rohprodukt wurde säulenchromatographisch an Kieselgel mit Petrolether/Essigsäureethylester (20:1) als Eluent gereinigt. Es wurden 5.86 g (44%) des Produktes als gelbes Öl erhalten.
- *R*_{f} = 0.18 (Petrolether/Essigsäureethylester 20:1). - ¹H-NMR (300 MHz, CDCl₃): δ = 1.40-1.46 (m, 4 H, 2 × C*H*₂), 1.23-1.35 (m, 25 H, 12 × C*H*₂ + OH), 1.14 (s, 3 H, C*H*₃), 0.88 (t, ³*J* = 6.6 Hz, 6 H, 2 × C*H*₃). - ¹³C-NMR (75 MHz, CDCl₃): δ = 73.0 (*C*ₐᵣ-OH), 42.0 (2 × *C*H₂), 32.1 (2 × *C*H₂), 30.4 (2 × *C*H₂), 29.8 (2 × *C*H₂), 29.5 (2 × *C*H₂), 27.1 (*C*H₃), 24.1 (2 × *C*H₂), 22.8 (2 × *C*H₂), 14.3 (2 × *C*H₃). - MS (EI), *m*/*z* (%): 270 (1) [M⁺], 255 (50) [(C₁₇H₃₅O)⁺], 157 (100) [(C₁₀H₂₁O)⁺]. - HRMS (C₁₈H₃₈O): ber. 270.2923, gef. 270.2894.

### Synthese der Imidazolester:

### Allgemeine Arbeitsvorschrift zur Darstellung von Imidazolestern (AAV 1):

Eine Suspension aus dem entsprechenden Alkohol (1.00 Äquiv.), 1,1-Carbonyldiimidazol (1.10 Äquiv.) und Kaliumhydroxid (0.01 Äquiv.) in Toluol (5.0 mL/mmol Alkohol) wurden bei 60 °C über 18 h gerührt. Das Lösungsmittel wurde unter vermindertem Druck entfernt und der übriggebliebene Rest mit Dichlormethan (50 mL) wieder aufgenommen. Die organische Phase wurde dreimal mit je 50 mL Wasser gewaschen, über Magnesiumsulfat getrocknet und das Lösungsmittel wurde unter vermindertem Druck entfernt. Das Rohprodukt wurde säulenchromatographisch an Kieselgel mit Petrolether/Essigsäureethylester-Gemischen als Eluent gereinigt.

### 1.12 2-Methylhexan-2-yl 1H-imidazol-1-carboxylat:

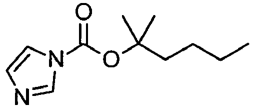

Die Synthese wurde ausgehend von 2-Methylhexan-2-ol (9.30 g, 80.0 mmol, 1.00 Äquiv.), 1,1-carbonyldiimidazol (15.7 g, 88.0 mmol, 1.10 Äquiv.) und Kaliumhydroxid (45.0 mg, 800 µmol, 0.01 Äquiv.) gemäß ***AAV 1*** durchgeführt. Nach säulenchromatographischer Reinigung (Kieselgel, Petrolether/ Essigsäureethylester 5:1) wurden 11.7 g (70%) des Imidazolesters als klare Flüssigkeit erhalten. - *R*_{f} = 0.18 (Petrolether/Essigsäureethylester 5:1). - ¹H-NMR (300 MHz, CDCl₃): δ = 8.04 (s, 1 H, *H*ₐᵣ), 7.34 (s, 1 H, *H*ₐᵣ), 7.01 (s, 1 H, *H*ₐᵣ), 1.83-1.89 (m, 2 H, C*H*₂), 1.58 (s, 6 H, 2 × C*H*₃), 1.30-1.38 (m, 4 H, 2 × C*H*₂), 0.89-0.92 (m, 3 H, C*H*₃). - ¹³C-NMR (75 MHz, CDCl₃): δ = 147.1 (*C*=O), 137.2 (*C*ₐᵣH), 130.4 (*C*ₐᵣH), 117.2 (*C*ₐᵣH), 88.1 (O-*C*_{q}), 40.6 (*C*H₂), 26.2 (*C*H₂), 25.9 (2 × *C*H₃), 22.6 (*C*H₂), 14.0 (CH₃). - FTIR: ṽ = 1752 cm⁻¹. - MS (EI), *m*/*z* (%): 210 (10) [M⁺], 153 (18) [(C₇H₉N₂O₂)⁺], 99 (100) [(C₇H₁₅)⁺], 95 (45) [(C₄H₃N₂O)⁺]. - HRMS (C₁₁H₁₈N₂O₂): ber. 210.1368, gef. 210.1355.

### 1.13 1-Butylcyclopentyl 1H-imidazol-1-carboxylat:

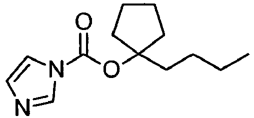

Die Synthese wurde ausgehend von 1-Butylcyclopentanol (2.13 g, 15.0 mmol, 1.00 Äquiv.), 1,1-carbonyldiimidazol (3.80 g, 18.8 mmol, 1.10 Äquiv.) und Kaliumhydroxid (8.00 mg, 150 µmol, 0.01 Äquiv.) gemäß ***AAV 1*** durchgeführt. Nach säulenchromatographischer Reinigung (Kieselgel, Petrolether/ Essigsäureethylester 5:1) wurden 1.04 g (29%) des Imidazolesters als klare Flüssigkeit erhalten.- *R*_{f} = 0.19 (Petrolether/Essigsäureethylester 5:1). - ¹H-NMR (300 MHz, CDCl₃): δ = 8.07 (s, 1 H, Cₐᵣ*H*), 7.37 (s, 1 H, Cₐᵣ*H*), 7.04 (s, 1 H, Cₐᵣ*H*), 2.23-2.34 (m, 2 H, C*H*₂), 2.04-2.10 (m, 2 H, C*H*₂), 1.66-1.84 (m, 6 H, 3 × C*H*₂), 1.27-1.37 (m, 4 H, 2 × C*H*₂), 0.89 (t, ³*J* = 7.1 Hz, 3 H, C*H*₃). - ¹³C-NMR (75 MHz, CDCl₃): δ = 145.4 (*C*=O), 137.2 (*C*ₐᵣH), 130.5 (*C*ₐᵣH), 117.2 (*C*ₐᵣH), 98.8 (O-*C*_{q}), 37.6 (2 × *C*H₂), 36.8 (*C*H₂), 26.8 (*C*H₂), 24.0 (2 × *C*H₂), 23.0 (*C*H₂), 14.1 (*C*H₃). - FTIR: ṽ = 2959, 2872, 1752, 1469, 1382, 1286, 1239, 1171, 999, 772 cm⁻¹. - MS (ESI), *m*/*z* (%): 275 [(M+K)⁺], 259 [(M+Na)⁺], 236 [M⁺]. - HRMS (C₁₃H₂₀N₂O₂Na): ber. 259.1422, gef. 259.1419. - HRMS (C₁₃H₂₀N₂O₂K): ber. 275.1162, gef. 275.1159.

### 1.14 2-Methyloct-3-in-2-yl 1H-imidazol-1-carboxylat:

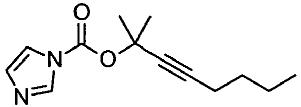

Die Synthese wurde ausgehend von 2-Methyloct-3-in-2-ol (1.01 g, 7.20 mmol, 1.00 Äquiv.), 1,1-carbonyldiimidazol (1.41 g, 7.92 mmol, 1.10 Äquiv.) und Kaliumhydroxid (4.00 mg, 72.0 µmol, 0.01 Äquiv.) gemäß ***AAV 1*** durchgeführt. Nach säulenchromatographischer Reinigung (Kieselgel, Petrolether/ Essigsäureethylester 10:1) wurden 1.25 g (74%) des Imidazolesters als klare Flüssigkeit erhalten. - *R*_{f} = 0.14 (Petrolether/Essigsäureethylester 10:1). - ¹H-NMR (300 MHz, CDCl₃): δ = 8.08 (s, 1 H, *H*ₐᵣ), 7.38 (s, 1 H, *H*ₐᵣ), 7.03 (s, 1 H, *H*ₐᵣ), 2.21 (t, ³*J* = 7.0 Hz, 2 H, C*H*₂), 1.79 (s, 6 H, 2 × C*H*₃), 1.32-1.53 (m, 4 H, 2 × C*H*₂), 0.89 (t, ³*J* = 7.2 Hz, 3 H, C*H*₃). - ¹³C-NMR (75 MHz, CDCl₃): δ = 146.7 (*C*=O), 137.3 (*C*ₐᵣH), 130.5 (*C*ₐᵣH), 117.3 (*C*ₐᵣH), 87.3 (C_{q}-*C*), 79.8 (*C*-CH₂), 77.7 (O-*C*_{q}), 30.6 (*C*H₂), 29.5 (2 × *C*H₃), 22.0 (*C*H₂), 18.5 (*C*H₂), 13.7 (CH₃). - FTIR: ṽ = 2989, 2933, 2689, 2247, 163, 1468, 1379, 1291, 1241, 1125, 1091, 998, 839, 769, 649 cm⁻¹. - MS (EI), *m*/*z* (%): 234 (4) [M⁺], 123 (100) [(C₉H₁₅)⁺], 81 (71) [(C₆H₉)⁺]. - HRMS (C₁₃H₁₈N₂O₂): ber. 234.1368, gef. 234.1366.

### 1.15 1-(Hex-1-in-1-yl)cyclohexyl 1H-imidazole-1-carboxylat

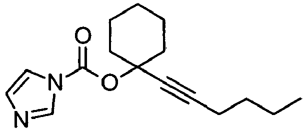

Die Synthese wurde ausgehend von 1-(Hex-1-in-1-yl)cyclohexanol (4.06 g, 22.5 mmol, 1.00 Äquiv.), 1,1-carbonyldiimidazol (4.41 g, 24.8 mmol, 1.10 Äquiv.) und Kaliumhydroxid (13.0 mg, 225 µmol, 0.01 Äquiv.) gemäß ***AAV 1*** durchgeführt. Nach säulenchromatographischer Reinigung (Kieselgel, Petrolether/ Essigsäureethylester 5:1) wurden 5.24 g (85%) des Imidazolesters als farbloser Feststoff erhalten. - *R*_{f} = 0.16 (Petrolether/Essigsäureethylester 5:1). - ¹H-NMR (300 MHz, CDCl₃): δ = 8.10 (s, 1 H, *H*ₐᵣ), 7.40 (s, 1 H, *H*ₐᵣ), 7.04 (s, 1 H, *H*ₐᵣ), 2.17-2.28 (m, 4 H, 2 × C*H*₂), 1.94-2.04 (m, 2 H, C*H*₂), 1.30-1.71 (m, 10 H, 5 × C*H*₂), 0.90 (t, ³*J* = 7.2 Hz, 3 H, C*H*₃). - ¹³C-NMR (75 MHz, CDCl₃): δ = 146.3 (*C*=O), 137.2 (*C*ₐᵣH), 130.4 (*C*ₐᵣH), 117.2 (*C*ₐᵣH), 89.0 (C_{q}-*C*), 81.2 (C-C), 78.5 (Cq), 37.5 (2 × *C*H₂), 30.6 (*C*H₂), 25.0 (*C*H₂), 22.9 (2 × *C*H₂), 22.0 (*C*H₂), 18.5 (*C*H₂), 13.6 (*C*H₃). - FTIR: ṽ = 2934, 2860, 2240, 1763, 1467, 1378, 1283, 1234, 1167, 1092, 996, 893, 830, 765, 742, 649 cm⁻¹. - MS (ESI), *m*/*z* (%): 313 [(M+K)⁺], 297 [(M+Na)⁺]. - HRMS (C₁₆H₂₂N₂O₂Na): ber. 297.1579, gef. 297.1574.

### 1.16 9-Methylheptadecan-9-yl 1H-imidazole-1-carboxylat

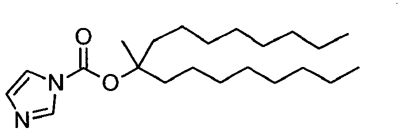

Die Synthese wurde ausgehend von 9-Methylheptadecan-9-ol (5.82 g, 21.5 mmol, 1.00 Äquiv.), 1,1-carbonyldiimidazol (5.45 g, 26.9 mmol, 1.10 Äquiv.) und Kaliumhydroxid (12.0 mg, 215 µmol, 0.01 Äquiv.) gemäß ***AAV 1*** durchgeführt. Nach säulenchromatographischer Reinigung (Kieselgel, Petrolether/ Essigsäureethylester 10:1) wurden 6.71 g (86%) des Imidazolesters als klare Flüssigkeit erhalten. - *R*_{f} = 0.09 (Petrolether/Essigsäureethylester 10:1). - ¹H-NMR (300 MHz, CDCl₃): δ = 8.05 (s, 1 H, *H*ₐᵣ), 7.35 (s, 1 H, *H*ₐᵣ), 7.03 (s, 1 H, *H*ₐᵣ), 1.79-1.99 (m, 4 H, 2 × C*H*₂), 1.56 (s, 3 H, C*H*₃), 1.23-1.36 (m, 24 H, 12 × C*H*₂), 0.87 (t, ³*J* = 6.7 Hz, 6 H, 2 × C*H*₃). - ¹³C-NMR (75 MHz, CDCl₃): δ = 147.1 (*C*=O), 137.2 (*C*ₐᵣH), 130.4 (*C*ₐᵣH), 117.2 (*C*ₐᵣH), 90.8 (Cq), 38.3 (2 × *C*H₂), 31.9 (2 × *C*H₂), 30.0 (2 × *C*H₂), 29.6 (2 × *C*H₂), 29.3 (2 × *C*H₂), 23.8 (2 × *C*H₂), 23.7 (*C*H₃), 22.8 (2 × *C*H₂), 14.2 (2 × C*H*₃). - FTIR: ṽ = 2925, 2854, 1753, 1466, 1380, 1317, 1281, 1237, 1187, 1091, 999, 835, 771, 742, 649 cm⁻¹. - MS (ESI), *m*/*z* (%): 730 [(M-H-M)⁺], 403 [(M+K)⁺], 387 [(M+Na)⁺]. - HRMS (C₂₂H₄₀N₂O₂Na): ber. 387.2987, gef. 387.2987.

### Synthese des Benzo[lmn][3,8]phenanthrolin-1,3,6,8-tetraon-4,9-diyl-Bausteins:

### 1.17 2,7-Dibromonaphthalen-1,8:4,5-tetracarbonsäure Dianhydrid:

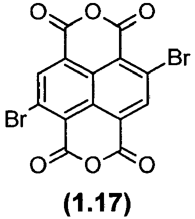

Naphthalen-1,4,5,8-tetracarboxyldianhydrid (20 mmol, 1 eq) wurde in konz. H₂SO₄ (97 %, 250 mL) gelöst. Dibromisocyanursäure (40 mmol, 2 eq) wurde zugegeben. Der Aufbau wurde mit Aluminiumfolie abgedeckt und bei Raumtemperatur für 7 Tage gerührt. Die Reaktionslösung wurde vorsichtig auf 1.5 kg Eis gegossen und für 1 Stunde bei Raumtemperatur gerührt. Die Suspension wurde für eine Stunde ruhen gelassen, der Niederschlag über Zentrifugation abgetrennt, jeweils dreimal mit Wasser und Methanol gewaschen und im Vakuum getrocknet. Der erhaltene Feststoff wurde in frischem Essigsäureanhydrid suspendiert und unter Argon für 5 Stunden bei 120 °C gerührt. Die Mischung wurde auf Raumtemperatur abgekühlt und über Nacht im Kühlschrank gelagert. Der Niederschlag wurde abfiltriert, mit Methanol gewaschen und im Vakuum getrocknet. Es wurden 4.80 g (56 %) eines gelben Feststoffes erhalten. Aus Derivatisierungsreaktionen ging hervor, dass dieses Rohprodukt etwa eine Stärke von 50 % hat.
IR (v in cm⁻¹): 1778 (s, O-C=O), 1747 (vs, O-C=O), 1568 (m). - MS (EI⁺), *m*/*z*: 268.0 [(C₁₄H₄O₆)⁺], 345.9 [(C₁₄H₃⁷⁹BrO₆)⁺], 423.8 [(C₁₄H₂⁷⁹Br₂O₆)⁺], 501.7 [(C₁₄H₁⁷⁹Br₃O₆)⁺].

### 1.18 2,7-Dibromonaphthalen-1,8:4,5-tetracarbonsäurebisimid-[N,N]-bis(hexan-6,1-diyl) Dipropionat

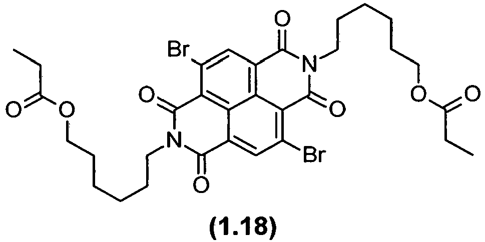

2,7-Dibromonaphthalen-1,8:4,5-tetracarbonsäure Dianhydrid (2.13 g Rohprodukt, 5 mmol) und 6-Aminohexanol (1.46 g, 12.5 mmol) wurden in 25 mL eines Gemisches aus Propionsäure und o-Xylen (1:1, v:v) suspendiert und unter über Nacht unter Rückfluss erhitzt. Die Reaktionsmischung wurde sehr langsam auf Raumtemperatur abgekühlt. Der Niederschlag wurde abfiltriert und mit Essigsäure gewaschen. Der erhaltene Feststoff wurde anschließend aus 30 mL Propionsäure:o-Xylene (1:1, v:v) umkristallisiert. Der Niederschlag wurde abfiltriert, mit Essigsäure und Methanol gewaschen und im Vakuum getrocknet. Das Rohprodukt wurde säulenchromatographisch an Kieselgel mit einem Gemisch aus Chloroform und Toluen (1:1, v:v) mit 3 % THF als Eluent gereinigt. Es wurden 1.13 g (30 % d.Th. für 100 % reinen Eduktes) des Produktes als gelber Feststoff erhalten. - *R*_{f} = 0.32 (Chloroform:Toluen (1:1) + 3 % THF). - ¹H-NMR (300 MHz, CDCl₃): δ = 8.99 (s, 2 H, 2 × C*H*ₐᵣ), 4.19 (t, ³*J* = 7.7 Hz, 4 H, 2 × N-C*H*₂), 4.06 (t, ³*J* = 6.6 Hz, 4 H, 2 × O-C*H*₂), 2.32 (q, ³*J* = 7.6 Hz, 4 H, (C=O)C*H*₂), 1.70-1.80 (m, 4 H, C*H*₂), 1.65 (m, 4 H, C*H*₂), 1.40-1.50 (m, 8 H, C*H*₂), 1.13 (t, ³*J* = 7.6 Hz, 6 H, C*H*₃). - ¹³C-NMR (75 MHz, CDCl₃): δ = 174.6 (2 × O-*C*=O), 160.7 (2 × N-*C*=O), 160.6 (2 × N-C=*O*), 139.1 (2 × *C*ₐᵣH), 128.3 (2 × *C_{q-ar}*), 127.7 (2 × *C_{q-ar}*), 125.3 (2 × *C*_{*q*-*ar*}), 124.0 (2 × *C*_{q-ar}), 64.2 (*C*H₂-O), 41.4 (*C*H₂-N), 28.5 (*C*H₂), 27.8 (*C*H₂), 27.6 ((C=O)-*C*H₂), 26.6 (*C*H₂), 25.6 (*C*H₂), 9.2 (*C*H₃). - HR-MS (DART, NH₄⁺-Addukt, [C₃₂H₄₀Br₂N₃O₈]⁺): ber. 752.1177, gef. 752.1166. - IR: (v in cm⁻¹): 1732 (s, O-C=O), 1701 (s, N-C=O), 1649 (vs, N-C=O), 1559 (m).

### 1.19 2,7-Bis(2'-thiophenyl)-naphthalen-1,8:4,5-tetracarbonsäurebisimid-[N,N]-bis(hexan-6,1-diyl) Dipropionat

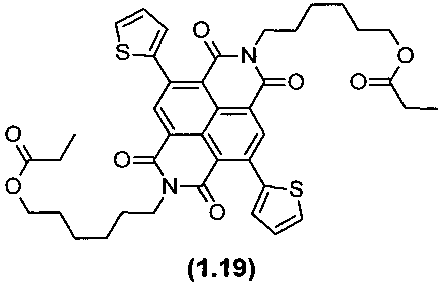

2,7-Dibromnaphthalen-1,8:4,5-tetracarbonsäurebisimid-[N,N]-bis(hexan-6,1-diyl) Dipropionat (368 mg, 0.5 mmol) und 2-(Tributylstannyl)thiophen (560 mg, 1.5mmol) wurden in 2.5 mL trockenem DMF gelöst und dreimal mittels Vakuumentgasung von Sauerstoff befreit.
Tetrakis(triphenylphosphine)palladium(0) (29 mg, 0.05 eq) wurde im Argongegenstrom zugegeben und die Reaktionslösung für 14 Stunden bei 100 °C gerührt. Die auf Raumtemperatur abgekühlte Reaktionslösung wurde mit 35 mL Methanol verdünnt und für 2 Stunden gerührt. Niederschläge wurden abfiltriert, mit MeOH gewaschen und im Vakuum getrocknet. Das Rohprodukt wurde säulenchromatographisch an Kieselgel mit einem Gemisch aus Chloroform und Toluen (1:1, v:v) mit 3 % THF als Eluent gereinigt. Es wurden 330 mg (90 % d.Th.) des Produktes als roter Feststoff erhalten.
- *R*_{f} = 0.33 (Chloroform:Toluen (1:1) + 3 % THF). - ¹H-NMR (300 MHz, CDCl₃): δ = 8.75 (s, 2 H, 2 × C*H*ₐᵣ), 7.58 (dd, ³*J* = 5.1 Hz, ⁴*J* = 1.2 Hz, 2 H, C*H*ₐᵣ), 7.29 (dd, ³*J* = 3.6 Hz, ⁴*J* = 1.2 Hz, 2 H, C*H*ₐᵣ), 7.21 (dd, ³*J* = 5.1 Hz, ³*J* = 3.6 Hz, 2 H, C*H*ₐᵣ), 4.11 (t, ³*J* = 7.7 Hz, 4 H, 2 × N-C*H*₂), 4.04 (t, ³*J* = 6.6 Hz, 4 H, 2 × O-C*H*₂), 2.32 (q, ³*J* = 7.6 Hz, 4 H, (C=O)C*H*₂), 1.58-1.73 (m, 8 H, C*H*₂), 1.36-1.43 (m, 8 H, C*H*₂), 1.12 (t, ³*J* = 7.6 Hz, 6 H, C*H*₃). - ¹³C-NMR (75 MHz, CDCl₃): δ = 174.6 (2 × O-*C*=O), 162.1 (2 × N-*C*=O), 161.9 (2 × N-*C*=O), 140.6 (2 x *C*_{q}-S), 140.3 (2 x C_{q}), 136.7 (2 × *C*ₐᵣH), 128.2 (2 × S*-CHₐᵣ*), 128.0 (2 × *Cₐᵣ*H), 127.5 (2 x C_{q}), 127.4 (2 × *Cₐᵣ*H), 125.3 (2 × (C=O)-*C*_{q-ar}), 123.4 (2 × (C=O)-*C*_{q-ar}), 64.2 (*C*H₂-O), 40.9 (*C*H₂-N), 28.5 (*C*H₂), 27.8 (*C*H₂), 27.6 ((C=O)-*C*H₂), 26.6 (*C*H₂), 25.6 (*C*H₂), 9.1 (*C*H₃). - HR-MS (DART, NH₄⁺-Addukt, [C₄₀H₄₆N₃O₈S₂]⁺): ber. 760.2721, gef. 760.2723.

### 1.20 2,7-Bis(4'-hexyl-2'-thiophenyl)-naphthalen-1,8:4,5-tetracarbonsäurebisimid-[N,N]-bis(hexan-6,1-diyl) Dipropionat

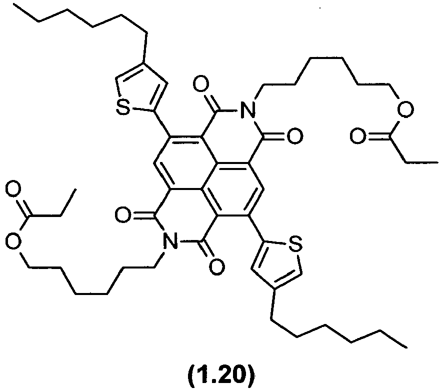

2,7-Dibromonaphthalen-1,8:4,5-tetracarbonsäurebisimid-[N,N]-bis(hexan-6,1-diyl) Dipropionat (368 mg, 0.5 mmol) und 2-(Tributylstannyl)-4-hexylthiophen (1.2 g, 5 mmol, 60 % Stärke) wurden in 5 mL trockenem DMF gelöst und dreimal mittels Vakuumentgasung von Sauerstoff befreit. Tetrakis(triphenylphosphin)palladium(0) (46 mg, 0.08 eq) wurde im Argongegenstrom zugegeben und die Reaktionslösung für 2 Stunden bei 100 °C gerührt. Die auf Raumtemperatur abgekühlte Reaktionslösung wurde mit 50 mL Methanol verdünnt, 3 Stunden lang intensiv gerührt und anschließend über Nacht im Kühlschrank gelagert. Niederschläge wurden abfiltriert, mit MeOH gewaschen und im Vakuum getrocknet. Das Rohprodukt wurde säulenchromatographisch an Kieselgel mit einem Gemisch aus Chloroform und Toluen (1:1, v:v) mit 3 % THF als Eluent gereinigt. Es wurden 349 mg (77 % d.Th.) des Produktes als roter Feststoff erhalten.
- *R*_{f} = 0.35 (Chloroform:Toluen (1:1) + 3 % THF). - ¹H-NMR (300 MHz, CDCl₃): δ = 8.74 (s, 2 H, 2 × C*H*ₐᵣ), 7.16 (s, 2 H, C*H*ₐᵣ), 7.13 (s, 2 H, C*H*ₐᵣ), 4.11 (t, ³*J* = 7.7 Hz, 4 H, 2 × N-C*H*₂), 4.04 (t, ³*J* = 6.6 Hz, 4 H, 2 × O-C*H*₂), 2.69 (t, ³*J* = 7.7 Hz, 4 H, 2 × C_{q}-C*H*₂), 2.30 (q, ³*J* = 7.6 Hz, 4 H, (C=O)C*H*₂), 1.66-1.72 (m, 8 H, C*H*₂), 1.59-1.64 (m, 6 H, C*H*₂), 1.36-1.43 (m, 10 H, C*H*₂), 1.30-1.36 (m, 8 H, C*H*₂), 1.12 (t, ³*J* = 7.6 Hz, 6 H, C*H*₃), 0.91 (t, ³*J* = 6.9 Hz, 6 H, C*H*₃). ¹³C-NMR (75 MHz, CDCl₃): δ = 174.6 (2 × O-*C*=*O*), 162.2 (2 × N-*C*=*O*), 162.0 (2 × N-*C*=*O*), 143.7 (2 x C_{q}), 140.6 (2 x C_{q}-S), 140.3 (2 x C_{q}), 136.7 (2 × *C*Hₐᵣ), 129.7 (2 × *C*H*ₐᵣ*), 127.3 (2 x C_{q}), 125.2 (2 × (C=O)-C_{q-ar}), 123.0 (2 × (C=O)-C_{q-ar}), 122.8 (2 × *C*H*ₐᵣ*), 64.2 (*C*H₂-O), 40.9 (*C*H₂-N), 31.7 (C_{q}-*C*H₂), 30.5 (*C*H₂), 30.3 (*C*H₂), 29.0 (*C*H₂), 28.5 (*C*H₂), 27.8 (*C*H₂), 27.6 ((C=O)-*C*H₂), 26.6 (*C*H₂), 25.6 (*C*H₂), 22.6 (*C*H₂), 14.1 (*C*H₃), 9.1 (*C*H₃). - HR-MS (ESI, Na⁺-Addukt, [C₅₂H₆₆N₂NaO₈S₂]⁺): ber. 933.4152, gef. 933.4160.

### 1.21 2,7-Bis(5'-bromo-2'-thiophenyl)-naphthalen-1,8:4,5-tetracarbonsäurebisimid-[N,N]-bis(hexan-6,1-diyl) Dipropionat

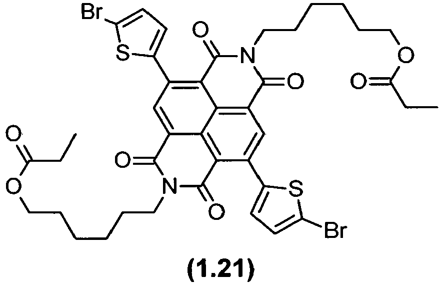

2,7-Bis(2'-thiophenyl)-naphthalen-1,8:4,5-tetracarbonsäurebisimid-[N,N]-bis(hexan-6,1-diyl) Dipropionat (330 mg, 0.44 mmol) wurden in einem trockenen 50-mL Schlenkkolben vorgelegt und eine Argonatmosphäre erzeugt. 22 mL trockenes N,N-Dimethylformamid und N-Bromsuccinimid (190 mg, 1.07 mmol) wurden hinzugefügt und das Reaktionsgemisch 5 Tage lang bei Raumtemperatur im dunkelen gerührt. Das Lösungsmittel wurde anschließend im Vakuum entfernt und der Rückstand in 5 mL Chlorofom gelöst. 30 mL MeOH wurde hinzugefügt und die Mischung für 3 Stunden stark gerührt. Niederschläge wurde abfiltriert, mit MeOH gewaschen und im Vakuum getrocknet. Das Rohprodukt wurde säulenchromatographisch an Kieselgel mit einem Gemisch aus Chloroform und Toluen (1:1, v:v) mit 2.5 % THF als Eluent gereinigt. Es wurden 372 mg (93 % d.Th.) des Produktes als roter Feststoff erhalten. - *R*_{f} = 0.30 (Chloroform:Toluen (1:1) + 3 % THF). - ¹H-NMR (300 MHz, CDCl₃): δ = 8.71 (s, 2 H, 2 × C*H*ₐᵣ), 7.16 (d, ³*J* = 3.8 Hz, 2 H, C*H*ₐᵣ), 7.07 (d, ³*J* = 3.8 Hz, 2 H, C*H*ₐᵣ), 4.12 (t, ³*J* = 7.7 Hz, 4 H, 2 × N-C*H*₂), 4.05 (t, ³*J* = 6.6 Hz, 4 H, 2 × O-C*H*₂), 2.30 (q, ³*J* = 7.6 Hz, 4 H, (C=O)C*H*₂), 1.58-1.73 (m, 8 H, C*H*₂), 1.36-1.43 (m, 8 H, C*H*₂), 1.12 (t, ³*J* = 7.6 Hz, 6 H, C*H*₃). - ¹³C-NMR (75 MHz, CDCl₃): δ = 174.6 (2 × O-*C*=*O*), 161.9 (2 × N-*C*=O), 161.8 (2 × N-*C*=O), 141.9 (2 x *C*_{q}-S), 139.1 (2 x C_{q}), 136.5 (2 × *C*ₐᵣH), 130.2 (2 × S-C*Hₐᵣ*), 128.8 (2 × *Cₐᵣ*H), 127.5 (2 x C_{q}), 125.6 (2 × (C=O)-*C*_{q-ar}), 123.2 (2 × (C=O)-*C*_{q-ar}), 115.4 (*C*_{q-ar}Br), 64.2 (*C*H₂-O), 41.0 (*C*H₂-N), 28.5 (*C*H₂), 27.8 (*C*H₂), 27.6 ((C=O)-*C*H₂), 26.6 (*C*H₂), 25.6 (*C*H₂), 9.1 (*C*H₃). - HR-MS (DART, NH₄⁺-Addukt, [C₄₀H₄₄Br₂N₃O₈S₂]⁺): ber. 916.0931, gef. 916.0915.

### 1.22 2,7-Bis(5'-bromo-4'-hexyl-2'-thiophenyl)-naphthalen-1,8:4,5-tetracarbonsäurebisimid-[N,N]-bis(hexan-6,1-diyl)Dipropionat

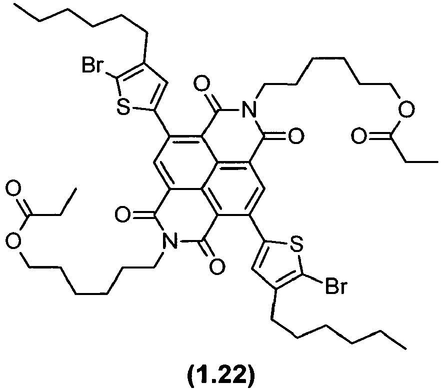

2,7-Bis(4'-hexyl-2'-thiophenyl)-naphthalen-1,8:4,5-tetracarbonsäurebisimid-[N,N]-bis(hexan-6,1-diyl) Dipropionat (328 mg, 0.36 mmol) und N-Bromsuccinimid (225 mg, 1.44 mmol) wurden in einem trockenen 25-mL Schlenkkolben vorgelegt und eine Argonatmosphäre erzeugt. 15 mL trockenes Tetrahydrofuran wurden hinzugefügt und das Reaktionsgemisch 4 Tage lang bei Raumtemperatur im dunkelen gerührt. Das Lösungsmittel wurde anschließend im Vakuum entfernt, der Rückstand in 5 mL Dichlormethan gelöst, auf eine kurze mit DCM equilibrierte Silicagelsäule aufgetragen und vorsichtig mit reinem Dichlormethan gewaschen. Das Produkt wurde mit einem Lösungsmittelgemisch von Dichlormethan und Tetrahydrofuran (100:2, v:v) eluiert. Dunkelrote Fraktionen wurden vereiningt und das Lösungsmittel entfernt. Das so erhaltene Rohprodukt wurde zweimal aus MeOH umkristallisiert. Es wurden 375 mg (97 % d.Th.) des Produktes als violetter Feststoff erhalten. - *R*_{f} = 0.37 (Chloroform:Toluen (1:1) + 3 % THF). - ¹H-NMR (300 MHz, CDCl₃): δ = 8.71 (s, 2 H, 2 × C*H*ₐᵣ), 7.03 (s, 2 H, C*H*ₐᵣ), 4.12 (t, ³*J* = 7.7 Hz, 4 H, 2 × N-C*H*₂), 4.05 (t, ³*J* = 6.6 Hz, 4 H, 2 × O-C*H*₂), 2.64 (t, ³*J* = 7.7 Hz, 4 H, 2 × C_{q}-C*H*₂), 2.30 (q, ³*J* = 7.6 Hz, 4 H, (C=O)C*H*₂), 1.60-1.71 (m, 14 H, C*H*₂), 1.41 (s, 10 H, C*H*₂), 1.34 (m, 8 H, C*H*₂), 1.12 (t, ³*J* = 7.6 Hz, 6 H, C*H*₃), 0.91 (t, ³*J* = 6.9 Hz, 6 H, C*H*₃). - ¹³C-NMR (75 MHz, CDCl₃): δ = 174.6 (2 × O-*C*=*O*), 162.1 (2 × N-*C*=O), 162.0 (2 × N-*C*=O), 142.5 (2 x C_{q}), 140.0 (2 x C_{q}), 139.5 (2 x C_{q}), 136.4 (2 × *C*Hₐᵣ), 129.6 (2 × *C*H*ₐᵣ*), 127.4 (2 x C_{q}), 125.4 (2 × (C=O)-*C*_{q-ar}), 122.9 (2 × (C=O)-*C*_{q}), 112.5 (2 × *C_{q-ar}*Br), 64.2 (*C*H₂-O), 41.0 (*C*H₂-N), 31.6 (C_{q}-*C*H₂), 29.7 (*C*H₂), 29.0 (*C*H₂), 28.5 (*C*H₂), 27.8 (*C*H₂), 27.6 ((C=O)-*C*H₂), 26.6 (*C*H₂), 25.6 (*C*H₂), 22.6 (*C*H₂), 14.1 (*C*H₃), 9.1 (*C*H₃). - HR-MS (ESI, Na⁺-Addukt, [C₅₂H₆₄Br₂N₂NaO₈S₂]⁺): ber. 1089.2363, gef. 1089.2371.

### 1.23 2,7-Bis(5'-bromo-2'-thiophenyl)-[N,N]-bis(hexan-1-yl-6-ol)-naphthalen-1,8:4,5-tetracarbonsäurebisimid

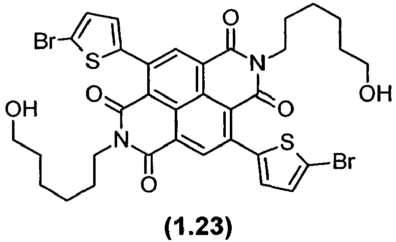

2,7-Bis(5'-bromo-4'-hexyl-2'-thiophenyl)-naphthalen-1,8:4,5-tetracarbonsäurebisimid-[N,N]-bis(hexan-6,1-diyl) Dipropionat (360 mg, 0.4 mmol) wurde in einer Mischung aus 16 mL Chloroform und 4 mL MeOH gelöst und mit 100 µL konzentrierter Salzsäure versetzt. Die Apparatur wurde entgast und unter Argon für 18 Stunden unter Rückfluss erhitzt, anschließend langsam auf Raumtemperatur abgekühlt und für 3 Stunden im Kühlschrank gelagert. Der Niederschlag wurde isoliert und nacheinander mit einer kalten Mischung aus Chlorofrom und Methanol (1:1, v:v) und reinem Methanol gewaschen. Nach Trocknung im Vakuum wurden 276 mg eines violetten Feststoffes erhalten, der aufgrund seiner Unlöslichkeit nicht weiter aufgereinigt wurde (88 % d. Theorie). - Smp.: 226 °C. - IR: (v in cm⁻¹): 3100 - 3300 (vbs, OH), 1698 (s, N-C=O), 1659 (vs, N-C=O), 1571 (m). - HR-MS (MALDI, DCTB-Matrix, [C₃₄H₃₃Br₂N₂O₆S₂]⁺: ber. 787.01413, gef. 787.01419.

### 1.24 2,7-Bis(5'-bromo-4'-hexyl-2'-thiophenyl)-[N,N]-bis(hexan-1-yl-6-ol)-naphthalen-1,8:4,5-tetracarbonsäurebisimid

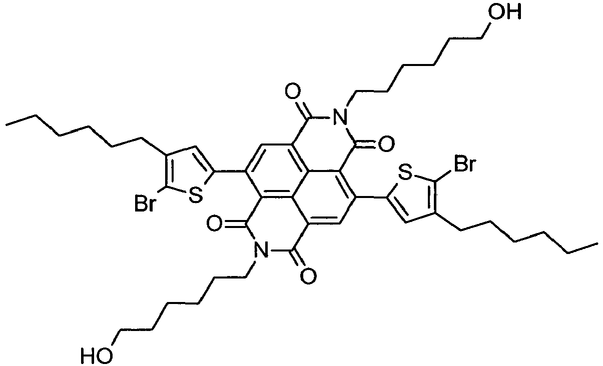

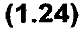

2,7-Bis(5'-bromo-4'-hexyl-2'-thiophenyl)-naphthalen-1,8:4,5-tetracarbonsäurebisimid-[N,N]-bis(hexan-6,1-diyl) Dipropionat (374 mg, 0.35 mmol) wurde in einer Mischung aus 16 mL Chloroform und 4 mL MeOH gelöst und mit 100 µL konzentrierter Salzsäure versetzt. Die Apparatur wurde entgast, unter Argon für 8 Stunden unter Rückfluss erhitzt und anschließend langsam auf Raumtemperatur abgekühlt. Das Lösungsmittel wurde im Vakuum entfernt. Der Rückstand wurde in 15 mL MeOH suspendiert, der Feststoff abfiltriert und mit MeOH gewaschen. Der violette Feststoff wurde in Chloroform wieder angelöst und das Lösungsmittel im Vakuum entfernt. Das Rohprodukt wurde säulenchromatographisch an Kieselgel mit einem Gemisch aus Chloroform und Toluen (1:1, v:v) mit 5 % Ethanol als Eluent gereinigt. Es wurden 330 mg (99 % d.Th.) des Produktes als violetter Feststoff erhalten. - *R*_{f} = 0.14 (Chloroform:Toluen (1:1) + 5 % EtOH). - ¹H-NMR (300 MHz, CDCl₃): δ = 8.70 (s, 2 H, 2 × C*H*ₐᵣ), 7.03 (s, 2 H, C*H*ₐᵣ), 4.12 (t, ³*J* = 7.7 Hz, 4 H, 2 × N-C*H*₂), 3.63 (t, ³*J* = 6.6 Hz, 4 H, 2 × O-C*H*₂), 2.64 (t, ³*J* = 7.7 Hz, 4 H, 2 × C_{q}-C*H*₂), 1.60-1.73 (m, 8 H, C*H*₂), 1.57 (m, 4 H, C*H*₂), 1.37-1.44 (m, 12 H, C*H*₂), 1.32-1.37 (m, 10 H, C*H*₂), 0.91 (t, ³*J* = 6.9 Hz, 6 H, C*H*₃). - ¹³C-NMR (75 MHz, CDCl₃): δ = 162.1 (2 × N-*C*=O), 162.0 (2 × N-*C*=O), 142.5 (2 x C_{q}), 140.0 (2 x C_{q}), 139.5 (2 x C_{q}), 136.4 (2 × *C*Hₐᵣ), 129.6 (2 × *C*H*ₐᵣ*), 127.4 (2 x C_{q}), 125.4 (2 × (C=O)-*C*_{q-ar}), 122.9 (2 × (C=O)-*C*_{q}), 112.5 (2 × *C_{q-ar}*Br), 62.8 (*C*H₂-O), 41.0 (*C*H₂-N), 32.6 (*C*H₂), 31.6 (C_{q}-*C*H₂), 29.7 (*C*H₂), 29.0 (*C*H₂), 27.9 (*C*H₂), 26.7 (*C*H₂), 25.3 (*C*H₂), 22.6 (*C*H₂), 14.1 (*C*H₃). - HR-MS (ESI, H⁺-Addukt, [C₄₆H₅₇Br₂N₂O₆S₂]⁺: ber. 955.2019, gef. 955.2025.

### 1.25 Bis(2-methylhexan-2-yl) Dicarbonat

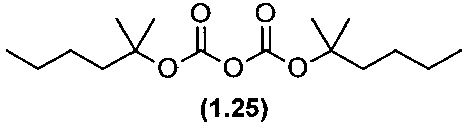

Zu einer Lösung von 2-Methyl-2-hexanol (15.1 g, 1.0 eq, 130 mmol) in 260 mL eines Gemisches aus trockenem Toluen und Tetrahydrofuran im Verhältnis 2:1 (v:v) wurde unter Eiskühlung im Schutzgasgegenstrom schrittweise 3.14 g pulverförmiges Natriumhydrid (1.05 eq, 136 mmol) gegeben. Die Reaktionsmischung wurde für 18 Stunden unter Rückfluss erhitzt, wobei sich ein Großteil des Feststoffes löste. Nach Abkühlen auf Raumtemperatur wurde die leicht trübe Lösung wurde auf -78 °C gekühlt und langsam unter Schutzgas auf einen großen Überschuss festes CO₂ bei ca. -100°C transferiert. Die Reaktionsmischung wurde langsam auf Raumtemperatur erwärmt. Weitere 2 Stunden wurde trockenes CO₂ durch das dickflüssige Reaktionsgemisch geleitet. Anschließend wurde die Reaktionsmischung wieder auf 0 °C gekühlt und katalytische Mengen DMF (51 µL, 0.005 eq, 0.6 mmol) zugegeben, gefolgt von Oxalylchlorid (5.6 mL, 0.5 eq, 65 mmol) und katalytischen Mengen an Benzyltrimethylammoniumchlorid (338 mg, 0.014 eq, 1.8 mmol) und Pyridin (315 µL, 0.03 eq, 3.9 mmol). Nach dem Ende der Gasentwicklung wurde die Reaktionsmischung auf Raumtemperatur erwärmt und für 2 Tage gerührt. Anschließend wurden 35 mL einer einprozentigen wässrigen Schwefelsäurelösung zugegeben. Die organische Phase wurde abgetrennt und mit Wasser gewaschen. Die wässrige Phase wurde dreimal mit Diethylether extrahiert. Die vereinigten organischen Extrakte wurden vereinigt, mit gesättigter Kochsalzlösung und MgSO₄ getrocknet und eingeengt. Das Rohprodukt wurde säulenchromatographisch an Silica mit einem Eluentengemisch aus Petrolether und Ethylacetat 20:1 (v:v) aufgereiningt. Es wurden 7.01 g (37 % d.Th.) des gewünschten Produktes als farbloses Öl gehalten. - Rf = 0.45 (Petrolether:Ethylacetat 20:1). -¹H-NMR (300 MHz, CDCl3): δ = 1.82-1.75 (m, 4H, C_{q}-C*H*₂), 1.50 (s, 12H, C_{q}-C*H*₃), 1.36-1.28 (m, 8H, C*H*₂), 0.91 (t, 6H, Cₛ-C*H*₃). - ¹³C-NMR (75 MHz, CDCl3): δ = 146.7 (O-(C=O)-O), 87.5 (C_{q}), 40.0 (*C*_{q}-CH₃), 25.6 (*C*H₂), 25.2 (*C*H₂), 22.8 (*C*H₂), 13.9 (*C*H₃). - Elementaranalyse: Theorie: C: 63.55, H: 10.00; gefunden: C: 63.80, H: 9.98.

### 1.26 Tetrabutyl-2,7-bis(4'-hexylthiophen-2'-yl)naphthalen-1,4,5,8-tetracarboxylat

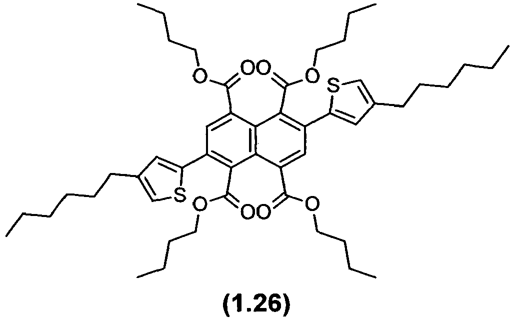

Tetrabutyl-2,7-dibromonaphthalen-1,4,5,8-tetracarboxylat (1.506 g, 2.2 mmol, 1.00 eq) und 4-Hexyl-2-(tributylstannyl)thiophen (3.02 g, 6.6 mmol, 3 eq) wurden in 12 mL trockenem N,N-Dimethylformamid gelöst und dreimal mit der *freeze-pump-thaw*-Methode entgast. Tetrakis(triphenylphospin)palladium (114 mg, 0.1 mmol, 0.045 eq) wurde zugegeben und die Reaktionsmischung unter Schutzgas für fünf Stunden bei 105 °C gerührt. Die Mischung wurde mit 45 mL einer 20-prozentigen wässrigen Kaliumfluorid-Lösung versetzt und die wässrige Phase dreimal mit je 40 mL Methyl-tert.-butylether extrahiert. Die vereinigten organischen Extrakte wurden zweimal mit Wasser gewaschen, mit gesättigter Kochsalzlösung und MgSO₄ getrocknet und eingeengt. Das Rohprodukt wurde säulenchromatographisch an Silica mit einem Eluentengemisch aus Petrolether und Dichlormethan im Verhältnis 9:1 -> 1:1 -> 1:5 (v:v) aufgereinigt. Es wurden 1.53 g (72 % d.Th.) der Zielsubstanz als viskoses gelbfarbenes Öl erhalten. - Rf = 0.34 (Petrolether/Ethylacetat 15:1) - ¹H-NMR (300.51 MHz, CDCl3): δ [ppm]= 7.95 (s, 2H, Hₐᵣ), 7.01 (d, ⁴J = 1.1 Hz, 2H, Hₐᵣ), 6.89 (d, ⁴J = 1.3 Hz, 2H, Hₐᵣ), 4.29 (t, ³J = 6.8 Hz, 4H, C*H*₂), 4.02 (t, ³J = 6.8 Hz, 4H, CH₂), 2.59 (t, ³J = 7.8 Hz, 4H, CH₂), 1.77 (p, ³J = 7.8 Hz, 4H, CH₂), 1.61-1.67 (m, 4H, CH₂), 1.26-1.53 (m, 20H, CH₂), 1.16 (m, 4H, CH₂) 0.97 (t, ³J = 7.3 Hz, 6H, CH₃), 0.92 (t, ³J = 8.0 Hz, 6H, CH₃), 0.82 (t, ³J = 7.3 Hz, 6H, CH3) - APCI⁺-MS: [M-BuO⁻]⁺ calc. m/z = 787.37, found: 787.32.

### 1.27 Tetrabutyl 2,7-bis(5'-bromo-4'-hexylthiophen-2'-yl)naphthalen-1,4,5,8-tetracarboxylat

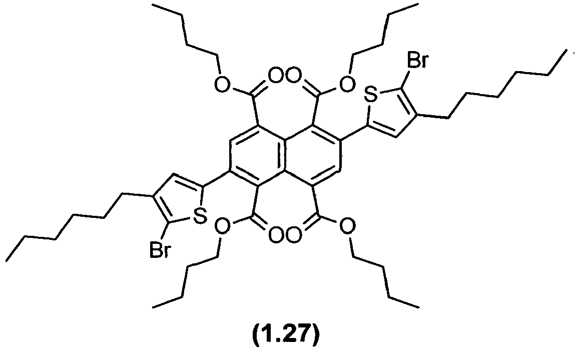

Zu einer Lösung von 1.52 g Tetrabutyl-2,7-bis(4'-hexylthiophen-2'-yl) naphthalen-1,4,5,8-tetracarboxylat (1.77 mmol, 1.00 eq) in 95 mL trockenem Tetrahydrofuran wurden 1.26 g N-Bromsuccinimid (7.08 mmol, 4.00 eq) unter Schutzgas gegeben. Die Reaktionslösung wurde zwei Tage unter Lichtausschluss gerührt. Das Lösungsmittel wurde unter vermindertem Druck entfernt und das Rohprodukt säulenchromatographisch an Silica mit einem Eluentengemisch aus Petrolether und Dichlormethan im Verhältnis 9:1 -> 1:1 -> 1:5 (v:v) aufgereinigt. Es wurden 1.80 g (99 % d.Th.) der Zielsubstanz als gelbfarbener Feststoff erhalten. - Rf = 0.35 (Petrolether:Ethylacetat 15:1) - ¹H-NMR (300.51 MHz, CDCl3): δ [ppm]= 7.95 (s, 2H, *H*ₐᵣ), 7.01 (d, ⁴J = 1.1 Hz, 2H, *H*ₐᵣ), 6.89 (d, ⁴J = 1.3 Hz, 2H, *H*ₐᵣ), 4.29 (t, ³J = 6.8 Hz, 4H, C*H*₂), 4.02 (t, ³J = 6.8 Hz, 4H, C*H*₂), 2.59 (t, ³J = 7.8 Hz, 4H, C*H*₂), 1.77 (p, ³J = 7.8 Hz, 4H, C*H*₂), 1.61-1.67 (m, 4H, C*H*₂), 1.26-1.53 (m, 20H, C*H*₂), 1.16 (m, 4H, C*H*₂) 0.97 (t, ³J = 7.3 Hz, 6H, C*H*₃), 0.92 (t, ³J = 8.0 Hz, 6H, C*H*₃), 0.82 (t, ³J = 7.3 Hz, 6H, C*H*₃). - APCI+-MS: [M-BuO⁻]⁺, C₄₆H₅₇Br₂O₇S₂⁺, berechnet: m/z = 943.19 : 945.19 : 947.19 (1:2:1), gefunden: 943.12 : 945.08 : 947.03 (1:2:1).

### 1.28 2,7-bis(5'-bromo-4'-hexylthiophen-2'-yl)naphthalen-1,4,5,8-tetracarbonsäuredianhydrid

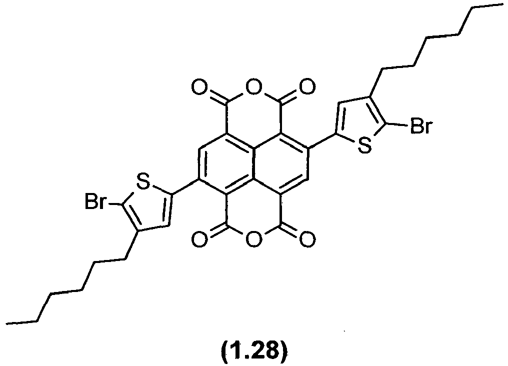

Tetrabutyl 2,7-bis(5'-bromo-4'-hexylthiophen-2'-yl)naphthalen-1,4,5,8-tetracarboxylat (1.80 g, 1.77 mmol, 1.00 eq) wurde in 72 mL Ethanol aufgeschlemmt und eine Lösung von 1.49 g Natriumhydroxid (15 mmol, 21.0 eq) in 4 mL Wasser zugegeben. Die Reaktionsapparatur wurde mit Schutzgas gespült und für 20 Stunden unter Rückfluss erhitzt. Nach dem Erkalten auf Raumtemperatur wurde auf 0°C gekühlt, 4.5 mL konzentrierte Salzsäure zugegeben und für weitere 20 Minuten gerührt. Das Reaktionsgemisch wurde im Vakuum eingeengt und der Rückstand in Petrolether aufgeschlemmt. Der anfallende Feststoff wurde durch Zentrifugation abgetrennt, noch zweimal mit Petrolether gewaschen und schließlich in 50 mL Aceton aufgenommen. Der zurückbleibende farblose Feststoff wurde durch Filtration abgetrennt, mit weiteren 100 mL Aceton gewaschen und das Filtrat aufgefangen. Nach Entfernen des Lösungsmittels wurde ein rot-brauner Feststoff erhalten, im Vakuum getrocknet und in einen 100 mL Kolben überführt. Unter Schutzgasathmosphäre wurden 35 mL Essigsäureanhydrid zugegeben und die Suspension für 5 Stunden bei 110 °C gerührt. Nach Abkühlen auf Raumtemperatur wurde das Reaktionsgemisch über Nacht im Kühlschrank bei 6 °C gelagert. Der Feststoff wurde abgetrennt und mit je 50 mL Essigsäureanhydrid und mL Methyl-tert.-butylether gewaschen. Der Rückstand wurde im Vakuum getrocknet und 966 mg (72 % d .Th.) des gewünschten Produktes als tiefvioletter Feststoff erhalten. - ¹H-NMR (600.24 MHz, o-C₆D₄Cl₂): δ [ppm]= 8.60 (s, 2 H, C*H*ₐᵣ), 7.28 (s, 2 H, C*H*ₐᵣ), 2.56 (t, ³J = 7.7 Hz, 4 H, C*H₂*), 1.57 (m, 4 H, C*H₂*), 1.37 (m, 4 H, C*H₂*), 1.28-1.19 (m, 8 H, C*H₂*), 0.86 (t, 6 H, C*H*₃) - ¹³C-NMR (150.9 MHz, o-C₆D₄Cl₂): 158.1 (C=O), 157.0 (C=O), 143.9 (C_{q}), 140.8 (C_{q}), 137.9 (C_{q}), 137.5 (*C*Hₐᵣ), 132.2 (*C*Hₐᵣ), 129.1 (C_{q}), 122.9 (C_{q}), 119.5 (C_{q}), 115.2 (C_{q}), 32.1 (*C*H₂), 30.1 (*C*H₂), 30.0 (*C*H₂), 29.4 (*C*H₂), 23.1 (*C*H₂), 14.5 (*C*H₃)-HR-FAB-MS: [M+H]⁺ C₃₄H₃₁O₆S₂⁷⁹Br₂, calc: m/z = 756.9923, found: m/z = 756.9929 - FTIR: v [cm⁻¹] = 2950 (w, C*H₂*), 2920 (m, C*H₂*), 2848 (w, C*H₂*), 1767 (s, O(*C*=O)₂), 1729 (s, O(*C*=O)₂), 1567 (m).

### 1.29 2,7-Bis(5'-bromo-4'-hexylthiophen-2'-yl)naphthalen-1,4,5,8-tetracarbonsäurediimid

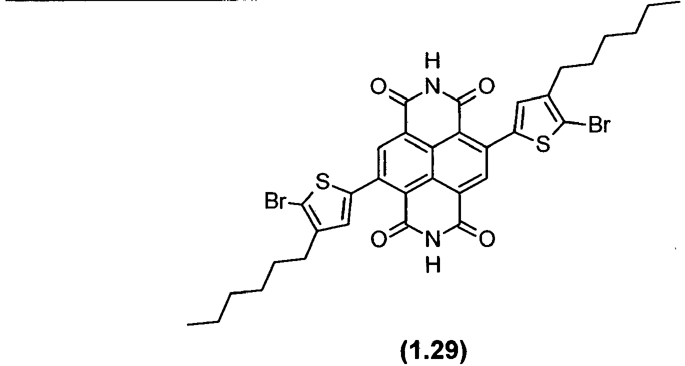

2,7-Bis(5'-bromo-4'-hexylthiophen-2'-yl)naphthalen-1,4,5,8-tetracarbonsäuredianhydrid (0.933 g, 1.23 mmol, 1.0 eq) und trockenes Ammoniumacetat (1.89 g, 24.6 mmol, 20 eq) wurden mit 13 mL Eisessig versetzt, entgast und für 2 Stunden unter Rückfluss erhitzt. Nach Erkalten wurde das Reaktionsgemisch über Nacht im Kühlschrank bei 6 °C gelagert. Das dickflüssige Reaktionsgemisch wurde mit 10 mL Essigsäure verdünnt und der Feststoff über Zentrifugation abgetrennt. Der so erhaltene Feststoff wurde je zweimal mit je 35 mL Essigsäure, Methyl-tert.-butylether und Dichlormethan gewaschen. Nach Trocknung wurden 865 mg (93 % d.Th.) des gewünschten Produktes als tief violetter Feststoff erhalten. - HR-MALDI-MS: Matrix: DCTB, [M+H]⁺, C₃₄H₃₃N₂O₄S₂⁷⁹Br₂, ber.: m/z = 757.0223, gefunden: m/z = 757.0233 - FT-IR: v [cm⁻¹] = 3171 (m, N-H), 3061 (m, N-H), 2951 (w, CH2) 2925 (m, CH2), 2852 (w, CH2), 1703 (s, O(*C*=O)₂), 1674 (s, O(*C*=O)₂), 1576 (m).

### Beispiel 2 - Monomere:

### 2.1 4,7-Bis(4,4,5,5-tetramethyl-1,3,2-dioxaborolan-2-yl)benzo[c][1,2,5]thiadiazol

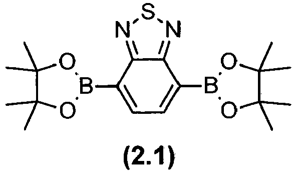

[1,1-Bis(diphenylphosphin)ferrocen] dichlorpalladium(II) (307 mg, 420 µmol, 0.02 Äquiv.) wurde zu einer Mischung aus 4,7-Dibrombenzo[c][1,2,5]thiadiazol (6.17 g, 22.0 mmol, 1.00 Äquiv.), Bis(pinacolato)diboron (11.7 g, 46.2 mmol, 2.20 Äquiv.) und Kaliumacetat (12.4 g, 126 mmol, 6.00 Äquiv.) in 1,4-Dioxan (100 mL) gegeben. Das Reaktionsgemisch wurde bei 80 °C für 18 h gerührt. Nachdem Abkühlen auf Raumtemnperatur wurde das Reaktionsgemisch auf Wasser (30 mL) gegeben und mit Essigsäureethylester (50 mL) extrahiert. Die vereinigten organischen Phasen wurden mit gesättigter Natriumchlorid-Lösung (50 mL) gewaschen, über Magnesiumsulfat getrocknet und das Lösungsmittel unter vermindertem Druck entfernt. Das Rohprodukt wurde säulenchromatographisch an Kieselgel mit Petrolether/Essigsäureethylester (1:1) als Eluent gereinigt. Es wurden 4.05 g (50%) des Produktes als gelber Feststoff erhalten. - *R*_{f} = 0.80 (Petrolether/Essigsäureethylester 1:1). - ¹H-NMR (300 MHz, CDCl₃): δ = 8.12 (s, 2 H, *H*ₐᵣ), 1.44 (s, 24 H, 8 × C*H*₃). - ¹³C-NMR (75 MHz, CDCl₃): δ = 157.1 (2 × C-N), 137.9 (2 × *C*ₐᵣH), 84.5 (4 × C_{q}), 25.0 (8 × *C*H₃). - MS (EI), *m*/*z* (%): 388 (23) [M⁺], 330 (100) [(C₁₈H₂₈B₂O₄)⁺]. - HRMS (C₁₄H₁₈BrIO₃S): ber. 388.1799, gef. 388.1823.

### Allgemeine Arbeitsvorschrift zur Darstellung der Carbonate (AAV 2):

Eine Lösung des entsprechenden Alkohols (1.00 Äquiv.) in Tetrahydrofuran wurde langsam bei 60 °C zu einer Lösung aus dem entsprechenden Imidazolester (1.10 Äquiv.) und Kaliumhydroxid (0.01 Äquiv.) in Tetrahydrofuran (4.0 mL/mmol Alkohol) getropft und bei 60 °C über 18 h gerührt. Das Lösungsmittel wurde unter vermindertem Druck entfernt und der übriggebliebene Rest mit Dichlormethan (50 mL) wieder aufgenommen. Die organische Phase wurde dreimal mit je 50 mL Wasser gewaschen, über Magnesiumsulfat getrocknet und das Lösungsmittel wurde unter vermindertem Druck entfernt. Das Rohprodukt wurde säulenchromatographisch an Kieselgel mit Petrolether/Essigsäureethylester-Gemischen als Eluent gereinigt.

### 2.2 2-(2-Brom-5-iodthiophen-3-yl)ethyl (2-methylhexan-2-yl) carbonat

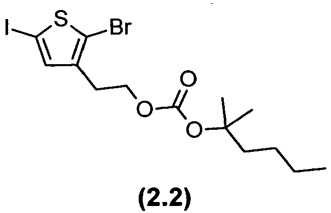

Die Synthese wurde ausgehend von 2-(2-Brom-5-iodthiophen-3-yl)ethanol (1.00 g, 3.00 mmol, 1.00 Äquiv.), 2-Methylhexan-2-yl 1*H*-imidazol-1-carboxylat (694 mg, 3.30 mmol, 1.10 Äquiv.) und Kaliumhydroxid (2.00 mg, 30.0 µmol, 0.01 Äquiv.) gemäß ***AAV 2*** durchgeführt. Nach säulenchromatographischer Reinigung (Kieselgel, Petrolether/ Essigsäureethylester 10:1) wurden 1.23 g (86%) des Carbonates als klares Öl erhalten. - *R*_{f} = 0.55 (Petrolether/Essigsäureethylester 5:1). - ¹H-NMR (300 MHz, CDCl₃): δ = 7.02 (s, 1 H, *H*ₐᵣ), 4.20 (t, ³*J* = 6.9 Hz, 2 H, C*H*₂-O), 2.91 (t, ³*J* = 6.9 Hz, 2 H, Cₐᵣ-C*H*₂), 1.73-1.78 (m, 2 H, C_{q}-C*H*₂), 1.45 (s, 6 H, 2 × C*H*₃), 1.26-1.35 (m, 4 H, 2 × C*H*₂), 0.91 (t, ³*J* = 6.7 Hz, 3 H, C*H*₃). - ¹³C-NMR (75 MHz, CDCl₃): δ = 153.4 (*C*=O), 139.3 (*C*ₐᵣ-CH₂), 138.2 (*C*ₐᵣ), 113.7 (*C*ₐᵣ₋Br), 84.7 (O-*C*_{q}), 71.7 (*C*ₐᵣ-I), 65.3 (*C*H₂-O), 40.4 (C_{q}-*C*H₂), 28.9 (Cₐᵣ-*C*H₂), 26.2 (*C*H₂), 25.8 (2 × *C*H₃), 23.1 (*C*H₂), 14.2 (*C*H₃). - FTIR: ν̃ = 2955, 1734, 1250 cm⁻¹. - MS (EI), *m*/*z* (%): 476/474 (9/9) [M⁺], 316/314 (100/94) [(C₆H₄BrIS)⁺]. - HRMS (C₁₄H₂₀BrIO₃S): ber. 473.9361, gef. 473.9370. - Abspaltungstemperatur: T_{On} (Starttemperatur) = 190 °C.

### 2.3 2-(2-Brom-5-iodthiophen-3-yl)ethyl (1-butylcyclopentyl) carbonat

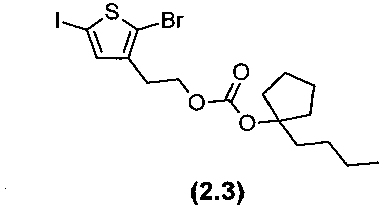

Die Synthese wurde ausgehend von 2-(2-Brom-5-iodthiophen-3-yl)ethanol (1.30 g, 3.90 mmol, 1.00 Äquiv.), 1-Butylcyclopentyl 1*H*-imidazol-1-carboxylat (1.01 g, 4.29 mmol, 1.10 Äquiv.) und Kaliumhydroxid (2.00 mg, 39.0 µmol, 0.01 Äquiv.) gemäß ***AAV 2*** durchgeführt. Nach säulenchromatographischer Reinigung (Kieselgel, Petrolether/ Essigsäureethylester 10:1) wurden 1.77 g (91%) des Carbonates als gelbes Öl erhalten. - *R*_{f} = 0.73 (Petrolether/Essigsäureethylester 10:1). - ¹H-NMR (300 MHz, CDCl₃): δ = 7.02 (s, 1 H, *H*ₐᵣ), 4.21 (t, ³*J* = 6.7 Hz, 2 H, C*H*₂-O), 2.91 (t, ³*J* = 6.7 Hz, 2 H, Cₐᵣ-C*H*₂), 2.07-2.18 (m, 2 H, C_{q}-C*H*₂), 1.89-1.97 (m, 2 H, C*H*₂), 1.57-1.79 (m, 6 H, 3 × C*H*₂), 1.25-1.35 (m, 4 H, 2 × C*H*₂), 0.90 (t, ³*J* = 6.9 Hz, 3 H, C*H*₃). - ¹³C-NMR (75 MHz, CDCl₃): δ = 153.5 (*C*=O), 139.3 (*C*ₐᵣ-CH₂), 138.2 (*C*ₐᵣH), 113.7 (*C*ₐᵣ-Br), 95.5 (O-*C*_{q}), 71.8 (*C*ₐᵣ-I), 65.4 (*C*H₂-O), 37.5 (2 × *C*H₂), 36.7 (*C*H₂), 29.0 (*C*H₂), 27.1 (*C*H₂), 24.1 (2 × *C*H₂), 23.1 (*C*H₂), 14.2 (*C*H₃). - FTIR: ṽ = 2959, 2870, 1735, 1453, 1388, 1251, 1172, 1102, 965, 792 cm⁻¹. - MS (Fab), *m*/*z* (%): 502/500 [M⁺]. - HRMS (C₁₆H₂₂BrIO₃S): ber. 499.9518, gef. 499.9489. - Abspaltungstemperatur: T_{On} = 158 °C.

### 2.4 2-(2-Brom-5-iodthiophen-3-yl)ethyl (2-methyloct-3-in-2-yl) carbonat

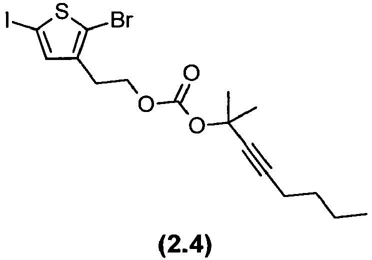

Die Synthese wurde ausgehend von 2-(2-Brom-5-iodthiophen-3-yl)ethanol (1.67 g, 5.00 mmol, 1.00 Äquiv.), 2-Methyloct-3-in-2-yl 1*H*-imidazol-1-carboxylat (1.29 g, 5.50 mmol, 1.10 Äquiv.) und Kaliumhydroxid (3.00 mg, 50.0 µmol, 0.01 Äquiv.) gemäß ***AAV 2*** durchgeführt. Nach säulenchromatographischer Reinigung (Kieselgel, Petrolether/ Essigsäureethylester 10:1) wurden 1.77 g (91%) des Carbonates als klares Öl erhalten. - *R*_{f} = 0.69 (Petrolether/Essigsäureethylester 10:1). - ¹H-NMR (300 MHz, CDCl₃): δ = 7.03 (s, 1 H, *H*ₐᵣ), 4.23 (t, ³*J* = 6.9 Hz, 2 H, C*H*₂-O), 2.92 (t, ³*J* = 6.9 Hz, 2 H, Cₐᵣ-C*H*₂), 2.21 (t, ³*J* = 6.9 Hz, 2 H, C-C*H*₂), 1.67 (s, 6 H, 2 × C*H*₃), 1.33-1.53 (m, 4 H, 2 × C*H*₂), 0.90 (t, ³*J* = 7.1 Hz, 3 H, C*H*₃). - ¹³C-NMR (75 MHz, CDCl₃): δ = 152.9 (*C*=O), 139.3 (*C*ₐᵣ-CH₂), 138.2 (*C*ₐᵣH), 113.7 (*C*ₐᵣ-Br), 85.6 (*C*-CH₂), 80.7 (*C*-C_{q}), 75.3 (O-*C*_{q}), 71.7 (*C*ₐᵣ-I), 65.7 (*C*H₂-O), 30.7 (*C*H₂), 29.3 (2 × *C*H₃), 28.9 (*C*H₂), 22.0 (*C*H₂), 18.5 (*C*H₂), 13.7 (*C*H₃). - FTIR: ṽ = 2958, 2930, 2867, 2245, 1748, 1463, 1384, 1250, 1194, 1132, 1100, 790 cm⁻¹. - MS (EI), *m*/*z* (%): 500/498 (12/12) [M⁺], 316/314 (100/95) [(C₆H₄BrIS)⁺], 236 (25) [(C₆H₅IS)⁺], 123 (61) [(C₉H₁₅)⁺]. - HRMS (C₁₆H₂₀BrIO₃S): ber. 497.9361, gef. 497.9385. - Abspaltungstemperatur: T_{On} = 186 °C.

### 2-5 2-(2-Bromo-5-iodothiophen-3-yl)ethyl (1-(hex-1-in-1-yl)cyclohexyl) carbonat

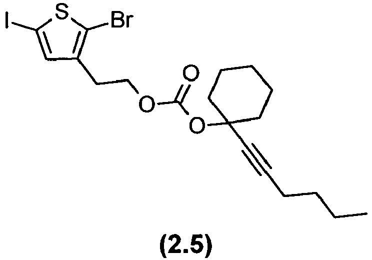

Die Synthese wurde ausgehend von 2-(2-Brom-5-iodthiophen-3-yl)ethanol (1.67 g, 5.00 mmol, 1.00 Äquiv.), 1-(Hex-1-in-1-yl)cyclohexyl 1*H*-imidazole-1-carboxylat (1.51 g, 5.50 mmol, 1.10 Äquiv.) und Kaliumhydroxid (3.00 mg, 50.0 µmol, 0.01 Äquiv.) gemäß ***AAV 2*** durchgeführt. Nach säulenchromatographischer Reinigung (Kieselgel, Petrolether/ Essigsäureethylester 20:1, 5% Triethylamin) wurden 2.17 g (80%) des Carbonates als gelbes Öl erhalten. - *R*_{f} = 0.50 (Petrolether/Essigsäureethylester 20:1, TEA). - ¹H-NMR (300 MHz, CDCl₃): δ = 7.03 (s, 1 H, *H*ₐᵣ), 4.24 (t, ³*J* = 7.2 Hz, 2 H, C*H*₂-O), 2.93 (t, ³*J* = 7.2 Hz, 2 H, Cₐᵣ-C*H*₂), 2.25 (t, ³*J* = 7.2 Hz, 2 H, C-C*H*₂), 2.09-2.17 (m, 2 H, C*H*₂), 1.77-1.86 (m, 2 H, C*H*₂), 1.60-1.68 (m, 4 H, 2 × C*H*₂), 1.39-1.45 (m, 6 H, 3 × C*H*₂), 0.91 (t, ³*J* = 7.2 Hz, 3 H, C*H*₃). - ¹³C-NMR (75 MHz, CDCl₃): δ = 152.8 (*C*=O), 139.3 (*C*ₐᵣ-CH₂), 138.2 (*C*ₐᵣH), 113.7 (*C*ₐᵣ-Br), 87.9 (*C*-CH₂), 79.5 (*C*-C_{q}), 79.0 (O-*C*_{q}), 71.9 (*C*ₐᵣ-I), 65.7 (*C*H₂-O), 37.5 (2 × *C*H₂), 30.9 (*C*H₂), 27.1 (*C*H₂), 25.3 (2 × *C*H₃), 23.1 (*C*H₂), 22.1 (*C*H₂), 18.7 (*C*H₂), 13.8 (*C*H₃). - FTIR: ṽ = 2930, 2857, 2240, 1745, 1447, 1267, 1231, 1182, 1125, 1014, 917, 782 cm⁻¹. - MS (FAB), *m*/*z* (%): 541/539 (67/100) [(M+H)⁺], 540/538 (50/47) [M⁺]. - HRMS (C₁₉H₂₄BrIO₃S): ber. 537.9674, gef. 537.9725. - Abspaltungstemperatur: T_{On} = 185 °C.

### 2.6 3-(2,7-Dibrom-9-methyl-fluoren-9-yl)propyl (2-methylhexan-2-yl) carbonat

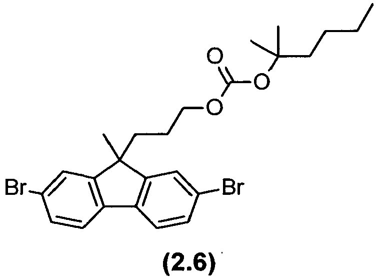

Die Synthese wurde ausgehend von 2,7-Dibrom-9-(3-hydroxypropyl)-9-methylfluoren (3.17 g, 8.00 mmol, 1.00 Äquiv.), 2-Methylhexan-2-yl 1*H-*imidazol-1-carboxylat (1.85 g, 8.80 mmol, 1.10 Äquiv.) und Kaliumhydroxid (4.00 mg, 80.0 µmol, 0.01 Äquiv.) gemäß ***AAV 2*** durchgeführt. Nach säulenchromatographischer Reinigung (Kieselgel, Petrolether/Essigsäureethylester 20:1) wurden 3.70 g (86%) des Carbonates als klares Öl erhalten. - *R*_{f} = 0.40 (Petrolether/Essigsäureethylester 20:1). - ¹H-NMR (300 MHz, CDCl₃): δ = 7.53 (d, ³*J* = 8.0 Hz, 2 H, 2 × *H*ₐᵣ), 7.49 (d, ⁴*J* = 1.8 Hz, 2 H, 2 × *H*ₐᵣ), 7.46 (d, ³*J* = 8.0 Hz, ⁴*J* = 1.8 Hz, 2 H, 2 × *H*ₐᵣ), 3.82 (t, ³*J* = 6.7 Hz, 2 H, C*H*₂-O), 1.99-2.06 (m, 2 H, C*H*₂), 1.69-1.76 (m, 2 H, C*H*₂-C_{q}), 1.47 (s, 3 H, C*H*₃), 1.42 (s, 6 H, 2 × C*H*₃), 1.25-1.32 (m, 4 H, 2 × C*H*₂), 0.94-1.04 (m, 2 H, C*H*₂), 0.86-0.92 (m, 3 H, C*H*₃). - ¹³C-NMR (75 MHz, CDCl₃): δ = 153.5 (C=O), 153.2 (2 × *C*ₐᵣ), 138.3 (2 × *C*ₐᵣ), 130.7 (2 × *C*ₐᵣH), 126.3 (2 × *C*ₐᵣH), 121.8 (2 × *C*ₐᵣ-Br), 121.5 (2 × *C*ₐᵣH), 84.4 (*C*_{q}), 66.9 (*C*H₂-O), 51.0 (*C*H₂), 40.3 (*C*H₂-C_{q}), 36.6 (*C*H₂), 26.5 (*C*H₂), 26.2 (*C*H₃), 25.8 (2 × *C*H₃), 23.9 (*C*H₂), 23.1 (*C*H₂), 14.1 (*C*H₃). - FTIR: ṽ = 2959, 2927, 2858, 1735, 1449, 1250 cm⁻¹. - MS (EI), *m*/*z* (%): 540/538/536 (47/92/48) [M⁺], 442/440/438 (34/63/33) [(C₁₈H₁₆Br₂O₃)⁺], 339/337/335 (52/100/52) [(C₁₄H₉Br₂)⁺], 258/256 (44/46) [(C₁₄H₉Br)⁺]. - HRMS (C₁₇H₁₆Br₂O): ber. 536.0562, gef. 536.0568.

### 2.7 3-(2,7-Diprom-9-methyl-9H-fluoren-9-yl)propyl (1-(hex-1-in-1-yl)cyclohexyl) carbonat

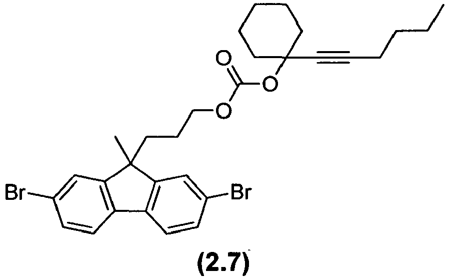

Die Synthese wurde ausgehend von 2,7-Dibrom-9-(3-hydroxypropyl)-9-methylfluoren (792 mg,28.00 mmol, 1.00 Äquiv.), 1-(Hex-1-in-1-yl)cyclohexyl 1*H*-imidazol-1-carboxylat (604 mg, 2.20 mmol, 1.10 Äquiv.) und Kaliumhydroxid (1.00 mg, 20.0 µmol, 0.01 Äquiv.) gemäß ***AAV 2*** durchgeführt. Nach säulenchromatographischer Reinigung (Kieselgel, Petrolether/Essigsäureethylester 10:1, 5% Triethylamin) wurden 895 mg (74%) des Carbonates als klares Öl erhalten.- *R*_{f} = 0.63 (Petrolether/Essigsäureethylester 10:1, TEA). - ¹H-NMR (300 MHz, CDCl₃): δ = 7.53 (d, ³*J* = 8.1 Hz, 2 H, 2 × *H*ₐᵣ), 7.49 (d, ⁴*J* = 1.7 Hz, 2 H, 2 × *H*ₐᵣ), 7.46 (d, ³*J* = 8.1 Hz, ⁴*J* = 1.7 Hz, 2 H, 2 × *H*ₐᵣ), 3.88 (t, ³*J* = 6.6 Hz, 2 H, C*H*₂-O), 2.22 (³*J* = 6.9 Hz, 2 H, C_{q}-C*H*₂), 2.01-2.14 (m, 4 H, 2 × C*H*₂), 1.73-1.84 (m, 2 H, C*H*₂-C_{q}), 1.55-1.67 (m, 4 H, 2 × C*H*₂), 1.40-1.51 (m, 9 H, 3 × C*H*₂ + C*H*₃), 0.95-1.05 (m, 2 H, C*H*₂), 0.89 (t, ³*J* = 7.1 Hz, 3 H, C*H*₃). - ¹³C-NMR (75 MHz, CDCl₃): δ = 153.2 (2 × *C*ₐᵣ), 152.8 (C=O), 138.3 (2 × *C*ₐᵣ), 130.7 (2 × *C*ₐᵣH), 126.4 (2 × *C*ₐᵣH), 121.9 (2 × *C*ₐᵣ-Br), 121.6 (2 × *C*ₐᵣH), 87.6 (*C*-CH₂), 79.5 (*C*-C_{q}), 78.6 (*C*_{q}), 67.2 (*C*H₂-O), 51.0 (*C*_{q}-CH₃), 37.5 (2 × *C*H₂), 36.7 (*C*H₂-C_{q}), 30.8 (*C*H₂), 27.1 (*C*H₂), 26.5 (*C*H₂), 23.9 (*C*H₂), 23.0 (2 × *C*H₂), 22.0 (*C*H₂), 18.6 (*C*H₂), 13.8 (*C*H₃). - FTIR: ṽ = 2931, 2857, 1744, 1447, 1414, 1268, 1236, 1181, 1013, 917, 812 cm⁻¹. - MS (FAB), *m*/*z* (%): 604/602/600 (60/100/53) [M⁺]. - HRMS (C₃₀H₃₄Br₂O₃): ber. 600.0875, gef. 600.0858.

### 2.8 2,7-Bis(5'-bromo-2'-thiophenyl)-[N,N]-bis(hexan-1-yl-6-(2-methylhexan-2-yl)-carbonate)-naphthalen-1,8:4,5-tetracarbonsäurebisimid

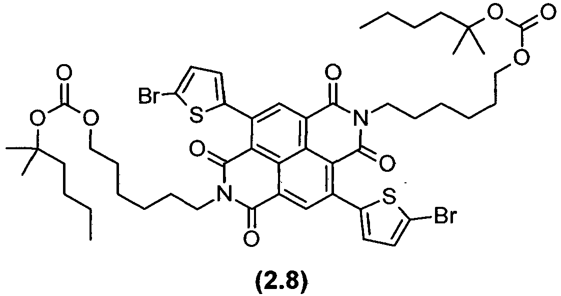

In einem trockenen Schlenkkolben wurde 2,7-Bis(5'-bromo-2'-thiophenyl)-[N,N]-bis(hexan-1-yl-6-ol)-naphthalen-1,8:4,5-tetracarbonsäurebisimid (213 mg, 270 mmol) und 2-methylhexan-2-yl-1-carbonylimidazol (227 mg, 1.08 mmol) in 6 mL trockenem DMF gelöst. Katalytische Mengen KOH (8 mg, 0.14 mmol) wurden zugegeben und das Reaktionsgemisch bei Raumtempertur gerührt. Nach zwei Stunden wurde mit 10 mL wässriger NH₄Cl-Lösung versetzt. Die erhaltene Suspension wurde für 1 Stunde stark gerührt. Die wässrige Phase wurde abgetrennt und der Rückstand in Dichlormethan gelöst, mit gesättigter NaCl-Lösung gewaschen und mit MgSO₄ getrocknet. Das Rohprodukt wurde säulenchromatographisch an Kieselgel mit einem Gemisch aus Chloroform und Toluen (1:1, v:v) mit 2 % THF als Eluent gereinigt und anschließend mehrmals aus MeOH umkristallisiert. Es wurden 140 mg (48 % d.Th.) des Produktes als roter Feststoff erhalten. - *R*_{f} = 0.32 (Chloroform:Toluen (1:1) + 3 % THF). - ¹H-NMR (300 MHz, CDCl₃): δ = 8.71 (s, 2 H, 2 × C*H*ₐᵣ), 7.15 (d, ³*J* = 3.8 Hz, 2 H, C*H*ₐᵣ), 7.07 (d, ³*J* = 3.8 Hz, 2 H, C*H*ₐᵣ), 4.11 (t, ³*J* = 7.7 Hz, 4 H, 2 × N-C*H*₂), 4.03 (t, ³*J* = 6.6 Hz, 4 H, 2 × O-C*H*₂), 1.62-1.77 (m, 12 H, C*H*₂), 1.39-1.46 (m, 8 H, C*H*₂), 1.44 (s, 12 H, C_{q}-C*H*₃), 1.24-1.32 (m, 8 H, C*H*₂), 1.12 (t, ³*J* = 7.6 Hz, 6 H, C*H*₃). - ¹³C-NMR (75 MHz, CDCl₃): - IR: (v in cm⁻¹): 1730 (vs, O-(C=O)-O), 1705 (s, N-C=O), 1662 (vs, N-C=O), 1578 (m). - MS (APCl⁻), *m*/*z*: 1070.3 [(C₅₀H₆₀⁷⁹Br₂N₂O₁₀S₂)⁻], 1072.3 [(C₅₀H₆₀⁷⁹Br⁸¹Br N₂O₁₀S₂)⁻], 1074.3 [(C₅₀H₆₀⁸¹Br₂N₂O₁₀S₂)⁻].

### 2.9 2,7-Bis(5'-bromo-4'-hexyl-2'-thiophenyl)-[N,N]-bis(hexan-1-yl-6-(2-methylhexan-2-yl)-carbonate)-naphthalen-1,8:4,5-tetracarbonsäurebisimid

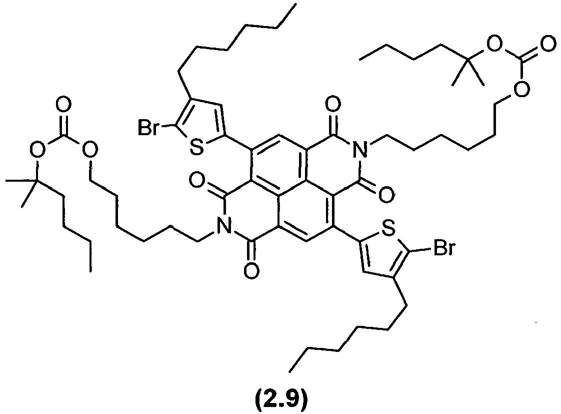

In einem trockenen mit Argon befülltem Schlenkrohr wurden 2,7-Bis(5'-bromo-4'-hexyl-2'-thiophenyl)-[N,N]-bis(hexan-1-yl-6-ol)-naphthalen-1,8:4,5-tetracarbonsäurebisimid (248 mg, 0.26 mmol) und 2-methylhexan-2-yl 1*H*-imidazole-1-carboxylat (219 mg, 1.04 mmol) in 5 mL trockenem DMF gelöst. Die Suspension wurde auf 0 °C gekühlt und katalytische Mengen KOH (7 mg, 0.13 mmol) zugegeben und für 3 Stunden bei 0 °C gerührt. Anschließend wurden 10 mL wässriger NH₄Cl-Lösung zugegeben und für eine weitere Stunde gerührt. Der Niederschlag wurde abfiltriert, mit Wasser gewaschen und mit Dichlormethan wieder extrahiert. Die organische Phase wurde mit ges. NaCl gewaschen und über MgSO₄ getrocknet. Das Rohprodukt wure säulenchromatographisch an Kieselgel mit einem Gemisch aus Chloroform und Toluen (1:1, v:v) mit 2 % THF als Eluent gereinigt und anschließend mehrmals aus MeOH umkristallisiert. Es wurden 180 mg (56 % d.Th.) des Produktes als violetter Feststoff erhalten. - ¹H-NMR (300 MHz, CDCl₃): δ = 8.70 (s, 2 H, 2 × C*H*ₐᵣ), 7.03 (s, 2 H, C*H*ₐᵣ), 4.12 (t, ³*J* = 7.7 Hz, 4 H, 2 × N-C*H*₂), 4.03 (t, ³*J* = 6.6 Hz, 4 H, 2 × O-C*H*₂), 2.64 (t, ³*J* = 7.7 Hz, 4 H, 2 × C_{q}-C*H*₂), 1.73-1.76 (m, 4 H, C_{q}-C*H*₂), 1.60-1.73 (m, 12 H, C*H*₂), 1.43 (s, 12 H, C_{q}-CH₃), 1.37-1.44 (m, 12 H, C*H*₂), 1.32-1.37 (m, 8 H, C*H*₂), 1.27-1.32 (m, 8 H, C*H*₂), 0.91 (t, ³*J* = 6.9 Hz, 6 H, C*H*₃), 0.89 (t, ³*J* = 6.9 Hz, 6 H, C*H*₃). - ¹³C-NMR (75 MHz, CDCl₃): δ = 162.1 (2 × N-*C*=*O*), 162.0 (2 × N-*C*=*O*), 153.6 (O-(C=O)-O), 142.5 (2 x C_{q}), 140.0 (2 x C_{q}), 139.5 (2 x C_{q}), 136.4 (2 × *C*Hₐᵣ), 129.6 (2 × *C*H*ₐᵣ*), 127.4 (2 x C_{q}), 125.4 (2 × (C=O)-*C*_{q-ar}), 122.9 (2 × (C=O)-*C*_{q}), 112.5 (2 × *C_{q-ar}*Br), 84.1 (*C*H₂-C_{q}), 66.9 (*C*H₂-O), 41.0 (*C*H₂-N), 40.2 (*C*H₂), 31.6 (C_{q}-*C*H₂), 29.7 (*C*H₂), 29.0 (*C*H₂), 28.6 (*C*H₂), 27.9 (*C*H₂), 26.7 (*C*H₂), 26.0 (*C*H₂), 25.7 (*C*H₃), 25.5 (*C*H₂), 22.9 (*C*H₂), 22.6 (*C*H₂), 14.1 (*C*H₃), 14.0 (*C*H₃). - HR-MS (ESI, Na⁺-Addukt, [C₆₂H₈₄Br₂N₂NaO₁₀S₂]⁺): ber. 1261.3826, gef. 1261.3831. - IR: (v in cm⁻¹): 1737 (vs, O-(C=O)-O), 1704 (s, N-C=O), 1667 (vs, N-C=O), 1574 (m). - TGA/DSC: Smp: 78.7 °C, Pyrolyse Onset: 150 °C, Pyrolyse Mittelpunkt: 223 °C. Gewichstverlust: 22.4 % (ber. 22.9 %).

### 2.10 2-Methylhexan-2-yl 2,7-dibrom-9H-carbazol-9-carboxylat (nicht erfindungsgemäß)

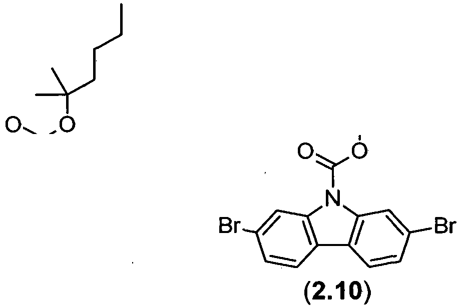

Zu einer Lösung aus 2,7-Dibromcarbazol (2.28 g, 7.00 mmol, 1.00 Äquiv.) in Acetonitril/Tetrahydrofuran (4:1, 25 mL) wurden bei Raumtemperatur 2-Methylhexan-2-yl 1*H*-imidazot-1-carboxylat (1.62 g, 7.70 mmol, 1.10 Äquiv.) und 1,8-Diazabicyclo[5.4.0]undec-7-en (213 mg, 1.40 mmol, 0.20 Äquiv.) gegeben. Das Reaktionsgemisch wurde bei Raumtemperatur über Nacht gerührt und anschließend mit 1 M Salzsäure (5 mL) versetzt und mit Essigsäureethylester (20 mL) extrahiert. Die vereinigten organischen Phasen wurden mit gesättigter Natriumchlorid-Lösung gewaschen, über Magnesiumsufat getrocknet und das Lösungsmittel wurde unter vermindertem Druck entfernt. Nach säulenchromatographischer Reinigung (Kieselgel, Petrolether/Essigsäureethylester 30:1) wurden 2.73 g (84%) des Carbonates als gelber Feststoff erhalten. - *R*_{f} = 0.66 (Petrolether/Essigsäureethylester 30:1). - ¹H-NMR (300 MHz, CDCl₃): δ = 8.48 (s, 2 H, 2 × *H*ₐᵣ), 7.77 (d, ³*J* = 8.3 Hz, 2 H, 2 × *H*ₐᵣ), 7.47 (d, ³*J* = 8.3 Hz, 2 H, 2 × *H*ₐᵣ), 2.05 (t, ³*J* = 7.2 Hz, 2 H, C*H*₂), 1.74 (s, 6 H, 2 × C*H*₃), 1.40-1.53 (m, 4 H, 2 × C*H*₂), 0.96 (t, ³*J* = 6.9 Hz, 3 H, C*H*₃). - ¹³C-NMR (75 MHz, CDCl₃): δ = 150.6 (C=O), 139.4 (2 × *C*ₐᵣ-N), 126.7 (2 × *C*ₐᵣH), 124.1 (2 × *C*ₐᵣ), 121.2 (2 × *C*ₐᵣ-Br), 120.8 (2 × *C*ₐᵣH), 119.9 (2 × *C*ₐᵣH), 87.9 (*C*_{q}), 41.1 (*C*H₂), 26.4 (*C*H₂), 26.3 (2 × *C*H₃), 23.3 (*C*H₂), 14.3 (*C*H₃). - FTIR: ṽ = 2955, 2867, 1729, 1590, 1439, 1406, 1347, 1326, 1277, 1207, 1150, 797 cm⁻¹. - MS (DART), *m*/*z* (%): 469/467/465 (15/30/15) [M⁺]. - HRMS (C₂₀H₂₁Br₂NO₂): ber. 464.9939, gef. 464.9922.-Abspaltungstemperatur: T_{On} = 159 °C.

### 2.11 9-Methylheptadecan-9-yl 2,7-dibrom-9H-carbazol-9-carboxylat (nicht erfindungsgemäß)

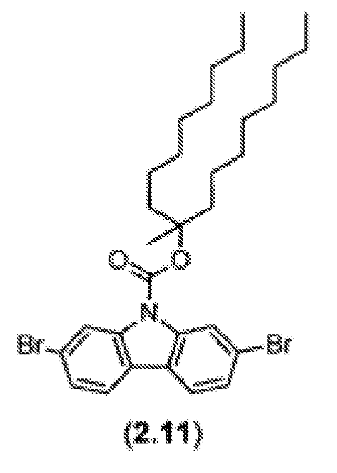

Zu einer Lösung aus 2,7-Dibromcarbazol (1.95 g, 6.00 mmol, 1.00 Äquiv.) in Acetonitril/Tetrahydrofuran (4:1, 25 mL) wurden bei Raumtemperatur 9-Methylheptadecan-9-yl 1*H*-imidazol-1-carboxylat (2.41 g, 6.60 mmol, 1.10 Äquiv.) und 1,8-Diazabicyclo[5.4.0]undec-7-en (183 mg, 1.20 mmol, 0.20 Äquiv.) gegeben. Das Reaktionsgemisch wurde bei Raumtemperatur über Nacht gerührt und anschließend mit 1 M Salzsäure (5 mL) versetzt und mit

Essigsäureethylester (20 mL) extrahiert. Die vereinigten organischen Phasen wurden mit gesättigter natriumchlorid-Lösung gewaschen, über Magnesiumsufat getrocknet und das Lösungsmittel wurde unter vermindertem Druck entfernt. Nach säulenchromatographischer Reinigung (Kieselgel, Petrolether/Essigsäureethylester 40:1) wurden 3.07 g (82%) des Carbonates als gelber Feststoff erhalten. - *R*_{f} = 0.81 (Petrolether/Essigsäureethylester 40:1). - ¹H-NMR (300 MHz, CDCl₃): δ = 8.48 (s, 2 H, 2 × *H*ₐᵣ), 7.78 (d, ³*J* = 8.3 Hz, 2 H, 2 × *H*ₐᵣ), 7.47 (d, ³*J* = 8.3 Hz, ⁴*J* = 1.7 Hz, 2 H, 2 × *H*ₐᵣ), 1.95-2.18 (m, 4 H, 2 × C*H*₂), 1.71 (s, 3 H, C*H*₃), 1.23-1.49 (m, 24 H, 12 × C*H*₂), 0.84-0.89 (m, 6 H, 2 × C*H*₃). - ¹³C-NMR (75 MHz, CDCl₃): δ = 150.4 (C=O), 139.4 (2 × *C*ₐᵣ-N), 126.6 (2 × *C*ₐᵣH), 124.1 (2 × *C*ₐᵣ), 121.2 (2 × *C*ₐᵣ-Br), 120.7 (2 × *C*ₐᵣH), 119.8 (2 × *C*ₐᵣH), 90.6 (*C*_{q}), 38.7 (2 × CH₂), 32.0 (2 × CH₂), 30.2 (2 × CH₂), 29.7 (2 × *C*H₂), 29.4 (2 × *C*H₂), 24.1 (*C*H₃), 24.0 (2 × *C*H₂), 22.8 (2 × *C*H₂), 14.3 (*C*H₃). - FTIR: ṽ = 2924, 2852, 1730, 1588, 1438, 1348, 1323, 1278, 1208, 1123, 973, 799 cm⁻¹. - MS (DART), *m*/*z* (%): 623/621/619 (7/15/7) [M⁺]. - HRMS (C₃₁H₄₃Br₂NO₂): ber. 619.1661, gef. 619.1673. - Abspaltungtemperatur: T_{On} = 151 °C.

### 2.12 Bis-(2-methyl-hexan-2-yl)-2,7-bis(5'-bromo-4'-hexylthiophen-2'-yl)naphthalen-1,4,5,8-tetracarbonsäurediimid-[N,N]-dicarboxylat (nicht erfindungsgemäß)

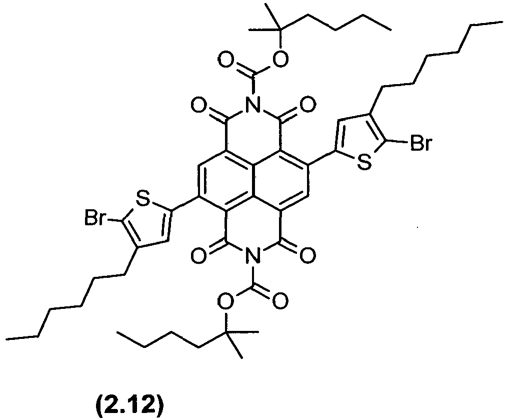

Bis(2-methylhexan-2-yl) Dicarbonat (0.404 g, 1.34 mmol, 4.00 eq) wurden unter Schutzgasatmosphäre zu einer Suspension von 2,7-bis(5'-bromo-4'-hexylthiophen-2'-yl)naphthalen-1,4,5,8-tetracarbonsäure Diimid (0.200 g, 0.334 mmol, 1.00 eq) und DMAP (0.100 g, 0.835 mmol, 2.50 eq) in 2.5 mL trockenem Tetrahydrofuran gegeben. Das Reaktionsgemisch wurde für 4.5 Stunden bei Raumtemperatur gerührt, anschließend mit 30 mL Chloroform verdünnt und auf 25 mL einer gesättigten wässrigen Ammoniumchlorid-Lösung gegeben. Die organische Phase wurde abgetrennt und die wässrige Phase zweimal mit je 35 mL Chloroform extrahiert. Die vereinigten organischen Phasen wurden mit gesättigter Kochsalzlösung und Na₂SO₄ getrocknet und eingeengt. Der erhaltene Feststoff wurde säulenchromatographisch an Kieselgel mit einem Lösungsmittelgemisch aus Chloroform, Petrolether, Toluen und Triethylamin (50:32:15:3, v:v) aufgereiningt und fünf Mal aus einem Gemisch aus Dichlormethan und Methanol umkristallisiert. Es wurden 98 mg (31 % d.Th.) des gewünschten Produktes erhalten. - Rf = 0.53 (Chloroform/Petrolether/Toluen 50:35:15 + 3 % Triethylamin) - ¹H-NMR (500 MHz, CDCl₃): δ [ppm]= 8.75 (s, 1H, *H*ₐᵣ), 7.12 (s, 2H, *H*ₐᵣ), 2.63 (t, ³J = 7.6 Hz, 4H, C*H₂*), 1.88 (m, 4H, C*H*₂), 1.64 (tt, ³J = 7.5 Hz, 4H, C*H₂*), 1.63 (s, 12H, C*H₃*), 1.33-1.45 (m, 20H, CH₂), 0.91-0.92 (m, 12H, C*H*₃) - ¹³C-NMR (125 MHz, CDCl₃): δ [ppm]= 159.8 (*C*_{q}), 159.7 (*C*_{q}), 1.47.5 (*C*_{q}), 142.7 (*C*_{q}), 140.0 (*C*_{q}), 136.7 (*C*Hₐᵣ), 136.5 (*C*_{q}), 130.9 (*C*Hₐᵣ), 128.1 (*C*_{q}), 125.4 (*C*_{q}), 122.3 (*C*-Br), 113.6 (*C*_{q}), 90.3 (*C*_{q}), 40.2 (C_{q}-*C*H₂), 31.6 (*C*H₂), 29.7 (*C*H₂), 29.6 (*C*H₂), 28.9 (*C*H₂), 25.7 (*C*H₂), 25.4 (*C*H₃), 22.9 (*C*H₂), 22.6 (*C*H₂), 14.1 (*C*H₃),14.0 (*C*H₃) - FT-IR: v [cm⁻¹] = 2953 (w, *CH₂*), 2923 (m, *CH₂*), 2856 (w, *CH₂*), 1775 (s, *C*=*O*), 1713 (s, N(*C*=*O*)₂), 1683 (s, N(*C*=*O*)₂), 1589 (w) - UV/Vis (CHCl₃): λₘₐₓ (ε) = 536 nm (1.23·10⁵ I mol⁻¹ cm⁻¹) - HR-ESI-MS (C₅₀H₆₀KN₂O₈S₂⁷⁹Br⁸¹Br): ber. 1079.177 (exp. 1079.179), (C₅₀H₆₀NaN₂O₈S₂⁷⁹Br⁸¹Br) ber. 1063.203 (exp. 1063.205).

### 2.13 2,7-Bis(5'-bromo-2'-thiophenyl)-[N,N]-bis(hexan-1-yl-6-(9-Methytheptadecan-9-yl)-carbonate)-naphthalen-1,8:4,5-tetracarbonsäurebisimid

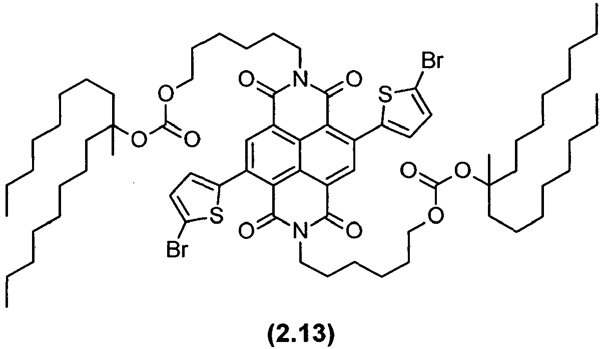

In einem trockenen Schlenkkolben wurde 2,7-Bis(5'-bromo-2'-thiophenyl)-[N,N]-bis(hexan-1-yl-6-ol)-naphthalen-1,8:4,5-tetracarbonsäurebisimid (434 mg, 550 mmol) und 9-Methylheptadecan-9-yl 1*H*-imidazol-1-carboxylat (802 mg, 2.2 mmol) in 11 mL trockenem DMF gelöst. Katalytische Mengen KOH (15 mg, 0.28 mmol) wurden zugegeben und das Reaktionsgemisch bei Raumtemperatur gerührt. Nach 18 Stunden wurde mit 200 mL wässriger NH₄Cl-Lösung versetzt. Die erhaltene Suspension wurde für 1 Stunde stark gerührt. Der tief rote organische Niederschlag wurde abgetrennt und in Dichlormethan gelöst, mit Wasser und gesättigter Kochsalzlösung gewaschen, mit MgSO₄ getrocknet und eingeengt. Das Rohprodukt wurde säulenchromatographisch an Kieselgel mit einem Gemisch aus Chloroform, Toluen und Petrolether (3:4:3, v:v) mit 2 % THF als Eluent gereinigt und anschließend mehrmals aus MeOH und Acetonitril umkristallisiert. Es wurden 228 mg (30 % d.Th.) des Produktes als roter Feststoff erhalten. - UPLC-Reinheit: 98.6 %. - *R*_{f} = 0.32 (Chloroform:Toluen:Petrolether (3:4:3, v:v) + 2 % THF). - ¹H-NMR (500 MHz, CDCl₃): δ = 8.71 (s, 2H, C*H*ₐᵣ), 7.15 (d, ³*J* = 3.8 Hz, 2H, C*H*ₐᵣ), 7.07 (d, ³*J* = 3.8 Hz, 2H, C*H*ₐᵣ), 4.11 (t, ³*J* = 7.7 Hz, 4H, N-C*H*₂), 4.03 (t, ³*J* = 6.6 Hz, 4H, O-C*H*₂), 1.62-1.82 (m, 16H, C*H*₂), 1.39-1.41 (m, 14H, C*H*₂), 1.21-1.31 (m, 48H, C*H*₂), 1.12 (t, ³*J* = 7.6 Hz, 12H, C*H*₃). - ¹³C-NMR (125 MHz, CDCl₃): 161.9 (*C*_{q}), 161.8 (*C*_{q}), 153.5 (*C*_{q}), 141.9 (*C*_{q}), 139.1 (*C*_{q}), 136.5 (*C*Hₐᵣ), 130.2 (*C*Hₐᵣ), 128.8 (*C*_{q}), 127.5 (*C*_{q}), 125.6 (*C*_{q}), 123.2 (*C*-Br), 115.4 (*C*_{q}), 86.6 (CH₂), 66.9 (CH₂), 41.0, 38.0, 31.8, 29.9, 29.5, 29.2, 28.6, 27.8, 26.7, 25.5, 23.6, 23.5, 22.6, 14.1. - HR-ESI-MS: [M+NH₄]⁺, (C₇₂H₁₀₈⁷⁹Br₂N₃O₁₀S₂): ber. 1396.584 (exp. 1396.585).

### Beispiel 3 - Synthese der Polymere

### Synthese nach Yokozawa:

### Allgemeine Arbeitsvorschrift zur Darstellung von Polythiophenen (AAV 3):

Eine 2.0 M Isopropylmagnesiumchlorid-Lösung (1.00 Äquiv.) in Tetrahydrofuran wurde bei 0 °C zu einer Lösung des entsprechenden Carbonates (1.00 Äquiv.) in Tetrahydrofuran (5.0 mL/mmol Carbonat) zugegeben und für 1 h bei bei dieser Temperatur gerührt. Dazu wurde eine Suspension von [1,3-Bis(diphenylphosphin)propan]nickelchlorid (0.005 Äquiv.) in Tetrahydrofuran (5 mL) gegeben. Das Reaktionsgemisch wurde bei Raumtemperatur für 24 h gerührt. Nach Zugabe von 5 M Salzsäure (10 mL) wurde das Reaktionsgemisch mit Chloroform extrahiert. Die organische Phase mit Wasser gewaschen, über Magnesiumsulfat getrocknet und das Lösungsmittel wurde unter vermindertem Druck entfernt. Der Rückstand wurde dann in Methanol (250 mL) gegeben und das unlösliche Material in einer Soxhlet-Apparatur mit Methanol, Aceton und Chloroform fraktioniert.

### 3.1 Poly(2-(thiophen-3-yl)ethyl (2-methylhexan-2-yl) carbonat)

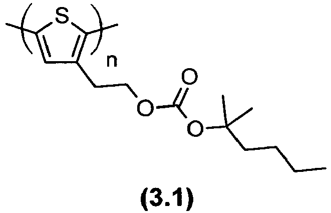

Die Synthese wurde ausgehend von 2-(2-Brom-5-iodthiophen-3-yl)ethyl (2-methylhexan-2-yl) carbonat (4.66 g, 9.80 mmol, 1.00 Äquiv.), Isopropylmagnesiumchlorid-Lösung (4.9 mL, 9.80 mmol, 1.00 Äquiv.) und [1,3-Bis(diphenylphosphin)propan]nickelchlorid (27.0 mg, 49.0 µmol, 0.005 Äquiv.) gemäß ***AAV 3*** durchgeführt. Nach der fraktionierten Reinigung mittels Soxhlet-Apparatur (Methanol, Aceton, Chloroform) wurden 1.02 g (39%) in der Aceton-Fraktion und 382 mg (15%) in der Chloroform-Fraktion des Polymers als dunkelroter Feststoff erhalten.
- Aceton: Mₙ = 15.3 kDa, M_{w} = 24.6 kDa, PDI = 1.61; Chloroform: Mₙ = 41.0 kDa, M_{w} = 45.6 kDa, PDI = 1.11. - Abspaltungstemperatur: T_{On} = 180 °C.

### Synthese nach Yamamoto:

Allgemeine Arbeitsvorschrift zur Darstellung von Homopolymeren (Poylfluorene und Polycarbazole) (**AAV 4**):

Eine Mischung aus Bis(1,5-cyclooctadien)nickel (2.25 Äquiv.), Cyclooctadien (2.25 Äquiv.) und 2,2-Bipyridin (2.25 Äquiv.) in N,N-Dimethylformamid (5 mL) wurde bei 50 °C für 30 Minuten gerührt. Dazu wurde eine Lösung aus dem entsprechenden Monomer (1.00 Äquiv.) in Tetrahydrofuran (10 mL/mmol Monomer) dazugegeben. Das Reaktionsgemisch wurde bei 70 °C für zwei Tage gerührt. Danach wurde der Endcapper (1.00 Äquiv.) dazugegeben und weitere 12 h bei gleicher Temperatur gerührt. Das Reaktionsgemisch wurde nach dem Abkühlen in eine Mischung aus Methanol/Salzsäure (2:1, 300 mL) gegeben und das unlösliche Material in einer Soxhlet-Apparatur mit Methanol, Aceton und Chloroform fraktioniert.

### 3.2 Poly(2,7-(3-(9-methyl-fluoren-9-yl)propyl) (2-methylhexan-2-yl) carbonat)

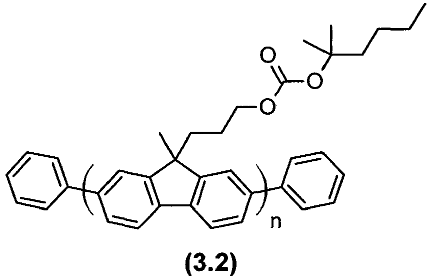

Die Synthese wurde ausgehend von 3-(2,7-Dibrom-9-methyl-fluoren-9-yl)propyl (2-methylhexan-2-yl) carbonat (807 mg, 1.50 mmol, 1.00 Äquiv.), Bis(1,5-cyclooctadien)nickel (928 mg, 3.38 mmol, 2.25 Äquiv.), Cyclooctadien (365 mg, 3.38 mmol, 2.25 Äquiv.), 2,2-Bipyridin (527 mg, 3.38 mmol, 2.25 Äquiv.) und Brombenzol (236 mg, 1.50 mmol, 1.00 Äquiv.) gemäß ***AAV 4*** durchgeführt. Nach der fraktionierten Reinigung mittels Soxhlet-Apparatur (Methanol, Aceton, Chloroform) wurden 471 mg (80%) in der Chloroform-Fraktion des Polymers als gelber Feststoff erhalten.
- Chloroform: Mₙ = 167 kDa, M_{w} = 577 kDa, PDI = 3.46. - Abspaltungstemperatur: T_{On} = 200 °C.

### 3.3 Poly-[5',5'1-2,7-bis(2'-thiophenyl)-[N,N]-bis(hexan-1-yl-6-(2-methylhexan-2-yl)-carbonate)-naphthalen-1,8:4,5-tetracarbonsäurebisimid

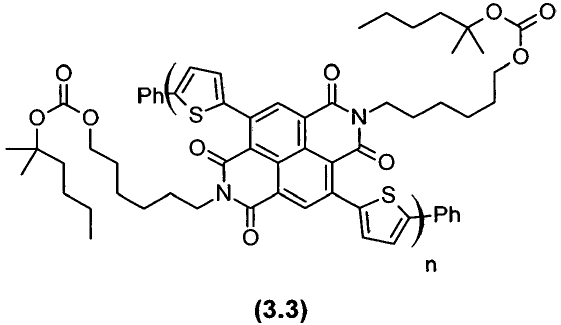

In der Glovebox wurden Bis(1,5-cyclooctadien)nickel(0) (75 mg, 0.27 mmol), 1,5-cyclooctadien (29 mg, 0.27 mmol) und 2,2'-Bipyridin (42 mg, 0.27 mmol) mit 3 mL trockenem und entgasten Tetrahydrofuran versetzt und für 30 min auf 70 °C erhitzt. 2,7-Bis(5'-bromo-2'-thiophenyl)-[N,N]-bis(hexan-1-yl-6-(2-methylhexan-2-yl)-carbonate)-naphthalen-1,8:4,5-tetracarbonsäurebisimid (91 mg, 0.85 mmol) wurde in 7 mL trockenem und entgasten Tetrahydrofuran gelöst, zu der auf 10 °C abgekühlten Nickel-Lösung gegeben und für 120 min bei 10 °C gerührt. Anschließend wurden 100 µL Brombenzol zugegeben und für 4 Stunden bei 60 °C gerührt. Nach dem Abkühlen auf Raumtemperatur wurde die Reaktionssuspension auf eine gerührte Mischung aus 100 mL MeOH und 50 mL konz. Salzsäure gegeben. Das Gemisch wurde für eine Stunde gerührt, die Niederschläge abfiltriert und mit MeOH gewaschen. Der Rückstand wurde mit 200 mL Chloroform extrahiert, wobei ein großer Anteil ungelöst zurück blieb. Der Extrakt wurde auf 3 mL eingeengt und langsam zu 50 mL MeOH getropft. Nach drei Stunden Rühren wurde abfiltriert und der Rückstand mit MeOH gewaschen. Dieser Vorgang wurde insgesamt zweimal wiederholt, wobei zunächst wieder MeOH, anschließend n-Hexan verwendet wurde. Der erhaltene violette Festoff wurde im Vakuum getrocknet, 2 Stunden mit 10 mL Benzol extrahiert, von Feststoffen abfiltriert und lyophilisiert. Es wurden 23 mg (30 % d.Th.) eines violetten Polymerschaumes erhalten.
- GPC: Mn: 10 kD, Mw: 21 kD, PDI: 2.1. - IR: (v in cm⁻¹): 2927 (m, CH₂), 2858 (m, CH₂),1735 (s, O-C=O)-O), 1703 (s, N-C=O), 1664 (vs, N-C=O), 1570 (m). - Abspaltungstemperatur: T_{On} = 155 °C.

### 3.4 Poly-[5',5']-2,7-Bis(4'-hexyl-2'-thiophenyl)-[N,N]-bis(hexan-1-yl-6-(2-methylhexan-2-yl)-carbonate)-naphthalen-1,8:4,5-tetracarbonsäurebisimid

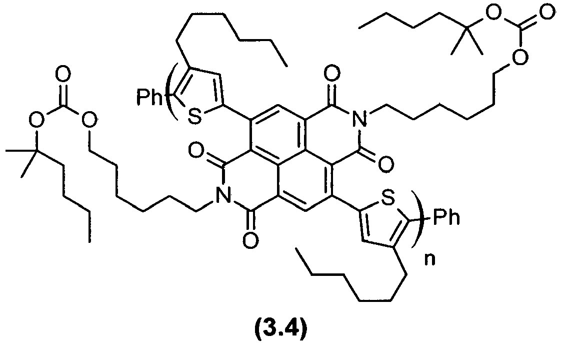

In der Glovebox wurden Bis(1,5-cyclooctadien)nickel(0) (81 mg, 0.29 mmol), 1,5-cyclooctadien (32 mg, 0.29 mmol) und 2,2'-Bipyridin (46 mg, 0.29 mmol) in 3 mL trockenem und entgasten Tetrahydrofuran suspendiert und für 30 min auf 70 °C erhitzt. 2,7-Bis(5'-bromo-4'-hexyl-2'-thiophenyl)-[N,N]-bis(hexan-1-yl-6-(2-methylhexan-2-yl)-carbonate)-naphthalen-1,8:4,5-tetracarbonsäurebisimid (114 mg, 0.09 mmol) wurde in 9 mL trockenem und entgasten Tetrahydrofuran gelöst, zu der auf Raumtemperatur (25 °C) abgekühlten Nickel-Lösung gegeben und für 4 Stunden bei Raumtemperatur gerührt. Der Reaktion wurden 100 µL Brombenzol (0.64 mmol) zugegeben, für weitere 3 Stunden auf 65 °C erhitzt und nach dem Abkühlen auf Raumtemperatur in eine gerührte Mischung aus 100 mL MeOH und 50 mL konz. HCl. gegeben. Die suspension wurde für eine Stunde gerührt, die Niederschläge abfiltriert und mit MeOH gewaschen. Der Rückstand wurde mit Chloroform extrahiert, auf 3 mL eingeengt und langsam zu 100 mL MeOH zugetropft. Nach einer Stunde Rühren wurde abfiltriert und der Rückstand mit MeOH gewaschen. Dieser Vorgang wurde insgesamt dreimal wiederholt, wobei zunächst wieder MeOH, anschließend ein Gemisch aus MeOH und Aceton (2:1, v:v) und letztlich n-Hexan verwendet wurden. Der erhaltene violette Festoff wurde im Vakuum getrocknet und aus Benzol lyophilisiert. Es wurden 58 mg (58 % d.Th.) eines violetten Polymerschaumes erhalten.
- GPC: Mn: 30 kD, Mw: 130 kD, PDI: 4.1. - IR: (v in cm⁻¹): 1735 (vs, O-C=O)-O), 1705 (s, N-C=O), 1663 (vs, N-C=O), 1572 (m). Abspaltungstemperatur: T_{On} = 157 °C.

### Synthese nach Suzuki:

### Allgemeine Arbeitsvorschrift zur Darstellung von Copolymeren (AAV 5):

Eine Mischung aus den entsprechenden Monomeren (jeweils 1.00 Äquiv.), Tetrakis(triphenylphosphan)palladium (0.05 Äquiv.), Kaliumcarbonat (2.50 Äquiv.), Aliquat 336 (0.01 Äquiv.) und Wasser (5 mL) in Toluol (5 mL/mmol Monomer) wurde bei 120 °C für 3 Tage gerührt. Danach wurden die Endcapper (jeweils 1.00 Äquiv.) dazugegeben und jeweils weitere 12 h bei gleicher Temperatur gerührt. Das Reaktionsgemisch wurde nach dem Abkühlen in eine Mischung aus Methanol/Salzsäure (2:1, 300 mL) gegeben und das unlösliche Material in einer Soxhlet-Apparatur mit Methanol, Aceton und Chloroform fraktioniert.

### 3.5 Poly[2,7-(3-(9-methyl-fluoren-9-yl)propyl (2-methylhexan-2-yl) carbonat)-4,7-(benzo[c][1,2,5]thiadiazol)]

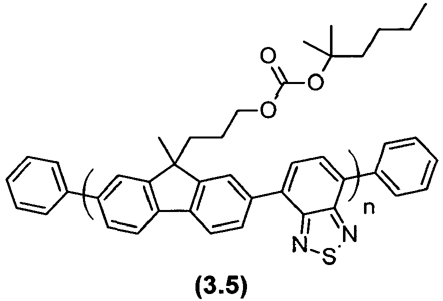

Die Synthese wurde ausgehend von 3-(2,7-Dibrom-9-methyl-fluoren-9-yl)propyl (2-methylhexan-2-yl) carbonat (1.35 g, 2.50 mmol, 1.00 Äquiv.), 4,7-Bis(4,4,5,5-tetramethyl-1,3,2-dioxaborolan-2-yl)benzo[*c*][1,2,5]thiadiazol (970 mg, 2.50 mmol, 1.00 Äquiv.), Tetrakis(triphenylphosphan) palladium (144 mg, 125 µmol, 0.05 Äquiv.), Kaliumcarbonat (864 mg, 6.25 mmol, 2.50 Äquiv.), Aliquat 336 (10.0 mg, 25.0 mmol, 2.50 Äquiv.), Phenylboronsäure (305 mg, 2.50 mmol, 1.00 Äquiv.) und Brombenzol (393 mg, 2.50 mmol, 1.00 Äquiv.) gemäß ***AAV 4*** durchgeführt. Nach der fraktionierten Reinigung mittels Soxhlet-Apparatur (Methanol, Aceton, Chloroform) wurden 1.07 g (83%) in der Chloroform-Fraktion des Polymers als gelber Feststoff erhalten. - Chloroform: Mₙ = 25.8 kDa, M_{w} = 55.8 kDa, PDI = 2.28. - Abspaltungstemperatur: T_{On} = 204 °C.

### Synthese nach Yamamoto:

### 3.6 Poly(9-Methylheptadecan-9-yl 2,7-dibrom-9H-carbazol-9-carboxylat) (nicht erfindungsgemäß)

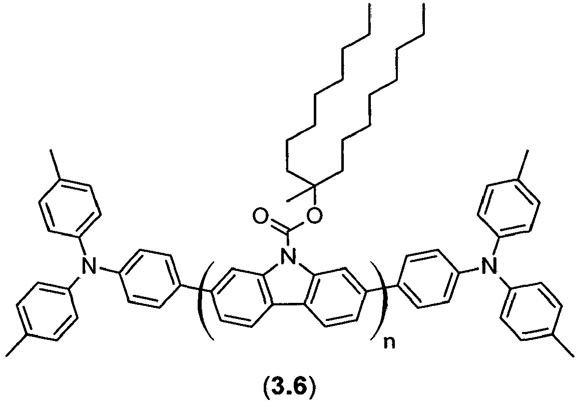

Die Synthese wurde ausgehend von 9-Methylheptadecan-9-yl 2,7-dibrom-9H-carbazol-9-carboxylat (932 mg, 1.50 mmol, 1.00 Äquiv.), Bis(1,5-cyclooctadien)nickel (928 mg, 3.38 mmol, 2.25 Äquiv.), Cyclooctadien (365 mg, 3.38 mmol, 2.25 Äquiv.), 2,2-Bipyridin (527 mg, 3.38 mmol, 2.25 Äquiv.) und 4-Brom-N,N-di-p-tolylanilin (5.00 mg, 15.0 µmol, 0.01Äquiv.) gemäß ***AAV 4*** durchgeführt. Nach der fraktionierten Reinigung mittels Soxhlet-Apparatur (Methanol, Aceton, Chloroform) wurden 661 mg (95%) in der Chloroform-Fraktion des Polymers als grauer Feststoff erhalten. - Chloroform: Mₙ = 95.9 kDa, M_{w} = 370 kDa, PDI = 3.86. - Abspaltungstemperatur: T_{On} = 160 °C.

### 3.7 Poly-[5'-5']-2,7-Bis(4'-hexylthiophen)-[N,N]-Bis(2-methyl-hexan-2-yl)-naphthalen-1,4,5,8-tetracarbonsäuredicarbamat (nicht erfindungsgemäß)

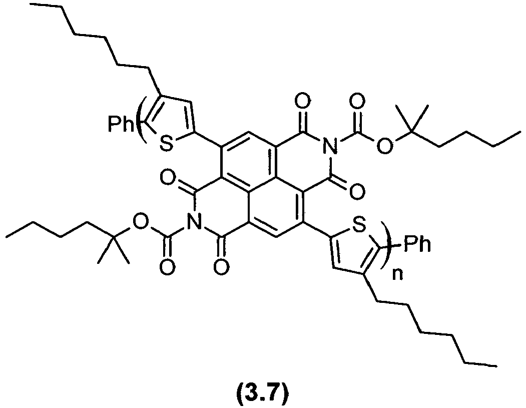

In der Glovebox wurden Bis(1,5-cyclooctadien)nickel(0) (81 mg, 0.29 mmol), 1,5-cyclooctadien (32 mg, 0.29 mmol) und 2,2'-Bipyridin (46 mg, 0.29 mmol) mit 3 mL trockenem und entgasten Tetrahydrofuran versetzt und für 30 min auf 70 °C erhitzt. Bis-(2-methyl-hexan-2-yl)-2,7-bis(5'-bromo-4'-hexylthiophen-2'-yl)naphthalen-1,4,5,8-tetracarbonsäurediimid-[N,N]-dicarboxylat (96 mg, 0.09 mmol) wurde in 9 mL trockenem und entgasten Tetrahydrofuran gelöst, zu der auf Raumtemperatur (25 °C) abgekühlten Nickel-Lösung gegeben und für 3.5 Stunden bei Raumtemperatur gerührt. Der Reaktion wurden 100 µL Brombenzol (0.64 mmol) zugegeben, für weitere 3 Stunden auf 65 °C erhitzt und nach dem Abkühlen auf Raumtemperatur in eine gerührte Mischung aus 100 mL MeOH und 50 mL konzentrierter Salzsäure gegeben. Das Gemisch wurde für eine Stunde gerührt, die Niederschläge abfiltriert und mit MeOH gewaschen. Der Rückstand wurde mit Chloroform extrahiert, auf 3 mL eingeengt und langsam zu 100 mL MeOH zugetropft. Nach einer Stunde Rühren wurde abfiltriert und der Rückstand mit MeOH gewaschen. Dieser Vorgang wurde insgesamt dreimal wiederholt, wobei zunächst wieder MeOH, anschließend zweimal n-Hexan verwendet wurde. Der erhaltene violette Festoff wurde im Vakuum getrocknet und aus Benzol lyophilisiert. Es wurden 48 mg (59 % d.Th.) eines violetten Polymerschaumes erhalten. - GPC: Mn: 12.7 kD, Mw: 26 kD, PDI: 2.1. - FT-IR: (v in cm⁻¹): 2926 (m, CH₂), 2856 (m, CH₂), 1783 (vs, N-C=O)-O), 1712 (s, N-C=O), 1684 (vs, N-C=O), 1576 (m). Abspaltungstemperatur: T_{On} = 103 °C.

### 3.8 Poly-[5',5']-2,7-bis(2'-thiophenyl)-[N,N]-bis(hexan-1-yl-6-(9-Methylheptadecan-9-yl)-carbonate)-carbonate)-naphthalen-1,8:4,5-tetracarbonsäurebisimid

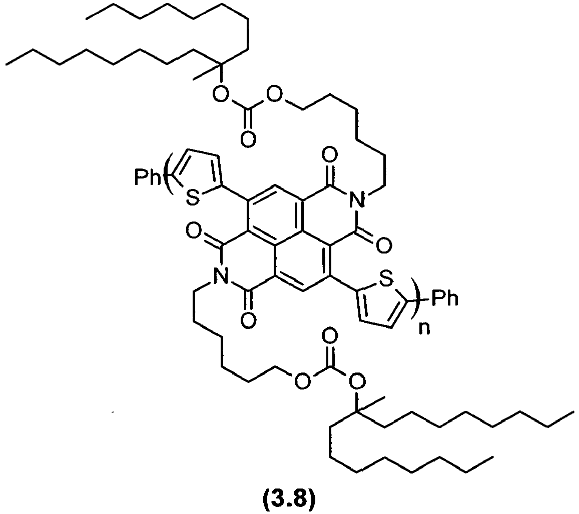

In der Glovebox wurden Bis(1,5-cyclooctadien)nickel(0) (66 mg, 0.24 mmol), 1,5-cyclooctadien (26 mg, 0.24 mmol) und 2,2'-Bipyridin (37 mg, 0.24 mmol) mit 3 mL trockenem und entgasten Tetrahydrofuran versetzt und für 30 min auf 70 °C erhitzt. 2,7-Bis(5'-bromo-2'-thiophenyl)-[N,N]-bis(hexan-1-yl-6-(9-Methylheptadecan-9-yl)-carbonate)-naphthalen-1,8:4,5-tetracarbonsäurebisimid (104 mg, 0.075 mmol) wurde in 6 mL trockenem und entgasten Tetrahydrofuran gelöst, zu der auf 10 °C abgekühlten Nickel-Lösung gegeben und für 110 min bei 10 °C gerührt. Anschließend wurden 200 µL Brombenzol zugegeben und für 3 Stunden bei 60 °C gerührt. Nach dem Abkühlen auf Raumtemperatur wurde die Reaktionslösung auf eine gerührte Mischung aus 100 mL MeOH und 50 mL konz. HCl. gegeben. Das Gemisch wurde für eine Stunde gerührt, die Niederschläge abfiltriert und mit MeOH gewaschen. Der Rückstand wurde in Chloroform aufgenommen auf 3 mL eingeengt und langsam zu 150 mL MeOH gegeben. Nach drei Stunden Rühren wurde abfiltriert und der Rückstand mit MeOH gewaschen. Dieser Vorgang wurde einmal wiederholt. Der erhaltene violette Festoff wurde im Vakuum getrocknet und aus Benzol lyophilisiert. Der so erhaltene Polymerschaum wurde anschließend für 3 Stunden in einer Soxhlet-Appartur mit n-Pentan gewaschen. Dieser Vorgang wurde einmal wiederholt. Es wurden 66 mg (72 % d.Th.) eines violetten Polymerschaumes erhalten.
- GPC: Mn: 12 kD, Mw: 39 kD, PDI: 3.1. - FT-IR: (v in cm⁻¹): 2922 (m, CH₂), 2853 (m, CH₂), 1735 (s, O-(C=O)-O), 1704 (s, N-C=O), 1664 (vs, N-C=O), 1572 (m). Abspaltungstemperatur: T_{On} = 175 °C.

### Beispiel 4 - Vorrichtungen

### 4.1 - 4.4 Solarzellen mit Polymer 3.1 und PCBM-C60

BHJ-Solarzellen wurden wie folgt hergestellt:
Alle organischen Solarzellen wurden auf mit Indium Zinn Oxid beschichtetem Glas aufgebracht (R□ ≈ 13 Ω/□). Dieses wurde zuvor mittels Salzsäure strukturiert und anschließend in Aceton und Isopropanol für jeweils 15 min gereinigt, letzte organische Rückstände wurden durch ein Sauerstoff Plasma entfernt.

Die organischen Schichten wurden mittels Spincoating unter Schutzgasatmosphäre (N₂) abgeschieden.

Die Lochinjektionsschicht bestehend aus Poly(3,4-ethylendioxythiophen): Poly(styrolsulfonat) (PEDOT:PSS) wurde im Verhältniss 1:1 mit Wasser verdünnt und bei 4000 rpm abgeschieden. Um letzte Wasserrückstände zu entfernen, wurden die Proben bei 120°C für 10 min im Vakuumofen getrocknet.

Der Absorber, bestehend aus dem Mischsystem Polymer 3.1 und PC₆₀BM (1:0,75), wurde in einer Konzentration von 40 mg/ml (Dichlorbenzol) gelöst. Anschließend wurde das Mischsystem 120 s lang bei 800 rpm aufgeschleudert, gefolgt von einem 10-minütigen Ausheizschritt bei 200°C zur thermischen Abspaltung der löslichkeitsvermittelnden Gruppen. Als Referenz wurden ebenfalls Solarzellen ohne diesen Ausheizschritt gebaut, wobei diese Referenz-Solarzellen lediglich zur Verdampfung von Lösemittel-Resten auf nur 150°C erhitzt wurden, so dass die Seitengruppen nicht abgespalten wurden.

Als Elektrode wurden mittels thermischer Sublimation 20 nm Kalzium gefolgt von 200 nm Aluminium abgeschieden.

Die Spannungs-Stromdichte (J-V) Kennlinie wurde mittels einer "Source Measurement Unit" (kurz: SMU, Keitley 238) aufgezeichnet. Anhand der so ermittelten Spannungs- und Stromwerte konnten die Füllfaktoren und Effizienzen berechnet werden.

Die photoaktive Schicht enthält eine Mischung aus Polymer 3.1 als Donor und PC₆₀BM als Akzeptor.

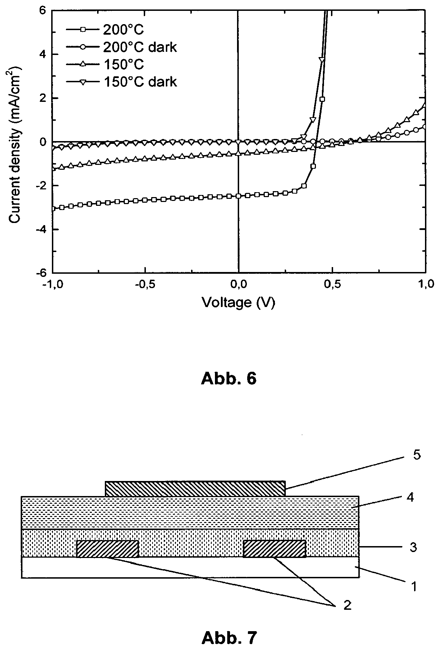

Die UJ-Kennlinie in **Abbildung 6** zeigt eine Zunahme des Photostroms nach Abspalten der Seitengruppen sowie eine gleichzeitige Abnahme des Serienwiderstandes der Solarzelle (inverse Steigung der Kennline zu großen Vorwärts-Spannungen). Das Abspalten der Seitengruppen beim Erhitzen der Schicht führt zu einer Volumenschrumpfung, wodurch sich die Polymerketten reorganisieren und sich die Distanz zwischen den Polymerketten verringert. Diese Verringerung des Abstandes führt zu einer besseren Photogeneration von Ladungsträgern und einem besseren intermolekularen Ladungstransfer ("bessere Leitfähigkeit").

Die Kennwerte für die OPV-Vorrichtung sind in Tabelle 1 gezeigt.

**Tabelle 1**

| Bsp. | Photoaktive Schicht | J_{SC} [mA/cm²] | V_{OC} [V] | FF [%] | η [%] | Rₛ [Ω] |
|---|---|---|---|---|---|---|
| 4.1 | Polymer 3.1 / PCBM Ausheiz Temp. 150°C | 0,6 | 0,62 | 34 | 0,12 | 1,5 |
| 4.2 | Polymer 3.1 / PCBM Ausheiz Temp. 200°C | 2,5 | 0,42 | 68 | 0,71 | 0,1 |

Darin bedeuten:
J_{SC} = Kurzschlussstrom (engl. "Short Circuit Current")
V_{OC} = Leerlaufspannung (engl. "Open Circuit Voltage")
FF = Füllfaktor (engl. "Fill Factor")
η = Wirkungsgrad (engl "Power Conversion Efficiency, PCE")
Rₛ = Serienwiederstand (engl "Serial Resistance")

### 4.5 - 4.6 OLEDs mit einer Emitter-Schicht enthaltend Polymer 3.2 bzw. 3.3

OLEDs wurden wie folgt hergestellt:
Alle OLEDs wurden auf mit Indium Zinn Oxide beschichtetem Glas abgeschieden (R□ ≈ 13 Ω/□). Dieses wurde zuvor mittels Salzsäure strukturiert und anschließend in Aceton und Isopropanol für jeweils 15 min gereinigt, letzte organische Rückstände wurden durch ein Sauerstoffplasma entfernt.

Die organischen Schichten wurden mittels Spincoating unter Schutzgasatmosphäre (N₂) abgeschieden.

Die Lochinjektionsschicht, bestehend aus Poly(3,4-ethylendioxythiophen) Poly(styrolsulfonat) (PEDOT:PSS), wurde im Verhältnis 1:1 mit Wasser verdünnt und bei 4000 rpm abgeschieden. Um letzte Wasserrückstände zu entfernen, kamen die Proben bei 120°C für 10 min in den Vakuumofen.

Der Emitter bestehend aus Polymer 3.2 wurde in einer Konzentration von 8 g/l in Toluol gelöst und bei 1000 rpm abgeschieden, gefolgt von einem Ausheizschritt bei 200°C über einen Zeitraum von 10 min zur thermischen Abspaltung der Löslichkeits-vermittelnden Gruppen. Als Referenz wurden ebenfalls OLEDs ohne diese Ausheizschritt gebaut.

Als Elektrode wurden mittels thermischer Sublimation 0,7 nm Lithiumfluorid, gefolgt von 200 nm Aluminium, abgeschieden.

Die Spannungs- Stromdichte (J-V) Kennlinie wurde mittels einer "Source Measuremnet Unit" (kurz: SMU, Keitley 238) aufgezeichnet. Mittels der so ermittelten Spannungs-, Stromwerten und der aus dem Spektrum brechneten Leuchtdichte konnte schließlich die Leistungs- und Strom-Effizienz errechnet werden.

Das Spektrometer wurde zuvor mit einem Halogenstandard (Philips FEL-1000W) kalibriert. Leistungs- und Strom Effizienz wurden unter Annahme einer Lambert'schen Lichtausbreitung berechnet.

Bei Polymer 3.2 handelt es sich um ein blau emittierendes Polymer. Die Löslichkeitsgruppe kann wie zuvor erwähnt durch Zufügen von thermischer Energie (200°C) über einen Zeitraum von 10 min abgespalten werden. Löslichkeitstests vor und nach dem Abspalten haben gezeigt, dass der Schichtdickenverlust bei etwa 10 nm liegt, ausgehend von einer Anfangsschichtdicke von ∼80 nm. Diese Abnahme entspricht einem Massenverlust durch das Verdampfen der Carboxyl- und der 1,1-Dimethylpentyl-Gruppen.

Zur Charakterisierung der Oberfläche wurden rasterkraftmikroskopische (AFM) Untersuchungen durchgeführt.

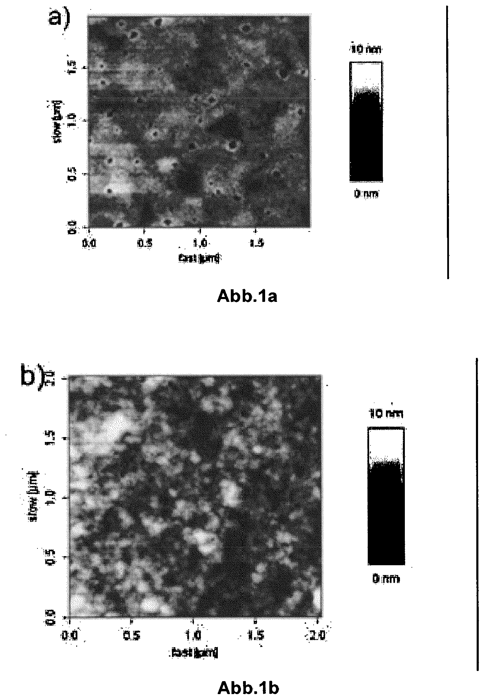

**Abbildung 1a****-c** zeigt AFM Aufnahmen der Topographie a) einer unbehandelten, b) einer thermisch behandelten (200°C, 10 min) und c) einer thermisch behandelten und mit Toluol abgespülten Polymer 3.2 Schicht.

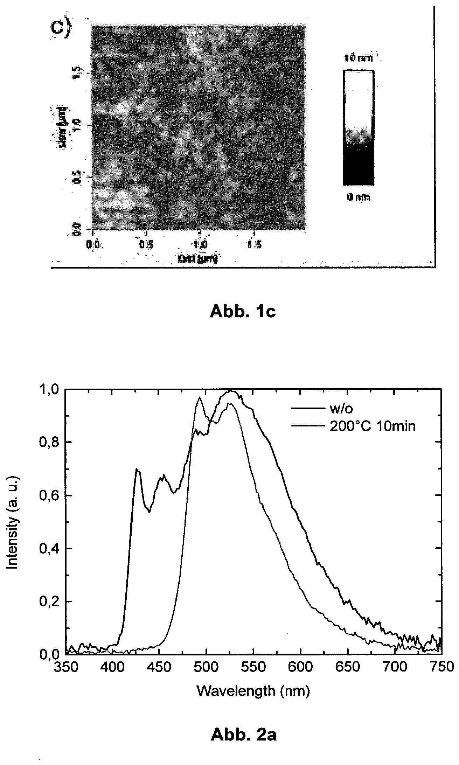

**Abbildung 1a** zeigt die Topographie einer Polymer 3.2 Schicht, die nicht thermisch behandelt wurde. Die AFM-Aufnahme zeigt deutlich Löcher in der Schicht. Diese Defekte können durch die thermische Nachbehandlung "ausgeheilt" werden, wie in **Abbildung 1b** ersichtlich wird. Die zum Abspalten der Seitengruppen notwendige Temperatur ist demnach hoch genug (oberhalb der Glas-Temperatur des Polymers), um eine Reorganisation des Polymerfilms zu ermöglichen und die Löcher zu schließen. Auch nach einem Abspülen der thermisch behandelten Schicht mit dem zur Schichtdeposition verwendeten Toluol bildet Polymer 3.2 einen geschlossenen Film (**Abbildung 1c**).

Wichtige Aussagen über den Emitter können im Allgemeinen aus dem Emissionsspektrum gewonnen werden. **Abbildung 2a****-b** zeigen das Emissionsspektrum und den Farbort einer OLED mit Polymer 3.2 vor und nach thermischer Nachbehandlung Das Elektrolumineszenz-Spektrum von Polymer 3.2 vor und nach der thermischen Behandlung bei 200°C zeigt Unterschiede (**Abbildung 2a**). Diese Beobachtung ist vermutlich ein direkter Nachweis der Abspaltung der Seitengruppen im thermisch behandelten Film.

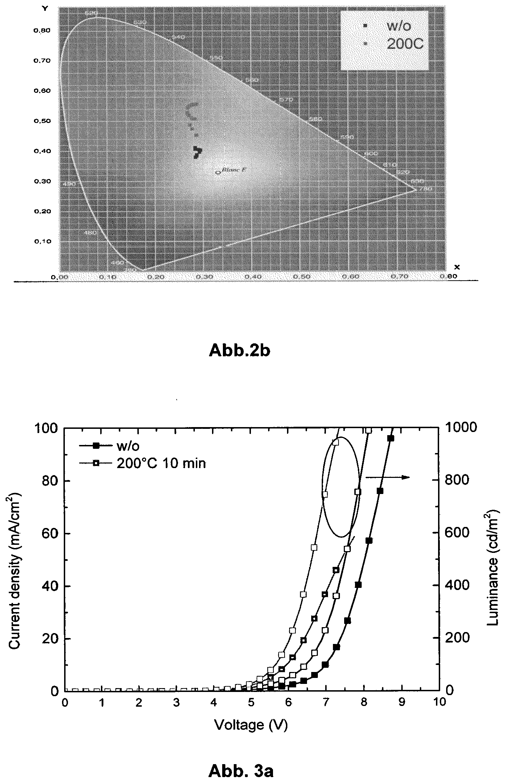

Entsprechend der Veränderung der spektralen Emission ändern sich auch die Farbkoordinaten der OLEDs mit der thermischen Behandlung (**Abbildung 2b**). Die Farbkoordinaten verschieben sich von der Nähe des Weißpunktes (CRI: ∼65) hin zu einer grünen Emission.

**Abbildung 3a****-d** zeigen die optoelektronischen Kenndaten einer OLED mit Polymer 3.2-Emitter. Die Einsatzspannung konnte von 3,9 V auf 3,6 V gesenkt und der Roll-Off der Effizienzen deutlich reduziert werden.

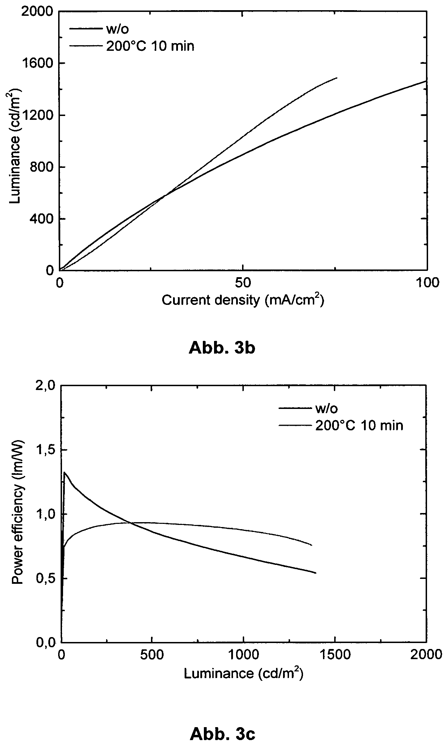

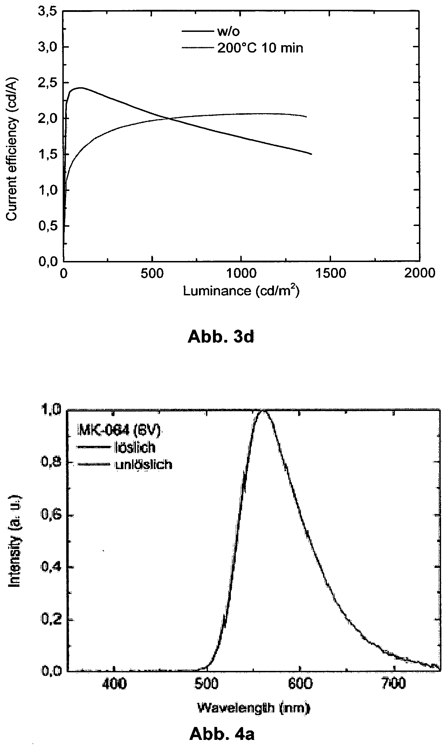

Die optoelektronische Charakterisierung (**Abbildung 3**) des Polymer 3.2-Emitters zeigt unter anderen, dass die Einsatzspannung durch die thermische Behandlung von ca. 3,9 V auf 3,6 V sinkt. Durch das Ausheizen konnten zudem der Roll-Off der Effizienzen zu höheren Leuchtdichten hin deutlich reduziert und Effizienzen von ∼0,9 lm/W und ∼2,1 cd/A bei 1000 cd/m² erreicht werden.

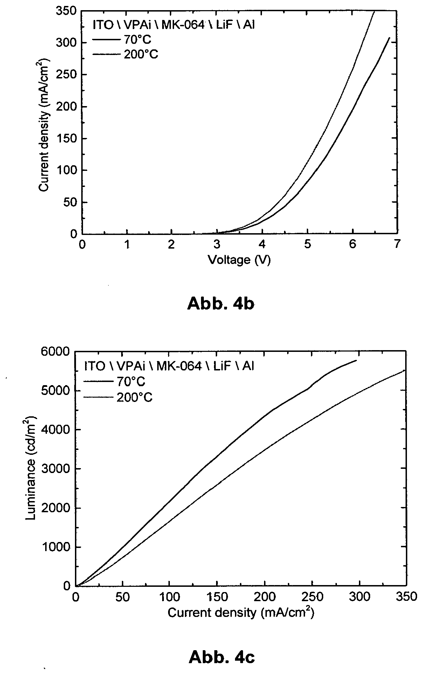

**Abbildung 4a** zeigt für Polymer 3.3 den Vergleich der Elektroluminszenz-Spektren bei einer Spannung von 6 V vor (löslich) und nach (unlöslich) thermischer Behandlung.

Gegenüber dem zuvor untersuchten Polymer 3.2, enthält das Polymer 3.3 Thiobenzodiazole-Akzeptoreinheiten. Das Emissions-Spektrum des Polymers 3.3 (**Abbildung 4a**) zeigt im Gegensatz zu Polymer 3.2 keine spektrale Verschiebung mehr. Die zusätzlichen Akzeptoreinheiten im Polymer erhöhen insgesamt dessen Akzeptorstärke, so dass die Wechselwirkung mit den Carboxyl-Seitengruppen durchaus anders sein kann. Auch hier müssen weitere spektroskopische Untersuchungen durchgeführt werden, um die Wechselwirkung der Carboxyl-Seitengruppen mit dem π-System des Polymers näher zu untersuchen.

**Abbildung 4b** zeigt die optoelektronischen Kenndaten einer OLED mit Polymer 3.3-Emitter: Die Einsatzspannung liegt bei ∼3,2V. Es wurden Leuchtdichten von bis zu 5500 cd/m² erreicht. Die Effizienzen weisen lediglich einen geringen Roll-Off auf.

Die optoelektronische Kenndaten (**Abbildung 4b****-d**) der Polymer 3.3-OLEDs zeigen eine Einsatzspannung von 3,2 V. Die Effizienzen nehmen nach dem Abspalten der Löslichkeitsgruppen minimal ab. Beide Bauteile weisen nur einen geringen Roll-Off in den Effizienzen auf. Die Effizienzen liegen bei 1000 cd/m² bei etwa 1,5 cd/A und 1,0 lm/W. Die maximal erreichte Leuchtdichte ist ∼5500 cd/m² bei einem Strom von 300 mA/cm².

Die Löslichkeitsschaltbarkeit der Polymere kann nun genutzt werden, um weitere funktionale Schichten wie z.B. eine lochblockierende Schicht auf den Emittern abzuscheiden. Dies wirkt sich i.A. sehr positiv auf das Ladungsträgergleichgewicht aus und sollte so die Effizienz der Bauteile weiter erhöhen.

### 4.7 - 4.9 OFETs mit einer aktiven Halbleiterschicht enthaltend Polymer 3.4 bzw. 3.8 und nicht erfindungsgemäßem Polymer 3.7

Organische Feld-Effekt-Transistoren (OFETs) wurden wie folgt hergestellt:
Alle OFETs wurden auf Glassubstraten hergestellt, die zuvor jeweils für 15 min in Aceton und Isopropanol im Ultraschallbad gereinigt wurden. Die Source- und Drain-Elektroden aus Gold mit einer Höhe von 50 nm wurden durch thermische Verdampfung über eine Schattenmaske im Hochvakuum strukturiert aufgedampft. Die Dimensionen der einzelnen OFETs betrugen: Kanallänge: 50 µm, Kanalbreite: 1000 µm, Elektrodenhöhe: 50 nm.

**Abbildung 7** zeigt den hier verwendeten Aufbau eines Top-Gate/Bottom-Contact OFET. Als Trägermaterial wurden Glassubstrate (1) verwendet. Die Source-(S)- und Drain-(D)-Elektroden (2) aus Silber wurden über eine Schattenmaske im Hochvakuum aufgedampft und besitzen eine Dicke von ca. 50 nm. Die aktive Schicht (3) wurde mittels Spincoating unter Schutzgasatmosphäre (N₂) abgeschieden.

Zur Abscheidung der aktiven Halbleiterschicht (3) mit einer Schichtdicke zwischen 80 und 130 nm wurde eine Lösung von 5-10 mg/L Polymer 3.4, 3.7 oder 3.8 in Chlorbenzol bei 1000 rpm mit einer Beschleunigung von 500 rpm/s und einer Gesamtdauer von 60 s abgeschieden. Um Lösungsmittelrückstände zu entfernen wurden die Proben anschließend bei 100°C für 180 s getempert. Zur thermischen Abspaltung der Löslichkeits-vermittelnden Gruppen wurde bei 180°C bis 220°C für 3-5 min ausgeheizt. Als Referenz wurden ebenfalls OFETs ohne diesen Ausheizschritt hergestellt

Als Dielektrikum (4) wurden 300 nm Parylen-C durch Gasphasenabscheidung mithilfe des Gerätes PDS2010 von "Special Coatings Systems" auf das gesamte Substrat aufgetragen, um die äußeren Einflüsse auf die Halbleiterschicht so gering wie möglich zu halten.

Die Gate-Elektroden (5) aus Silber wurden über eine Schattenmaske im Hochvakuum aufgedampft.

Dünnschicht-Transistor (TFT) Transfer Messungen wurden bei Raumtemperatur unter Raumluft mit einem "Semiconductor Parameter Analyzer" (Agilent 4155C) durchgeführt. Mittels der so erhaltenen TFT Transfer-Kennlinien konnte schließlich die Mobilität des halbleitenden Polymers in diesem Aufbau bestimmt werden.

Bei den Polymeren 3.4 und 3.8 handelt es sich um halbleitende Polymere mit n-Kanal-Charakter (Elektronenleitung). Die Löslichkeitsvermittelnde Gruppe kann wie zuvor erwähnt durch Zufügen von thermischer Energie (200 - 220 °C) über einen Zeitraum von 5 min abgespalten werden. Löslichkeitstests vor und nach dem Abspalten haben gezeigt, dass die Löslichkeit nach der Pyrolyse deutlich abnimmt. Der Schichtdickenverlust während der Pyrolyse liegt zwischen 20 und 35 %, ausgehend von einer Anfangsschichtdicke zwischen 25 und 40 nm. Diese Abnahme entspricht dem Massenverlust durchVerdampfen der Carboxyl- und der AlkylGruppen sowie einem geringen Maß an Verdichtung der Schicht.

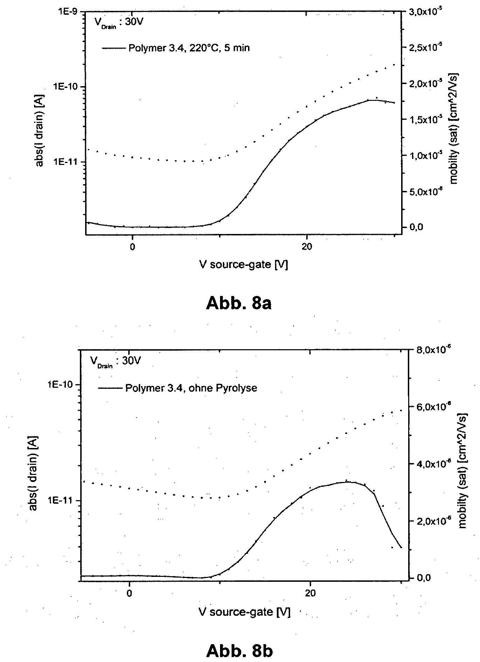

Bei Polymer 3.7 handelt es sich um ein nicht erfindungsgemäßes halbleitendes Polymer mit n-Kanal-Charakter (Elektronenleitung). Die Löslichkeitsvermittelnde Gruppe kann durch Zufügen von thermischer Energie (180 - 200 °C) über einen Zeitraum von 5 min abgespalten werden. Nach der thermischen Behandlung zeigt die Schicht eine sehr gute Lösungsmittelbeständigkeit. **Abbildung 8a** zeigt die TFT Transfer Kennlinien eines OFET (Source-Drain-Spannung 30 V) im gesättigten Regime mit Polymer 3.4 als Halbleiter nach einer thermischen Behandlung von 220 °C für 5 min. Der Kennlinie ist zu entnehmen, dass es sich bei dem beobachteten Phänomen um Elektronenleitung handelt. Bei einer Source-Gate-Spannung von 35 V wurde die höchste Mobilität gemessen, sie beträgt µₑ = 1,7•10⁻⁵ cm²/Vs. Das On/Off-Verhältnis des Transistors wurde mit 1:20 bestimmt.

**Abbildung 8b** zeigt die TFT Transfer Kennlinien eines OFET (Source-Drain-Spannung 30 V) im gesättigten Regime mit Polymer 3.4 als Halbleiter ohne thermische Behandlung. Der Kennlinie ist zu entnehmen, dass das Polymer 3.4 auch ohne Abspaltung der Löslichkeitsgruppen bereits elektronenleitenden Charakter besitzt. Die maximal bestimmte Mobilität beträgt in diesem Fall µₑ = 3,3•10⁻⁶ cm²/Vs und das On/Off-Verhältnis wurde mit 1:4 bestimmt. Damit liegen diese Werte deutlich unterhalb dessen, was für dasselbe Polymer nach der thermischen Behandlung bestimmt wurde.

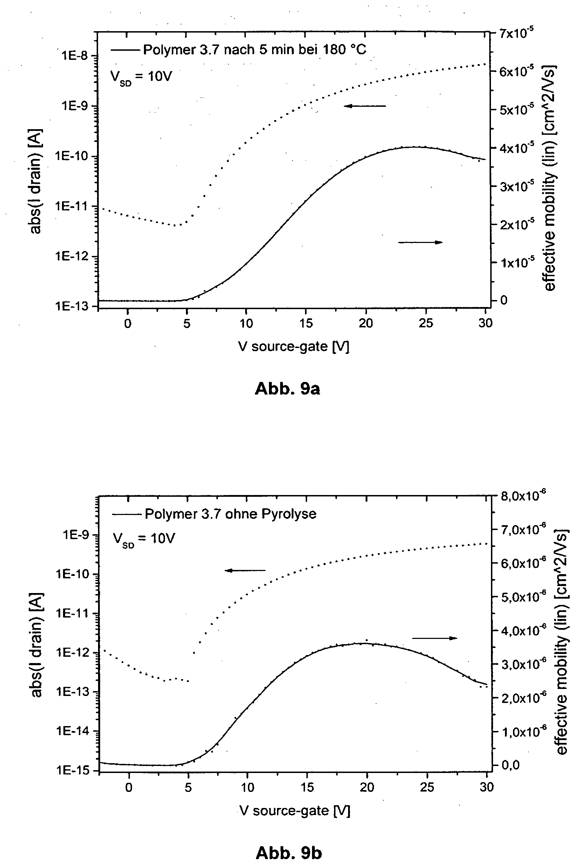

**Abbildung 9a** zeigt die TFT Transfer Kennlinien eines OFET (Source-Drain Spannung 10 V) im linearen Regime mit nicht erfindungsgemäßem Polymer 3.7 als Halbleiter nach einer thermischen Behandlung von 180 °C für 5 min. Der Kennlinie ist zu entnehmen, dass es sich bei dem beobachteten Phänomen um Elektronenleitung handelt. Bei einer Source-Gate-Spannung von 23 V wurde die höchste effektive Mobilität gemessen, sie beträgt µₑ = 4•10⁻⁵ cm²/Vs. Das On/Off-Verhältnis des Transistors wurde mit 10³ bestimmt.

**Abbildung 9b** zeigt die TFT Transfer Kennlinien eines OFET (Source-Drain Spannung 10 V) im linearen Regime mit nicht erfindungsgemäßem Polymer 3.7 als Halbleiter ohne thermische Behandlung. Der Kennlinie ist zu entnehmen, dass das Polymer 3.7 auch vor der Abspaltung der Löslichkeitsgruppen bereits elektronenleitenden Charakter besitzt. Die maximal bestimmte effektive Mobilität beträgt in diesem Fall µₑ = 3,5•10⁻⁶ cm²/Vs und das On/Off-Verhältnis wurde mit 10³ bestimmt. Die effektive Mobiltät des ungespaltenen Materials liegt somit etwa eine Größenordnung unterhalb jenes nach der Spaltung, wobei das On/Off-Verhältnis vergleichbar bleibt

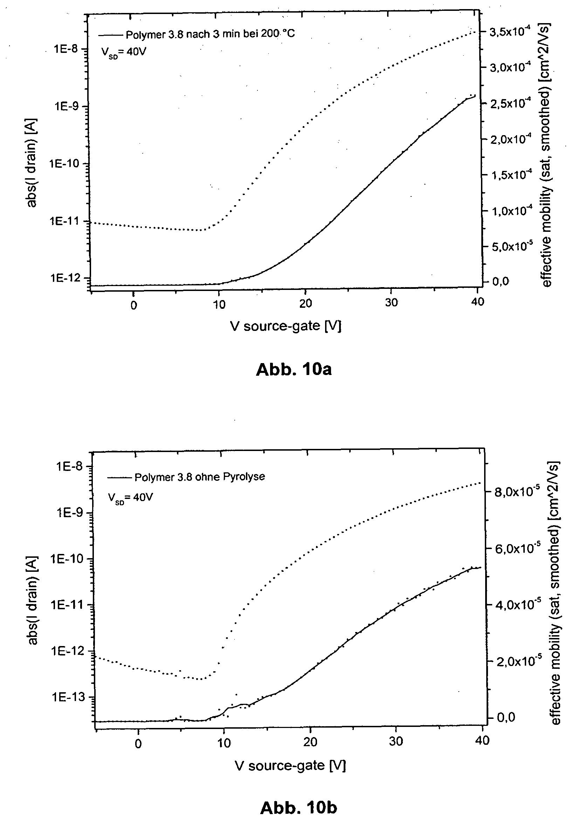

**Abbildung 10a** zeigt die TFT Transfer Kennlinien eines OFET (Source-Drain Spannung 40 V) im gesättigten Regime mit Polymer 3.8 als Halbleiter nach einer thermischen Behandlung von 200 °C für 3 min. Der Kennlinie ist zu entnehmen, dass es sich bei dem beobachteten Phänomen um Elektronenleitung handelt. Bei einer Source-Gate-Spannung von 40 V wurde die höchste effektive Mobilität gemessen, sie beträgt µₑ = 2.6•10⁻⁴ cm²/Vs. Das On/Off-Verhältnis des Transistors wurde mit 10⁴ bestimmt.

**Abbildung 10b** zeigt die TFT Transfer Kennlinien eines OFET Source-Drain Spannung 40 V) im gesättigten Regime mit Polymer 3.8 als Halbleiter ohne thermische Behandlung. Der Kennlinie ist zu entnehmen, dass das Polymer 3.8 auch vor der Abspaltung der Löslichkeitsgruppen bereits elektronenleitenden Charakter besitzt. Die maximal bestimmte effektive Mobilität beträgt in diesem Fall µₑ = 5.3•10⁻⁵ cm²/Vs und das On/Off-Verhältnis wurde mit 10⁴ bestimmt. Die effektive Mobiltät des ungespaltenen Materials liegt somit etwa eine Größenordnung unterhalb jener nach der Spaltung, wobei das On/Off-Verhältnis vergleichbar bleibt.

Die Löslichkeitsschaltbarkeit der Polymere kann nun genutzt werden, um weitere funktionale Schichten wie z.B. lösungsprozessierte Dielektrika und Elektroden auf der Halbleiterschicht abzuscheiden. Je mehr aufeinanderfolgende Prozessschritte auf lösungsmittelbasierten Abscheidetechniken beruhen, desto effizienter kann die Herstellung der OFET-Bauelemente gestaltet werden.

## Patentansprüche

1. Konjugiertes Polymer enthaltend eine oder mehrere gleiche oder verschiedene Wiederholungseinheiten der Formel I 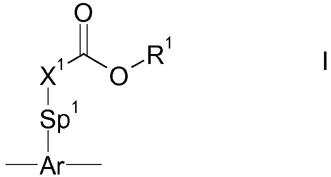 worin die einzelnen Reste folgende Bedeutung besitzen:
Ar mono- oder polycyclisches Aryl oder Heteroaryl, welches zusätzlich in einer oder mehreren Positionen substituiert sein kann,
Sp¹ Alkylen mit 1 bis 20 C-Atomen, welches unsubstituiert oder ein- oder mehrfach durch F, Cl, Br, I oder CN substituiert ist, und worin auch eine oder mehrere nicht benachbarte CH₂-Gruppen jeweils unabhängig voneinander so durch -O-, -S-, -NH-, -NR⁰-, -SiR⁰R⁰⁰-, -CO-, -COO-, -OCO-, -OCO-O-, -S-CO-, -CO-S-,-CR⁰=CR⁰⁰- oder -C≡C- ersetzt sein können, dass O- und/oder S-Atome nicht direkt miteinander verknüpft sind,
X¹ NR⁰ oder O,
R⁰ und R⁰⁰ jeweils unabhängig voneinander H oder Alkyl mit 1 bis 12 C-Atomen, und
R¹ ein Hydrocarbylrest mit 1 bis 40 C-Atomen,
ausgenommen
Wiederholungseinheiten der Formel I, worin Ar Phenylen bedeutet, welches ein- oder mehrfach mit -NH-C(O)-OR¹ substituiert ist, und welches in der Polymerhauptkette direkt benachbart zu einer gegebenenfalls substituierten Benzo[lmn][3,8]phenanthrolin-1,3,6,8-tetraon-4,9-diyl-Einheit ist, sowie
Wiederholungseinheiten der Formel I, worin Ar Pyrrolo[3,4-c]pyrrol-1,4-dion-3,6-diyl bedeutet worin beide N-Atome mit -C(O)-OR¹ substituiert sind.

2. Konjugiertes Polymer nach Anspruch 1, worin die Wiederholungseinheiten der Formel I aus folgenden Formeln ausgewählt sind 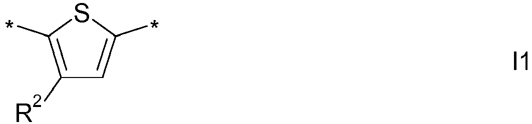 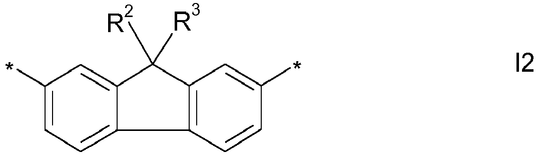 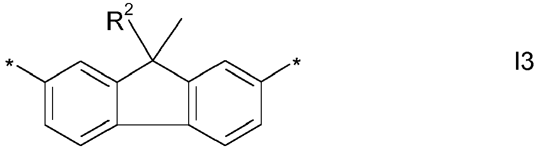 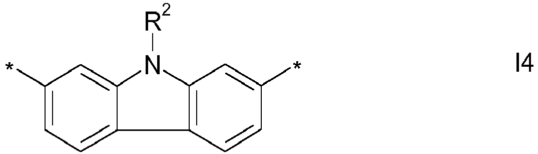 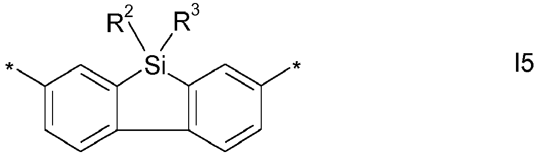 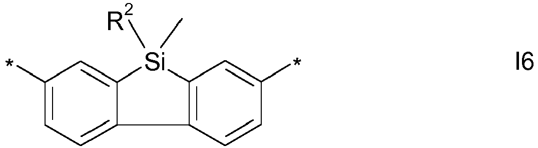 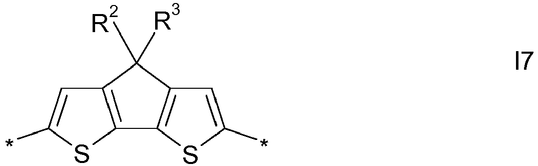 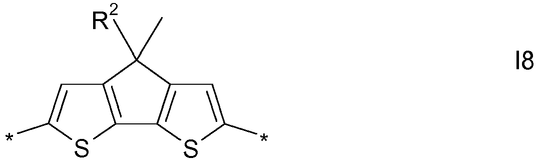 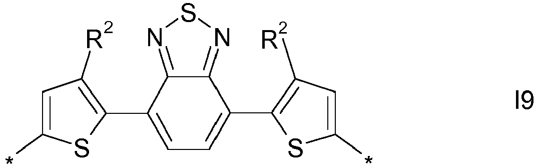 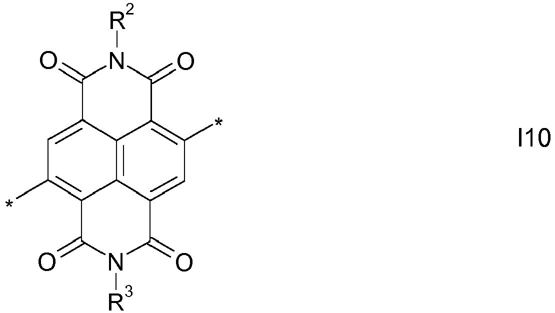 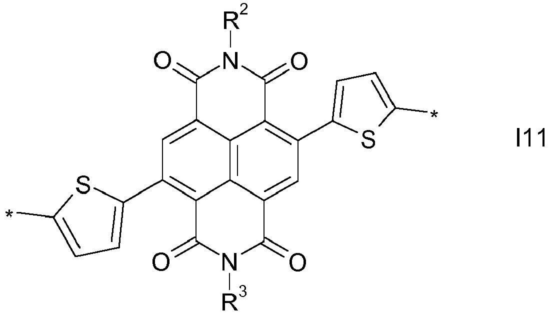 worin R² und R³ jeweils unabhängig voneinander, und bei jedem Auftreten gleich oder verschieden, einen Rest -Sp¹-X¹-C(O)-O-R¹ bedeutet, und Sp¹, X¹ und R¹ die in Anspruch 1 angegebenen Bedeutungen besitzen.

3. Konjugiertes Polymer nach Anspruch 1 oder 2, worin der Rest Sp¹ Alkylen mit 1 bis 20 C-Atomen bedeutet.

4. Konjugiertes Polymer nach einem der Ansprüche 1 bis 3, worin der Rest R¹ geradkettiges, verzweigtes oder cyclisches Alkyl mit 1 bis 25 C-Atomen bedeutet, welches unsubstituiert oder ein- oder mehrfach durch F, Cl, Br, I oder CN substituiert ist, und worin auch eine oder mehrere nicht benachbarte CH₂-Gruppen jeweils unabhängig voneinander so durch -O-, -S-, -C(O)-, -C(S)-, -C(O)-O-, -O-C(O)-,-NR⁰-, -SiR⁰R⁰⁰-, -CF₂-, -CHR⁰=CR⁰⁰-, -CY¹=CY²- oder -C≡C- ersetzt sein können, dass O- und/oder S-Atome nicht direkt miteinander verknüpft sind, wobei R⁰ und R⁰⁰ die in Anspruch 1 angegebene Bedeutung besitzen.

5. Konjugiertes Polymer nach einem der Ansprüche 1 bis 4, worin der Rest X¹ O oder NH bedeutet.

6. Konjugiertes Polymer nach einem der Ansprüche 1 bis 5, worin der Rest -Sp¹-X¹-C(O)-O-R¹ aus folgender Formel ausgewählt ist: 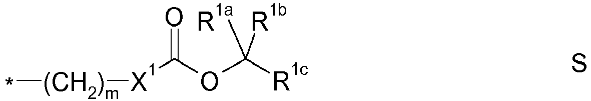 worin X¹ die in Anspruch 1 oder 5 angegebene Bedeutung hat, R^{1a}, R^{1b} und R^{1c} jeweils unabhänging voneinander H oder einen geradkettigen, verzweigten oder cyclischen Alkylrest mit 1 bis 25 C-Atomen oder einen geradkettigen, verzweigten oder cyclischen Alkenylrest oder Alkinylrest mit jeweils 2 bis 25 C-Atomen bedeuten, wobei auch zwei der Reste R^{1a}, R^{1b} und R^{1c} zusammen einen cyclischen Alkylrest, Alkenylrest oder Alkinylrest mit jeweils 5 bis 12 C-Atomen bilden können, m eine ganze Zahl von 1 bis 12, vorzugsweise 2, 3, 4, 5 oder 6 bedeutet, und das Symbol * die Verknüpfung mit dem Rest Ar bedeutet.

7. Konjugiertes Polymer nach einem der Ansprüche 1 bis 6, worin der Rest -Sp¹-X¹-C(O)-O-R¹ aus folgenden Formeln ausgewählt ist: 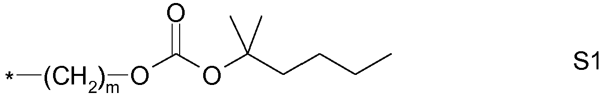 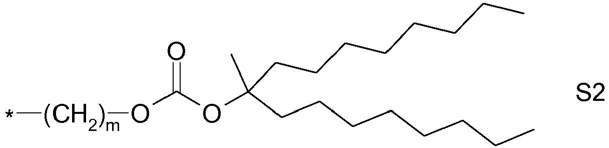 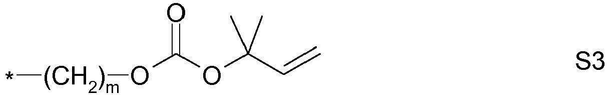 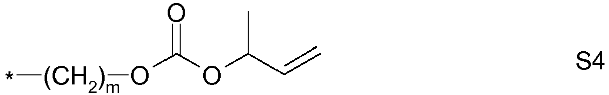 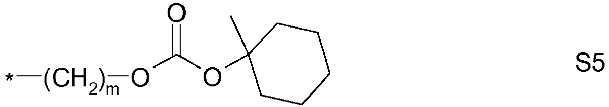 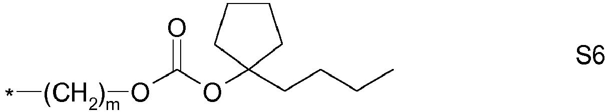 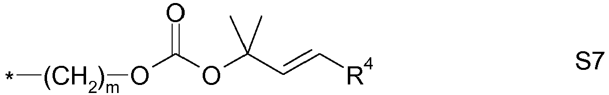 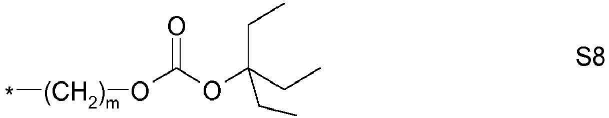 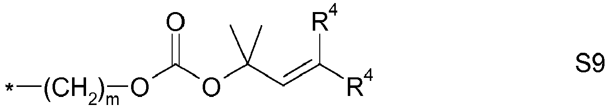 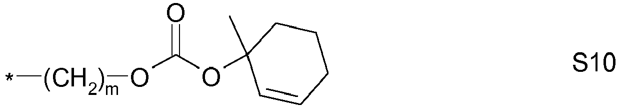 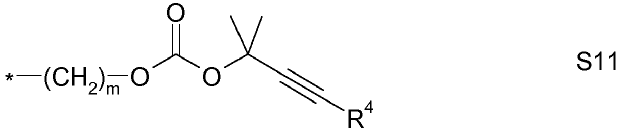 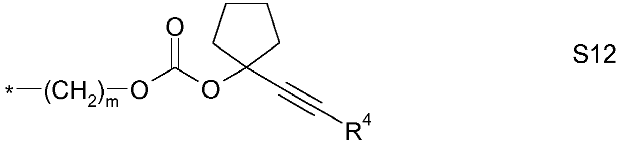 worin R⁴ bei jedem Auftreten gleich oder verschieden H oder einen geradkettigen oder verzweigten Alkylrest mit 1 bis 25 C-Atomen bedeutet, m eine ganze Zahl von 1 bis 12 bedeutet, und das Symbol * die Verknüpfung mit dem Rest Ar bedeutet.

8. Konjugiertes Polymer nach einem der Ansprüche 1 bis 7, enthaltend eine oder mehrere Wiederholungseinheiten der Formel IIa oder IIb:
-[(Ar¹)ₐ-(U)_{b}-(Ar²)_{c}-(Ar³)_{d}]- IIa
-[(U)_{b}-(Ar¹)ₐ-(U)_{b}-(Ar²)_{c}-(Ar³)_{d}]- IIb
worin die einzelnen Reste folgende Bedeutung besitzen
U eine Einheit der Formel I oder I1 bis I11 wie in einem der Ansprüche 1 bis 7 definiert,
Ar¹, Ar², Ar³ bei jedem Auftreten gleich oder verschieden, und jeweils unabhängig voneinander, Aryl oder Heteroaryl welches von U verschieden ist, vorzugsweise 5 bis 30 Ringatome aufweist, und gegebenenfalls substitutiert ist, vorzugsweise mit einer oder mehreren Gruppen R^{S},
R^{S} bei jedem Auftreten gleich oder verschieden F, Br, Cl, -CN,-NC, -NCO, -NCS, -OCN, -SCN, -C(O)NR⁰R⁰⁰, -C(O)X⁰,-C(O)R⁰, -NH₂, -NR⁰R⁰⁰, -SH, -SR⁰, -SO₃H, -SO₂R⁰, -OH,-NO₂, -CF₃, -SF₅, gegebenenfalls substitutiertes Silyl oder Hydrocarbyl mit 1 bis 40 C-Atomen, welches gegebenenfalls substitutiert ist und gegebenenfalls ein oder mehrere Heteroatome enthält,
R⁰ und R⁰⁰ wie in Anspruch 1 definiert,
X⁰ Halogen, vorzugsweise F, Cl oder Br,
a, b und c bei jedem Auftreten gleich oder verschieden und jeweils unabhängig voneinander 0, 1 oder 2,
d bei jedem Auftreten gleich oder verschieden 0 oder eine ganze Zahl von 1 bis 10,
wobei das konjugierte Polymer mindestens eine Wiederholungseinheit der Formel IIa oder IIb enthält, worin b mindestens 1 ist.

9. Konjugiertes Polymer nach einem Ansprüche 1 bis 8, zusätzlich enthaltend eine oder mehrere, gegebenenfalls substituierte, mono- oder polycyclische Aryl- oder Heteroaryleinheiten.

10. Konjugiertes Polymer nach Anspruch 9, worin die zusätzlichen, gegebenenfalls substituierten, mono- oder polycyclischen Aryl- oder Heteroarylgruppen aus Formel IIIa oder IIIb ausgewählt sind
-[(Ar¹)ₐ-(Ar⁴)_{b}-(Ar²)_{c}-(Ar³)_{d}]- IIIa
-[(Ar⁴)_{b}-(Ar¹)ₐ-(Ar⁴)_{b}-(Ar²)_{c}-(Ar³)_{d}]- IIIb
worin Ar¹, Ar², Ar³, a, b, c und d wie in Anspruch 8 definiert sind, und Ar⁴ eine Aryl- oder Heteroarylgruppe bedeutet, welche von U und Ar¹⁻³ verschieden ist, vorzugsweise 5 bis 30 Ringatome aufweist, und gegebenenfalls substitutiert ist, vorzugsweise mit einer oder mehreren Gruppen R^{S}, wobei das konjugierte Polymer mindestens eine Wiederholungseinheit der Formel IIIa oder IIIb enthält worin b mindestens 1 ist.

11. Konjugiertes Polymer nach einem der Ansprüche 1 bis 10, ausgewählt aus Formel IV: 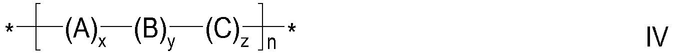 worin die einzelnen Reste folgende Bedeutung besitzen
A, B, C jeweils unabhängig voneinander eine verschiedene Einheit der Formel I, I1 bis I9, IIa, IIb, IIIa oder IIIb wie in einem der Ansprüche 1 bis 10 definiert,
x > 0 und ≤ 1,
y ≥ 0 und < 1,
z ≥ 0 und < 1,
x+y+z 1, und
n eine ganze Zahl >1.

12. Konjugiertes Polymer nach Anspruch 11, ausgewählt aus folgenden Unterfomeln
*-[(Ar¹-U-Ar²)ₓ-(Ar³)_{y}]ₙ-* IVa
*-[(Ar¹-U-Ar²)ₓ-(Ar³-Ar³)_{y}]ₙ-* IVb
*-[(Ar¹-U-Ar²)ₓ-(Ar³-Ar³-Ar³)_{y}]ₙ-* IVc
*-[(Ar¹)ₐ-(U)_{b}-(Ar²)_{c}-(Ar³)_{d}]ₙ-* IVd
*-([(Ar¹)ₐ-(U)_{b}-(Ar²)_{c}-(Ar³)_{d}]ₓ-[(Ar¹)ₐ-(Ar)_{b}-(Ar²)_{c}-(Ar³)_{d}]_{y})ₙ-* IVe
*-[(U-Ar¹-U)ₓ-(Ar²-Ar³)_{y}]ₙ-* IVf
*-[(U-Ar¹-U)ₓ-(Ar²-Ar³-Ar²)_{y}]ₙ-* IVg
*-[(U)_{b}-(Ar¹)ₐ-(U)_{b}-(Ar²)_{c}]ₙ-* IVh
*-([(U)_{b}-(Ar¹)ₐ-(U)_{b}-(Ar²)_{c}]ₓ-[(A^{c})_{b}-(Ar¹)ₐ-(Ar⁴)_{b}-(Ar²)_{d}]_{y})ₙ-* IVi
*-[(U-Ar¹)ₓ-(U-Ar²)_{y}-(U-Ar³)_{z}]ₙ-* IVk
worin U, Ar¹, Ar², Ar³, a, b, c und d bei jedem Auftreten gleich oder verschieden eine der in Anspruch 8 angegebenen Bedeutungen besitzen, Ar⁴ bei jedem Auftreten gleich oder verschieden eine der in Anspruch 10 angegebenen Bedeutungen besitzt, und x, y, z und n wie in Anspruch 11 definiert sind, wobei diese Polymere alternierende oder statistische Copolymere sein können, und wobei in Formel IVd und IVe in mindestens einer Wiederholungseinheit [(Ar¹)ₐ-(U)_{b}-(Ar²)_{c}-(Ar³)_{d}] und in in mindestens einer Wiederholungseinheit [(Ar¹)ₐ-(Ar⁴)_{b}-(Ar²)_{c}-(Ar³)_{d}] b mindestens 1 ist, und in Formel IVh und IVi in mindestens einer Wiederholungseinheit [(U)_{b}-(Ar¹)ₐ-(U)_{b}-(Ar²)_{d}] und in mindestens einer Wiederholungseinheit [(U)_{b}-(Ar¹)ₐ-(U)_{b}-(Ar²)_{d}] b 1 ist.

13. Konjugiertes Polymer nach einem der Ansprüche 1 bis 12, worin Ar¹, Ar², Ar³ und Ar⁴, jeweils unabhängig voneinander, und bei jedem Auftreten gleich oder verschieden, ausgewählt sind aus der Gruppe bestehend aus 1,4-Phenylen, Thiophen-2,5-diyl, Selenophen-2,5-diyl, Thieno[3,2-*b*]thiophen-2,5-diyl, Thieno[2,3-*b*]thiophen-2,5-diyl, Selenopheno[3,2-*b*]selenophen-2,5-diyl, Selenopheno[2,3-b]selenophen-2,5-diyl, Selenopheno[3,2-*b*]thiophen-2,5-diyl, Selenopheno[2,3-*b*]thiophen-2,5-diyl, Benzo[1,2-*b*:4,5-*b*']dithiophen-2,6-diyl, 2,2-Dithiophen, 2,2-Diselenophen, Dithieno[3,2-*b*:2',3'-*d*]silol-5,5-diyl, 4*H*-Cyclopenta[2,1-*b*:3,4-*b*']dithiophen-2,6-diyl, Carbazol-2,7-diyl, Fluoren-2,7-diyl, Indaceno[1,2-*b*:5,6-*b*']dithiophen-2,7-diyl, Benzo[1",2":4,5;4",5":4',5']bis(silolo[3,2-*b*:3',2'-*b*']thiophen)-2,7-diyl, Phenanthro[1,10,9,8-*c*,*d*,*e*,*f*,*g*]carbazol-2,7-diyl, Benzo[2,1,3]thiadiazol-4,7-diyl, Benzo[2,1,3]selenadiazol-4,7-diyl, Benzo[2,1,3]oxadiazol-4,7-diyl, 2H-Benzotriazol-4,7-diyl, 3,4-Difluorthiophen-2,5-diyl, Thieno[3,4-*b*]pyrazin-2,5-diyl, Chinoxalin-5,8-diyl, Thieno[3,4-*b*]thiophen-4,6-diyl, Thieno[3,4-*b*]thiophen-6,4-diyl, 3,6-Di-thien-2-yl-pyrrolo[3,4-c]pyrrol-1,4-dion oder [1,3]Thiazolo[5,4-d][1,3]thiazol-2,5-diyl, wobei die vorstehenden Reste alle unsubstituiert oder ein- oder mehrfach, vorzugsweise mit R^{S} wie in Anspruch 8 definiert, substituert sein können.

14. Konjugiertes Polymer nach einem der Ansprüche 1 bis 13, ausgewählt aus folgenden Formeln: 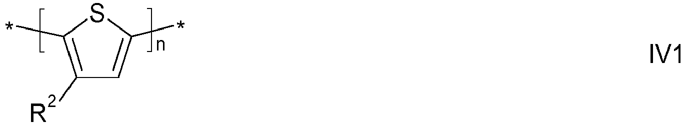 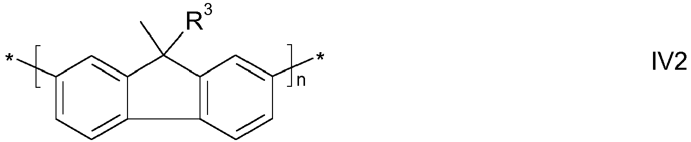 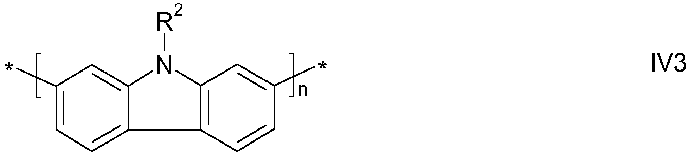 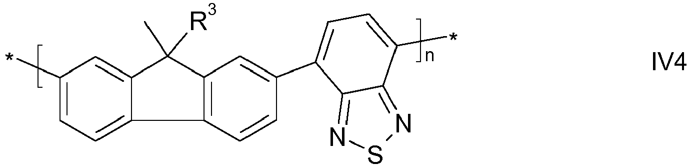 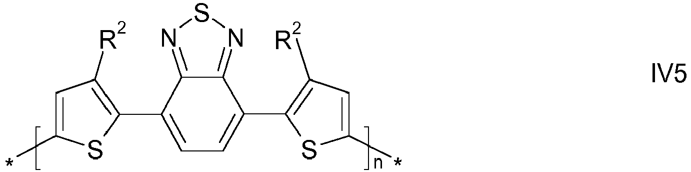 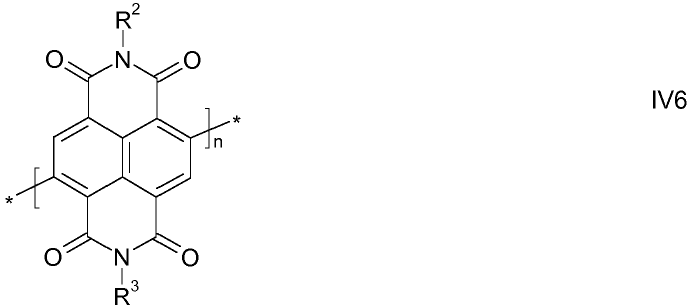 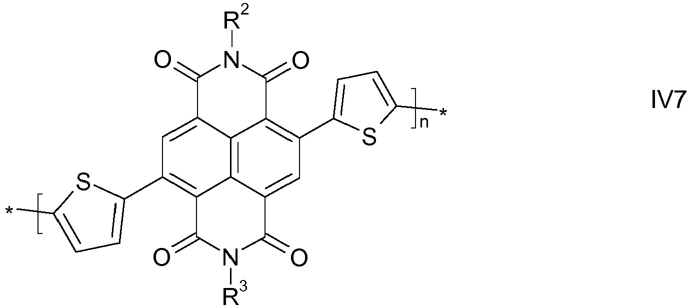 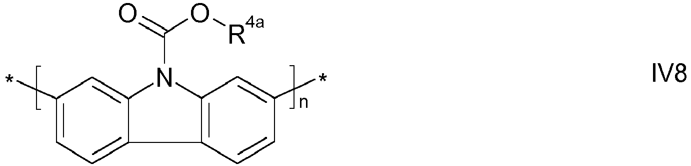 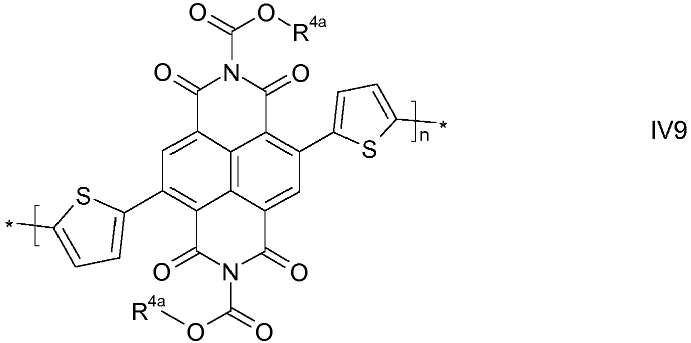 worin R² und R³ die in Anspruch 2 angegebene Bedeutung besitzen, R^{4a} einen geradkettigen oder verzweigten Alkylrest mit 1 bis 25 C-Atomen, vorzugsweise 2-Methylhexyl oder 9-Methylheptadecyl bedeutet, und n die in Anspruch 11 angegebene Bedeutung besitzt.

15. Konjugiertes Polymer nach einem der Ansprüche 1 bis 14, ausgewählt aus folgender Formel:
R⁵-Kette-R⁶ V
worin "Kette" eine Polymerkette ausgewählt aus den Formeln IV, IVa bis IVk und IV1 bis IV9 wie in Anspruch 11, 12 und 14 definiert bedeutet, und R⁵ und R⁶ jeweils unabhängig voneinander eine der für R^{S} in Anspruch 8 angegebenen Bedeutungen besitzen, oder H, F, Br, Cl, I, -CH₂Cl, -CHO, -CR'=CR"₂, -SiR'R"R''', -SiR'X'X", -SiR'R"X',-SnR'R"R"', -BR'R", -B(OR')(OR"), -B(OH)₂, -O-SO₂-R', -C≡CH, -C≡C-SiR'₃ oder -ZnX' bedeuten, worin X' und X" Halogen bedeuten, R', R" und R''' jeweils unabhängig voneinander eine der für R⁰ in Anspruch 1 angegebenen Bedeutungen besitzen, und zwei der Reste R', R" und R''' zusammen mit dem jeweiligen Heteroatom, an das sie gebunden sind, auch eine Cyclosilyl-, Cyclostannyl-, Cycloboran- oder Cycloboronatgruppe mit 2 bis 20 C-Atomen bilden können.

16. Mischung oder Polymerblend enthaltend ein oder mehrere Polymere nach einem der Ansprüche 1 bis 15 und eine oder mehrere Verbindungen oder Polymeren mit Halbleiter-, Ladungstransport-, Loch/Elektronentransport-, loch/elektronenblockierenden, elektrisch leitenden, photoleitenden oder lichtemittierenden Eigenschaften.

17. Mischung oder Polymerblend nach Anspruch 16, enthaltend ein oder mehrere Polymere gemäß der vorliegenden Erfindung und eine oder mehrere zusätzliche Verbindungen ausgewählt aus Elektronenakzeptoren oder organischen Halbleitern des n-Typs.

18. Mischung oder Polymerblend nach Anspruch 17, worin der organische Halbleiter des n-Typs ausgewählt ist aus der Gruppe bestehend aus Fullerenen und substituierten Fullerenen.

19. Formulierung enthaltend ein oder mehrere Polymere, Mischungen oder Polymerblends nach einem der Ansprüche 1 bis 18 und ein oder mehrere Lösungsmitteln, vorzugsweise ausgewählt aus organischen Lösungsmitteln.

20. Verwendung eines Polymers, einer Mischung, eines Polymerblends oder einer Formulierung nach einem der Ansprüche 1 bis 19 als Ladungstransport-, Halbleiter-, elektrisch leitendes, photoleitendes oder lichtemittierendes Material in einer optischen, elektrooptischen, elektronischen, Elektrolumineszenz- oder Photolumineszenz-Vorrichtung, in einer Komponente einer solchen Vorrichtung, oder in einem Produkt enhaltend eine solche Vorrichtung.

21. Ladungstransport-, Halbleiter-, elektrisch leitendes, photoleitendes oder lichtemittierendes Material, enthaltend ein Polymer, eine Mischung ein Polymerblend oder eine Formulierung nach einem der Ansprüche 1 bis 19.

22. Vorrichtung mit optischen, elektrooptischen, elektronischen, Elektrolumineszenz- oder Photolumineszenz-Eigenschaften, Komponente einer solchen Vorrichtung, oder Produkt enthaltend eine solche Vorrichtung, enthaltend ein Polymer, eine Mischung, ein Polymerblend oder eine Formulierung nach einem der Ansprüche 1 bis 19.

23. Vorrichtung nach Anspruch 22, ausgewählt aus der Gruppe bestehend aus organischen Feldeffekttransistoren (organic field effect transistors - OFETs), organischen Dünnschichttransistoren (organic thin film transistors - OTFTs), organischen Leuchtdioden (organic light emitting diodes - OLEDs), organischen lichtemittierende Transistoren (organic light emitting transistors - OLETs), organischen Photovoltaikbauteilenvorrichtungen (OPV), organischen Photodetektoren (OPD), organischen Solarzellen, Schottky-Dioden, Laserdioden, und organischen Photoleiter.

24. OFET, OPV-Vorrichtung, organische Solarzelle oder OPD nach Anspruch 23, worin die organischen Halbleiter in der photoaktiven Schicht einen Volumen-Heteroübergang bilden.

25. Komponente nach Anspruch 22, ausgewählt aus der Gruppe bestehend aus Ladungsinjektionsschichten, Ladungstransportschichten, Zwischenschichten, Planarisierungsschichten, Antistatikfolien, Polymerelektrolytmembranen (PEMs), leitenden Substraten und leitenden Mustern.

26. Produkt nach Anspruch 22, ausgewählt aus der Gruppe bestehend aus integrierten Schaltungen (integrated circuits - ICs), Kondensatoren, RFID-Tags (RFID - radio frequency identification), RFID-Tags enthaltende Sicherheitsmarkierungen oder Sicherheitsvorrichtungen, Flachbildschirmen, Hintergrundbeleuchtungen für Anzeigen, elektrophotographischen Vorrichtungen, elektrophotographischen Aufzeichnungsvorrichtungen, organischen Speichervorrichtungen, Sensorvorrichtungen, Biosensoren und Biochips.

27. Verwendung eines Polymers, einer Mischung, eines Polymerblends oder einer Formulierung nach einem der Ansprüche 1 bis 19 in Batterien, oder in Komponenten oder Vorrichtungen für den Nachweis und die Unterscheidung von DNA-Sequenzen.

28. Monomer der Formel VIa oder VIb
R⁷-(Ar¹)ₐ-U-(Ar²)_{c}-R⁸ VIa
R⁷-U-(Ar¹)ₐ-U-R⁸ VIb
worin U, Ar¹, Ar², a und b die in Anspruch 8 oder 13 angegebene Bedeutung besitzen und R⁷ und R⁸ jeweils unabhängig voneinander ausgewählt sind aus der Gruppe bestehend aus Cl, Br, I, O-Tosylat, O-Triflat, O-Mesylat, O-Nonaflat, -SiMe₂F, -SiMeF₂, -O-SO₂Z¹,-B(OZ²)₂, -CZ³=C(Z³)₂, -C≡CH, - C≡CSi(Z¹)₃, -ZnX⁰ und -Sn(Z⁴)₃, worin X⁰ Halogen, vorzugsweise Cl, Br oder I bedeutet, Z¹⁻⁴ ausgewählt ist aus der Gruppe bestehend aus Alkyl und Aryl, welche gegebenenfalls substitutiert sein können, und zwei Reste Z² zusammen mit den B- und O-Atomen auch eine Cycloboronatgruppe mit 2 bis 20 C-Atomen bilden können, ausgenommen Monomere der Formel VIa, worin a und c 0 sind und U mit einer Carbamatgruppe substituiertes Phenylen-2,5,diyl bedeutet.

29. Monomer nach Anspruch 28, ausgewählt aus folgenden Formeln:
R⁷-Ar¹-U-Ar²-R⁸ VI1
R⁷-U-R⁸ VI2
R⁷-Ar¹-U-R⁸ VI3
R⁷-U-Ar²-R⁸ VI4
R⁷-U-Ar¹-U-R⁸ VI5
worin U, Ar¹, Ar², R⁷ und R⁸ wie in Anspruch 28 definiert sind.

30. Verfahren zur Herstellung eines Polymers nach einem der Ansprüche 1 bis 15 durch Reaktion eines oder mehrerer Monomere nach Anspruch 28 oder 29, worin R⁷ und R⁸ ausgewählt sind aus Cl, Br, I, -B(OZ²)₂ und -Sn(Z⁴), miteinander und/oder mit einem oder mehreren Monomeren ausgewählt aus folgenden Formeln
R⁷-(Ar¹)ₐ-A^{c}-(Ar²)_{c}-R⁸ VIII
R⁷-Ar¹-R⁸ IX
R⁷-Ar³-R⁸ X
worin Ar¹, Ar², a und c wie in Anspruch 28 definiert sind, Ar³ wie in Anspruch 8 oder 13 definiert ist, und Ar⁴ wie in Anspruch 10 oder 13 definiert ist, in einer Aryl-Aryl-Kupplungsreaktion.

31. Verfahren zur teilweisen oder vollständigen Abspaltung der Carbonat- oder Carbamatgruppen eines Polymers nach einem der Ansprüche 1 bis 15, durch Erhitzen des Polymers, oder einer Schicht enthaltend das Polymer, auf eine Temperatur von ≤200°C.

## Claims

1. Conjugated polymer containing one or more identical or different recurring units of the formula I 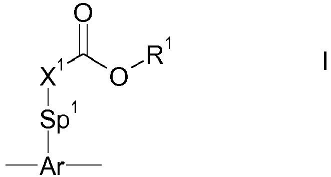 in which the individual radicals have the following meanings:
Ar denotes mono- or polycyclic aryl or heteroaryl, which may additionally be substituted in one or more positions,
Sp¹ denotes alkylene having 1 to 20 C atoms, which is unsubstituted or mono- or polysubstituted by F, Cl, Br, I or CN, and in which, in addition, one or more non-adjacent CH₂ groups may in each case be replaced, independently of one another, by -O-, -S-, -NH-, -NR⁰-, -SiR⁰R⁰⁰-, -CO-, -COO-, -OCO-, -OCO-O-, -S-CO-, -CO-S-, -CR⁰=CR⁰⁰- or -C≡C- in such a way that O and/or S atoms are not linked directly to one another,
X¹ denotes NR⁰ or O,
R⁰ and R⁰⁰ in each case, independently of one another, denote H or alkyl having 1 to 12 C atoms, and
R¹ denotes a hydrocarbyl radical having 1 to 40 C atoms,
except for
recurring units of the formula I in which Ar denotes phenylene which is mono- or polysubstituted by -NH-C(O)-OR¹, and which is directly adjacent to an optionally substituted benzo[lmn][3,8]phenanthroline-1,3,6,8-tetraone-4,9-diyl unit in the main polymer chain, and recurring units of the formula I in which Ar denotes pyrrolo[3,4-c]-pyrrole-1,4-dione-3,6-diyl, in which both N atoms are substituted by -C(O)-OR¹.

2. Conjugated polymer according to Claim 1, in which the recurring units of the formula I are selected from the following formulae: 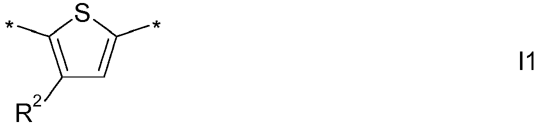 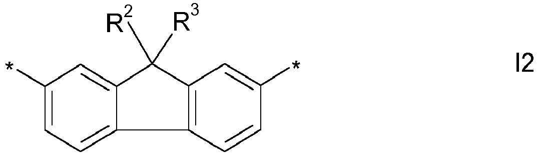 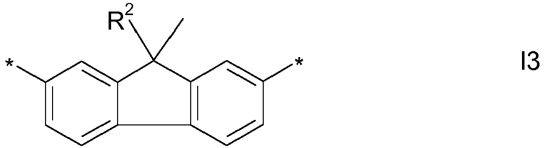 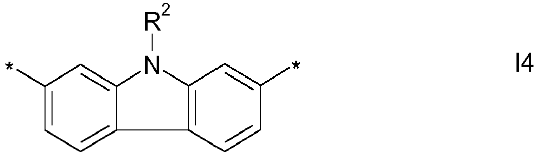 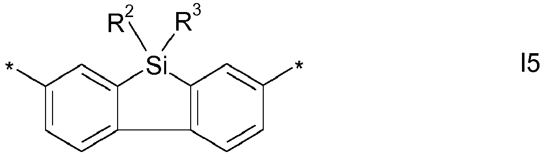 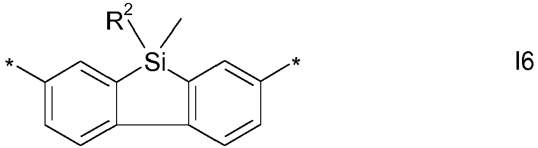 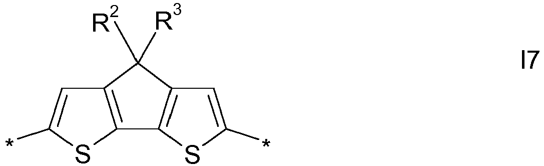 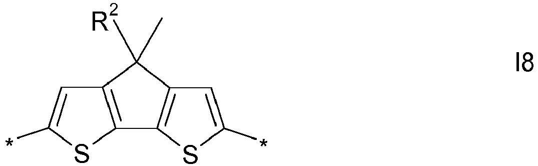 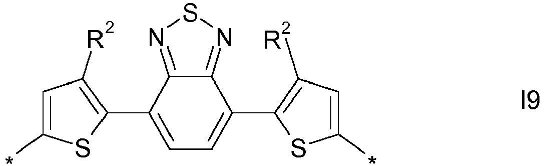 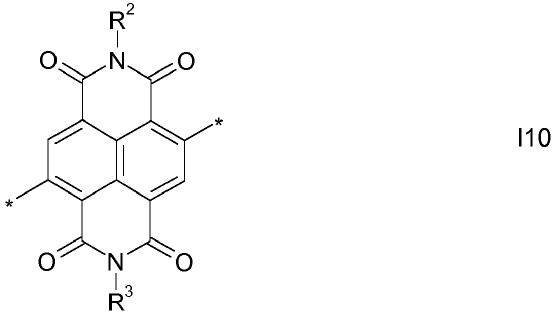 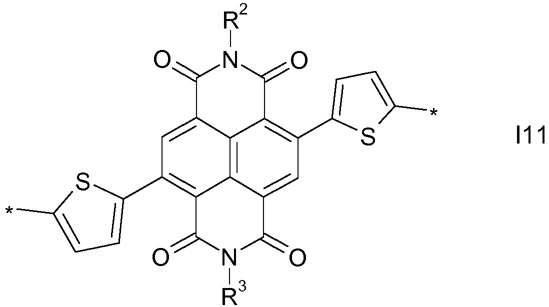 in which R² and R³ each, independently of one another, and identically or differently on each occurrence, denote a radical -Sp¹-X¹-C(O)-O-R¹, and Sp¹, X¹ and R¹ have the meanings indicated in Claim 1.

3. Conjugated polymer according to Claim 1 or 2, in which the radical Sp¹ denotes alkylene having 1 to 20 C atoms.

4. Conjugated polymer according to one of Claims 1 to 3, in which the radical R¹ denotes straight-chain, branched or cyclic alkyl having 1 to 25 C atoms, which is unsubstituted or mono- or polysubstituted by F, Cl, Br, I or CN, and in which, in addition, one or more non-adjacent CH₂ groups may each be replaced, independently of one another, by -O-, -S-, -C(O)-, -C(S)-, -C(O)-O-, -O-C(O)-, -NR⁰-, -SiR⁰R⁰⁰-, -CF₂-, -CHR⁰=CR⁰⁰-, -CY¹=CY²- or -C≡C- in such a way that O and/or S atoms are not linked directly to one another, where R⁰ and R⁰⁰ have the meanings indicated in Claim 1.

5. Conjugated polymer according to one of Claims 1 to 4, in which the radical X¹ denotes O or NH.

6. Conjugated polymer according to one of Claims 1 to 5, in which the radical -Sp¹-X¹-C(O)-O-R¹ is selected from the following formula: 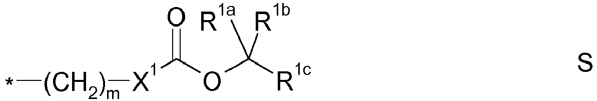 in which X¹ has the meaning indicated in Claim 1 or 5, R^{1a}, R^{1b} and R^{1c} each, independently of one another, denote H or a straight-chain, branched or cyclic alkyl radical having 1 to 25 C atoms or a straight-chain, branched or cyclic alkenyl radical or alkynyl radical, each having 2 to 25 C atoms, where, in addition, two of the radicals R^{1a}, R^{1b} and R^{1c} together may form a cyclic alkyl radical, alkenyl radical or alkynyl radical, each having 5 to 12 C atoms, m denotes an integer from 1 to 12, preferably 2, 3, 4, 5 or 6, and the symbol * denotes the link to the radical Ar.

7. Conjugated polymer according to one of Claims 1 to 6, in which the radical -Sp¹-X¹-C(O)-O-R¹ is selected from the following formulae: 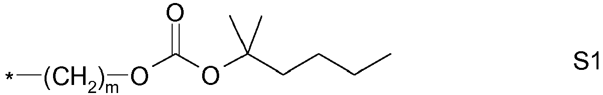 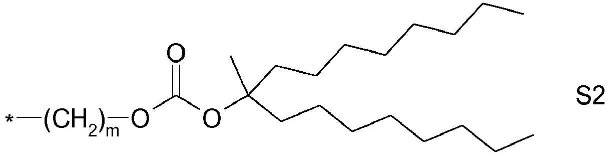 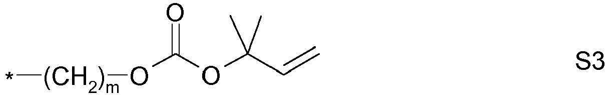 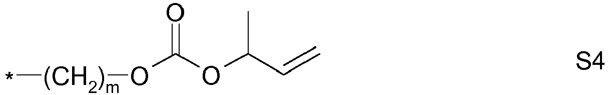 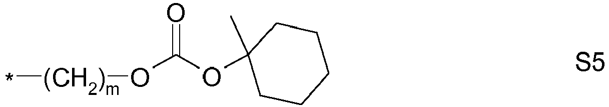 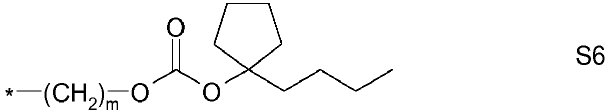 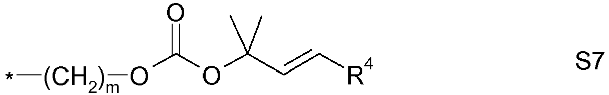 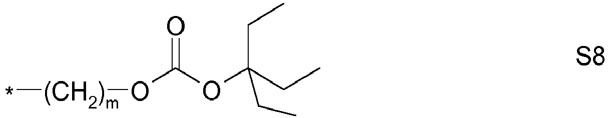 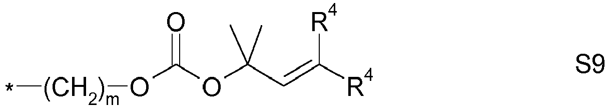 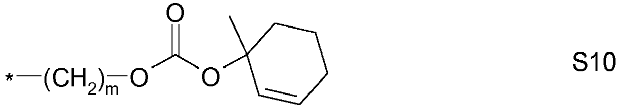 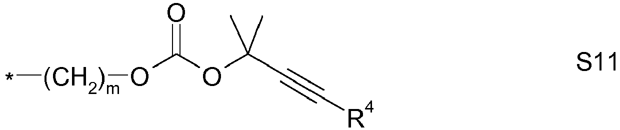 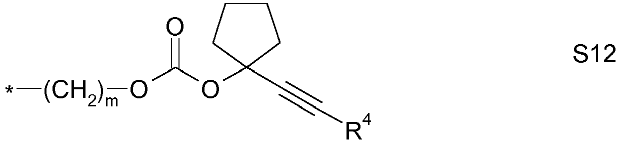 in which R⁴ on each occurrence, identically or differently, denotes H or a straight-chain or branched alkyl radical having 1 to 25 C atoms, m denotes an integer from 1 to 12, and the symbol * denotes the link to the radical Ar.

8. Conjugated polymer according to one of Claims 1 to 7, containing one or more recurring units of the formula IIa or IIb:
-[(Ar¹)ₐ-(U)_{b}-(Ar²)_{c}-(Ar³)_{d}]- IIa
-[(U)_{b}-(Ar¹)ₐ-(U)_{b}-(Ar²)_{c}-(Ar³)_{d}]- IIb
in which the individual radicals have the following meanings:
U denotes a unit of the formula I or I1 to 117 as defined in one of Claims 1 to 7,
Ar¹, Ar², Ar³ on each occurrence, identically or differently, and in each case independently of one another, denote aryl or heteroaryl which is different from U, preferably has 5 to 30 ring atoms, and is optionally substituted, preferably by one or more groups R^{S},
R^{S} on each occurrence, identically or differently, denotes F, Br, Cl, -CN, -NC, -NCO, -NCS, -OCN, -SCN, -C(O)NR⁰R⁰⁰, -C(O)X⁰, -C(O)R⁰, -NH₂, -NR⁰R⁰⁰, -SH, -SR⁰, -SO₃H, -SO₂R⁰, -OH, -NO₂, -CF₃, -SF₅, optionally substituted silyl or hydrocarbyl having 1 to 40 C atoms, which is optionally substituted and optionally contains one or more heteroatoms,
R⁰ and R⁰⁰ are defined as in Claim 1,
X⁰ denotes halogen, preferably F, Cl or Br,
a, b and c on each occurrence, identically or differently and in each case independently of one another, denote 0, 1 or 2,
d on each occurrence, identically or differently, denotes 0 or an integer from 1 to 10,
where the conjugated polymer contains at least one recurring unit of the formula IIa or IIb in which b is at least 1.

9. Conjugated polymer according to one of Claims 1 to 8, additionally containing one or more optionally substituted mono- or polycyclic aryl or heteroaryl units.

10. Conjugated polymer according to Claim 9, in which the additional optionally substituted mono- or polycyclic aryl or heteroaryl groups are selected from formulae IIIa and IIIb
-[(Ar¹)ₐ-(Ar⁴)_{b}-(Ar²)_{c}-(Ar³)_{d}]- IIIa
-[(Ar⁴)_{b}-(Ar¹)ₐ-(Ar⁴)_{b}-(Ar²)_{c}-(Ar³)_{d}]- IIIb
in which Ar¹, Ar², Ar³, a, b, c and d are defined as in Claim 8, and Ar⁴ denotes an aryl or heteroaryl group which is different from U and Ar¹⁻³, preferably has 5 to 30 ring atoms, and is optionally substituted, preferably by one or more groups R^{S}, where the conjugated polymer contains at least one recurring unit of the formula IIIa or IIIb in which b is at least 1.

11. Conjugated polymer according to one of Claims 1 to 10, selected from formula IV: 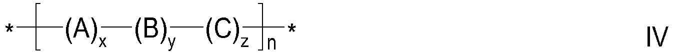 in which the individual radicals have the following meanings:
A, B, C each, independently of one another, denote a different unit of the formula I, I1 to 19, IIa, IIb, IIIa or IIIb as defined in one of Claims 1 to 10,
x is > 0 and ≤ 1,
y is ≥ 0 and < 1,
z is ≥ 0 and < 1,
x+y+z is 1, and
n is an integer >1.

12. Conjugated polymer according to Claim 11, selected from the following sub-formulae:
*-[(Ar¹-U-Ar²)ₓ-(Ar³)_{y}]ₙ-* IVa
*-[(Ar¹-U-Ar²)ₓ-(Ar³-Ar³)_{y}]ₙ-* IVb
*-[(Ar¹-U-Ar²)ₓ-(Ar³-Ar³-Ar³)_{y}]ₙ-* IVc
*-[(Ar¹)ₐ-(U)_{b}-(Ar²)_{c}-(Ar³)_{d}]ₙ-* IVd
*-([(Ar¹)ₐ-(U)_{b}-(Ar²)_{c}-(Ar³)_{d}]ₓ-[(Ar¹)ₐ-(Ar⁴)_{b}-(Ar²)_{c}-(Ar³)_{d}]_{y})ₙ-* IVe
*-[(U-Ar¹-U)ₓ-(Ar²-Ar³)_{y}]ₙ-* IVf
*-[(U-Ar¹-U)ₓ-(Ar²-Ar³-Ar²)_{y}]ₙ-* IVg
*-[(U)_{b}-(Ar¹)ₐ-(U)_{b}-(Ar² )_{c}]ₙ-* IVh
*-([(U)_{b}-(Ar¹)ₐ-(U)_{b}-(Ar²)_{c}]ₓ-[(A^{c})_{b}-(Ar¹)ₐ-(Ar⁴)_{b}-(Ar²)_{d}]_{y})ₙ-* IVi
*-[(U-Ar¹)ₓ-(U-Ar²)_{y}-(U-Ar³)_{z}]ₙ-* IVk
in which U, Ar¹, Ar², Ar³, a, b, c and d on each occurrence, identically or differently, have one of the meanings indicated in Claim 8, Ar⁴ on each occurrence, identically or differently, has one of the meanings indicated in Claim 10, and x, y, z and n are defined as in Claim 11, where these polymers may be alternating or random copolymers, and where, in formulae IVd and IVe, b is at least 1 in at least one recurring unit [(Ar¹)ₐ-(U)_{b}-(Ar²)_{c}-(Ar³)_{d}] and in at least one recurring unit [(Ar¹)ₐ-(Ar⁴)_{b}-(Ar²)_{c}-(Ar³)_{d}], and, in formulae IVh and IVi, b is 1 in at least one recurring unit [(U)_{b}-(Ar¹)ₐ-(U)_{b}-(Ar²)_{d}] and in at least one recurring unit [(U)_{b}-(Ar¹)ₐ-(U)_{b}-(Ar²)_{d}].

13. Conjugated polymer according to one of Claims 1 to 12, in which Ar¹, Ar², Ar³ and Ar⁴, in each case independently of one another, and identically or differently on each occurrence, are selected from the group consisting of 1,4-phenylene, thiophene-2,5-diyl, selenophene-2,5-diyl, thieno[3,2-*b*]thiophene-2,5-diyl, thieno[2,3-*b*]thiophene-2,5-diyl, selenopheno[3,2-*b*]selenophene-2,5-diyl, selenopheno[2,3-*b*]-selenophene-2,5-diyl, selenopheno[3,2-*b*]thiophene-2,5-diyl, seleno-pheno[2,3-*b*]thiophene-2,5-diyl, benzo[1,2-*b*:4,5-*b*']dithiophene-2,6-diyl, 2,2-dithiophene, 2,2-diselenophene, dithieno[3,2-*b*:2',3'-*d*]silole-5,5-diyl, 4*H*-cyclopenta[2,1-*b*:3,4-*b'*]dithiophene-2,6-diyl, carbazole-2,7-diyl, fluorene-2,7-diyl, indaceno[1,2-*b*:5,6-*b'*]dithiophene-2,7-diyl, benzo[1",2":4,5;4",5":4',5']bis(silolo[3,2-*b*:3',2'-*b'*]thiophene)-2,7-diyl, phenanthro[1,10,9,8-*c,d,e,f,g*]carbazole-2,7-diyl, benzo-2,1,3-thiadiazole-4,7-diyl, benzo-2,1,3-selenadiazole-4,7-diyl, benzo-2,1,3-oxa-diazole-4,7-diyl, 2H-benzotriazole-4,7-diyl, 3,4-difluorothiophene-2,5-diyl, thieno[3,4-*b*]pyrazine-2,5-diyl, quinoxaline-5,8-diyl, thieno[3,4-*b*]-thiophene-4,6-diyl, thieno[3,4-b]thiophene-6,4-diyl, 3,6-dithien-2-yl-pyrrolo[3,4-c]pyrrole-1,4-dione or 1,3-thiazolo[5,4-d]-1,3-thiazole-2,5-diyl, where the above radicals may all be unsubstituted or mono- or polysubstituted, preferably by R^{S} defined as in Claim 8.

14. Conjugated polymer according to one of Claims 1 to 13, selected from the following formulae: 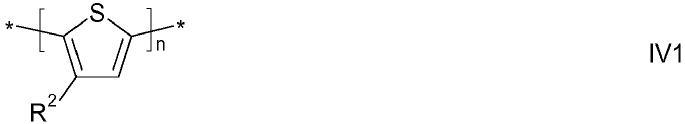 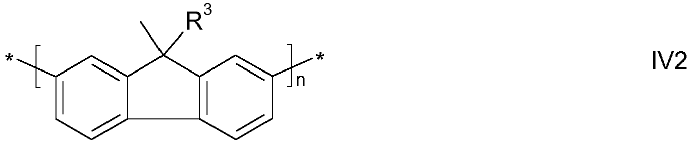 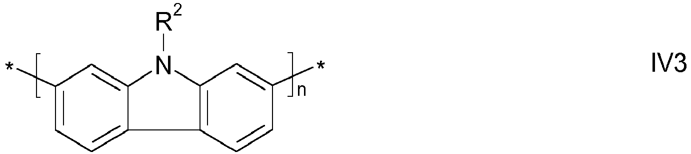 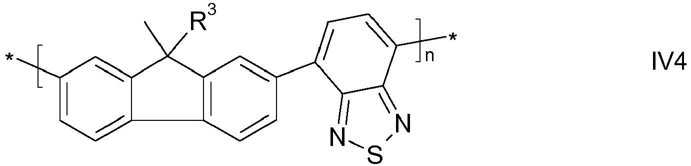 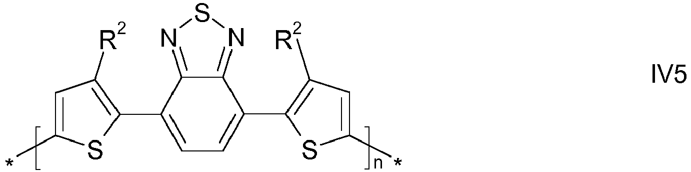 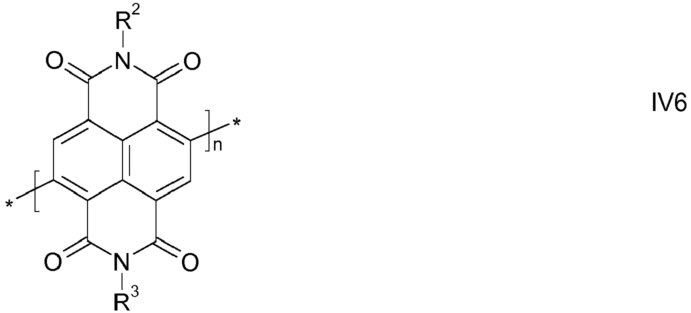 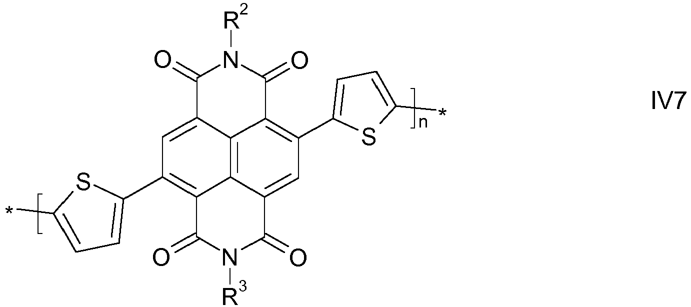 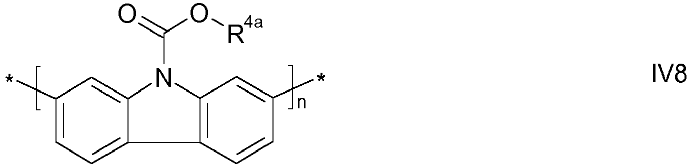 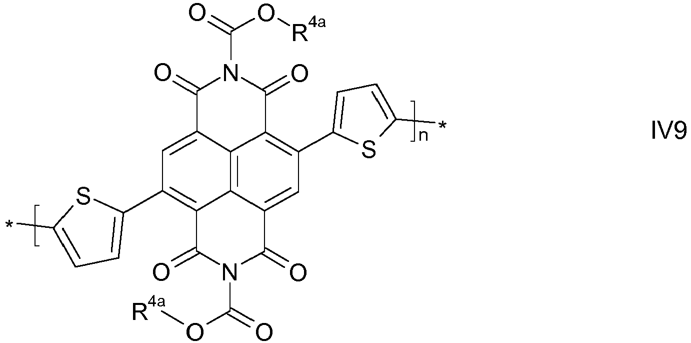 in which R² and R³ have the meanings indicated in Claim 2, R^{4a} denotes a straight-chain or branched alkyl radical having 1 to 25 C atoms, preferably 2-methylhexyl or 9-methylheptadecyl, and n has the meaning indicated in Claim 11.

15. Conjugated polymer according to one of Claims 1 to 14, selected from the following formula:
R⁵-chain-R⁶ V
in which "chain" denotes a polymer chain selected from the formulae IV, IVa to IVk and IV1 to IV9 as defined in Claims 11, 12 and 14, and R⁵ and R⁶ each, independently of one another, have one of the meanings indicated for R^{S} in Claim 8, or denote H, F, Br, Cl, I, CH₂Cl, -CHO, -CR'=CR"₂, -SiR'R"R"', -SiR'X'X", -SiR'R"X', -SnR'R"R"', -BR'R", -B(OR')(OR"), -B(OH)₂, -O-SO₂-R', -C≡CH, -C≡C-SiR'₃ or -ZnX', in which X' and X" denotes halogen, R', R" and R'" each, independently of one another, have one of the meanings indicated for R⁰ in Claim 7, and two of the radicals R', R" and R'" together with the respective heteroatom to which they are bonded may also form a cyclosilyl, cyclostannyl, cycloborane or cycloboronate group having 2 to 20 C atoms.

16. Mixture or polymer blend comprising one or more polymers according to one of Claims 1 to 15 and one or more compounds or polymers having semiconductor, charge-transport, hole/electron-transport, hole/electron-blocking, electrically conducting, photoconducting or light-emitting properties.

17. Mixture or polymer blend according to Claim 16, comprising one or more polymers in accordance with the present invention and one or more additional compounds selected from electron acceptors or n-type organic semiconductors.

18. Mixture or polymer blend according to Claim 17, in which the n-type organic semiconductor is selected from the group consisting of fullerenes and substituted fullerenes.

19. Formulation comprising one or more polymers, mixtures or polymer blends according to one of Claims 1 to 18 and one or more solvents, preferably selected from organic solvents.

20. Use of a polymer, mixture, polymer blend or formulation according to one of Claims 1 to 19 as charge-transport, semiconductor, electrically conducting, photoconducting or light-emitting material in an optical, electro-optical, electronic, electroluminescent or photoluminescent device, in a component of such a device, or in a product containing such a device.

21. Charge-transport, semiconductor, electrically conducting, photoconducting or light-emitting material comprising a polymer, mixture, polymer blend or formulation according to one of Claims 1 to 19.

22. Device having optical, electro-optical, electronic, electroluminescent or photoluminescent properties, component of such a device, or product containing such a device, comprising a polymer, mixture, polymer blend or formulation according to one of Claims 1 to 19.

23. Device according to Claim 22, selected from the group consisting of organic field-effect transistors (OFETs), organic thin-film transistors (OTFTs), organic light-emitting diodes (OLEDs), organic light-emitting transistors (OLETs), organic photovoltaic devices (OPVs), organic photodetectors (OPDs), organic solar cells, Schottky diodes, laser diodes and organic photoconductors.

24. OFET, OPV device, organic solar cell or OPD according to Claim 23, in which the organic semiconductors in the photoactive layer form a volume heterojunction.

25. Component according to Claim 22, selected from the group consisting of charge-injection layers, charge-transport layers, interlayers, planarisation layers, antistatic films, polymer electrolyte membranes (PEMs), conducting substrates and conducting patterns.

26. Product according to Claim 22, selected from the group consisting of integrated circuits (ICs), capacitors, RFID (radio frequency identification) tags, security markings or security devices containing RFID tags, flat-panel displays, backlights for displays, electrophotographic devices, electrophotographic recording devices, organic memory devices, sensor devices, biosensors and biochips.

27. Use of a polymer, mixture, polymer blend or formulation according to one of Claims 1 to 19 in batteries, or in components or devices for the identification and differentiation of DNA sequences.

28. Monomer of the formula Via or VIb
R⁷-(Ar¹)ₐ-U-(Ar²)_{c}-R⁸ Via
R⁷-U-(Ar¹)ₐ-U-R⁸ VIb
in which U, Ar¹, Ar², a and b have the meanings indicated in Claim 8 or 13 and R⁷ and R⁸ are each selected, independently of one another, from the group consisting of CI, Br, I, O-tosylate, O-triflate, O-mesylate, O-nonaflate, -SiMe₂F, -SiMeF₂, -O-SO₂Z¹, -B(OZ²)₂, -CZ³=C(Z³)₂, -C≡CH, -C≡CSi(Z¹)₃, -ZnX⁰ and -Sn(Z⁴)₃, in which X⁰ denotes halogen, preferably Cl, Br or I, Z¹⁻⁴ are selected from the group consisting of alkyl and aryl, which may optionally be substituted, and two radicals Z² together with the B and O atoms may also form a cycloboronate group having 2 to 20 C atoms, except for monomers of the formula Via in which a and c are 0 and U denotes phenylene-2,5-diyl which is substituted by a carbamate group.

29. Monomer according to Claim 28, selected from the following formulae:
R⁷-Ar¹-U-Ar²-R⁸ VI1
R⁷-U-R⁸ VI2
R⁷-Ar¹-U-R⁸ VI3
R⁷-U-Ar²-R⁸ VI4
R⁷-U-Ar¹-U-R⁸ VI5
in which U, Ar¹, Ar², R⁷ and R⁸ are defined as in Claim 28.

30. Process for the preparation of a polymer according to one of Claims 1 to 15 by reaction of one or more monomers according to Claim 28 or 29 in which R⁷ and R⁸ are selected from Cl, Br, I, -B(OZ²)₂ and -Sn(Z⁴), with one another and/or with one or more monomers selected from the following formulae:
R⁷-(Ar¹)ₐ-A^{c}-(Ar²)_{c}-R⁸ VIII
R⁷-Ar¹-R⁸ IX
R⁷-Ar³-R⁸ X
in which Ar¹, Ar², a and c are defined as in Claim 28, Ar³ is defined as in Claim 8 or 13, and Ar⁴ is defined as in Claim 10 or 13, in an aryl-aryl coupling reaction.

31. Process for cleaving off some or all of the carbonate or carbamate groups of a polymer according to one of Claims 1 to 15 by heating the polymer, or a layer comprising the polymer, to a temperature of ≤ 200°C.

## Revendications

1. Polymère conjugué contenant une ou plusieurs unité(s) récurrente(s) identiques ou différentes de la formule I : 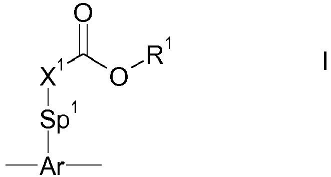 dans laquelle les radicaux individuels présentent les significations qui suivent :
Ar représente aryle ou hétéroaryle mono- ou polycyclique, lequel peut de façon additionnelle être substitué au niveau d'une ou de plusieurs position(s) ;
Sp¹ représente alkylène qui comporte de 1 à 20 atome(s) de C, lequel est non substitué, mono- ou polysubstitué par F, Cl, Br, I ou CN, et où, en outre, un ou plusieurs groupe(s) CH₂ non adjacents peut/peuvent dans chaque cas être remplacé(s), de manière indépendante les uns des autres, par -O-, -S-, -NH-, -NR⁰-, -SiR⁰R⁰⁰-, -CO-, -COO-, -OCO-, -OCO-O-, -S-CO-, -CO-S-, -CR⁰=CR⁰⁰- ou -C≡C- de telle sorte que des atomes de O et/ou de S ne soient pas liés directement les uns aux autres ;
X¹ représente NR⁰ ou O ;
R⁰ et R⁰⁰ représentent dans chaque cas, de manière indépendante l'un de l'autre, H ou alkyle qui comporte de 1 à 12 atome(s) de C ; et
R¹ représente un radical hydrocarbyle qui comporte de 1 à 40 atome(s) de C ;
à l'exception
d'unités récurrentes de la formule I pour lesquelles Ar représente phénylène qui est mono- ou polysubstitué par -NH-C(O)-OR¹, et qui est directement adjacent à une unité benzo[lmn][3,8]phénanthroline-1,3,6,8-tétraone-4,9-diyle en option substituée dans la chaîne polymère principale ; et
d'unités récurrentes de la formule I pour lesquelles Ar représente pyrrolo[3,4-c]pyrrole-1,4-dione-3,6-diyle, où les 2 atomes de N sont substitués par -C(O)-OR¹.

2. Polymère conjugué selon la revendication 1, dans lequel les unités récurrentes de la formule I sont sélectionnées à partir des formules qui suivent : 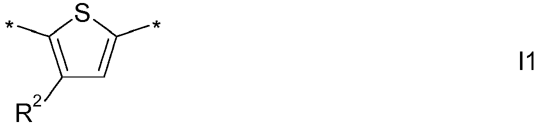 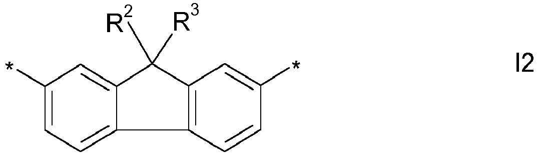 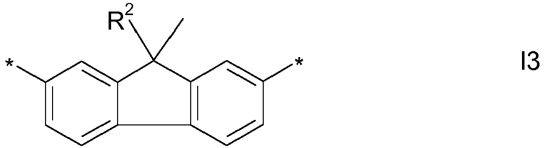 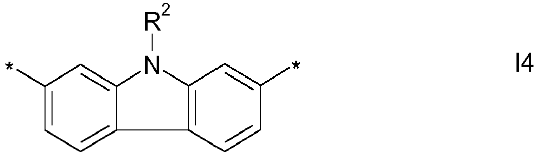 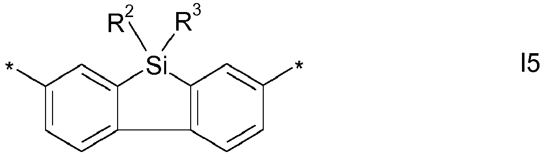 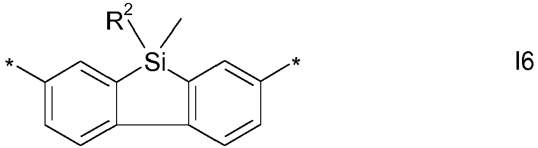 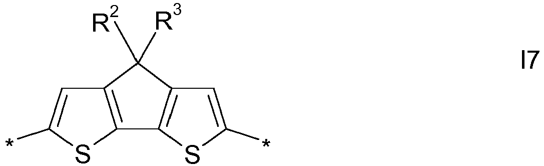 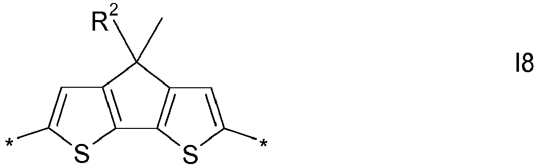 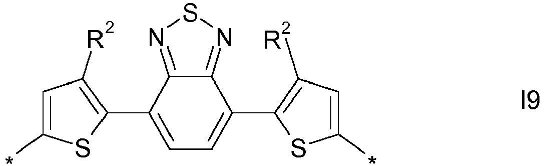 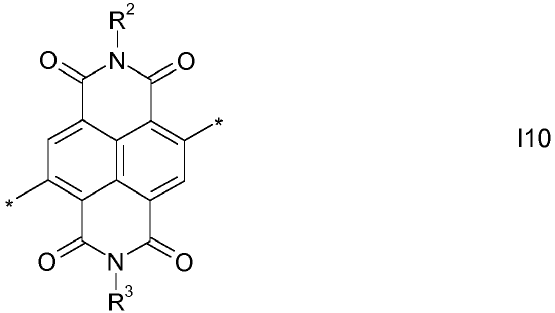 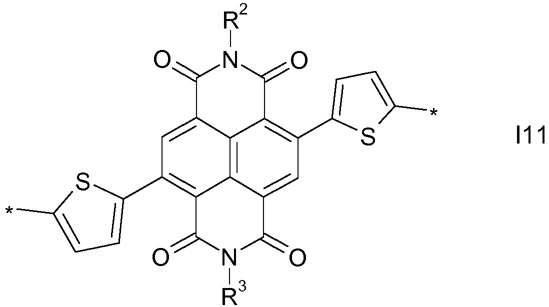 dans lesquelles R² et R³ représentent chacun, de manière indépendante l'un de l'autre, et de manière identique ou différente pour chaque occurrence, un radical -Sp¹-X¹-C(O)-O-R¹, et Sp¹, X¹ et R¹ présentent les significations qui ont été indiquées selon la revendication 1.

3. Polymère conjugué selon la revendication 1 ou 2, dans lequel le radical Sp¹ représente alkylène qui comporte de 1 à 20 atome(s) de C.

4. Polymère conjugué selon l'une des revendications 1 à 3, dans lequel le radical R¹ représente alkyle en chaîne droite, ramifié ou cyclique qui comporte de 1 à 25 atome(s) de C, lequel est non substitué ou mono- ou polysubstitué par F, Cl, Br, I ou CN, et où, en outre, un ou plusieurs groupe(s) CH₂ non adjacents peut/peuvent chacun être remplacé(s), de manière indépendante les uns des autres, par -O-, -S-, -C(O)-, -C(S)-, -C(O)-O-, -O-C(O)-, -NR⁰-, -SiR⁰R⁰⁰-, -CF₂-, -CHR⁰=CR⁰⁰-, -CY¹=CY²- ou -C≡C- de telle sorte que des atomes de O et/ou de S ne soient pas liés directement les uns aux autres, où R⁰ et R⁰⁰ présentent les significations qui ont été indiquées selon la revendication 1.

5. Polymère conjugué selon une ou plusieurs des revendications 1 à 4, dans lequel le radical X¹ représente O ou NH.

6. Polymère conjugué selon une ou plusieurs des revendications 1 à 5, dans lequel le radical -Sp¹-X¹-C(O)-O-R¹ est sélectionné à partir de la formule qui suit : 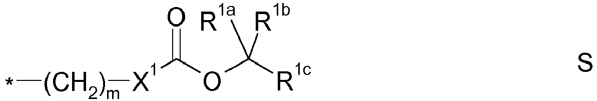 dans laquelle X¹ présente la signification qui a été indiquée selon la revendication 1 ou 5, R^{1a}, R^{1b} et R^{1c} représentent chacun, de manière indépendante les uns des autres, H ou un radical alkyle en chaîne droite, ramifié ou cyclique qui comporte de 1 à 25 atome(s) de C ou un radical alkényle ou alkynyle en chaîne droite, ramifié ou cyclique dont chacun comporte de 2 à 25 atomes de C, où, en outre, deux des radicaux R^{1a}, R^{1b} et R^{1c} peuvent en association former un radical alkyle cyclique, un radical alkényle cyclique ou un radical alkynyle cyclique dont chacun comporte de 5 à 12 atomes de C, m représente un entier de 1 à 12, de façon préférable 2, 3, 4, 5 ou 6, et le symbole * représente la liaison sur le radical Ar.

7. Polymère conjugué selon une ou plusieurs des revendications 1 à 6, dans lequel le radical -Sp¹-X¹-C(O)-O-R¹ est sélectionné à partir des formules qui suivent : 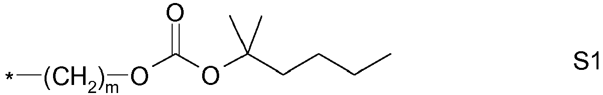 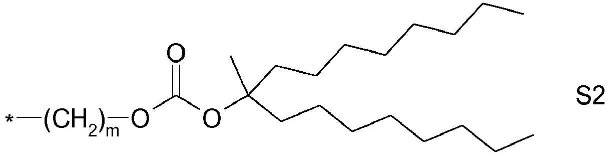 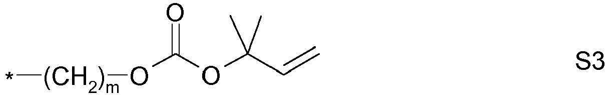 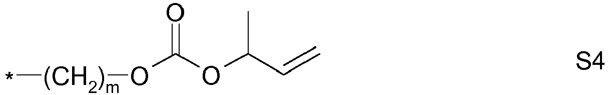 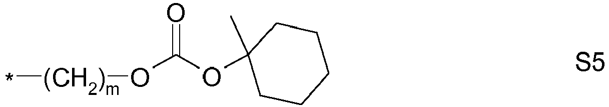 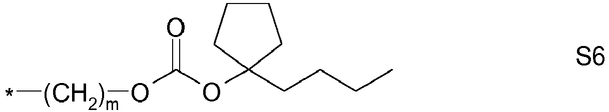 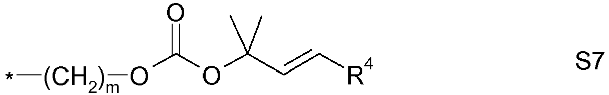 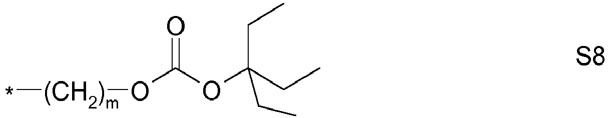 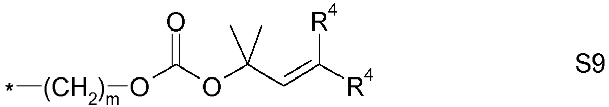 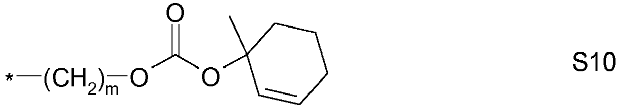 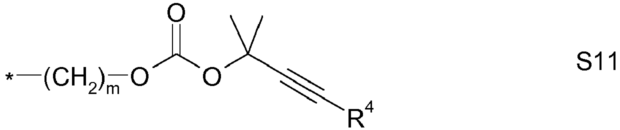 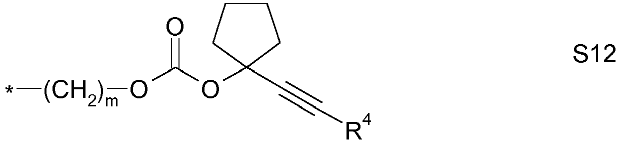 dans lesquelles R⁴ représente pour chaque occurrence, de manière identique ou différente, H ou un radical alkyle en chaîne droite ou ramifié qui comporte de 1 à 25 atome(s) de C, m représente un entier de 1 à 12, et le symbole * représente la liaison sur le radical Ar.

8. Polymère conjugué selon une ou plusieurs des revendications 1 à 7, contenant une ou plusieurs unité(s) récurrente(s) de la formule IIa ou IIb :
-[(Ar¹)ₐ-(U)_{b}-(Ar²)_{c}-(Ar³)_{d}]- IIa
-[(U)_{b}-(Ar¹)ₐ-(U)_{b}-(Ar²)_{c}-(Ar³)_{d}]- IIb
dans lesquelles les radicaux individuels présentent les significations qui suivent :
U représente une unité de la formule I ou I1 à I17 comme défini selon l'une des revendications 1 à 7 ;
Ar¹, Ar², Ar³ représentent pour chaque occurrence, de manière identique ou différente, et dans chaque cas de manière indépendante les uns des autres, aryle ou hétéroaryle qui est différent de U, de façon préférable qui comporte de 5 à 30 atomes de cycle, et qui est en option substitué, de façon préférable par un ou par plusieurs groupe(s) R^{S} ;
R^{S} représente pour chaque occurrence, de manière identique ou différente, F, Br, Cl, -CN, -NC, -NCO, -NCS, -OCN, -SCN, -C(O)NR⁰R⁰⁰, -C(O)X⁰, -C(O)R⁰, -NH₂, -NR⁰R⁰⁰, -SH, -SR⁰, -SO₃H, -SO₂R⁰, -OH, -NO₂, -CF₃, -SF₅, silyle ou hydrocarbyle en option substitué qui comporte de 1 à 40 atome(s) de C, lequel est en option substitué et contient en option un ou plusieurs hétéroatome(s) ;
R⁰ et R⁰⁰ sont définis tel que selon la revendication 1 ;
X⁰ représente halogène, de façon préférable F, Cl ou Br ;
a, b et c représentent pour chaque occurrence, de manière identique ou différente et dans chaque cas, de manière indépendante les uns des autres, 0, 1 ou 2 ;
d représente pour chaque occurrence, de manière identique ou différente, 0 ou un entier de 1 à 10 ;
où le polymère conjugué contient au moins une unité récurrente de la formule IIa ou IIb dans laquelle b est au moins 1.

9. Polymère conjugué selon une ou plusieurs des revendications 1 à 8, contenant de façon additionnelle une ou plusieurs unité(s) aryle ou hétéroaryle mono- ou polycyclique(s), en option substituée(s).

10. Polymère conjugué selon la revendication 9, dans lequel les groupes aryle ou hétéroaryle mono- ou polycycliques, en option substitués, sont sélectionnés à partir des formules IIIa et IIIb :
-[(Ar¹)ₐ-(Ar¹)_{b}-(Ar²)_{c}-(Ar³)_{d}]- IIIa
-[(Ar⁴)_{b}-(Ar¹)ₐ-(Ar⁴)_{b}-(Ar²)_{c}-(Ar³)_{d}]- IIIb
dans lesquelles Ar¹, Ar², Ar³, a, b, c et d sont définis tel que selon la revendication 8, et Ar⁴ représente un groupe aryle ou hétéroaryle qui est différent de U et de Ar¹⁻³, de façon préférable qui comporte de 5 à 30 atomes de cycle et qui est en option substitué, de façon préférable par un ou par plusieurs groupe(s) R^{S}, où le polymère conjugué contient au moins une unité récurrente de la formule IIIa ou IIIb dans laquelle b est au moins 1.

11. Polymère conjugué selon l'une des revendications 1 à 10, lequel est sélectionné à partir de la formule IV : 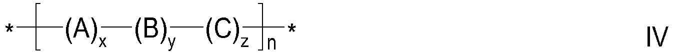 dans laquelle les radicaux individuels présentent les significations qui suivent :
A, B, C représentent chacun, de manière indépendante les uns des autres, une unité différente de la formule I, I1 à I9, IIa, IIb, IIIa ou IIIb tel que défini selon une ou plusieurs des revendications 1 à 10 ;
x est > 0 et ≤ 1 ;
y est ≥ 0 et < 1 ;
z est ≥ 0 et < 1 ;
x + y + z est 1 ; et
n est un entier > 1.

12. Polymère conjugué selon la revendication 11, lequel est sélectionné à partir des sous-formules qui suivent :
*-[(Ar¹-U-Ar²)ₓ-(Ar³)_{y}]ₙ-* IVa
*-[(Ar¹-U-Ar²)ₓ-(Ar³-Ar³)_{y}]ₙ-* IVb
*-[(Ar¹-U-Ar²)ₓ-(Ar³-Ar³-Ar³)_{y}]ₙ-* IVc
*-[(Ar¹)ₐ-(U)_{b}-(Ar²)_{c}-(Ar³)_{d}]ₙ-* IVd
*-([(Ar¹)ₐ-(U)_{b}-(Ar²)_{c}-(Ar³)_{d}]ₓ-[(Ar¹)ₐ-(Ar⁴)_{b}-(Ar²)_{c}-(Ar³)_{d}]_{y})ₙ-* IVe
*-[(U-Ar¹-U)ₓ-(Ar²-Ar³)_{y}]ₙ-* IVf
*-[(U-Ar¹-U)ₓ-(Ar²-Ar³-Ar²)_{y}]ₙ-* IVg
*-[(U)_{b}-(Ar¹)a-(U)_{b}-(Ar²)_{c}]ₙ-* IVh
*-([(U)_{b}-(Ar¹)ₐ-(U)_{b}-(Ar²)_{c}]ₓ-[(A^{c})_{b}-(Ar¹)ₐ-(Ar⁴)_{b}-(Ar²)_{d}]_{y})ₙ-* IVi
*-[(U-Ar¹)ₓ-(U-Ar²)_{y}-(U-Ar³)_{z}]ₙ-* IVk
dans lesquelles U, Ar¹, Ar², Ar³, a, b, c et d présentent, pour chaque occurrence, de manière identique différente, l'une des significations qui ont été indiquées selon la revendication 8, Ar⁴ présente, pour chaque occurrence, de manière identique ou différente, l'une des significations qui ont été indiquées selon la revendication 10, et x, y, z et n sont définis tel que selon la revendication 11, où ces polymères peuvent être des copolymères alternés ou aléatoires, et où, dans les formules IVd et IVe, b est au moins 1 dans au moins une unité récurrente [(Ar¹)ₐ-(U)_{b}-(Ar²)_{c}-(Ar³)_{d}] et dans au moins une unité récurrente [(Ar¹)ₐ-(Ar⁴)_{b}-(Ar²)_{c}-(Ar³)_{d}], et, dans les formules IVh et IVi, b est 1 dans au moins une unité récurrente [(U)_{b}-(Ar¹)ₐ-(U)_{b}-(Ar²)_{d}] et dans au moins une unité récurrente [(U)_{b}-(Ar¹)ₐ-(U)_{b}-(Ar²)_{d}].

13. Polymère conjugué selon l'une des revendications 1 à 12, dans lequel Ar¹, Ar², Ar³ et Ar⁴ sont sélectionnés, dans chaque cas de manière indépendante les uns des autres et de manière identique ou différente, parmi le groupe qui est constitué par 1,4-phénylène, thio-phène-2,5-diyle, sélénophène-2,5-diyle, thiéno[3,2-*b*]thiophène-2,5-diyle, thiéno[2,3-*b*]thiophène-2,5-diyle, sélénophéno[3,2-*b*]séléno-phène-2,5-diyle, sélénophéno[2,3-*b*]sélénophène-2,5-diyle, séléno-phéno[3,2-*b*]thiophène-2,5-diyle, sélénophéno[2,3-*b*]thiophène-2,5-diyle, benzo[1,2-*b*:4,5-*b'*]dithiophène-2,6-diyle, 2,2-dithiophène, 2,2-disélénophène, dithiéno[3,2-*b*:2',3'-*d*]silole-5,5-diyle, 4*H*-cyclopenta-[2,1-*b*:3,4-*b'*]dithiophène-2,6-diyle, carbazole-2,7-diyle, fluorène-2,7-diyle, indacéno[1,2-*b*:5,6-*b'*]dithiophène-2,7-diyle, benzo-[1",2":4,5;4",5":4',5']bis(silolo[3,2-*b*:3',2'-*b*']thiophène)-2,7-diyle, phén-anthro[1,10,9,8-*c,d,e,f,g*]carbazole-2,7-diyle, benzo-2,1,3-thiadiazole-4,7-diyle, benzo-2,1,3-sélénadiazole-4,7-diyle, benzo-2,1,3-oxa-diazole-4,7-diyle, 2H-benzotriazole-4,7-diyle, 3,4-difluorothiophène-2,5-diyle, thiéno[3,4-*b*]pyrazine-2,5-diyle, quinoxaline-5,8-diyle, thiéno[3,4-*b*]thiophène-4,6-diyle, thiéno[3,4-*b*]thiophène-6,4-diyle, 3,6-dithién-2-yl-pyrrolo[3,4-c]pyrrole-1,4-dione ou 1,3-thiazolo[5,4-d]-1,3-thiazole-2,5-diyle, où les radicaux qui ont été mentionnés ci-avant peuvent tous être non substitués ou mono- ou polysubstitués, de façon préférable par R^{S} qui est défini tel que selon la revendication 8.

14. Polymère conjugué selon l'une des revendications 1 à 13, lequel est sélectionné à partir des formules qui suivent : 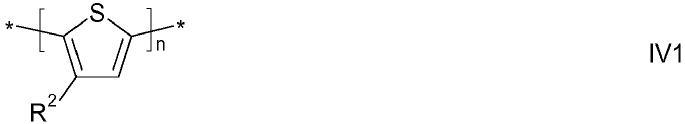 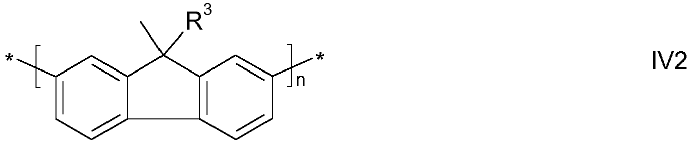 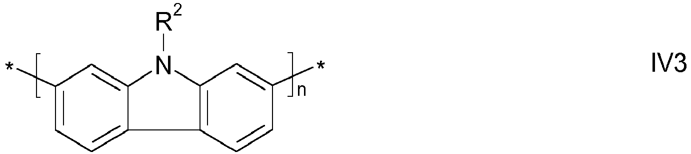 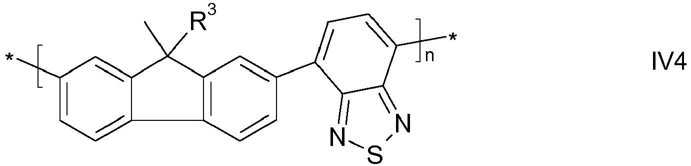 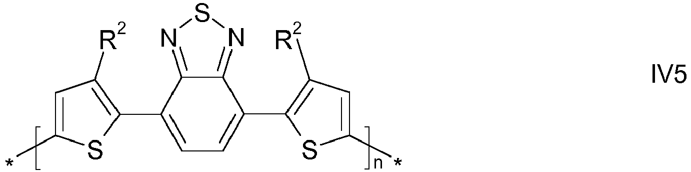 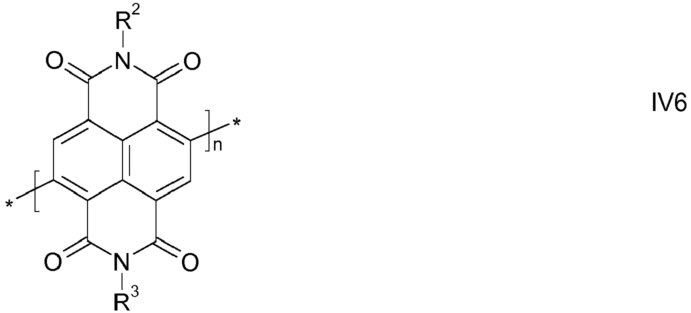 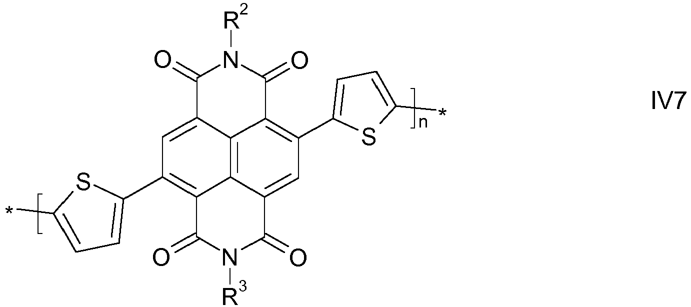 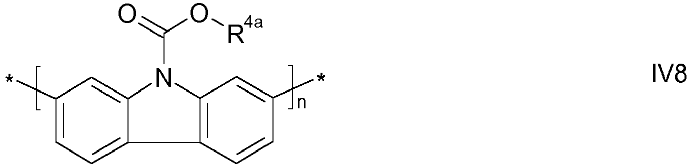 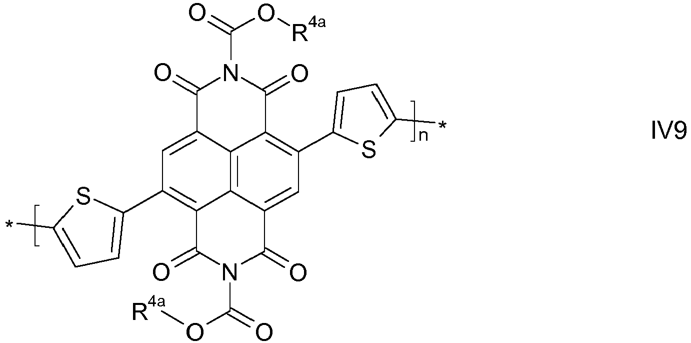 dans lesquelles R² et R³ présentent les significations qui ont été indiquées selon la revendication 2, R^{4a} représente un radical alkyle en chaîne droite ou ramifié qui comporte de 1 à 25 atome(s) de C, de façon préférable 2-méthylhexyle ou 9-méthylheptadécyle, et n présente la signification qui a été indiquée selon la revendication 11.

15. Polymère conjugué selon l'une des revendications 1 à 14, lequel est sélectionné à partir de la formule qui suit :
R⁵-chaîne-R⁶ V
dans laquelle "chaîne" représente une chaîne polymère qui est sélectionnée à partir des formules IV, IVa à IVk et IV1 à IV9 comme défini selon les revendications 11, 12 et 14, et R⁵ et R⁶ présentent chacun, de manière indépendante l'un de l'autre, l'une des significations qui ont été indiquées pour R^{S} selon la revendication 8, ou représentent H, F, Br, Cl, I, CH₂Cl, -CHO, -CR'=CR"₂, -SiR'R"R"', -SiR'X'X", -SiR'R"X', -SnR'R"R"', -BR'R", -B(OR')(OR"), -B(OH)₂, -O-SO₂-R', -C≡CH, -C≡C-SiR'₃ ou -ZnX', où X' et X" représentent halogène, R', R" et R'" présentent chacun, de manière indépendante les uns des autres, l'une des significations qui ont été indiquées pour R⁰ selon la revendication 7, et deux des radicaux R', R" et R'", en association avec l'hétéroatome respectif auquel ils sont liés, peuvent également former un groupe cyclosilyle, cyclostannyle, cycloborane ou cycloboronate qui comporte de 2 à 20 atomes de C.

16. Mixture ou mélange de polymères comprenant un ou plusieurs polymère(s) selon l'une des revendications 1 à 15 et un ou plusieurs composé(s) ou polymère(s) qui présente(nt) des propriétés de semiconducteur, de transport de charges, de transport de trous/d'électrons, de blocage de trous/d'électrons, de conduction électrique, de photoconduction ou d'émission de lumière.

17. Mixture ou mélange de polymères selon la revendication 16, comprenant un ou plusieurs polymère(s) conformément à la présente invention et un ou plusieurs composé(s) additionnel(s) qui est/sont sélectionné(s) parmi les accepteurs d'électrons ou les semiconducteurs organiques de type n.

18. Mixture ou mélange de polymères selon la revendication 17, dans laquelle ou lequel le semiconducteur organique de type n est sélectionné parmi le groupe qui est constitué par les fullerènes et par les fullerènes substitués.

19. Formulation comprenant un ou plusieurs polymère(s), une ou plusieurs mixture(s) ou un ou plusieurs mélange(s) de polymères selon l'une des revendications 1 à 18 et un ou plusieurs solvant(s), qui est/sont de façon préférable sélectionné(s) parmi les solvants organiques.

20. Utilisation d'un polymère, d'une mixture, d'un mélange de polymères ou d'une formulation selon l'une des revendications 1 à 19 en tant que matériau de transport de charges, semiconducteur, électriquement conducteur, photoconducteur ou à émission de lumière dans un dispositif optique, électrooptique, électronique, électroluminescent ou photoluminescent, dans un composant d'un tel dispositif ou dans un produit qui contient un tel dispositif

21. Matériau de transport de charges, semiconducteur, électriquement conducteur, photoconducteur ou à émission de lumière qui comprend un polymère, une mixture, un mélange de polymères ou une formulation selon l'une des revendications 1 à 19.

22. Dispositif qui présente des propriétés optiques, électrooptiques, électroniques, électroluminescentes ou photoluminescentes, composant d'un tel dispositif, ou produit qui contient un tel dispositif, comprenant un polymère, une mixture, un mélange de polymères ou une formulation selon l'une des revendications 1 à 19.

23. Dispositif selon la revendication 22, lequel est sélectionné parmi le groupe qui est constitué par les transistors à effet de champ organiques (OFET), les transistors à film mince organiques (OTFT), les diodes à émission de lumière/électroluminescentes organiques (OLED), les transistors à émission de lumière/électroluminescents organiques (OLET), les dispositif photovoltaïques organiques (OPV), les photodétecteurs organiques (OPD), les cellules solaires organiques, les diodes Schottky, les diodes laser et les photoconducteurs organiques.

24. Dispositif OFET, OPV, cellule solaire organique ou OPD selon la revendication 23, dans lequel ou laquelle les semiconducteurs organiques dans la couche photoactive forment une hétérojonction en volume.

25. Composant selon la revendication 22, lequel est sélectionné parmi le groupe qui est constitué par les couches d'injection de charges, les couches de transport de charges, les intercouches, les couches de planarisation, les films antistatiques, les membranes électrolytiques polymère(s) (PEM), les substrats conducteurs et les motifs conducteurs.

26. Produit selon la revendication 22, lequel est sélectionné parmi le groupe qui est constitué par les circuits intégrés (IC), les condensateurs, les étiquettes RFID (d'identification radiofréquence), les marquages de sécurité ou les dispositifs de sécurité qui contiennent des étiquettes RFID, les affichages à écran plat, les rétroéclairages pour affichages, les dispositifs électrophotographiques, les dispositifs d'enregistrement électrophotographique, les dispositifs de mémoire organique, les dispositifs de capteur, les biocapteurs et les biopuces.

27. Utilisation d'un polymère, d'une mixture, d'un mélange de polymères ou d'une formulation selon l'une des revendications 1 à 19 dans des batteries, ou dans des composants ou dispositifs pour l'identification et la différentiation de séquences d'ADN.

28. Monomère de la formule VIa ou VIb :
R⁷-(Ar¹)ₐ-U-(Ar²)_{c}-R⁸ VIa
R⁷-U-(Ar¹)ₐ-U-R⁸ VIb
dans lesquelles U, Ar¹, Ar², a et b présentent les significations qui ont été indiquées selon la revendication 8 ou 13 et R⁷ et R⁸ sont chacun sélectionnés, de manière indépendante l'un de l'autre, parmi le groupe qui est constitué par Cl, Br, I, O-tosylate, O-triflate, O-mésylate, O-nonaflate, -SiMe₂F, -SiMeF₂, -O-SO₂Z¹, -B(OZ²)₂, -CZ³=C(Z³)₂, -C≡CH, -C≡CSi(Z¹)₃, -ZnX⁰ et -Sn(Z⁴)₃, où X⁰ représente halogène, de façon préférable Cl, Br ou I, Z¹⁻⁴ sont sélectionnés parmi le groupe qui est constitué par alkyle et aryle, lequel peut en option être substitué, et deux radicaux Z², en association avec les atomes de B et de O, peuvent également former un groupe cycloboronate qui comporte de 2 à 20 atomes de C, à l'exception des monomères de la formule VIa pour lesquels a et c sont 0 et U représente phénylène-2,5-diyle, lequel est substitué par un groupe carbamate.

29. Monomère selon la revendication 28, lequel est sélectionné à partir des formules qui suivent :
R⁷-Ar¹-U-Ar²-R⁸ VI1
R⁷-U-R⁸ VI2
R⁷-Ar¹-U-R⁸ VI3
R⁷-U-Ar²-R⁸ VI4
R⁷-U-Ar¹-U-R⁸ VI5
dans lesquelles U, Ar¹, Ar², R⁷ et R⁸ sont définis tel que selon la revendication 28.

30. Procédé pour la préparation d'un polymère selon l'une des revendications 1 à 15 au moyen de la réaction d'un ou de plusieurs monomère(s) selon la revendication 28 ou 29, où R⁷et R⁸ sont sélectionnés parmi Cl, Br, I, -B(OZ²)₂ et -Sn(Z⁴), avec un autre monomère et/ou avec un ou plusieurs monomère(s) qui est/sont sélectionné(s) à partir des formules qui suivent :
R⁷-(Ar¹)ₐ-A^{c}-(Ar²)_{c}-R⁸ VIII
R⁷-Ar¹-R⁸ IX
R⁷-Ar³-R⁸ X
dans lesquelles Ar¹, Ar², a et c sont définis tel que selon la revendication 28, Ar³ est défini tel que selon la revendication 8 ou 13, et Ar⁴ est défini tel que selon la revendication 10 ou 13, selon une réaction par couplage aryle-aryle.

31. Procédé pour séparer par clivage certains groupes ou tous les groupes carbonate ou carbamate d'un polymère selon l'une des revendications 1 à 15 en chauffant le polymère, ou une couche qui comprend le polymère, jusqu'à une température ≤ 200°C.
